(19) Europäisches Patentamt
European Patent Office
Office européen des brevets

(11) Veröffentlichungsnummer: **0 042 026**
**B1**

(12) # EUROPÄISCHE PATENTSCHRIFT

(45) Veröffentlichungstag der Patentschrift: **08.01.86**

(51) Int. Cl.⁴: **C 07 D 205/08**, C 07 F 9/65, C 07 D 499/00, C 07 D 417/12

(21) Anmeldenummer: **81100569.3**

(22) Anmeldetag: **30.01.79**

(60) Veröffentlichungsnummer der früheren Anmeldung nach Art. 76 EPÜ: **0003960**

(54) **3,4-Disubstituierte Azetidin-2-onverbindungen und Verfahren zu ihrer Herstellung.**

(30) Priorität: **02.02.78 CH 1140/78**

(43) Veröffentlichungstag der Anmeldung: **23.12.81 Patentblatt 81/51**

(45) Bekanntmachung des Hinweises auf die Patenterteilung: **08.01.86 Patentblatt 86/2**

(84) Benannte Vertragsstaaten: **BE CH DE FR GB IT LU NL SE**

(56) Entgegenhaltungen:
**EP-A-0 002 210**
**DE-A-2 205 123**
**DE-A-2 819 655**
**US-A-4 123 539**

(73) Patentinhaber: **CIBA-GEIGY AG, Klybeckstrasse 141, CH-4002 Basel (CH)**

(72) Erfinder: **Gosteli, Jacques, Dr., Anwilerstrasse 10, CH-4059 Basel (CH)**
Erfinder: **Ernest, Ivan, Dr., Rüttihard 10, CH-4127 Birsfelden (CH)**
Erfinder: **Lang, Marc, Dr., Rue de Thann 4, CH-68200 Mulhouse (FR)**
Erfinder: **Woodward, Robert Burns, Prof. Dr., 12 Oxford Street, Cambridge Massachusetts 02138 (US)**

(74) Vertreter: **Zumstein, Fritz, Dr. et al, Bräuhausstrasse 4, D-8000 München 2 (DE)**

**EP 0 042 026 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue 3,4-disubstituierte Azetidin-2-onverbindungen und Verfahren zu ihrer Herstellung. Die Verbindungen sind Zwischenprodukte zur Herstellung von 6-substituierten 2-Penem-3-carbonsäureverbindungen mit antibiotischen Eigenschaften.

Der vorliegenden Erfindung liegt die Aufgabe zugrunde, 3,4-disubstituierte Azetidin-2-onverbindungen herzustellen, aus denen einen β-Lactamring enthaltende bicyclische Thia-azaverbindungen hergestellt werden können, die das in 6-Stellung substituierte 2-Penem-Ringsystem besitzen, und die sowohl gegen Penicillin-empfindliche als auch gegen Penicillin-resistente Keime wirksam sind.

Die erfindungsgemässe Herstellung der neuen Zwischenprodukte, eröffnet darüber hinaus neue Gebiete, auf denen nach weiteren, technisch verwertbaren Verbindungen geforscht werden kann.

Die Erfindung betrifft insbesondere Verbindungen der Formel

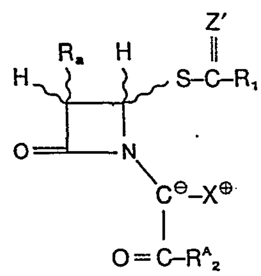

(II),

worin $R_a$ ein gesättigter oder ungesättigter, gegebenenfalls substituierter, aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, ein gegebenenfalls substituierter Heterocycly- oder Heterocyclylniederalkylrest mit bis zu 10 Kohlenstoffatomen und bis zu 4 Ringheteroatomen aus der Gruppe Stickstoff, Sauerstoff und/oder Schwefel, in welchen Resten $R_a$ die gegebenenfalls vorhandenen Substituenten Hydroxy, Niederalkoxy, Niederalkanoyloxy, in Salzform vorliegendes Hydroxysulfonyloxy, Halogen, Mercapto, Niederalkylmercapto, Carboxy, Niederalkoxycarbonyl, Carbamoyl, Cyan, Nitro, in Salzform vorliegendes Sulfo, oder gegebenenfalls durch Niederalkyl oder $C_1$–$C_{20}$-Acyl mono- oder disubstituiertes oder durch Niederalkylen disubstituiertes Amino sind,

gegebenenfalls geschütztes Carboxyl,

Hydroxy,

Mercapto,

durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy oder Mercapto, worin

die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$–$C_{20}$-Acyl mono- oder disubstituiertes oder durch Niederalkylen disubstituiertes Amino sind,

durch einen $C_1$–$C_{20}$-Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen verestertes Hydroxy oder Mercapto, worin

die gegebenenfalls vorhandenen Substituenten Hydroxy, Niederalkoxy, Phenyloxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$–$C_{20}$-Acyl mono- oder disubstituiertes oder durch Niederalkylen disubstituiertes Amino sind, oder Halogen ist,

$R_1$ Wasserstoff, ein gesättigter oder ungesättigter, gegebenenfalls substituierter aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, oder

ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel ist,

in welchen Resten $R_1$ die gegebenenfalls vorhandenen Substituenten Hydroxy, Niederalkoxy, Niederalkanoyloxy, Halogen, Mercapto, Niederalkylmercapto, gegebenenfalls durch Niederalkyl, Hydroxyniederalkyl, Carboxyniederalkyl, Aminoniederalkyl, Diniederalkylaminoniederalkyl, in Salzform vorliegendes Sulfoniederalkyl, Cycloalkyl, Aryl, Arylniederalkyl, Halogen, Amino, Mono- oder Di-niederalkylamino, Nitro, Hydroxy, Niederalkoxy, Carboxyl, Niederalkoxycarbonyl, Carbamoyl, N-Mono- oder N,N-Diniederalkylcarbamoyl, Cyan, Oxo oder Oxido substituiertes Heterocyclylmercapto, Carboxyl, Niederalkoxycarbonyl, Carbamoyl, Cyan, Nitro, Amino, Niederalkylamino, Diniederalkylamino, $C_1$–$C_{20}$-Acylamino, Di-$C_1$–$C_{20}$-acylamino oder Niederalkylenamino sind, oder worin $R_1$ eine durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen, verätherte Mercaptogruppe ist,

worin die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, gegebenenfalls durch Niederalkyl oder $C_1$–$C_{20}$-Acyl mono- oder disubstituiertes oder durch Niederalkylen disubstituiertes Amino sind, oder worin $R_1$

durch Niederalkyl substituiertes Phenylthio oder Phenylniederalkylthio ist, oder worin $R_1$ eine gegebenenfalls geschützte Carboxylgruppe ist, oder worin $R_1$ eine durch einen gegebenenfalls substituierten heterocyclischen Rest verätherte Mercaptogruppe ist, worin der Heterocyclylrest

über ein Ringkohlenstoffatom an die Mercaptogruppe gebunden ist und 1 bis 4 Ringstickstoffatome und gegebenenfalls ein weiteres Ringheteroatom der Gruppe Sauerstoff und Schwefel enthält und worin die gegebenenfalls vorhandenen Substituenten Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$–$C_{20}$-Acylamino oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl, Oxazolyl, Halogen, gegebenenfalls durch Niederalkyl mono- oder disubstituiertes Amino, $C_1$–$C_{20}$-Acylamino, Nitro, Hydroxy, Niederalkoxy, Carboxy, Niederalkoxycarbonyl, gegebenenfalls N-mono- oder N,N-diniederalkyliertes Carbamoyl, Cyan, Oxo oder Oxido sind, wobei funktionelle Gruppen in diesen Resten $R_a$ und $R_1$ vorzugsweise in geschützter Form vorliegen und die mit «Nieder» bezeichneten Gruppen bis zu 7, insbesondere bis zu 4 Kohlenstoffatome enthalten, Z′Sauerstoff, Schwefel oder eine gegebenenfalls einen oder zwei Substituenten tragende Methylidengruppe bedeutet, $X^{\oplus}$ entweder eine dreifach substituierte Phosphoniogruppe oder eine zweifach veresterte Phosphonogruppe zusammen mit einem Kation bedeutet und $R_2^A$ zusammen mit der Carbonylgruppe $-C(=O)-$ eine geschützte Carboxylgruppe darstellt, in racemischer oder optisch aktiver Form, Verfahren zu ihrer Herstellung und die zur Herstellung der Verbindungen der Formel II benötigten Zwischenprodukte.

In den erfindungsgemässen Zwischenprodukten haben die Substituenten $R_a$, $R_1$, $R_2^A$, Z′ und $X^{\oplus}$ die folgenden Bedeutungen.

Ein gesättigter oder ungesättigter, gegebenenfalls substituierter, aliphatischer, cycloaliphatischer, cycloaliphatisch-aliphatischer, aromatischer oder araliphatischer Kohlenwasserstoffrest $R_a$ hat bis zu 18, bevorzugt bis zu 10 Kohlenstoffatome, und ist insbesondere gegebenenfalls substituiertes Niederalkyl oder Niederalkenyl, oder gegebenenfalls substituiertes Cycloalkyl, Cycloalkenyl, Cycloalkylniederalkyl, Cycloalkylniederalkenyl, Cycloalkenylniederalkyl, Phenyl, Phenylniederalkyl, oder Phenylniederalkenyl. Der Rest $R_a$ kann auch eine gegebenenfalls geschützte Carboxylgruppe oder ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel sein. Substituenten von solchen Resten $R_a$ sind Hydroxy, Niederalkoxy, z.B. Methoxy oder Äthoxy, Niederalkanoyloxy, z.B. Acetyloxy oder Propionyloxy, in Salzform vorliegendes Hydroxysulfonyloxy, Halogen, z.B. Chlor oder Brom, Mercapto, Niederalkylmercapto, z.B. Methylthio, Carboxyl, Niederalkoxycarbonyl, z.B. Methoxycarbonyl oder Äthoxycarbonyl, Carbamoyl oder Cyan, ferner Nitro, in Salzform vorliegendes Sulfo, oder gegebenenfalls durch Niederalkyl, z.B. Methyl oder Äthyl, durch $C_1$–$C_{20}$-Acyl, wie Niederalkanoyl, z.B. Acetyl, mono- oder disubstituiertes, oder durch Niederalkylen, z.B. 1,4-Butylen oder 1,5-Pentylen, disubstituiertes Amino.

Ein Niederalkylrest $R_a$ enthält bis zu 7, insbesondere bis zu 4 Kohlenstoffatome, und ist z.B. Methyl, Äthyl, Propyl, Isopropyl, Butyl, Isobutyl, tert.-Butyl oder Pentyl. Substituiertes Niederalkyl $R_a$ ist in erster Linie substituiertes Methyl, Äthyl oder Propyl, wobei Substituenten insbesondere in 1-, aber auch in 2- oder in 3-Stellung stehen, und ist beispielsweise Hydroxyniederalkyl, wie Hydroxymethyl, Hydroxyäthyl oder Hydroxypropyl, Niederalkoxyniederalkyl, wie Niederalkoxymethyl, Niederalkoxyäthyl oder Niederalkoxypropyl, z.B. Methoxymethyl, Methoxyäthyl oder Methoxypropyl, Niederalkanoyloxyniederalkyl, wie Niederalkanoyloxymethyl, Niederalkanoyloxyäthyl oder Niederalkanoyloxypropyl, z.B. Acetyloxymethyl, Propionyloxymethyl, Acetyloxyäthyl, Acetyloxypropyl, in Salzform, z.B. als Alkalimetall-, wie Natriumsalz, oder als Ammoniumsalz vorliegendes Hydroxysulfonyloxyniederalkyl, wie Hydroxysulfonyloxymethyl, Hydroxysulfonyloxyäthyl oder Hydroxysulfonyloxypropyl, Halogenniederalkyl, wie Halogenmethyl, Halogenäthyl oder Halogenpropyl, z.B. Chlor- oder Bromäthyl oder Chlor- oder Brompropyl, Niederalkylthioniederalkyl, wie Methylthiomethyl, Methyltioäthyl, Methylthiopropyl oder tert.-Butylthiomethyl, Niederalkoxycarbonylniederalkyl, wie Niederalkoxycarbonylmethyl oder Niederalkoxycarbonyläthyl, z.B. Methoxycarbonylmethyl, Methoxycarbonyläthyl, Äthoxycarbonylmethyl oder Äthoxycarbonyläthyl, Cyanniederalkyl, wie Cyanmethyl oder Cyanäthyl, Sulfoniederalkyl, wie Sulfomethyl, Sulfoäthyl oder Sulfopropyl, worin die Sulfogruppe in Salzform, z.B. als Alkalimetall-, wie Natriumsalz oder auch als Ammoniumsalz vorliegt, oder gegebenenfalls geschütztes, z.B. auch acetyliertes, Aminoniederalkyl, wie Aminomethyl, Aminoäthyl oder Aminopropyl.

Ein Niederalkenylrest $R_a$ enthält 2 bis 7, insbesondere 2 bis 4 Kohlenstoffatome, und ist z.B. Vinyl, Allyl oder 2- oder 3-Butenyl. Substituiertes Niederalkenyl kann die gleichen Substituenten tragen wie substituiertes Niederalkyl.

Eine gegebenenfalls geschützte Carboxylgruppe $R_a$ ist eine freie oder eine der unter den Gruppen $-C(=O)-R_2^A$ genannten, beispielsweise veresterten oder amidierten Carboxylgruppen, wie Niederalkoxycarbonyl, z.B. Methoxy-, Äthoxy- oder tert.-Butoxycarbonyl, Arylniederalkoxycarbonyl, wie Benzyloxy-, p-Nitro-benzyloxy- oder Diphenylmethoxycarbonyl, Aryloxycarbonyl, wie gegebenenfalls, z.B. durch Halogen, wie Chlor, Niederalkoxy, wie Methoxy, oder Nitro, substituiertes Phenyloxycarbonyl, wie Phenyloxycarbonyl, o-, m- oder p-Chlorphenyloxy-, Pentachlorphenyloxy, o-, m- oder p-Methoxyphenyloxy- oder p-Nitrophenyloxycarbonyl, Aminocarbonyl oder substituiertes, wie durch Niederalkyl, z.B. Methyl oder Äthyl, mono- oder disubstituiertes Aminocarbonyl.

Ein Cycloalkylrest $R_a$ weist z.B. 3 bis 7 Kohlenstoffatome auf und ist z.B. Cyclopropyl, Cyclobutyl, Cyclopentyl oder Cyclohexyl, während ein Cycloalkylniederalkylrest $R_a$ beispielsweise 4 bis 7 Kohlenstoffatome enthält und z. B. Cyclopropylmethyl, Cyclobutylmethyl, Cyclopentylmethyl oder Cyclohexylmethyl darstellt.

Ein Cycloalkenylrest $R_a$ ist ein entsprechender Cycloalkylrest mit ein oder gegebenenfalls zwei C–C-Doppelbindungen, wie Cyclohexenyl, z. B. 1-Cyclohexenyl, oder Cyclohexadienyl, z. B. 1,4-Cyclohexadienyl.

Ein Cycloalkylniederalkenylrest oder Cycloalkenylniederalkylrest $R_a$ ist z. B. Cyclohexylvinyl, Cyclohexylallyl, bzw. Cyclohexenylmethyl oder 1,4-Cyclohexadienylmethyl.

Ein Phenyl- oder ein Phenylniederalkyl-, z. B. Benzyl- oder 1- oder 2-Phenyläthylrest $R_a$ kann, vorzugsweise im aromatischen Rest, z. B. durch Niederalkyl, wie Methyl oder Äthyl, Niederalkoxy, wie Methoxy, oder Halogen, wie Fluor oder Chlor, ferner durch Nitro oder Amino substituiert sein, wobei Phenylniederalkyl in α-Stellung z. B. durch Hydroxy, Hydroxysulfonyloxy, Carboxy, Sulfo oder Amino substituiert sein kann.

In einem Heterocycly- oder Heterocyclyniederalkylrest $R_a$ ist Heterocyclyl ein über ein Kohlenstoffatom gebundener Rest vorzugsweise aromatischen Charakters, wie Pyridyl, z. B. 2-, 3- oder 4-Pyridyl, Thienyl, z. B. 2-Thienyl, oder Fluryl, z. B. 2-Fluryl, oder ein entsprechender Pyridyl-, Thienyl- oder Furylniederalkyl-, insbesondere -methylrest, wobei Heterocyclylniederalkyl in α-Stellung z. B. durch Hydroxy, Hydroxysulfonyloxy, Carboxy, Sulfo oder Amino substituiert sein kann.

Ein Phenyl- oder Naphthyl-niederalkenylrest $R_a$ ist ein wie ein entsprechender Niederalkylrest substituierter Niederalkenylrest, z. B. Phenylvinyl.

Durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy $R_a$ ist insbesondere gegebenenfalls suibstituiertes Niederalkoxy, Cycloalkoxy, Cycloalkylniederalkoxy, Phenoxy, Naphthyloxy oder Phenylniederalkoxy. Substituenten von solchen Resten sind Hydroxy, Niederalkoxy, z. B. Methoxy oder Äthoxy, Niederalkanoyloxy, z. B. Acetyloxy oder Propionyloxy, Halogen, z. B. Chlor oder Brom, Mercapto, Niederalkylmercapto, z. B. Methylthio, Carboxyl, Niederalkoxycarbonyl, z. B. Methoxycarbonyl oder Äthoxycarbonyl, Carbamoyl oder Cyan, ferner Nitro oder gegebenenfalls durch Niederalkyl, z. B. Methyl oder Äthyl, durch $C_1$–$C_{20}$-Acyl, wie Niederalkanoyl, z. B. Acetyl, mono- oder disubstituiertes, oder durch Niederalkylen, z. B. 1,4-Butylen oder 1,5-Pentylen disubstituiertes Amino.

Ein Niederalkoxyrest $R_a$ enthält bis zu 7, insbesondere bis zu 4 Kohlenstoffatome, und ist u. a. Methoxy, Äthoxy, Propoxy, Isopropoxy, Butoxy, Isobutoxy, tert.-Butoxy oder Pentoxy. Substituiertes Niederalkoxy $R_a$ ist in erster Linie substituiertes Methoxy, Äthoxy oder Propoxy, wobei Substituenten in 1-, 2- oder 3-Stellung stehen, wie Methoxymethoxy, Äthoxymethoxy, Methoxyäthoxy oder Methoxypropoxy, Niederalkanoyloxymethoxy, Niederalkanoyloxyäthoxy oder Niederalkanoyloxypropoxy, wie Acetoxymethoxy, Acetoxyäthoxy oder Acetoxypropoxy, Halogenmethoxy, Halogenäthoxy oder Halogenpropoxy, z. B. Chlor- oder Bromäthoxy oder Chlor- oder Brompropoxy, Niederalkoxycarbonylmethoxy oder Niederalkoxycarbonyläthoxy, z. B. Methoxycarbonylmethoxy, Äthoxycarbonylmethoxy oder Methoxycarbonyläthoxy, Cyanmethoxy, Cyanäthoxy, oder gegebenenfalls geschütztes Aminomethoxy, Aminoäthoxy oder Aminopropoxy.

Eine Cycloalkoxygruppe $R_a$ wiest z. B. 3 bis 7 Kohlenstoffatome auf und ist z. B. Cyclopropyloxy, Cyclobutyloxy, Cyclopentyloxy oder Cyclohexyloxy.

Ein Cycloalkylniederalkoxyrest $R_a$ hat z. B. 4 bis 7 Kohlenstoffatome und ist z. B. Cyclopropylmethoxy, Cyclobutylmethoxy, Cyclopentylmethoxy oder Cyclohexylmethoxy.

Ein Phenoxy- oder ein Phenylniederalkoxy-, z. B. Benzyl- oder 1- oder 2-Phenyläthoxyrest $R_a$ kann, vorzugsweise im aromatischen Rest, z. B. durch Niederalkyl, wie Methyl oder Äthyl, Niederalkoxy, wie Methoxy, Halogen, wie Fluor oder Chlor, Nitro oder Amino substituiert sein.

Durch einen $C_1$–$C_{20}$-Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen veresterte Hydroxygruppen $R_a$ sind insbesondere gegebenenfalls substituiertes Niederalkanoyloxy, Cycloalkanoyloxy, Cycloalkyl-niederalkanoyloxy, Benzoyloxy, oder Phenylniederalkanoyloxy. Substituenten solcher Reste sind Hydroxy, Niederalkoxy, z. B. Methoxy, oder Äthoxy, Phenyloxy, Niederalkanoyloxy, z. B. Acetyloxy oder Propionyloxy, Halogen, z. B. Chlor oder Brom, Mercapto, Niederalkylmercapto, z. B. Methylthio, Carboxyl, Niederalkoxycarbonyl, z. B. Methoxycarbonyl oder Äthoxycarbonyl, Carbamoyl oder Cyan, ferner Nitro oder gegebenenfalls durch Niederalkyl, z. B. Methyl oder Äthyl, durch $C_1$–$C_{20}$-Acyl, wie Niederalkanoyl, z. B. Acetyl, mono- oder disubstituiertes, oder durch Niederalkylen, z. B. 1,4-Butylen oder 1,5-Pentylen, disubstituiertes Amino.

Ein Niederalkanoyloxyrest $R_a$ enthält bis zu 7, insbesondere bis zu 4 Kohlenstoffatome und ist z. B. Formyloxy, Acetoxy, Propionyloxy oder Butyryloxy. Substituiertes Niederalkanoyloxy $R_a$ ist in erster Linie substituiertes Acetoxy, z. B. Hydroxyacetoxy, Methoxyacetoxy, Phenyloxyacetoxy, Halogenacetoxy, z. B. Chlor- oder Bromacetoxy, Cyanacetoxy, oder gegebenenfalls geschütztes Glycyloxy.

Ein Cycloalkanoyloxyrest $R_a$ hat 4 bis 8 Kohlenstoffatome und ist z. B. Cyclopropylcarbonyloxy, Cyclobutylcarbonyloxy, Cyclopentylcarbonyloxy oder Cyclohexylcarbonyloxy, oder ein entsprechender, z. B. in 1-Stellung, z. B. durch Hydroxy oder Amino, substituierter Rest.

Ein Cycloalkylniederalkanoyloxyrest $R_a$ hat 5 bis 9 Kohlenstoffatome und ist z. B. Cyclopropylacetoxy, Cyclobutylacetoxy, Cyclohexylacetoxy oder Cyclohexylpropionoxy, oder ein entsprechender, z.B. in 1-Stellung, z.B. durch Hydroxy oder Amino, substituierter Rest.

Ein Benzoyloxy- oder Phenylniederalkanoyloxy-, z.B. Phenylacetyloxyrest $R_a$ kann, vorzugsweise im aromatischen Rest, z.B. durch Niederalkyl, wie Methyl oder Äthyl, Niederalkoxy, wie Methoxy, Halogen, wie Fluor oder Chlor, Nitro oder gegebenenfalls geschütztes Hydroxy oder Amino, substituiert sein. Im Phenylniederalkanoyloxyrest kann gegebenenfalls substituiertes, z.B. geschütztes Hydroxy oder gegebenenfalls substituiertes, z.B. geschütztes Amino auch im aliphatischen Teil stehen, insbesondere in 2-Stellung.

Durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Mercapto $R_a$ ist insbesondere gegebenenfalls substituiertes Niederalkylthio, Cycloalkylthio, Cycloalkylniederalkylthio, Phenylthio oder Phenylniederalkylthio. Substituenten von solchen Resten sind Hydroxy, Niederalkoxy, z.B. Methoxy oder Äthoxy, Niederalkanoyloxy, z.B. Acetyloxy oder Propionyloxy, Halogen, z.B. Chlor oder Brom, Mercapto, Niederalkylmercapto, z.B. Methylthio, Carboxyl, Niederalkoxycarbonyl, z.B. Methoxycarbonyl oder Äthoxycarbonyl, Carbamoyl oder Cyan, ferner Nitro oder gegebenenfalls durch Niederalkyl, z.B. Methyl oder Äthyl, durch $C_1$–$C_{20}$-Acyl, wie Niederalkanoyl, z.B. Acetyl, mono- oder disubstituiertes, oder durch Niederalkylen, z.B. 1,4-Butylen oder 1,5-Pentylen disubstituiertes Amino.

Ein Niederalkylthiorest $R_a$ enthält bis zu 7, insbesondere bis zu 4 Kohlenstoffatome, und ist u.a. Methylthio, Äthylthio, Propylthio, Isopropylthio, Butylthio, Isobutylthio, tert.-Butylthio oder Pentylthio. Substituiertes Niederalkylthio $R_a$ ist in erster Linie substituiertes Methylthio, Äthylthio oder Propylthio, wobei Substituenten in 1-, 2- oder 3-Stellung stehen, wie Methoxymethylthio, Äthoxymethylthio, Methoxyäthylthio oder Methoxypropylthio, Niederalkanoyloxymethylthio, Niederalkanoyloxyäthylthio oder Niederalkanoyloxypropylthio, wie Acetoxymethylthio, Acetoxyäthylthio oder Acetoxypropylthio, Halogenmethylthio, Halogenäthylthio oder Halogenpropylthio, z.B. Chlor- oder Bromäthylthio oder Chlor- oder Brompropylthio, Niederalkoxycarbonylmethylthio oder Niederalkoxycarbonyläthylthio, z.B. Methoxycarbonyläthylthio, Cyanmethylthio, Cyanäthylthio oder gegebenenfalls geschütztes, z.B. N-acyliertes Aminomethylthio, Aminoäthylthio oder Aminopropylthio.

Eine Cycloalkylthiogruppe $R_a$ weist z.B. 3 bis 7 Kohlenstoffatome auf und ist z.B. Cyclopropylthio, Cyclobutylthio, Cyclopentylthio oder Cyclohexylthio.

Ein Cycloalkylniederalkylthiorest $R_a$ hat z.B. 4 bis 7 Kohlenstoffatome und ist z.B. Cyclopropylmethylthio, Cyclobutylmethylthio, Cyclomethylthio oder Cyclohexylmethylthio.

Ein Phenylthio- oder ein Phenylniederalkylthio-, z.B. Benzyl- oder 1- oder 2-Phenylniederalkylthio-, z.B. Benzyl- oder 1- oder 2-Phenyläthylthiorest $R_a$ kann, vorzugsweise im aromatischen Rest, z.B. durch Niederalkyl, wie Methyl oder Äthyl, Niederalkoxy, wie Methoxy, Halogen, wie Fluor oder Chlor, Nitro oder Amino substituiert sein.

Durch einen Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen veresterte Mercaptogruppen $R_a$ sind insbesondere gegebenenfalls substituierte Niederalkanoylthio, Cycloalkylniederalkanoylthio, Benzoylthio oder Phenylniederalkanoylthio. Substituenten solcher Reste sind Hydroxy, Niederalkoxy, z.B. Methoxy oder Äthoxy, Aryloxy, z.B. Phenyloxy, Niederalkanoyloxy, z.B. Acetyloxy oder Propionyloxy, Halogen, z.B. Chlor oder Brom, Mercapto, Niederalkylmercapto, z.B. Methylthio, Carboxyl, Niederalkoxycarbonyl, z.B. Methoxycarbonyl oder Äthoxycarbonyl, Carbamoyl oder Cyan, ferner Nitro oder gegebenenfalls durch Niederalkyl, z.B. Methyl oder Äthyl, durch $C_1$–$C_{20}$-Acyl, wie Niederalkanoyl, z.B. Acetyl, mono- oder disubstituiertes oder durch Niederalkylen, z.B. 1,4-Butylen oder 1,5-Pentylen, disubstiertes Amino.

Ein Niederalkanoylthiorest $R_a$ enthält bis zu 7, insbesondere bis zu 4 Kohlenstoffatome und ist z.B. Formylthio, Acetylthio, Propionylthio oder Butyrylthio. Substituiertes Niederalkanoylthio $R_a$ oder $R_b$ ist in erster Linie substituiertes Acetylthio, z.B. Hydroxyacetylthio, Methoxyacetylthio, Phenyloxyacetylthio, Halogenacetylthio, z.B. Chlor- oder Bromacetylthio, Cyanacetylthio oder gegebenenfalls geschütztes Glycylthio.

Ein Cycloalkanoylthiorest $R_a$ hat 4 bis 8 Kohlenstoffatome und ist z.B. Cyclopropylcarbonylthio, Cyclobutylcarbonylthio, Cyclopentylcarbonylthio oder Cyclohexylcarbonylthio, oder ein entsprechender, z.B. in 2-Stellung, z.B. durch Hydroxy oder Amino, substituierter Rest.

Ein Cycloalkylniederalkanoylthiorest $R_a$ hat 5 bis 9 Kohlenstoffatome und ist z.B. Cyclopropylacetylthio, Cyclobutylacetylthio, Cyclohexylacetylthio oder Cyclohexylpropionylthio, oder ein entsprechender, z.B. in 2-Stellung, z.B. durch Hydroxy oder Amino, substituierter Rest.

Ein Benzoylthio- oder Phenylniederalkanoylthio-, z.B. Phenylacetylthiorest $R_a$ kann, vorzugsweise im aromatischen Rest, z.B. durch Niederalkyl, wie Methyl oder Äthyl, Niederalkoxy, wie Methoxy, Halogen, wie Fluor oder Chlor, Nitro, oder gegebenenfalls geschütztes Hydroxy oder Amino, substituiert sein. Im Phenylniederalkanoylthiorest kann gegebenenfalls substituiertes, z.B. geschütztes Hydroxy oder gegebenenfalls substituiertes, z.B. geschütztes Amino auch im aliphatischen Teil stehen, insbesondere in 2-Stellung.

$R_a$ als Halogen ist Jod oder insbesondere Fluor, Chlor oder Brom.

Ein gesättigter oder ungesättigter, gegebenenfalls substituierter aliphatischer, cycloaliphatischer, cycloaliphatisch-aliphatischer, aromatischer oder araliphatischer Kohlenwasserstoffrest $R_1$ mit bis zu 18, bevorzugt bis zu 10 Kohlenstoffatomen, ist insbesondere gegebenenfalls substituiertes Niederalkyl oder Niederalkenyl, oder gegebenenfalls substituiertes Cycloalkyl, Cycloalkenyl, Cycloalkylniederalkyl, Cycloalkylniederalkenyl, Cycloalkenylniederalkyl, Phenyl, Phenylniederalkyl, oder Phenylniederalkenyl.

Substituenten von solchen Resten $R_1$ und von gegebenenfalls substituierten Heterocyclyl- oder Heterocyclylniederalkylresten $R_1$ mit bis zu 10 Kohlenstoffatomen und bis zu 4 Ringheteroatomen aus der Gruppe Stickstoff, Sauerstoff und/oder Schwefel sind Hydroxy, Niederalkoxy, z.B. Methoxy oder Äthoxy, Niederalkanoyloxy, z.B. Acetyloxy oder Propionyloxy, Halogen, z.B. Chlor oder Brom, Mercapto, Niederalkylmercapto, z.B. Methylthio, oder Heterocyclylmercapto [dieser Heterocyclylrest ist gegebenenfalls substituiert, hat aromatische Eigenschaften oder ist partiell gesättigt; Substituenten sind u.a. Niederalkyl, insbesondere Methyl, Hydroxy-niederalkyl, z.B. Hydroxymethyl, Carboxyniederalkyl, z.B. Carboxymethyl oder 1- oder 2-Carboxyäthyl, Aminoniederalkyl, Diniederalkylaminoniederalkyl, z.B. Dimethylaminoäthyl, in Salzform vorliegendes Sulfoniederalkyl, z.B. als Natriumsalz vorliegendes Sulfomethyl oder 1- oder 2-Sulfoäthyl, Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder funktionelle Gruppen, wie Halogen, z.B. Fluor, Chlor oder Brom, Amino, durch Niederalkyl mono- oder disubstituiertes Amino, z.B. Amino, Methylamino oder Dimethylamino, Nitro, Hydroxy, Niederalkoxy, z.B. Methoxy oder Äthoxy, Carboxyl, Niederalkoxycarbonyl, z.B. Methoxycarbonyl oder Äthoxycarbonyl, gegebenenfalls N-mono- oder N,N-diniederalkyliertes Carbamoyl, z.B. N-Methylcarbamoyl, oder N,N-Dimethylcarbamoyl, oder Cyan, sowie Oxo oder Oxido, wobei einer oder mehrere solcher Substituenten, die in erster Linie mit Ringkohlenstoffatomen, aber auch, insbesondere Niederalkyl und Oxido, mit Ringstickstoffatomen verbunden sind, vorhanden sein können; solche heterocyclische Reste sind in erster Linie gegebenenfalls die obgenannten Substituenten enthaltende, monocyclische, fünfgliedrige diaza-, triaza-, tetraza-, thiaza, thiadiaza-, thiatriazaoxaza- oder oxadiazacyclische Reste aromatischen Charakters oder entsprechende, gegebenenfalls substituierte, z.B. die obgenannten Substituenten enthaltende Reste mit ankondensiertem Benzolring, wie benzodiaza- oder benzooxazacyclische Reste, gegebenenfalls die obgenannten Substituenten, in erster Linie Oxido enthaltende, monocyclische, sechsgliedrige monoaza- oder diazacyclische Reste aromatischen Charakters oder entsprechende, partiell gesättigte, gegebenenfalls substituierte, z.B. die obgenannten Substituenten, in erster Linie Oxo, enthaltende Reste, oder gegebenenfalls substituierte, z.B. die obgenannten Substituenten enthaltende, bicyclische triaza- oder tetrazacyclische Reste aromatischen Charakters oder entsprechende partiell gesättigte, gegebenenfalls substituierte, z.B. die obgenannten Substituenten, in erster Linie Oxo, enthaltende Reste; Beispiele solcher Heterocyclylreste sind Imidazolyl, z.B. 2-Imidazolyl, gegebenenfalls durch Niederalkyl und/oder Phenyl substituiertes Triazolyl, z.B.
1,2,3-Triazol-4-yl,
1-Methyl-1H-1,2,3-triazol-4-yl,
1H-1,2,4-Triazol-3-yl,
5-Methyl-1H-1,2,4-triazol-3-yl,
3-Methyl-1-phenyl-1H-1,2,4-triazol-5-yl,
4,5-Dimethyl-4H-1,2,4-triazol-3-yl oder
4-Phenyl-4H-1,2,4-triazol-3-yl,
gegebenenfalls durch Niederalkyl, Phenyl oder Halogenphenyl substituiertes Tetrazolyl, z.B.
1H-Tetrazol-5-yl, 1-Methyl-1H-tetrazol-5-yl,
1-Phenyl-1H-tetrazol-5-yl,
1-(4-Chlorphenyl)-1H-tetrazol-5-yl,
1-Carboxymethyl-1H-tetrazol-5-yl,
1-(2-Dimethylaminoäthyl)-1H-tetrazol-5-yl
oder 1-Natriumsulfomethyl-1H-tetrazol-5-yl,
gegebenenfalls durch Niederalkyl oder Thienyl substituiertes Thiazolyl oder Isothiazolyl, z.B.
2-Thiazolyl, 4-(2-Thienyl)-2-thiazolyl,
4,5-Dimethyl-2-thiazolyl, 3-Isothiazolyl,
4-Isothiazolyl oder 5-Isothiazolyl,
gegebenenfalls durch Niederalkyl substituiertes Thiadiazolyl z.B.
1,2,3-Thiadiazol-4-yl, 1,2,3-Thiadiazol-5-yl,
1,3,4-Thiadiazol-2-yl,
2-Methyl-1,3,4-thiadiazol-5-yl,
1,2,4-Thiadiazol-5-yl oder
1,2,5-Thiadiazol-3-yl, Thiatriazolyl, z.B.
1,2,3,4-Thiatriazolyl-5-yl,
gegebenenfalls durch Niederalkyl oder Phenyl substituiertes Oxazolyl oder Isoxazolyl, z.B.
5-Oxazolyl, 4-Methyl-5-oxazolyl,
2-Oxazolyl, 4,5-Diphenyl-2-oxazolyl
oder 3-Methyl-5-isoxazolyl,
gegebenenfalls durch Niederalkyl, Phenyl, Nitrophenyl oder Thienyl substituiertes Oxadiazolyl, z.B.
1,2,4-Oxadiazol-5-yl,
2-Methyl-1,3,4-oxadiazol-5-yl,
2-Phenyl-1,3,4-oxadiazol-5-yl,
5-(4-Nitrophenyl)-1,3,4-oxadiazol-2-yl
oder 2-(Thienyl)-1,3,4-oxadiazol-5-yl,
gegebenenfalls durch Halogen substituiertes Benzimidazolyl, z.B. 2-Benzimidazolyl oder 5-Chlor-2-benzimidazolyl, oder gegebenenfalls durch Halogen oder Nitro substituiertes Benzoxazolyl, z.B. 2-Benzoxazolyl, 5-Nitro-2-benzoxazolyl oder 5-Chlor-2-benzoxazolyl,
1-Oxido-pyridyl, z.B. 1-Oxido-2-pyridyl oder 4-Chlor-1-oxido-2-pyridyl, gegebenenfalls durch Hydroxy substituiertes Pyridazinyl, z.B. 3-Hydroxy-6-pyridazinyl, gegebenenfalls durch Niederalkyl, Niederalkoxy oder Halogen substituiertes N-Oxido-pyridazinyl, z.B.
2-Oxido-6-pyridazinyl,
3-Chlor-1-oxido-6-pyridazinyl,
3-Methyl-2-oxido-6-pyridazinyl,
3-Methoxy-1-oxido-6-pyridazinyl,
3-Äthoxy-1-oxido-6-pyridazinyl,

3-n-Butyloxy-1-oxido-6-pyridazinyl oder
3-(2-Äthylhexyloxy)-1-oxido-6-pyridazinyl,
oder gegebenenfalls durch Niederalkyl, Amino, Diniederalkylamino der Carboxy substituiertes 2-Oxo-1,2-dihydro-pyrimidinyl, z.B.
2-Oxo-1,2-dihydro-4-pyrimidinyl,
6-Methyl-2-oxo-1,2-dihydro-4-pyrimidinyl,
5-Methyl-2-oxo-1,2-dihydro-4-pyrimidinyl,
6-Amino-2-oxo-1,2-dihydro-4-pyrimidinyl,
6-Dimethylamino-2-oxo-1,2-dihydro-4-pyrimidinyl,
5-Carboxy-2-oxo-1,2-dihydro-4-pyrimidinyl
oder
6-Carboxy-2-oxo-1,2-dihydro-4-pyrimidinyl,
Triazolopyridyl, z.B.
s-Triazolo[4,3-a]pyrid-3-yl oder
3H-v-Triazolo[4,5-b]pyrid-5-yl,
oder gegebenenfalls durch Halogen und/oder Niederalkyl substituiertes Purinyl, z.B.
Purinyl, 6-Purinyl oder
8-Chlor-2-methyl-6-purinyl, ferner 2-oxo-1,2-dihydro-purinyl, z.B.
2-Oxo-1,2-dihydro-6-purinyl
Carboxyl, Niederalkoxycarbonyl, z.B. Methoxycarbonyl oder Äthoxycarbonyl, Carbamoyl oder Cyan, ferner Nitro oder gegebenenfalls durch Niederalkyl, z.B. Methyl oder Äthyl, durch $C_1$–$C_{20}$-Acyl, wie Niederalkanoyl, z.B. Acetyl, mono- oder disubstituiertes, oder durch Niederalkylen, z.B. 1,4-Butylen oder 1,5-Pentylen, disubstituiertes Amino.

Ein Niederalkylrest $R_1$ enthält bis zu 7, insbesondere bis zu 4 Kohlenstoffatome, und ist z.B. Methyl, Äthyl, Propyl, Isopropyl, Butyl, Isobutyl, tert.-Butyl oder Pentyl. Substituiertes Niederalkyl ist in erster Linie substituiertes Methyl, Äthyl oder Propyl, wobei Substituenten insbesondere in 1-, aber auch in 2- oder in 3-Stellung stehen, wie
Hydroxymethyl, Hydroxyäthyl oder
Hydroxypropyl, Niederalkoxymethyl,
Niederalkoxyäthyl, oder Niederalkoxypropyl,
z.B. Methoxymethyl, Methoxyäthyl oder
Methoxypropyl, Niederalkanoyloxymethyl,
Niederalkanoyloxyäthyl oder
Niederalkanoyloxypropyl, z.B.
Acetyloxymethyl, Propionyloxymethyl,
Acetyloxyäthyl oder Acetyloxypropyl,
Halogenmethyl, Halogenäthyl
oder Halogenpropyl, z.B.
Chlor- oder Bromäthyl,
oder Chlor- oder Brompropyl, Methylthiomethyl,
Methylthioäthyl,
Methylthiopropyl, tert.-Butylthiomethyl,
1,2,3-Triazol-4-ylthiomethyl,
1H-Tetrazol-5-ylthiomethyl,
1-Methyl-1H-tetrazol-5-ylthiomethyl,
1-Carboxymethyl-1H-tetrazol-5-ylthiomethyl,
1-(2-Dimethylaminoäthyl)-1H-tetrazol-5-ylthiomethyl oder
1-Natriumsulfomethyl-1H-tetrazol-5-ylthiomethyl,
1H-Tetrazol-5-ylthioäthyl,
(1-Methyl-1H-tetrazol-5-ylthio)-äthyl,
2-Methyl-1,3,4-thiadiazol-5-ylthiomethyl,
Niederalkoxycarbonylmethyl oder
Niederalkoxycarbonyläthyl, z.B.
Methoxycarbonylmethyl, Äthoxycarbonylmethyl oder Methoxycarbonyläthyl, Cyanmethyl, Cyanäthyl, oder gegebenenfalls geschütztes Aminomethyl, Aminoäthyl oder Aminopropyl.

Ein Niederalkenylrest $R_1$ enthält 2 bis 7, insbesondere 2 bis 4 Kohlenstoffatome, und ist z.B. Vinyl, Allyl oder 2- oder 3-Butenyl. Substituiertes Niederalkenyl kann die gleichen Substituenten tragen wie substituiertes Niederalkyl und ist beispielsweise 2-Amino-vinyl oder 2-Acylamino-, wie 2-Acetylaminovinyl.

Eine gegebenenfalls geschützte Carboxylgruppe $R_1$ ist eine freie oder eine der unter den Gruppen $-C(=O)-R_2^A$ genannten, beispielsweise veresterten oder amidierten Carboxylgruppen, wie Niederalkoxycarbonyl, z.B. Methoxy-, Äthoxy- oder tert.-Butoxycarbonyl, Arylniederalkoxycarbonyl, wie Benzyloxy-, p-Nitro-benzyl- oder Diphenylmethoxycarbonyl, Aryloxycarbonyl, wie gegebenenfalls, z.B. durch Halogen, wie Chlor, Niederalkoxy, wie Methoxy, oder Nitro, substituiertes Phenyloxycarbonyl, wie Phenyloxycarbonyl, o-, m- oder p-Chlorphenyloxy-, Pentachlorphenyloxy-, o-, m- oder p-Methoxyphenyloxy- oder p-Nitrophenyloxycarbonyl, Aminocarbonyl oder substituiertes, wie durch Niederalkyl, z.B. Methyl oder Äthyl, mono- oder disubstituiertes Aminocarbonyl.

Ein Cycloalkylrest $R_1$ weist z.B. 3 bis 7 Kohlenstoffatome auf und ist z.B. Cyclopropyl, Cyclobutyl, Cyclopentyl oder Cyclohexyl, während ein Cycloalkylniederalkylrest $R_1$ beispielsweise 4 bis 7 Kohlenstoffatome enthält und z.B. Cyclopropylmethyl, Cyclobutylmethyl, Cyclopentylmethyl darstellt.

Ein Cycloalkenylrest $R_1$ ist ein entsprechender Cycloalkylrest mit ein oder gegebenenfalls zwei C–C-Doppelbindungen, wie Cyclohexenyl, z.B. 1-Cyclohexenyl, oder Cyclohexadienyl, z.B. 1,4-Cyclohexadienyl.

Ein Cycloalkylniederalkenylrest oder Cycloalkenylniederalkylrest $R_1$ ist z.B. Cyclohexylvinyl, Cyclohexylallyl, bzw. Cyclohexenylmethyl oder 1,4-Cyclohexadienylmethyl.

Ein Phenyl- oder ein Phenylniederalkyl-, z.B. Benzyl- oder 1- oder 2-Phenyläthylrest $R_1$ kann, vorzugsweise im aromatischen Rest, z.B. durch Niederalkyl, wie Methyl oder Äthyl, Niederalkoxy, wie Methoxy, oder Halogen, wie Fluor oder Chlor, ferner durch Nitro oder Amino substituiert sein.

Ein Rest $R_1$ kann auch einen, über ein Kohlenstoffatom gebundenen heterocyclischen oder heterocyclisch-aliphatischen Rest, vorzugsweise aromatischen Charakters darstellen, wie Pyridyl, z.B. 2-, 3- oder 4-Pyridyl, Thienyl, z.B. 2-Thienyl, oder Fluryl, z.B. 2-Fluryl, oder entsprechende Pyridyl-, Thienyl oder Furylniederalkyl-, insbesondere -methylreste, darstellen.

Ein Phenyl- oder Heterocyclylniederalkenylrest $R_1$ ist ein wie ein entsprechender Niederalkylrest substituierter Niederalkenylrest, z.B. Phenylvinyl oder Furylallyl.

Durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere

bis zu 10 Kohlenstoffatomen verätherte Mercaptogruppen $R_1$ sind gegebenenfalls substituiertes Niederalkylthio, Niederalkenylthio, Cycloalkylthio, Cycloalkylniederalkylthio, Phenylthio oder Phenylniederalkylthio. Substituenten von solchen Resten sind Hydroxy, Niederalkoxy, z.B. Methoxy oder Äthoxy, Niederalkanoyloxy, z.B. Acetyloxy oder Propionyloxy, Halogen, z.B. Chlor oder Brom, Mercapto, Niederalkylmercapto, z.B. Methylthio, Carboxyl, Niederalkoxycarbonyl, z.B. Methoxycarbonyl oder Äthoxycarbonyl, Carbamoyl oder Cyan, ferner Nitro oder gegebenenfalls durch Niederalkyl, z.B. Methyl oder Äthyl, durch $C_1$–$C_{20}$ Acyl, wie Niederalkanoyl, z.B. Acetyl, mono- oder disubstituiertes, oder durch Niederalkylen, z.B. 1,4-Butylen oder 1,5-Pentylen disubstituiertes Amino, oder die unten bei den einzelnen verätherten Mercaptogruppen $R_1$ aufgeführten Substituenten.

Ein Niederalkylthiorest $R_1$ enthält bis zu 7, insbesondere bis zu 4 Kohlenstoffatome, und ist z.B. Methylthio, Äthylthio, Isopropylthio, Butylthio, Isobutylthio, tert.-Butylthio oder Pentylthio. Substituiertes Niederalkylthio $R_1$ ist in erster Linie substituiertes Methylthio, Äthylthio oder Propylthio, wobei Substituenten in 1-, 2- oder 3-Stellung stehen, wie
Methoxymethylthio, Äthoxymethylthio,
Methoxyäthylthio, oder Methoxypropylthio,
Niederalkanoyloxymethylthio,
Niederalkanoyloxyäthylthio oder
Niederalkanoyloxypropylthio, wie
Acetoxymethylthio, Acetoxyäthylthio oder
Acetoxypropylthio, Halogenmethylthio,
Halogenäthylthio oder Halogenpropylthio, z.B.
Chlor- oder Bromäthylthio oder
Chlor- oder Brompropylthio,
Niederalkoxycarbonylmethylthio oder
Niederalkoxycarbonyläthylthio, z.B.
Methoxycarbonyläthylthio, Cyanmethylthio,
Cyanäthylthio oder gegebenenfalls geschütztes, z.B. acetyliertes, Aminomethylthio, Aminoäthylthio oder Aminopropylthio.

Ein Niederalkenylthiorest $R_1$ enthält 2 bis 7, insbesondere 2 bis 4 Kohlenstoffatome und ist insbesondere 1-Niederalkenylthio, z.B. Vinylthio, 1-Propenylthio, 1-Butenylthio oder 1-Pentenylthio oder auch 2-Niederalkenylthio, z.B. Allylthio. Substituiertes Niederalkenylthio $R_1$ ist in erster Linie in 2-Stellung substituiert, wobei als Substituenten vor allem Niederalkoxy, Niederalkanoyloxy und gegebenenfalls geschütztes Amino in Frage kommen, und ist beispielsweise 2-Methoxyvinylthio, 2-Acetoxyvinylthio, 2-Acetylaminovinylthio oder entsprechend substituiertes 1-Propenylthio.

Eine Cycloalkylthiogruppe $R_1$ weist z.B. 3 bis 7 Kohlenstoffatome auf und ist z.B. Cyclopropylthio, Cyclobutylthio, Cyclopentylthio oder Cyclohexylthio.

Ein Cycloalkylniederalkylthiorest $R_1$ hat z.B. 4 bis 7 Kohlenstoffatome und ist z.B. Cyclopropylmethylthio, Cyclobutylmethylthio, Cyclopentylmethylthio oder Cyclohexylmethylthio.

Ein Phenylthio- oder ein Phenylniederalkylthio-, z.B. Benzyl- oder 1- oder 2-Phenyläthylthiorest $R_1$ kann, vorzugsweise im aromatischen Rest, z.B. durch Niederalkyl, wie Methyl oder Äthyl, Niederalkoxy, wie Methoxy, Halogen, wie Fluor oder Chlor, Nitro oder Amino substituiert sein.

Durch einen gegebenenfalls substituierten, über ein Ringkohlenstoffatom an die Mercaptogruppe gebundenen, heterocyclischen Rest mit 1 bis 4 Ringstickstoffatomen und gegebenenfalls einem weiteren Ringheteroatom der Gruppe Sauerstoff und Schwefel verätherte Mercaptogruppen $R_1$ sind in erster Linie gegebenenfalls substituierte, z.B. die unten genannten Substituenten enthaltende, monocyclische, fünfgliedrige diaza-, triaza-, tetraza-, thiaza-, thiadiaza-, thiatriaza-, oxaza- oder oxadiazacyclische Reste aromatischen Charakters, oder gegebenenfalls substituierte monocyclische sechsgliedrige aza- oder diazacyclische Reste aromatischen oder partiell gesättigten Charakters.

Substituenten solcher Heterocyclylreste sind Niederalkyl, insbesondere Methyl, sowie Äthyl, n-Propyl, Isopropyl oder geradkettiges oder verzweigtes Butyl, oder durch Hydroxy, Niederalkanoyloxy, Halogen, wie Chlor, Carboxy, Niederalkoxycarbonyl, Sulfo, amidiertes Sulfo, Amino, Mono- oder Diniederalkylamino, $C_1$–$C_{20}$-Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Äthoxycarbonylmethyl, 2-Äthoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, durch Halogen, z.B. Chlor, oder Nitro substituiertes Phenyl, Benzyl, Furyl, z.B. 2-Furyl, Thienyl, z.B. 2-Thienyl, Oxazolyl, z.B. 2- oder 5-Oxazolyl, Halogen, z.B. Fluor, Chlor oder Brom, gegebenenfalls durch Niederalkyl mono- oder di-substituiertes Amino, z.B. Amino, Methylamino oder Dimethylamino, $C_1$–$C_{20}$-Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Äthoxy, n-Butyloxy oder 2-Äthylhexyloxy, Carboxy, Niederalkoxycarbonyl, z.B. Methoxycarbonyl oder Äthoxycarbonyl, gegebenenfalls N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte heterocyclisch verätherte Mercaptogruppen $R_1$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio,

z. B.
1H-1,2,3-Triazol-4-ylthio,
1-Methyl-1H-1,2,3-triazol-4-ylthio,
1H-1,2,4-Triazol-3-ylthio,
5-Methyl-1H-1,2,4-triazol-3-ylthio,
3-Methyl-1-phenyl-1H-1,2,4-triazol-5-ylthio,
4,5-Dimethyl-4H-1,2,4-triazol-3-ylthio oder
4-Phenyl-4H-1,2,4-triazol-3-ylthio,
insbesondere gegebenenfalls wie angegeben substituiertes Tetrazolylthio, z. B.
1H-Tetrazol-5-ylthio,
1-Methyl-1H-tetrazol-5-ylthio,
1-Carboxymethyl-1H-tetrazol-5-ylthio,
1-(2-Carboxyäthyl)-1H-tetrazol-5-ylthio,
1-Sulfomethyl-1H-tetrazol-5-ylthio,
1-(2-Sulfoäthyl)-1H-tetrazol-5-ylthio,
1-(2-Dimethylaminoäthyl)-1H-tetrazol-5-ylthio,
1-Phenyl-1H-tetrazol-5-ylthio oder
1-(4-Chlorphenyl)-1H-tetrazol-5-ylthio,
gegebenenfalls durch Niederalkyl oder Thienyl substituiertes Thiazolylthio oder Isothiazolylthio, z. B.
2-Thiazolylthio,
4-(2-Thienyl)-2-thiazolylthio,
4,5-Dimethyl-2-thiazolylthio,
3-Isothiazolylthio, 4-Isothiazolylthio oder
5-Isothiazolylthio,
insbesondere auch gegebenenfalls wie angegeben substituiertes Thiadiazolylthio, z. B.
1,2,3-Thiadiazol-4-ylthio,
1,2,3-Thiadiazol-5-ylthio,
1,3,4-Thiadiazol-2-ylthio,
2-Methyl-1,3,4-thiadiazol-5-ylthio,
2-(3-Carboxypropionylamino)-1,3,4-thiadiazol-5-ylthio,
1,2,4-Thiadiazol-5-ylthio oder
1,2,5-Thiadiazol-3-ylthio,
Thiatriazolylthio, z. B.
1,2,3,4-Thiatriazolyl-5-ylthio,
gegebenenfalls wie angegeben substituiertes Oxazolylthio oder Isoxazolylthio, z. B.
5-Oxazolylthio, 4-Methyl-5-oxazolylthio,
2-Oxazolylthio,
4,5-Diphenyl-2-oxazolylthio oder
3-Methyl-5-isoxazolylthio,
oder gegebenenfalls wie angegeben substituiertes Oxadiazolylthio, z. B.
1,2,4-Oxadiazol-5-ylthio,
2-Methyl-1,3,4-oxadiazol-5-ylthio,
2-Phenyl-1,3,4-oxadiazol-5-ylthio,
5-(4-Nitrophenyl)-1,3,4-oxadiazol-2-ylthio
oder 2-(2-Thienyl)-1,3,4-oxadiazol-5-ylthio.

Bevorzugte heterocyclisch verätherte Mercaptogruppen $R_1$, worin der heterocyclische Rest einen entsprechenden monocyclischen, sechsgliedrigen Rest oder einen entsprechenden partiell gesättigten Rest darstellt, sind u.a. gegebenenfalls durch Halogen substituiertes 1-Oxido-pyridylthio, z. B. 1-Oxido-2-pyridylthio oder 4-Chlor-1-oxido-2-pyridylthio, gegebenenfalls durch Hydroxy substituiertes Pyridazinylthio, z. B. 3-Hydroxy-6-pyridazinylthio, gegebenenfalls durch Niederalkyl, Niederalkoxy oder Halogen substituiertes N-Oxidopyridazinylthio, z. B. 2-Oxido-6-pyridazinylthio,
3-Chlor-1-oxido-6-pyridazinylthio,
3-Methyl-2-oxido-6-pyridazinylthio,
3-Methoxy-1-oxido-6-pyridazinylthio,
3-Äthoxy-1-oxido-6-pyridazinylthio,
3-n-Butyloxy-1-oxido-6-pyridazinylthio oder
3-(2-Äthylhexyloxy)-1-oxido-6-pyridazinylthio,
oder gegebenenfalls durch Niederalkyl, Amino, Diniederalkylamino oder Carboxy substituiertes 2-Oxo-1,2-dihydro-pyrimidinylthio, z. B.
2-Oxo-1,2-dihydro-4-pyrimidinylthio,
6-Methyl-2-oxo-1,2-dihydro-4-pyrimidinylthio,
5-Methyl-2-oxo-1,2-dihydro-4-pyrimidinylthio,
6-Amino-2-oxo-1,2-dihydro-4-pyrimidinylthio,
6-Dimethylamino-2-oxo-1,2-dihydro-4-pyrimidinylthio,
5-Carboxy-2-oxo-1,2-dihydro-4-pyrimidinylthio oder
6-Carboxy-2-oxo-1,2-dihydro-4-pyrimidinylthio.

Eine geschützte Carboxylgruppe der Formel $-C(=O)-R_2^A$ ist in erster Linie eine veresterte Carboxylgruppe, worin $R_2^A$ eine durch einen organischen Rest oder eine organische Silyl- oder Stannylgruppe verätherte Hydroxygruppe darstellt. Organische Reste, auch als Substituenten in organischen Silyl- oder Stannylgruppen, sind aliphatische, cycloyliphatische, cycloaliphatischaliphatische, aromatische oder aralyphatische Reste, insbesondere gegebenenfalls substituierte Kohlenwasserstoffreste dieser Art, sowie heterocyclische oder heterocyclisch-aliphatische Reste, vorzugsweise mit bis zu 18 Kohlenstoffatomen.

Eine verätherte Hydroxygruppe $R_2^A$ bildet zusammen mit der Carbonylgruppierung eine, vorzugsweise leicht spaltbare, z. B. reduktiv, wie hydrogenoletisch, oder solvolytisch, wie acidolytisch oder insbesondere basisch oder neutral hydrolytisch, sowie oxidatic oder unter physiologischen Bedingungen spaltbare, oder leicht in eine andere funktionell abgewandelte Carboxylgruppe, wie in eine andere veresterte Carboxylgruppe oder in eine Hydrazinocarbonylgruppe umwandelbare, veresterte Carboxylgruppe. Eine solche Gruppe $R_2^A$ ist z. B. 2-Halogen-niederalkoxy, worin Halogen vorzugsweise ein Atomgewicht von über 19 hat, z. B. 2,2,2-Trichloräthoxy oder 2-Jodäthoxy, ferner 2-Chloräthoxy oder 2-Bromäthoxy, das sich leicht in letzteres überführen lässt, oder 2-Niederalkylsulfonyl-niederalkoxy, z. B. 2-Methylsulfonyläthoxy. Die Gruppe $R_2^A$ ist ferner eine durch gegebenenfalls substituierte Kohlenwasserstoffreste, insbesondere gesättigte aliphatische oder aromatische Kohlenwasserstoffreste, wie Niederalkyl, z.B. Methyl und/oder Phenyl, polysubstituierte Methoxygruppe oder eine durch einen ungesättigten aliphatischen Kohlenwasserstoffrest, wie Niederalkenyl, z.B. 1-Niederalkenyl, wie Vinyl, durch eine Elektronen-abgebende Substituenten aufweisende carbocyclische Arylgruppe oder durch eine Sauerstoff oder Schwefel als Ringglied aufweisende heterocyclische Gruppe aromatischen Charakters monosubstituierte Methoxygruppe, wie tert.-Niederalkoxy, z. B. tert.-Butyloxy oder tert.-Pentyloxy, gegebenenfalls substituiertes Diphenylmethoxy, z. B. Diphenylmethoxy

oder 4,4'-Dimethoxydiphenylmethoxy, Niederalkenyloxy, insbesondere 2-Niederalkenyloxy, z.B. Allyloxy, Niederalkoxy-phenylniederalkoxy, z.B. Niederalkoxy-benzyloxy, wie Methoxybenzyloxy (wobei Methoxy in erster Linie in 3-, 4- und/oder 5-Stellung steht), in erster Linie 3- oder 4-Methoxybenzyloxy, 3,4-Dimethoxybenzyloxy, oder vor allem Nitrobenzyloxy, z.B. 4-Nitrobenzyloxy, 2-Nitrobenzyloxy oder 4,5-Dimethoxy-2-nitro-benzyloxy, bzw. Furfuryloxy, wie 2-Furfuryloxy. Die Gruppe $R_2^A$ ist ferner eine 2-Oxoäthoxygruppe, die in 2-Stellung gegebenenfalls durch Niederalkyl, wie Methyl, Niederalkoxy, wie Methoxy oder Äthoxy, durch Aralkyl, wie Benzyl, oder durch Aryl, wie Phenyl, und in 1-Stellung durch Niederalkyl, wie Methyl, Niederalkoxycarbonyl, wie Methoxycarbonyl, Niederalkylcarbonyl, wie Methylcarbonyl, Aralkylcarbonyl, wie Benzylcarbonyl oder Arylcarbonyl, wie Benzoyl, substituiert ist, und stellt beispielsweise Acetonyloxy, Phenacyloxy, 2,4-Dioxo-3-pentyloxy, 1-Methoxycarbonyl-2-oxo-propyloxy oder 1-Äthoxycarbonyl-2-oxo-propyloxy dar. Die Gruppe $R_2^A$ ist auch eine 2-Cyanäthoxygruppe, die gegebenenfalls in 1- und/oder in 2-Stellung beispielsweise durch Niederalkyl, wie Methyl, oder Aryl, wie gegebenenfalls substituiertes Phenyl, substituiert ist, und stellt beispielsweise 2-Cyanäthoxy oder 2-Cyan-2-penyläthoxy dar. $R_2^A$ ist auch eine 2-$(S_1)(S_2)(S_3)$-Silyläthoxygruppe, worin die Substituenten $S_1$, $S_2$ und $S_3$ unabhängig voneinander je einen gegebenenfalls substituierten Kohlenwasserstoffrest bedeuten und die einzelnen Reste durch eine einfache C–C-Bindung verknüpft sein können. Ein Kohlenwasserstoffrest $S_1$, $S_2$ und $S_3$ ist beispielsweise ein Alkyl-, Cycloalkyl- oder Arylrest, vorzugsweise ein solcher mit höchstens 12 Kohlenstoffatomen, wobei der Rest einer Art durch einen Rest einer anderen Art oder durch Niederalkoxy, wie Methoxy, oder Halogen, wie Fluor oder Chlor, substituiert sein kann, und ist insbesondere Niederalkyl mit bis zu 7, bevorzugt bis zu 4 Kohlenstoffatomen, wie Methyl, Äthyl, Propyl oder Butyl, Cycloalkyl mit bis zu 7 Kohlenstoffatomen, wie Cyclopropyl oder Cyclohexyl, Cycloalkylalkyl, wie Cyclopentylmethyl, Aryl mit bis zu 10 Kohlenstoffatomen, wie Phenyl, Tolyl oder Xylyl, Arylniederalkyl, wie Benzyl oder Phenyläthyl. Hervorzuhebende Reste $R_2^A$ dieser Art sind 2-Triniederalkylsilyläthoxy, wie 2-Trimethylsilyläthoxy oder 2-(Dibutyl-methyl-silyl)-äthoxy, sowie 2-Triarylsilyläthoxy-, wie 2-Triphenylsilyläthoxy.

$R_2^A$ kann auch 2-Oxa- oder 2-Thia-cycloalkoxy oder -cycloalkenyloxy mit 5–7 Ringgliedern, wie 2-Tetrahydrofuryloxy, 2-Tetrahydropyranyloxy oder 2,3-Dihydro-2-pyranyloxy oder eine entsprechende Thiagruppe sein oder $R_2^A$ bildet zusammen mit der –C(=O)-Gruppierung eine aktivierte Estergruppe und ist beispielsweise Nitrophenyloxy, z.B. 4-Nitrophenyloxy oder 2,4-Dinitrophenyloxy, oder Polyhalogenphenyloxy, z.B. Pentachlorphenyloxy. $R_2^A$ kann aber auch Niederalkoxy, z.B. Methoxy oder Äthoxy, sein.

Eine organische Silyloxy- oder organische Stanyloxygruppe $R_2^A$ ist insbesondere eine durch 1 bis 3 gegebenenfalls substituierte Kohlenwasserstoffreste, vorzugsweise mit bis zu 18 Kohlenstoffatomen, substituierte Silyloxy- oder Stannyloxygruppe. Sie enthält als Substituenten vorzugsweise gegebenenfalls substituierte, beispielsweise durch Niederalkoxy, wie Methoxy, oder durch Halogen, wie Chlor, substituierte aliphatische, cycloaliphatische, aromatische oder araliphatische Kohlenwasserstoffreste, wie Niederalkyl, Halogen-niederalkyl, Cycloalkyl, Phenyl oder Phenylniederalkyl, und stellt in erster Linie Triniederalkylsilyloxy, z.B. Trimethylsilyloxy, Halogenniederalkoxy-niederalkylsilyloxy, z.B. Chlormethoxy-methyl-silyloxy, oder Triniederalkylstannyloxy, z.B. Trin-n-butylstannyloxy, dar.

Die Gruppe $R_2^A$ kann auch eine verätherte Hydroxygruppe sein, die zusammen mit der Carbonylgruppierung –C(=O)– eine unter physiologischen Bedingungen spaltbare veresterte Carboxylgruppe bildet.

Diese Estergruppen verleihen den eigentlich wirksamen Carbonsäuren eine verbesserte Absorption bei oraler Applikation und/oder eine verlängerte Wirksamkeit. Zahlreiche solcher Estergruppen sind auf dem Gebiet der Penicilline und Cephalosporine bekannt. Genannt seien beispielsweise –C(=O)–$R_2^A$-Gruppen, worin $R_2^A$ eine durch Acyl, Acyloxy, Acylthio, Acylamino, oder veräthertes Hydroxy und gegebenenfalls einen weiteren organischen Rest substituierte Methyloxygruppe, wobei die Methylgruppe mit dem Carbonyl der Acylgruppe auch über eine Kohlenstoff enthaltende Brücke verbunden sein kann, oder eine 2-Amino-aliphatyloxygruppe darstellt. In solchen Gruppen stellt Acyl den Rest einer organischen Carbonsäure mit etwa bis zu 18 C-Atomen dar und ist beispielsweise gegebenenfalls substituiertes Alkanoyl, Cycloalkanoyl, Aroyl, Heterocyclylcarbonyl, z.B. auch der Heterocyclylcarbonylrest einer Carbonsäure der Formel I, oder einer biologisch aktiven Penam-3- oder Cephem-4-carbonsäure, oder der Acylrest eines Halbesters der Kohlensäure. Veräthertes Hydroxy in der Methoxygruppe ist durch einen Kohlenwasserstoffrest, insbesondere durch Niederalkyl veräthert. Der gegebenenfalls die Methyloxygruppe zusätzlich substituierende organische Rest hat bis zu 7-C-Atome und ist insbesondere Niederalkyl, wie Methyl, oder Aryl, wie Phenyl. Die genannte Kohlenstoffbrücke enthält ein bis drei, insbesondere 2 C-Atome, so dass ein Lacton, insbesondere ein $\gamma$-Lacton vorliegt. Die Aliphatylgruppe in der genannten 2-Aminoaliphatylgruppe kann aliphatischer oder cycloaliphatischer Natur sein und ist gesättigt oder ungesättigt. Die 2-Aminogruppe ist bevorzugt durch zwei Niederalkylgruppen oder gegebenenfalls eine Oxagruppe enthaltendes Alkylen substituiert. In solchen physiologisch spaltbaren Estergruppen –C(=O)–$R_2^A$ ist $R_2^A$ beispielsweise Niederalkanoyloxy-methoxy, z.B. Acetyloxymethoxy oder Pivaloyloxymethoxy, Aminoniederalkanoyloxymethoxy, insbesondere $\alpha$-Aminoniederalkanoyloxymethoxy, z.B. Glycyloxymethoxy, L-Valyloxymethoxy, L-Leucyloxymethoxy, Niederalkoxycarbonyloxymethoxy oder 1-Niederalkoxycarbonyloxyäthoxy, z. B. 1-Äthyloxycarbonyloxyäthoxy, Niederalkanoylthiomethoxy, z. B. Acetylthiomethoxy oder Pivaloylthiomethoxy, Niederalkanoylaminomethoxy, worin Niederalkanoyl gegebenenfalls durch Halogen, wie Chlor, substituiert sein kann, z. B. Acetylaminomethoxy oder 2,2-Dichloracetylaminomethoxy, Aroylaminomethoxy, z. B. Benzoylaminomethoxy, oder, als Beispiel für ein Lacton enthaltendes $R_2^A$, Phtalidyloxy. Die verätherte Hydroxymethoxygruppe $R_2^A$ ist beispielsweise Niederalkoxymethoxy, insbesondere Methoxymethoxy. Eine 2-Aminoaliphatyloxygruppe $R_2^A$ ist beispielsweise 2-Aminoniederalkoxy, wie eine 2-Aminoäthyloxygruppe, worin Amino durch zwei Niederalkyl oder gegebenenfalls eine Oxagruppe enthaltendes Alkylen substituiert ist, z. B. 2-Dimethylaminoäthoxy, 2-Diäthylaminoäthoxy oder 2-(1-Morpholino)-äthoxy, oder 2-Aminocycloalkyloxy, z. B. 2-Dimethylaminocyclohexyloxy.

Eine zusammen mit einer –C(=O)-Gruppierung eine gegebenenfalls substituierte Hydrazinocarbonylgruppe bildender Rest $R_2^A$ ist z. B. Hydrazino oder 2-Niederalkylhydrazino, z. B. 2-Methylhydrazino.

Bevorzugte Gruppen $R_2^A$ sind solche, die unter neutralen, basischen oder auch unter physiologischen Bedingungen in eine freie Hydroxygruppe übergeführt werden können.

Eine gegebenenfalls einen oder zwei Substituenten tragende Methylidengruppe Z' ist insbesondere eine Gruppe, die durch Oxidation in eine Oxogruppe Z übergeführt werden kann. Ein Substituent dieser Methylidengruppe ist ein organischer Rest, beispielsweise einer der unter $R_1$ erwähnten organischen Reste, wie einer der genannten gegebenenfalls substituierten Niederalkyl-, Cycloalkyl-, Cycloalkylniederalkyl-, Phenyl- oder Phenylniederalkylreste, und insbesondere eine der geschützten, wie veresterten, inklusive mit einem optisch aktiven Alkohol, wie 1-Menthol, veresterten Carboxylgruppen. Diese Methylidengruppe trägt bevorzugt einen der genannten Substituenten. Hervorzuheben sind die 2-Carbomethoxymethyliden- und die 2-Carbo-1-methyloxymethylidengruppe Z'. Letztere kann zur Herstellung optisch aktiver Verbindungen der Formeln IV bis VI und II verwendet werden.

Die Gruppe $X^{\oplus}$ in der Verbindung der Formel II ist eine der bei Wittig-Kondensationsreaktionen gebräuchlichen Phosphonio- oder Phosphonogruppen, insbesondere eine Triaryl-, z. B. Triphenyl-, oder Triniederalkyl-, z. B. Tributylphosphoniogruppe, oder eine durch Niederalkyl, z. B. Äthyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall, z. B. Lithium-, Natrium- oder Kaliumion, umfasst. Bevorzugt als Gruppe $X^{\oplus}$ sind einerseits Triphenylphosphonio und anderseits Diäthylphosphono zusammen mit einem Alkalimetall-, z. B. Natriumion.

In den Verbindungen der Formel II können die beiden asymmetrischen Kohlenstoffatome in 3- und 4-Stellung in der R-, der S- oder der racemischen R,S-Konfiguration vorliegen. Bevorzugt sind die Verbindungen, in denen die Konfiguration des 4-Kohlenstoffatoms derjenigen des natürlichen Penicillins entspricht (4R-Konfiguration). Die Substituenten in 3- und 4-Stellung können cis oder trans zueinander stehen.

Die Erfindung betrifft insbesondere Verbindungen der Formel II, worin $R_a$ Niederalkyl, Hydroxyniederalkyl, Niederalkoxyniederalkyl, Niederalkanoyloxyniederalkyl, in Salzform vorliegendes Hydroxysulfonyloxyniederalkyl, Hydroxy, Niederalkoxy, Niederalkanoyloxy, Niederalkanoyloxy substituiert durch Phenyloxy, Halogen, Amino oder Cyan, Phenylniederalkanoyloxy, oder durch Hydroxy oder Amino substituiertes Phenylniederalkanoyloxy ist, $R_1$ Wasserstoff,
Niederalkyl, Hydroxyniederalkyl,
Niederalkoxyniederalkyl,
Niederalkanoyloxyniederalkyl,
Niederalkylthioniederalkyl,
Heterocyclylthioniederalkyl,
worin Heterocyclyl einen gegebenenfalls durch Niederalkyl, Carboxyniederalkyl, Diniederalkylaminoniederalkyl oder in Salzform vorliegendes Sulfoniederalkyl substituierten fünfgliedrigen aromatischen diaza-, triaza-, tetraaza-, thiaza-, thiadiaza-, thiatriaza-, oxaza- oder oxadiazacyclischen Rest darstellt, Aminoniederalkyl, Acylaminoniederalkyl, worin Acyl Niederalkanoyl oder eine als Aminoschutzgruppe gebräuchliche substituierte Oxycarbonylgruppe, z. B. eine tert.-Butyl-, 2,2,2-Trichloräthyl-, Diphenylmethyl- oder p-Nitrobenzyloxycarbonylgruppe ist, Carboxyniederalkyl, Niederalkoxycarbonylniederalkyl, Phenylniederalkyl, Phenyl, Nydroxyphenyl, Aminophenyl, Furyl, Thienyl, Pyridyl, Niederalkylthio, durch Amino, Mono- oder Diniederalkylamino oder Niederalkanoylamino substituiertes Niederalkylthio oder Niederalkenylthio, oder gegebenenfalls durch Niederalkyl, Sulfoniederalkyl, Carboxyniederalkyl oder Diniederalkylaminoniederalkyl substituiertes Tetrazolylthio oder Thiadiazolylthio darstellt, wobei funktionelle Gruppen in solchen Resten $R_a$ und $R_1$ vorzugsweise in geschützter Form vorliegen, und Z', $X^{\oplus}$ und $R_2^A$ die oben angegebenen Bedeutungen haben in racemischer oder optisch aktiver Form.

In erster Linie steht in einer Verbindung der Formel II $R_a$ für Niederalkyl mit bis zu 4 Kohlenstoffatomen, z. B. Methyl, Äthyl, Propyl oder Butyl, Hydroxyniederalkyl, insbesondere 1-Hydroxyniederalkyl, mit bis zu 4 Kohlenstoffatomen, z. B. Hydroxymethyl, Hydroxyäthyl, oder Hydroxypropyl, Niederalkoxyniederalkyl, insbesondere 1-Niederalkoxyniederalkyl, worin Niederalkyl bis zu 4 Kohlenstoffatome enthält, z. B. Methoxymethyl, 1-Methoxyäthyl oder 1-Methoxypropyl, Niederalkanoyloxyniederalkyl, insbesondere 1-Niederalkanoyloxyniederalkyl, worin Niederalkanoyloxy und Niederalkyl je bis zu 4 Kohlenstoffatome enthalten, z. B. Acetoxymethyl, Propionyloxymethyl oder 1-Acetyloxyäthyl, in Salzform vorliegendes Hydroxysulfonyloxyniederalkyl, worin Niederalkyl bis zu 4 Kohlenstoffatome enthält, insbesondere

1-Hydroxysulfonyloxyniederalkyl, z. B. Hydroxysulfonyloxymethyl, 1-Hydroxysulfonyloxyäthyl oder 1-Hydroxysulfonyloxypropyl, Hydroxy, Niederalkoxy mit bis zu 4 Kohlenstoffatomen, z. B. Methoxy, Äthoxy, Propoxy oder Butoxy, Niederalkanoyloxy mit bis zu 4 Kohlenstoffatomen, z. B. Formyloxy, Acetoxy oder Propionyloxy, oder solches Niederalkanoyloxy substituiert durch Phenyloxy, Hydroxy, Halogen, Amino oder Cyan, z. B. Phenoxyacetoxy, Hydroxyacetoxy, Halogenacetoxi Aminoacetoxy oder Cyanacetoxy, Phenylniederalkanoyloxy, worin Niederalkanoyloxy bis zu 4 Kohlenstoffatome enthält, z. B. Phenylacetoxy, oder durch Hydroxy oder Amino substituiertes Phenylniederalkanoyloxy, worin Niederalkanoyl bis zu 4 Kohlenstoffatome enthält, z. B. Hydroxy- oder Aminophenylacetoxy, wobei Hydroxy oder Amino bevorzugt in p-Stellung stehen, oder α-Hydroxy, oder α-Amino-phenylacetoxy, $R_1$ für Wasserstoff, Niederalkyl mit bis zu 4 Kohlenstoffatomen, z. B. Methyl, Äthyl, Propyl oder Butyl, Hydroxyniederalkyl, insbesondere ω-Hydroxyniederalkyl, mit bis zu 4 Kohlenstoffatomen, z. B. Hydroxymethyl, Hydroxyäthyl oder Hydroxypropyl, Niederalkoxyniederalkyl, insbesondere ω-Niederalkoxyniederalkyl, worin Niederalkoxy und Niederalkyl bis zu 4 Kohlenstoffatome enthalten, z. B. Methoxymethyl, Methoxyäthyl oder Methoxypropyl, Niederalkanoyloxyniederalkyl, insbesondere ω-Niederalkanoyloxyniederalkyl, worin Niederalkanoyloxy und Niederalkyl je bis zu 4 Kohlenstoffatome enthalten, z. B. Acetoxymethyl, Acetoxyäthyl oder Acetoxypropyl, Niederalkylthioniederalkyl, insbesondere ω-Niederalkylthioniederalkyl, worin Niederalkyl bis 4 Kohlenstoffatome enthält, z. B. Methylthiomethyl, tert. Butylthiomethyl, Methylthioäthyl oder Methylthiopropyl, Heterocyclylthioniederalkyl, worin Niederalkyl bis zu 4 Kohlenstoffatome enthält und Heterocyclyl einen gegebenenfalls durch Niederalkyl, wie Methyl, Carboxyniederalkyl, z. B. Carboxymethyl oder 1- oder 2-Carboxyäthyl, gegebenenfalls N-substituiertes Aminoniederalkyl, wie Diniederalkylaminoniederalkyl, z. B. Dimethylaminoäthyl, in Salzform vorliegendes Sulfoniederalkyl, z. B. als Natriumsalz vorliegendes Sulfomethyl oder 1- oder 2-Sulfoäthyl, substituierten fünfgliedrigen aromatischen diaza-, triaza-, tetraza-, thiaza-, thiadiaza-, thiatriaza-, oxaza- oder oxadiazacyclischen Rest darstellt, z. B.

2-Imidazolylthiomethyl, 1,2,3-Triazol-4-ylthiomethyl,
1-Methyl-1H-1,2,3-triazol-4-ylthiomethyl,
1H-Tetrazol-5-ylthiomethyl,
1-Methyl-1H-tetrazol-5-ylthiomethyl,
1-Carboxymethyl-1H-tetrazo-5-ylthiomethyl,
1-(2-Dimethylaminoäthyl)-1H-tetrazol-5-ylthiomethyl,
1-Natriumsulfomethyl-1H-tetrazol-5-ylthiomethyl oder
2-Methyl-1,3,4-thiadiazol-5-ylthiomethyl

oder ein entsprechend in 2-Stellung substituierter Äthylrest, Aminoniederalkyl, insbesondere ω-Aminoniederalkyl, worin Niederalkyl bis 4 Kohlenstoffatome enthält, z. B. Aminomethyl, Aminoäthyl oder Aminopropyl, Acylaminoniederalkyl, worin Acyl Niederalkanoyl oder eine als Aminoschutzgruppe gebräuchliche substituierte Oxycarbonylgruppe ist, z. B. Acetylaminomethyl, Acetylaminoäthyl, Acetylaminopropyl, oder tert.-Butyl-, 2,2,2-Trichloräthyl-, Diphenylmethyl- oder p-Nitrobenzyl-oxycarbonylaminomethyl, -äthyl oder -propyl, Carboxyniederalkyl, worin Niederalkyl bis zu 4 Kohlenstoffatome enthält und Carboxy insbesondere in ω-Stellung steht, z. B. Carboxymethyl, Carboxyäthyl oder Carboxypropyl, Niederalkoxycarbonylniederalkyl, worin Niederalkoxy und Carbonylniederalkyl je bis zu 4 Kohlenstoffatome enthalten, z. B. Methoxy-, Äthoxy- oder tert.-Butoxycarbonylmethyl oder -äthyl, oder Phenylniederalkyl, worin Niederalkyl bis zu 4 Kohlenstoffatome enthält, z. B. Benzyl, Phenyläthyl oder 1Phenylpropyl, Phenyl, Hydroxyphenyl, Aminophenyl, Furyl, Thienyl oder Pyridyl, wie 2-Furyl, 2-Thienyl, 2-, 3- oder 4-Pyridyl, Niederalkylthio, z. B. Methyl-, Äthyl- oder Propylthio, Niederalkenylthio, z. B. Vinylthio oder Allylthio, oder durch Amino, Mono- oder Diniederalkylamino oder Niederalkanoylamino, insbesondere in ω-Stellung, substituiertes Niederalkylthio oder Niederalkenylthio, z. B. 2-Amino-, 2-Methylamino-, 2-Dimethylamino-, 2-Acetylaminoäthylthio, 3-Amino-, 3-Methylamino-, 3-Dimethylamino-, oder 3-Acetylaminopropylthio, 2-Acetylaminovinylthio, gegebenenfalls durch Niederalkyl, Sulfoniederalkyl, Carboxyniederalkyl, oder Diniederalkylaminoniederalkyl substituiertes Tetrazolylthio oder Thiadiazolylthio, insbesondere

1-Methyl-1H-tetrazol-5-ylthio,
1-Sulfomethyl-1H-tetrazol-5-ylthio,
1-Carboxymethyl-1H-tetrazol-5-ylthio,
1-(2-Dimethyl-aminoäthyl)-1H-tetrazol-5-ylthio,
2-Methyl-1,3,4-thiadiazol-5-yltio oder
1,3,4-thiadiazol-2-ylthio,

$R_2^A$ für gegebenenfalls α-polyverzweigtes Niederalkoxy, z. B. Methoxy oder tert.-Butyloxy, oder 2-Halogenniederalkoxy, z. B. 2,2,2-Trichloräthoxy, 2-Jodäthoxy oder das leicht in dieses überführbare 2-Chloräthoxy oder 2-Bromäthoxy, ferner Phenacyloxy, 1-Phenylniederalkoxy mit 1–3, gegebenenfalls durch Niederalkoxy und/oder Nitro substituierten Phenylresten, z. B.

4-Methoxybenzyloxy, 4-Nitrobenzyloxy,
2-Nitro-4,5-dimethoxy-benzyloxy,
Diphenylmethoxy,
4,4′-Dimethoxydiphenylmethoxy oder
Trityloxy, Acetonyloxy, 2-Cyanäthoxy,
2-Triniederalkylsilyläthoxy, z. B.
2-Trimethylsilyläthoxy,
Niederalkanoyloxymethoxy, z. B.
Acetyloxymethoxy oder Pivaloyloxymethoxy,
α-Aminonideralkanoyloxymethoxy, z. B.
Glycyloxymethoxy, Phthalidyloxy,
Pentachlorphenyloxy,

ferner Triniederalkylsilyloxy, z. B. Trimethylsilyloxy, sowie Niederalkenyloxy, wie 2-Niederalkenyloxy, z. B. Allyloxy, und worin Z′ und $X^{\oplus}$ die oben angegebenen Bedeutungen haben.

Die Erfindung betrifft in erster Linie Verbindungen der Formel II, worin $R_a$ Niederalkyl mit bis zu 4 Kohlenstoffatomen, insbesondere Methyl, Äthyl, Propyl, Isopropyl oder Butyl, 1-Hydroxyniederalkyl mit bis zu 4 Kohlenstoffatomen, insbesondere Hydroxymethyl, 1-Hydroxyäthyl, 1-Hydroxypropyl oder 1-Hydroxyisopropyl, Phenylniederalkyl mit bis zu 10 Kohlenstoffatomen, insbesondere Benzyl, Phenoxyniederalkanoyloxy, mit bis zu 10 Kohlenstoffatomen, insbesondere Phenoxyacetyloxy, oder Niederalkoxy mit bis zu 4 Kohlenstoffatomen, insbesondere Methoxy, ist, und $R_1$ Wasserstoff, Niederalkyl mit bis zu 4 Kohlenstoffatomen, insbesondere Methyl, Aminoniederalkyl, insbesondere ω-Aminoniederalkyl, worin Niederalkyl bis 4 Kohlenstoffatome enthält, z.B. Aminomethyl, Aminoäthyl oder Aminopropyl, Acylaminoniederalkyl, worin Acyl Niederalkanoyl oder eine als Aminoschutzgruppe gebräuchliche substituierte Oxycarbonylgruppe ist, z.B.
Acetylaminomethyl, Acetylaminoäthyl,
Acetylaminopropyl, oder tert.-Butyl,
2,2,2-Trichloräthyl-, Diphenylmethyl- oder
p-Nitrobenzyl-oxycarbonylaminomethyl,
-äthyl oder -propyl,
Niederalkylthio, insbesondere Äthylthio, durch Amino, Mono- oder Diniederalkylamino oder Niederalkanoylamino, insbesondere in ω-Stellung, substituiertes Niederalkylthio oder Niederalkenylthio, z.B.
2-Amino-, 2-Methylamino-, 2-Dimethylamino-,
2-Acetylaminoäthylthio, 3-Amino-,
3-Methylamino-, 3-Dimethylamino-, oder
3-Acetylaminopropylthio,
2-Acetylaminovinylthio,
1-Methyl-1H-tetrazol-5-yl-thio,
1-(2-Dimethylaminoäthyl)-1H-tetrazol-
5-ylthio,
2-Methyl-1,3,4-thiadiazol-5-ylthio oder
1,3,4-Thiadiazol-2-ylthio,
bedeutet, und $R_2^A$, Z' und $X^{\oplus}$ die oben angegebenen Bedeutungen haben.

Die Erfindung betrifft in erster Linie die in den Beispielen genannten erfindungsgemässen Zwischenprodukte.

Die Verbindungen der Formel II können zur Herstellung von pharmakologisch wirksamen $2-R_1-6-R_a-2$-penem-3-carbonsäuren, deren Salzen und Estern verwendet werden. Die genannten Penem-Verbindungen und das Verfahren zu ihrer Herstellung aus den Verbindungen der Formel II sind Gegenstand des Europäischen Patentes Nr. 3960.

In der Verbindung II und in den nachfolgend beschriebenen Verbindungen IV bis XV, IIIa und IVa kann eine Gruppe $R_a$, $R_1$ bzw $R_2^A$ nach an sich bekannten Methoden in eine andere Gruppe $R_a$, $R_1$ bzw. $R_2^A$ übergeführt werden.

So kann in einer verfahrensgemäss erhältlichen Verbindung, die eine freie Carboxylgruppe enthält, eine solche in an sich bekannter Weise in eine geschützte Carboxylgruppe übergeführt werden. So erhält man Ester z.B. durch Behandeln mit einer geeigneten Diazoverbindung, wie einem Diazoniederalkan, z.B. Diazomethan oder Diazobutan, oder einem Phenyldiazoniederalkan, z.B. Diphenyldiazomethan, wenn notwendig, in Gegenwart einer Lewissäure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodimid, sowie Carbonyldiimidazol, ferner mit einem N,N'-disubstituierten O- bzw. S-substituierten Isoharnstoff oder Isothioharnstoff, worin ein O- und S-Substituent z.B. Niederalkyl, insbesondere tert.-Butyl, Phenylniederalkyl oder Cycloalkyl, und N- bzw. N'-Substituenten z.B. Niederalkyl, insbesondere Isopropyl, Cycloalkyl oder Phenyl sind, oder nach irgendeinem anderen bekannten und geeigneten Veresterungsverfahren, wie Reaktion eines gegebenenfalls in situ hergestellten Salzes der Säure mit einem reaktionsfähigen Ester eines Alkohols und einer starken anorganischen Säure, sowie einer starken organischen Sulfonsäure. Ferner können Säurehalogenide, wie -chloride (hergestellt z.B. durch Behandeln mit Oxalylchlorid), aktivierte Ester (gebildet z.B. mit N-Hydroxystickstoffverbindungen, wie N-Hydroxysuccinimid) oder gemischte Anhydride (erhalten z.B. mit Halogenameisensäure-niederalkylestern, wie Chlorameisensäureäthyl- oder Chlorameisensäureisobutylester, oder mit Halogenessigsäurehalogeniden, wie Trichloressigsäurechlorid) durch Umsetzen mit Alkoholen, gegebenenfalls in Gegenwart einer Base, wie Pyridin, in eine veresterte Carboxylgruppe übergeführt werden.

In einer verfahrensgemäss erhältlichen Verbindung mit einer veresterten Carboxylgruppe kann diese in eine andere veresterte Carboxylgruppe übergeführt werden, z.B. 2-Chloräthoxycarbonyl oder 2-Bromäthoxycarbonyl durch Behandeln mit einem Jodsalz, wie Natriumjodid, in Gegenwart eines geeigneten Lösungsmittels, wie Aceton, in 2-Jodäthoxycarbonyl.

In einer verfahrensgemäss erhältlichen Verbindung mit einer freien Carboxylgruppe kann eine solche auch in eine gegebenenfalls substituierte Hydrazinocarbonylgruppe übergeführt werden, wobei man vorzugsweise reaktionsfähige funktionell abgewandelte Derivate, wie die obgenannten aktivierten Ester, oder gemischte Anhydride der entsprechenden Säure mit Hydrazinen umsetzt.

Eine durch eine organische Silyl- oder Stannylgruppe geschützte Carboxylgruppe kann in an sich bekannter Weise gebildet werden, z.B. indem man die Carboxyl enthaltende Verbindung oder ein Salz davon, wie Alkalimetall-, z.B. Natriumsalz davon, mit einem geeigneten Silylierungs- oder Stannylierungsmittel behandelt.

Im vorstehenden Verfahren, sowie in gegebenenfalls oder notwendigerweise durchzuführenden Zusatzmassnahmen, sind, wenn notwendig, an der Reaktion nicht teilnehmende, freie funktionelle Gruppen, wie freie Aminogruppen, z.B. durch Acylieren, Tritylieren oder Silylieren, freie Hydroxy- und Mercaptogruppen z.B. durch Veräthern oder Verestern, inkl. Silylieren, in an sich bekannter Weise vorzübergehend geschützt und

können, falls erwünscht, nach erfolgter Reaktion, in an sich bekannter Weise einzeln oder gemeinsam freigesetzt werden. So können in einem Ausgangsmaterial vorhandene Amino-, Hydroxy-, Mercapto-, Carboxyl- oder Sulfogruppen z. B. in Form von Acylamino-, wie den obgenannten, z. B. 2,2,2-Trichloräthoxycarbonylamino-, 2-Bromäthoxycarbonylamino-, 4-Methoxybenzyloxycarbonylamino-, oder tert.-Butyloxycarbonylamino-, von Aryl- oder Arylniederalkylthio-amino-, z. B. 2-Nitro-phenylthioamino-, oder Arylsulfonylamino-, z. B. 4-Methylphenylsulfonylamino-, von 1-Niederalkoxycarbony-2-propylidenaminogruppen, oder von o-Nytrophenyloxyacetylaminogruppen bzw. von Acyloxy-, wie den obgenannten, z. B. tert.-Butyloxycarbonyloxy-, 2,2,2-Trichloräthoxycarbonyloxy-, 2-Bromäthoxycarbonyloxy- oder p-Nitrobenzyloxycarbonyloxygruppen, oder entsprechenden Acylmercaptogruppen, bzw. von veresterten Carboxy-, wie den obgenannten, z. B. Diphenylmethoxycarbonyl-, p-Nitrobenzyloxycarbonyl-, Acetonyloxycarbonyl- oder 2-Cyanäthyloxycarbonylgruppen, bzw. von substituierten Sulfo-, wie den obgenannten Niederalkylsulfo-, z. B. Methylsulfogruppen, geschützt sein und nach erfolgter Reaktion, gegebenenfalls nach Umwandlung der Schutzgruppe, freigesetzt werden. Beispielsweise kann eine 2,2,2-Trichloräthoxycarbonylamino- oder 2-Jod-äthoxycarbonylaminogruppe oder auch eine p-Nitrobenzyloxycarbonylaminogruppe durch Behandeln mit geeigneten Reduktionsmitteln, wie Zink in Gegenwart von wässriger Essigsäure bzw. Wasserstoff in Gegenwart eines Palladiumkatalysators, eine Diphenylmethoxycarbonylamino- oder tert.-Butylcarbonylaminogruppe durch Behandeln mit Ameisen- oder Trifluoressigsäure, eine Aryl- oder Arylniederalkylthioaminogruppe durch Behandeln mit einem nucleophilen Reagens, wie schwefliger Säure, eine Arylsulfonylaminogruppe mittels elektrolytischer Reduktion, eine 1-Niederalkoxycarbonyl-2-propylidenaminogruppe durch Behandeln mit wässriger Mineralsäure bzw. eine tert.-Butyloxycarbonyloxygruppe durch Behandeln mit Ameisen- oder Trifluoressigsäure, oder eine 2,2,2-Trichloräthoxycarbonyloxygruppe oder p-Nitrobenzyloxycarbonyloxygruppe durch Behandeln mit einem chemischen Reduktionsmittel, wie Zink in Gegenwart von wässriger Essigsäure, oder mit Wasserstoff in Gegenwart eines Palladiumkatalysators, bzw. eine Diphenylmethoxycarbonylgruppe durch Behandeln mit Ameisen- oder Trifluoressigsäure oder durch Hydrogenolyse, eine Acetonyloxy- oder Cyanäthoxycarbonylgruppe durch Behandeln mit Basen, wie Natriumcarbonat oder 1,5-Diazabicyclo [5.4.0]undec-5-en, bzw. eine substituierte Sulfogruppe durch Behandeln mit einem Alkalimetallhalogenid, wenn erwünscht stufenweise, abgespalten werden.

Ferner kann man in einer erhaltenen Verbindung funktionelle Substituenten, wie freie Amino-, Hydroxy-, Mercapto-, Carboxy- oder Sulfogruppen nach an sich bekannten Verfahren, z. B. durch Alkylieren, Acylieren bzw. Verestern bzw. Substituieren, funktionell abwandeln.

So kann man eine Amino-, Hydroxy-, Mercapto-, Carboxy- oder Sulfogruppe durch Behandeln mit einem Alkylierungsreagens, wie einer Diazoverbindung, z. B. Diazomethan, oder einem reaktionsfähigen Ester eines Alkohols, z. B. Dimethylsulfat, alkylieren, z. B. methylieren, oder Amino-, Hydroxy- oder Mercaptogruppen durch Behandeln mit einem reaktionsfähigen funktionellen Derivat einer Säure, z. B. einem Anhydrid oder Säurechlorid, wie Acetanhydrid oder Acetylchlorid, acylieren, z. B. acetylieren. Ferner lässt sich z. B. eine Aminogruppe durch Behandeln mit Schwefeltrioxyd, vorzugsweise in der Form eines Komplexes mit einer organischen Base, wie einem Tri-niederalkylamin, z. B. Triäthylamin, in eine Sulfoaminogruppe umwandeln.

Eine Hydroxygruppe im Substituent $R_a$, insbesondere die Hydroxygruppe in einem 1-Hydroxyniederalkylrest, lässt sich durch Behandeln mit einem Schwefeltrioxyd Komplex, z. B. dem Komplex mit Dioxan oder mit einer tertiären Stickstoffbase, wie Triniederalkylamin, z. B. Triäthylamin, N,N-Diniederalkylanilin, z. B. N,N-Dimethylanilin oder insbesondere mit Pyridin oder auch durch Behandeln mit der Amidosulfonsäure, gegebenenfalls in Gegenwart von Pyridin, in eine in entsprechender Salzform vorliegende Hydroxysulfonyloxygruppe umwandeln, die durch doppelten Umsatz mit einem entsprechenden Metallhydroxyd, Metallcarbonat oder Metallhydrogencarbonat, wie einem Alkalimetall-, z. B. Natriumhydroxyd, -carbonat oder -hydrogencarbonat, in eine in Metallsalzform vorliegende Hydroxysulfonyloxygruppe umgewandelt werden kann.

In erfindungsgemäss erhältlichen Verbindungen können ferner, auf an sich bekannte Weise, primäre und sekundäre Hydroxygruppen durch Oxidation, z. B. nach Pfitzner-Moffatt, in Aldehyd- bzw. Ketogruppen übergeführt werden, oder, gegebenenfalls nach Acylierung, zusammen mit einem benachbarten aktivierten Wasserstoffatom unter Ausbildung einer C–C-Doppelbindung abgespalten werden. Aldehyd- oder Ketogruppen können durch Reduktion, z. B. mit komplexen Metallhydriden, in Hydroxygruppen oder durch Behandeln mit Alkoholen in Acetale bzw. Ketale, durch Behandeln mit einem Amin, Hydroxylamin oder Hydrazin in die entsprechenden Imine, Oxime oder Hydrazone, oder durch Behandeln mit einem Wittig-Reagens in die entsprechenden Methylidenverbindungen übergeführt werden. Erhaltene Acetale oder Ketale können in die entsprechenden Aldehyde bzw. Ketone überführt werden, z. B. durch Behandeln mit Trimethyljodsilan. In erfindungsgemäss erhaltenen Verbindungen können ferner, auf an sich bekannte Weise, C–C-Doppelbindungen reduziert werden, z. B. mit katalytisch angeregtem Wasserstoff. Halogen-, wie Brom- oder Jodsubstituenten können, z. B. durch Behandeln mit Zink/Silber in Methanol oder Methanol-Essigsäure, durch Wasserstoff ersetzt werden oder durch Behandeln mit einer metallorganischen Verbindung, wie Methylmagnesiumbromid oder Butyllithium, gefolgt von einem Aldehyd, z. B. Acetaldehyd, in 1-substituierte-1-Hydroxymethyl-

gruppen, z.B. die 1-Hydroxyäthylgruppe, übergeführt werden. Eine Nitro- oder Azidogruppe kann man z.B. durch Behandeln mit katalytisch, wie durch einen Palladium- bzw. Platinoxidkatalysator aktivierten Wasserstoff in eine Aminogruppe, umwandeln. Die genannten Nachfolgereaktionen können sowohl an entsprechenden Stellen in den Resten $R_a$ als auch in den Resten $R_1$, durchgeführt werden.

Das nachfolgend beschriebene Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbedingungen, gebildet werden. Beispielsweise kann ein Ausgangsmaterial der Formel II, worin Z Sauerstoff ist, aus einer Verbindung der Formel II, worin Z eine gegebenenfalls substituierte Methylidengruppe ist, durch Ozonisierung und anschliessende Reduktion des gebildeten Ozonids, analog dem in der Stufe 2.5 angegebenen Verfahren, in situ hergestellt werden, worauf, insbesondere wenn $R_1$ Wasserstoff ist, in der Reaktionslösung die Cyclisierung zur Verbindung der Formel I erfolgt.

Die vorliegende Erfindung betrifft ebenfalls die Herstellung der in den folgenden Reaktionsschemata 1, 2 und 3 beschriebenen Verfahren und die neuen Zwischenprodukte der Formeln II, IV, IVa, V, VI, VIII, IX, X, Xa, XI, XII, XIII, XIV, XV und IIIa.

Reaktionsschema 1

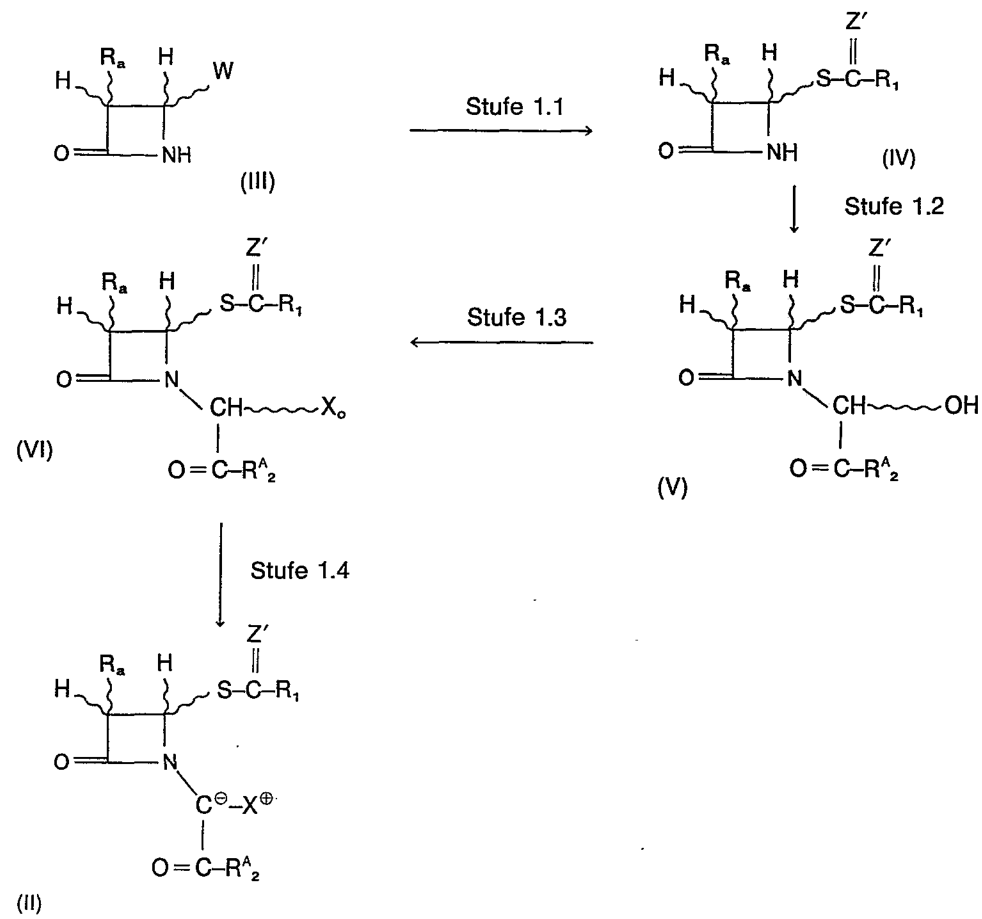

(III) (IV) (V) (VI) (II)

Stufe 1.1: Ein Thio-azetidinon der Formel IV wird erhalten, indem man ein 4-W-Azetidinon der Formel III, worin W eine nucleofuge Abgangsgruppe bedeutet, mit einer Mercaptoverbindung $R_1$–C(=Z′)–SH oder einem Salz, z.B. einem Alkalimetall-, wie Natrium- oder Kaliumsalz davon, behandelt, und gewünschtenfalls ein erhaltenes Isomerengemisch in die einzelnen Isomeren auftrennt, und/oder gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt.

Die nucleofuge Abgangsgruppe W in einem Ausgangsmaterial der Formel III ist ein durch den nucleophylen Rest $R_1$–C(=Z′)–S– ersetzbarer Rest. Solche Gruppen W sind beispielsweise Acyloxyreste, Sulfonylreste $R_0$–$SO_2$–, worin $R_0$ ein organischer Rest ist, Azido, oder Halogen. In einem Acyloxyrest W ist Acyl der Rest einer organischen Carbonsäure, inclusive einer optischen aktiven Carbonsäure, und hat beispielsweise die gleiche Bedeutung wie der Acylrest $R_1$–CO–, worin $R_1$

Wasserstoff oder einen der genannten, über ein Kohlenstoffatom gebundenen organischen Reste, z.B. einen der genannten, gegebenenfalls substituierten Niederalkyl-, Cycloalkyl-, Cycloalkylniederalkyl-, Phenyl- oder Phenylniederalkylreste, darstellt. In einem Sulfonylrest $R_0$–$SO_2$– ist $R_0$ beispielsweise ein gegebenenfalls substituierter aliphatischer, araliphatischer oder aromatischer Kohlenwasserstoffrest mit bis zu 12 C-Atomen, und ist insbesondere Niederalkyl, wie Methyl, Äthyl, oder ein durch einen optisch aktiven Rest substituiertes Methyl, wie Campheryl, oder Benzyl, Phenyl oder Toluyl. Ein Halogenrest W ist Brom, Jod oder insbesondere Chlor. W ist bevorzugt Acetoxy oder Chlor.

Die nucleophile Substitution kann unter neutralen oder schwach basischen Bedingungen in Gegenwart von Wasser und gegebenenfalls einem, mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Äthanol, Ketone, z.B. Niederalkanone, wie Aceton, Amide, z.B. Niederalkancarbonsäureamide, wie Dimethylformamid, und ähnliche verwendet werden. Die Reaktion wird üblicherweise bei Raumtemperatur durchgeführt, kann aber auch bei erhöhter oder erniedrigter Temperatur durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

In die Reaktion können sowohl optisch inaktive cis- oder trans-Verbindungen der Formel III, als auch Mischungen davon, oder entsprechende optisch aktive Verbindungen eingesetzt werden. Die eintretende Gruppe $R_1$–C(=Z')–S– wird von der Gruppe $R_a$ bevorzugt in die trans-Stellung dirigiert, unabhängig davon, ob W zur Gruppe $R_a$ in cis- oder trans-Stellung steht. Obwohl die trans-Isomeren überwiegend gebildet werden, können doch gelegentlich auch die cis-Isomeren isoliert werden. Die Auftrennung der cis- und trans-Isomeren erfolgt nach konventionellen Methoden, insbesondere durch Chromatographie und/oder durch Kristallisation.

Die nachträglich Ozonisierung einer Methyliden-Gruppe Z' kann wie weiter unten angegeben durchgeführt werden. Ein erhaltenes Racemat der Formel IV kann in die optisch aktiven Verbindungen getrennt werden.

Die Verbindungen der Formel IV sind neu.

Die von der Formel IV umfassten optisch aktiven Verbindungen der Formel IVa können auch nach dem unten angegebenen Reaktionsschema 2 hergestellt werden.

Azetidinone der Formel III, worin $R_a$ Methyl ist und W Acetyloxy, Phenylsulfonyl oder Campher-10-sulfonyl ist, sind bekannt (Deutsche Offenlegungsschrift 1 906 401 oder K. Clauss et al., Liebigs Ann. Chem., 1974, 539–560). Die übrigen Verbindungen der Formel III sind neu. Sie können nach den an sich bekannten Methoden hergestellt werden.

Die Azetidinone der Formel III werden beispielsweise durch Addition von Chlorsulfonylisocyanat an entsprechend substituierte Vinylester und nachträgliche Abspaltung der Chlorsulfonylgruppe hergestellt. Bei dieser Synthese werden gewöhnlich Mischungen von cis- und trans-Isomeren erhalten, die gewünschtenfalls in die reinen cis- oder trans-Isomeren, z.B. durch Chromatographie, und/oder Kristallisation oder Destillation aufgetrennt werden können. Die reinen cis- und trans-Isomeren liegen als Racemate vor und können in ihre optischen Antipoden getrennt werden, beispielsweise wenn Acyl in einem Acyloxyrest W in Verbindungen der Formel III von einer optisch aktiven Säure stammt. Die von der Formel III umfassten optisch aktiven Verbindungen der Formel IIIa können auch nach dem unten angegebenen Reaktionsschema 3 hergestellt werden.

Stufe 1.2: Eine α-Hydroxycarbonsäureverbindung der Formel V wird erhalten, indem man eine Verbindung der Formel IV mit einer Glyoxylsäure-Verbindung der Formel OHC–C(=O)–$R_2^A$ oder einem geeigneten Derivat, wie einem Hydrat, Hemihydrat oder Halbacetal, z.B. einem Halbacetal mit einem Niederalkanol, z.B. Methanol oder Äthanol, umsetzt, und gewünschtenfalls ein erhaltenes Isomerengemisch in die einzelnen Isomeren auftrennt, und/oder gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die Verbindung V erhält man üblicherweise als Gemisch der beiden Isomeren (bezüglich der Gruppierung >CH∼∼OH). Man kann aber auch die reinen Isomeren davon isolieren.

Die Anlagerung der Glyoxylsäureesterverbindung an das Stickstoffatom des Lactamrings findet bei Raumtemperatur oder, falls notwendig, unter Erwärmen, z.B. bis etwa 100 °C, und zwar in Abwesenheit eines eigentlichen Kondensationsmittels und/oder ohne Bildung eines Salzes statt. Bei Verwendung des Hydrats der Glyoxylsäureverbindung wird Wasser gebildet, das, wenn notwendig, durch Destillation, z.B. azeotrop, oder durch Verwendung eines geeigneten Dehydratationsmittels, wie eines Molekularsiebs, entfernt wird. Vorzugsweise arbeitet man in Gegenwart eines geeigneten Lösungsmittels, wie z.B. Dioxan, Toluol oder Dimethylformamid, oder Lösungsmittelgemisches, wenn erwünscht oder notwendig, in der Atmosphäre eines Inertgases, wie Stickstoff.

In die Reaktion können sowohl reine optisch inaktive cis- oder trans-Verbindungen der Formel IV, als auch Mischungen davon, oder entsprechende optisch aktive Verbindungen eingesetzt

werden. Ein erhaltenes Racemat der Formel V kann in die optisch aktiven Verbindungen getrennt werden.

Stufe 1.3: Verbindungen der Formel VI, worin $X_0$ für eine reaktionsfähige veresterte Hydroxygruppe, insbesondere für Halogen oder organisches Sulfonyloxy steht, werden hergestellt, indem man in einer Verbindung der Formel V die sekundäre Hydroxygruppe in eine reaktionsfähige veresterte Hydroxygruppe, insbesondere in Halogen, z.B. Chlor oder Brom, oder in eine organische Sulfonyloxygruppe, wie Niederalkylsulfonyloxy, z.B. Methylsulfonyloxy, oder Arylsulfonyloxy, z.B. 4-Methylphenylsulfonyloxy, umwandelt, gewünschtenfalls ein erhaltenes Isomerengemisch in die einzelnen Isomeren auftrennt, und gewünschtenfalls in einer erhaltenen Verbindung eine Gruppe $R_a$ und/oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die Verbindung VI kann man in Form von Gemischen der Isomeren (bezüglich der Gruppierung $>CH\sim\sim X_0$) oder in Form von reinen Isomeren erhalten.

Die obige Reaktion wird durch Behandeln mit einem geeigneten Veresterungsmittel durchgeführt, indem man z.B. ein Halogenierungsmittel, wie ein Thionylhalogenid, z.B. -chlorid, ein Phosphoroxyhalogenid, besonders -chlorid, oder ein Halogenphosphoniumhalogenid, wie Triphenylphosphindibromid oder -dijodid, sowie ein geeignetes organisches Sulfonsäurehalogenid, wie -chlorid, vorzugsweise in Gegenwart eines basischen, in erster Linie eines organischen basischen Mittels, wie eines aliphatischen tertiären Amins, z.B. Triäthylamin, Diisopropyläthylamin oder «Polystyrol-Hünigbase», oder einer heterocyclischen Base vom Pyridintyp, z.B. Pyridin oder Collidin, verwendet. Vorzugsweise arbeitet man in Gegenwart eines geeigneten Lösungsmittels, z.B. Dioxan oder Tetrahydrofuran, oder eines Lösungsmittelgemisches, wenn notwendig, unter Kühlen und/oder in der Atmosphäre eines Intergases, wie Stickstoff.

In einer so erhältlichen Verbindung der Formel VI kann eine reaktionsfähige veresterte Hydroxygruppe $X_0$ in eine andere reaktionsfähige veresterte Hydroxygruppe in an sich bekannter Weise umgewandelt werden. So kann man z.B. ein Chloratom durch Behandeln der entsprechenden Chlorverbindung mit einem geeigneten Brom- oder Jodreagens, insbesondere mit einem anorganischen Bromid- oder Jodidsalz, wie Lithiumbromid, vorzugsweise in Gegenwart eines geeigneten Lösungsmittels, wie Äther, durch ein Brom- bzw. Jodatom austauschen.

In die Reaktion können sowohl reine optisch inaktive cis- oder trans-Verbindungen der Formel V als auch Mischungen davon, oder entsprechende optisch aktive Verbindung eingesetzt werden. Ein erhaltenes Racemat der Formel VI kann in die optisch aktiven Verbindungen getrennt werden.

Stufe 1.4: Ein Ausgangsmaterial der Formel II wird erhalten, indem man eine Verbindung der Formel VI, worin $X_0$ für eine reaktionsfähige veresterte Hydroxygruppe steht, mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldimethylphosphit behandelt, wobei man je nach Wahl des Reagenzes eine Verbindung der Formel IIA bzw. IIB erhalten kann, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die obige Reaktion wird vorzugsweise in Gegenwart eines geeigneten inerten Lösungsmittels, wie eines Kohlenwasserstoffs, z.B. Hexan, Cyclohexan, Benzol, Toluol oder Xylol, oder eines Äthers, z.B. Dioxan, Tetrahydrofuran oder Diäthylenglykol-dimethyläther, oder eines Lösungsmittelgemisches vorgenommen. Je nach Reaktionsfähigkeit arbeitet man unter Kühlen oder bei erhöhter Temperatur, etwa zwischen $-10°$ und $+100°$, bevorzugt bei etwa 20° bis 80°, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man bei der Verwendung einer Phosphinverbindung üblicherweise in Gegenwart eines basischen Mittels, wie einer organischen Base, z.B. eines Amins, wie Triäthylamin, Diisopropyl-äthyl-amin oder «Polystyrol-Hünigbase», und gelangt so direkt zum Phosphoranyliden-Ausgangsmaterial der Formel IIA, das aus dem entsprechenden Phosphoniumsalz gebildet wird.

In die Reaktion können sowohl reine, optisch inaktive cis- oder trans-Verbindungen der Formel VI, als auch Mischungen davon, oder entsprechende optisch aktive Verbindung eingesetzt werden. Ein erhaltenes Racemat der Formel II kann in die optisch aktiven Verbindungen getrennt werden.

In den Verbindungen der Formeln II bis VI bedeutet $R_a$ bevorzugt einen der genannten, über ein Kohlenstoffatom mit dem Ringkohlenstoffatom verbundenen organischen Reste, oder auch eine verätherte Hydroxygruppe, wobei gegebenenfalls vorhandene funktionelle Gruppen in einem solchen Rest $R_a$ bevorzugt in geschützter Form vorliegen.

Die Auftrennung der vorstehend genannten cis, trans-Verbindungen in die reinen cis- und trans-Isomere erfolgt nach den üblichen Trennungsmethoden, z.B. chromatographisch und/oder durch Destillation oder Kristallisation.

Die Spaltung der vorstehend genannten Racemate in ihre optischen Antipoden erfolgt nach an sich bekannten Methoden.

Eine dieser Methoden besteht darin, dass man ein Racemat mit einem optisch aktiven Hilfsstoff reagieren lässt, das dabei entstandene Gemisch

zweier diastereomerer Verbindungen mit Hilfe von geeigneten physikalisch-chemischen Methoden trennt und die einzelnen diastereomeren Verbindungen dann in die optisch aktiven Verbindungen spaltet.

Zur Trennung in Antipoden besonders geeignete Racemate sind solche, die eine saure Gruppe besitzen, wie z.B. Racemate von Verbindungen der Formel I. Andere der beschriebenen Racemate kann man durch einfache Reaktionen in saure Racemate umwandeln. Beispielsweise reagieren Aldehyde- oder Ketogruppen tragende Racemate mit einem saure Gruppen tragenden Hydrazindederivat, z.B. 4-(4-Carboxyphenyl)-semicarbazid zu den entsprechenden Hydrazonderivaten oder Alkoholgruppen enthaltende Verbindungen mit einem Dicarbonsäureanhydrid, z.B. Phthalsäureanhydrid, zum Racemat eines sauren Halbesters.

Diese sauren Racemate können mit optisch aktiven Basen, z.B. Estern von optisch aktiven Aminosäuren, oder (−)-Brucin, (+)-Chinidin, (−)-Chinin, (+)-Chinchonin, (+)-Dehydroabietylamin, (+)- und (−)-Ephedrin, (+)- und (−)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen, z.B. auch in Racematen, die eine funktionell abgewandelte Carboxymethylidengruppe Z′ enthalten, kann diese Carboxylgruppe durch einen optisch aktiven Alkohol, wie (−)-Menthol, (+)-Borneol, (+)- oder (−)-2-Octanol verestert sein oder werden, worauf nach erfolgter Isolierung des gewünschten Diastereomeren die Carboxylgruppe freigesetzt wird, oder der die veresterte Carboxylgruppe enthaltende Molekülteil, z.B. der veresterte Carboxymethylidenrest, abgespalten wird.

Hydroxygruppen enthaltende Racemate können ebenfalls in ihre optischen Antipoden gespalten werden, wobei insbesondere optisch aktive Säuren oder deren reaktionsfähige, funktionelle Derivate Verwendung finden, die mit den genannten Alkoholen diastereomere Ester bilden. Solche Säuren sind beispielsweise (−)-Abietinsäure, D(+)- und L(−)-Äpfelsäure, N-acylierte optisch aktive Aminosäuren, (+) und (−)-Camphansäure, (+) und (−)-Ketopinsäure, L(+)-Ascorbinsäure, (+)-Camphersäure, (+)-Campher-10-sulfonsäure (β), (+) oder (−)-α-Bromcampher-π-sulfonsäure, D(−)-Chinasäure, D(−)-Isoascorbinsäure, D(−)- und L(+)-Mandelsäure, (+)-1-Menthoxyessigsäure, D(−)- und L(+)-Weinsäure und deren Di-O-benzoyl- und Di-O-p-toluylderivate. Die Acylreste der genannten optisch aktiven Säuren können beispielsweise als Acyl in Verbindungen der Formel III oder als $R_1$–C(=O)– in Verbindungen der Formeln II und IV bis VI vorliegen und die Racematspaltung solcher Verbindungen ermöglichen. Wenn erwünscht oder notwendig kann nach erfolgter Racematspaltung die optisch aktive Gruppe $R_1$–C(=O)– in eine gewünschte optisch inaktive Gruppe $R_1$–C(=O)– übergeführt werden.

Hydroxygruppen enthaltende Racemate können in ein Gemisch diastereomerer Urethane umgewandelt werden, beispielsweise durch Umsetzung mit optisch aktiven Isocyanaten, wie mit (+)- oder (−)-1-Phenyläthylisocyanat.

Basische Racemate können mit den optisch aktiven Säuren diastereomere Salze bilden. Doppelbindungen enthaltende Racemate können beispielsweise mit Platinchlorid und (+)-1-Phenyl-2-aminopropan in Gemische diastereomerer Komplexsalze übergeführt werden.

Zur Trennung der Diastereomerengemische eignen sich physikalisch-chemische Methoden, in erster Linie die fraktionierte Kristallisation. Brauchbar sind aber auch chromatographische Methoden, vor allem Fest-flüssig-Chromatographie. Leichtflüchtige Diastereomerengemische können auch durch Destillation oder Gaschromatographie getrennt werden.

Die Spaltung der aufgetrennten Diastereomeren in die optisch aktiven Ausgangsmaterialien erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromathographie an optisch aktiven Absorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produkten aus.

Erfindungsgemäss verwendbare optisch aktive trans-Verbindungen der Formel IVa können auch nach dem folgenden Reaktionsschema hergestellt werden:

Reaktionsschema 2

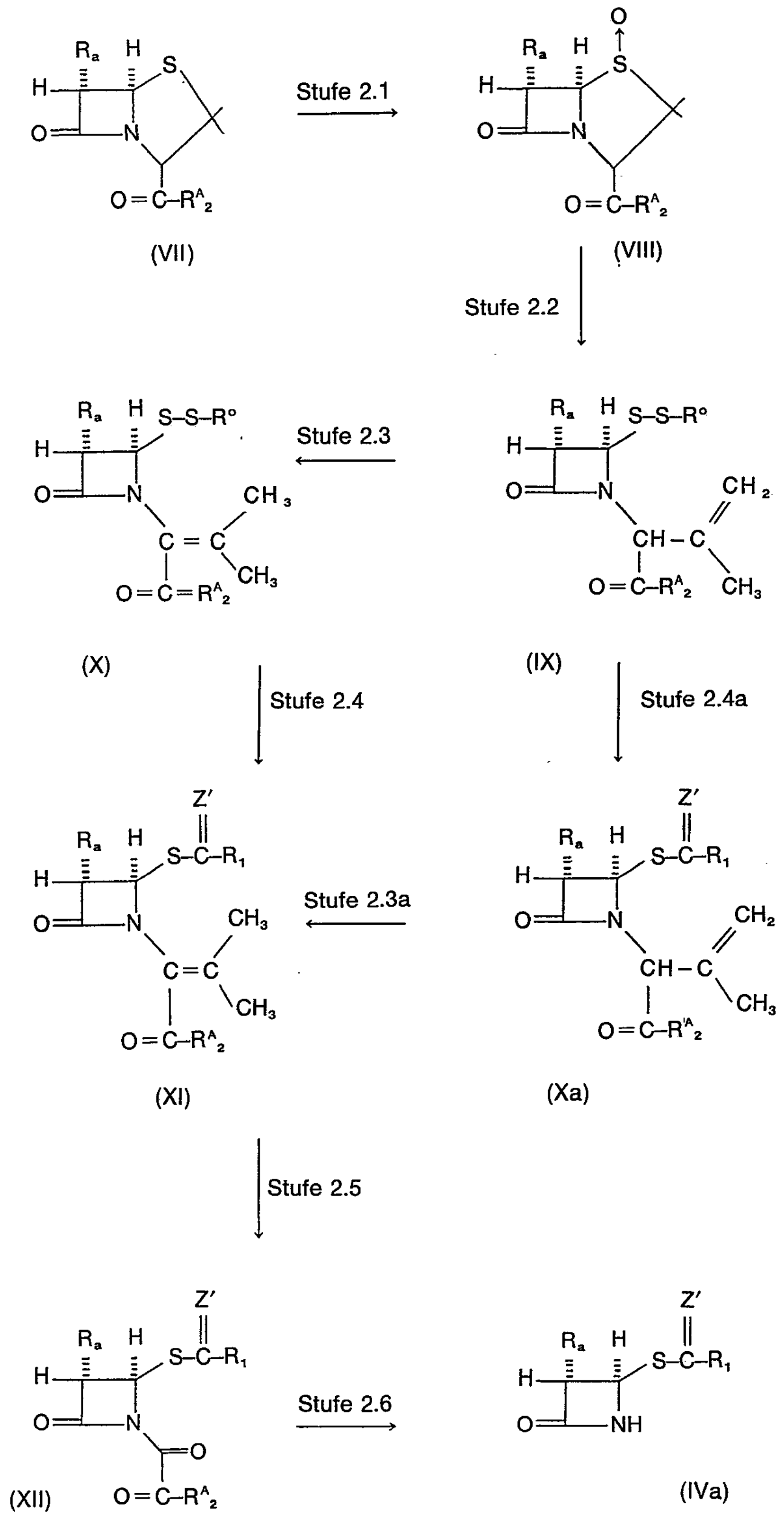


(VII) (VIII) (X) (IX) (XI) (Xa) (XII) (IVa)

Stufe 2.1: Ein Oxid einer Penicillansäureverbindung der Formel VIII wird erhalten, indem man eine Penicillansäureverbindung der Formel VII in 1-Stellung oxidiert, und gewünschtenfalls in einer erhaltenen Verbindung eine Gruppe $R_a$ in eine andere Gruppe $R_a$ überführt.

Die Oxidation erfolgt auf an sich bekannte Weise mit geeigneten Oxidationsmitteln, wie Wasserstoffperoxid oder anorganischen oder organischen Persäuren. Geeignete anorganische Persäuren sind z.B. Perjod- oder Perschwefelsäure. Geeignete organische Persäuren sind z.B. Percarbonsäuren, wie Perameisensäure, Peressigsäure, Trifluorperessigsäure, Permaleinsäure, Perbenzoesäure, 3-Chlor-perbenzoesäure oder Monoperphthalsäure, oder Persulfonsäuren, z.B. p-Toluolpersulfonsäure. Die Persäuren können auch in situ aus Wasserstoffperoxid und den entsprechenden Säuren hergestellt werden. Die Oxidation erfolgt unter milden Bedingungen, z.B. bei Temperaturen von etwa $-50°$ bis etwa $+100°$, vorzugsweise bei etwa $-10°$ bis etwa $+40°$, in einem inerten Lösungsmittel.

Racemische 1-Oxide der Formel VIII, worin $R_a$ Phenoxy oder Methoxy und $R_2^A$ Methoxy bedeuten, sind bekannt [A.K. Bose et al., Tetrahedron 28, 5977 (1972)]. Die optisch aktiven Verbindungen der Formel VIII sind neu und ebenfalls Gegenstand der vorliegenden Erfindung.

Ausgangsverbindungen der Formel VII sind bekannt oder können analog bekannten Verfahren hergestellt werden. Beispielsweise können sie nach D. Hauser und H.P. Sigg, Helv. Chimica Acta 50, 1327 (1967), erhalten werden, indem man einen 6-Diazopenicillansäureester, der gegebenenfalls in situ aus einem 6-Aminopenicillansäureester und salpetriger Säure hergestellt wird, mit Wasser oder einem Alkohol oder einer Säure der Formel $H-R_a$ umsetzt. Verbindungen der Formel VII, worin $R_a$ eine Acyloxygruppe ist, können ebenfalls nach D. Hauser erhalten werden, indem man einen entsprechenden 6α- oder 6β-N-Nitrosoacylaminopenicillansäureester in einem inerten Lösungsmittel pyrolysiert. Verbindungen der Formel VII, worin $R_a$ Hydroxy ist, sind auch durch J.C. Sheehan et al., J. Org. Chem. 39, 1444 (1974) beschrieben worden (Herstellung aus entsprechenden 6-Diazopenicillansäureverbindungen). Weitere Ausgangsstoffe der Formel VII, worin $R_a$ gegebenenfalls geschütztes 1-Hydroxyäthyl, Brom oder Jod ist, sind durch DiNinno et al. bekannt geworden (J. Org. Chem. 42 (1967), 2960). In einer erhaltenen Verbindung der Formel VIII kann eine Gruppe $R_a$ in eine andere Gruppe $R_a$ übergeführt werden.

Stufe 2.2: Eine 3-Methylenbuttersäureverbindung der Formel IX wird erhalten, indem man ein 1-Oxid einer Penicillansäureverbindung der Formel VIII mit einer Mercaptoverbindung $R^0$–SH behandelt, und gewünschtenfalls in einer erhaltenen Verbindung eine Gruppe $R_a$ in eine andere Gruppe $R_a$ umwandelt.

In der Mercaptoverbindung $R^0$–SH und in dem Reaktionsprodukt der Formel IX ist $R^0$ ein gegebenenfalls substituierter aromatischer heterocyclischer Rest mit bis zu 15, bevorzugt bis zu 9 Kohlenstoffatomen, und mindestens einem Ringstickstoffatom und gegebenenfalls einem weiteren Ringheteroatom, wie Sauerstoff oder Schwefel, welcher Rest mit einem seiner Ringkohlenstoffatome, das mit einem Ringstickstoffatom durch eine Doppelbindung verbunden ist, an die Thiogruppe –S– gebunden ist. Solche Reste sind monocyclisch oder bicyclisch und können beispielsweise durch Niederalkyl, wie Methyl oder Äthyl, Niederalkoxy, wie Methoxy oder Äthoxy, Halogen, wie Fluor oder Chlor, oder Aryl, wie Phenyl, substituiert sein.

Solche Reste $R^0$ sind z.B. monocyclische fünfgliedrige thiadiazacyclische, thiatriazacyclische, oxadiazacyclische oder oxatriazacyclische Reste aromatischen Charakters, insbesondere aber monocyclische fünfgliedrige diazacyclische, oxazacyclische und thiazacyclische Reste aromatischen Charakters, und/oder in erster Linie die entsprechenden benzdiazacyclischen, benzoxazacyclischen oder benzthiazacyclischen Reste, worin der heterocyclische Teil fünfgliedrig ist und aromatischen Charakter aufweist, wobei in Resten $R^0$ ein substituierbares Ringstickstoffatom z.B. durch Niederalkyl substituiert sein kann. Repräsentativ für solche Gruppen $R^0$ sind
1-Methyl-imidazol-2-yl, 1,3-Thiazol-2-yl,
1,3,4-Thiadiazol-2-yl, 1,3,4,5-Thiatriazol-2-yl,
1,3-Oxazol-2-yl, 1,3,4-Oxadiazol-2-yl,
1,3,4,5-Oxatriazol-2-yl, 2-Chinolyl,
1-Methyl-benzimidazol-2-yl, Benzoxazol-2-yl
und insbesondere Benzthiazol-2-yl.

Die Reaktion wird in einem inerten Lösungsmittel, wie einem aliphatischen oder aromatischen Kohlenwasserstoff z.B. Benzol oder Toluol, unter Erwärmen bis zur Rückflusstemperatur des verwendeten Lösungsmittels ausgeführt.

Stufe 2.3: Eine 3-Methylcrotonsäureverbindung der Formel X wird erhalten, indem man eine 3-Methylenbuttersäureverbindung der Formel IX durch Behandeln mit einem geeigneten basischen Mittel isomerisiert, und gewünschtenfalls in einer erhaltenen Verbindung eine Gruppe $R_a$ in eine andere Gruppe $R_a$ umwandelt.

Geeignete basische Mittel sind beispielsweise organische Stickstoffbasen, wie tertiäre Amine, z.B. Triniederalkylamine, wie Triäthylamin oder Hünigbase, oder auch anorganische Basen, die in einem inerten Lösungsmittel, wie einem gegebenenfalls halogenierten Kohlenwasserstoff, z.B. Methylenchlorid, bei Raumtemperatur oder gegebenenfalls leicht erniedrigter oder erhöhter Temperatur, zur Anwendung gelangen.

Stufe 2.4: Eine Thioverbindung der Formel XI wird erhalten, indem man eine Verbindung der Formel X mit einem geeigneten Reduktionsmittel behandelt und gleichzeitig oder nachträglich mit einem Acylierungsderivat einer Säure der Formel $R_1$–C(=Z)–OH, oder, wenn Z' eine gegebenenfalls durch Y substituierte Methylidengruppe bedeutet, mit einem Alkin der Formel $R_1$–C≡C–Y umsetzt, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z' in eine Oxogruppe Z überführt.

Geeignete Reduktionsmittel sind beispielsweise Hydridreduktionsmittel, wie Alkalimetallborhydride, z.B. Natriumborhydrid, oder auch Zink in Gegenwart einer Carbonsäure, z.B. einer Carbonsäure der Formel ($R_1$–C(=O)–OH. Die Hydridreduktionsmittel werden üblicherweise in Gegenwart von geeigneten Lösungsmitteln, wie Dimethylformamid eingesetzt. Die Hydridreduktion wird bevorzugt in Dimethylformamid mit Natriumborhydrid bei Temperaturen von etwa −50° bis etwa −10°, bevorzugt bei etwa −20°, durchgeführt, worauf bei der gleichen Temperatur das Acylierungsmittel und gegebenenfalls eine tertiäre Base, wie Pyridin, zugefügt wird. Die Reduktion mit Zink und einer Carbonsäure wird gegebenenfalls in einem Lösungsmittel, wozu die Carbonsäure, falls sie flüssig ist, selbst dienen kann, bei Temperaturen von etwa −10 bis etwa +50° bevorzugt bei etwa 0° bis Raumtemperatur durchgeführt. Das Acylierungsmittel kann der Reduktionsmischung von Anfang an zugegeben werden oder nach Beendigung der Reduktion und gegebenenfalls nach Abdampfen der verwendeten Carbonsäure und/oder des Lösungsmittels. Geeignete Acylierungsmittel sind insbesondere die Anhydride der genannten Carbonsäuren, wie symmetrische Anhydride, z.B. Acetanhydrid, oder gemischte Anhydride, vorzugsweise solche mit Halogenwasserstoffsäuren, d.h. die entsprechenden Carbonsäurehalogenide, z.B. die Chloride und Bromide, wie Acetylbromid. Beispielsweise kann eine Verbindung der Formel X mit Zink in einer Mischung von Essigsäure und Acetanhydrid bei 0° bis etwa 20° in eine Verbindung der Formel XI übergeführt werden, worin $R_1$ Methyl ist. Wegen der geringeren Racemisierungsgefahr wird die Zink/Carbonsäure-Reduktion bevorzugt. Das Alkin kann ebenfalls von Anfang an oder auch erst nach Beendigung der Reduktion zum Reduktionsgemisch zugegeben werden. Die Addition des bei der Reduktion intermediär entstehenden 4-Mercaptoazetidin-2-ons an die Dreifachbindung des Alkins erfolgt spontan bei der Reduktionstemperatur.

Stufe 2.3a: Eine Thioverbindung der Formel XI wird auch erhalten, indem man eine Verbindung der Formel Xa gemäss den Reaktionsbedingungen der Stufe 2.3 durch Behandeln mit einem geeigneten basischen Mittel isomerisiert, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z' in eine Oxogruppe Z überführt.

Stufe 2.4a: Eine Verbindung der Formel Xa wird erhalten, indem man eine 3-Methylenbuttersäureverbindung der Formel IX gemäss den Reaktionsbedingungen der Stufe 2.4 mit einem geeigneten Reduktionsmittel behandelt und gleichzeitig oder nachträglich mit einem Acylierungsderivat einer Carbonsäure der Formel $R_1$–C(=Z)–OH, oder, wenn Z' eine gegebenenfalls durch Y substituierte Methylidengruppe bedeutet, mit einem Alkin der Formel $R_1$–C≡C–Y, umsetzt, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z' in eine Oxogruppe Z überführt.

Stufe 2.5: Eine 2-Oxoessigsäureverbindung der Formel XII wird erhalten, indem man eine Verbindung der Formel XI ozonisiert und das gebildete Ozonid reduktiv zur Oxo-Verbindung spaltet, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die Ozonisierung wird üblicherweise mit einem Ozon-Sauerstoff-Gemisch in einem inerten Lösungsmittel, wie einem Niederalkanol, z.B. Methanol oder Äthanol, einem Niederalkanon, z.B. Aceton, einem gegebenenfalls halogenierten aliphatischen, cycloaliphatischen oder aromatischen Kohlenwasserstoff, z.B. einem Halogenniederalkan, wie Methylenchlorid oder Tetrachlorkohlenstoff, oder in einem Lösungsmittelgemisch, inkl. einem wässrigen Gemisch, vorzugsweise unter Kühlen, z.B. bei Temperaturen von etwa −90° bis etwa 0° durchgeführt.

Ein als Zwischenprodukt erhaltenes Ozonid wird, üblicherweise ohne isoliert zu werden, reduktiv zu einer Verbindung der Formel XII gespalten, wobei man katalytisch aktivierten Wasserstoff, z.B. Wasserstoff in Gegenwart eines Schwermetallhydrierkatalysators, wie eines Nikkel-, ferner Palladiumkatalysators, vorzugsweise auf einem geeigneten Trägermaterial, wie Calciumcarbonat oder Kohle, oder chemische Reduktionsmittel, wie reduzierende Schwermetalle, inkl. Schwermetallegierungen oder -amalgame, z.B. Zink, in Gegenwart eines Wasserstoffdonators, wie einer Säure, z.B. Essigsäure, oder eines Alkohols, z.B. Niederalkanols, reduzierende anorganische Salze, wie Alkalimetalljodide, z.B. Natriumjodid, oder Alkalimetallhydrogensulfite, z.B. Natriumhydrogensulfit, in Gegenwart eines Wasserstoffdonators, wie einer Säure, z.B. Essigsäure, oder Wasser, oder reduzierende organische Verbindungen, wie Ameisensäure, verwendet. Als Reduktionsmittel kommen auch Verbindungen zum Einsatz, die leicht in entsprechende Epoxiverbindungen oder Oxide umgewandelt werden können, wobei die Epoxidbildung aufgrund einer C,C-Doppelbindung und die Oxidbildung aufgrund eines vorhandenen oxidbildenden Hetero-, wie Schwefel-, Phosphor- oder Stickstoffatoms erfolgen kann. Solche Verbindungen sind z.B. geeignet substituierte Äthenverbindungen (die in der Reaktion in Äthylenoxidverbindungen umgewandelt werden), wie Tetracyanäthylen, dann insbesondere geeignete Sulfidverbindungen (die in der Reaktion in Sulfoxidverbindungen umgewandelt werden), wie Diniederalkylsulfide, in erster Linie Dimethylsulfid, geeignete organische Phosphorverbindungen, wie ein Phosphin, das gegebenenfalls substituierte aliphatische oder aromatische Kohlenwasserstoffreste als Substituenten enthält (und das in der Reaktion in ein Phosphinoxid umgewandelt wird), wie Triniederalkyl-phosphine, z.B. Tri-n-butylphosphin, oder Triarylphosphine, z.B. Triphenylphosphin, ferner Phosphite, welche gegebenenfalls substituierte aliphatische Kohlenwasserstoffreste als Substituenten enthalten (und in der Reaktion in Phosphorsäuretriester übergeführt werden), wie Triniederalkyl-phosphite, üblicherweise in der Form von entsprechenden Alkoholadduktverbindungen, wie Trimethylphosphit, oder Phosphorigsäure-triamide, welche gegebenenfalls substituierte aliphatische Kohlenwasserstoffreste als Substituenten enthalten, wie Hexaniederalkyl-phosphorigsäuretriamide, z. B. Hexamethylphosphorigsäuretriamid, letzteres vorzugsweise in der Form eines Methanoladdukts, ferner geeignete Stickstoffbasen (die in der Reaktion in die entsprechenden N-Oxide umgewandelt werden), wie heterocyclische Stickstoffbasen aromatischen Charakters, z. B. Basen vom Pyridintyp und insbesondere Pyridin selber. Die Spaltung des üblicherweise nicht isolierten Ozonids erfolgt normalerweise unter den Bedingungen, die man zu seiner Herstellung anwendet, d. h. in Gegenwart eines geeigneten Lösungsmittels oder Lösungsmittelgemisches, sowie unter Kühlen oder leichtem Erwärmen, wobei man vorzugsweise bei Temperaturen von etwa −10 °C bis etwa +25 °C arbeitet und die Reaktion üblicherweise bei Raumtemperatur abschliesst.

Stufe 2.6: Eine Verbindung der Formel IVa wird erhalten, indem man eine Verbindung der Formel XII solvolysiert, und wenn erwünscht in einer erhaltenen Verbindung eine Gruppe $R_a$ oder $R_1$ in eine andere Gruppe $R_a$ oder $R_1$ überführt, und/oder gewünschtenfalls eine gegebenenfalls substituierte Methylidengruppe Z′ in eine Oxogruppe Z überführt.

Die Solvolyse kann als Hydrolyse, als Alkoholyse oder auch als Hydrazinolyse durchgeführt werden. Die Hydrolyse wird mit Wasser, gegebenenfalls in einem mit Wasser mischbaren Lösungsmittel, durchgeführt. Die Alkoholyse wird üblicherweise mit einem Niederalkanol, z. B. Methanol oder Äthanol, vorzugsweise in Gegenwart von Wasser und eines organischen Lösungsmittels, wie eines Niederalkancarbonsäure-niederalkylesters, z. B. Essigsäure-äthylester, vorzugsweise bei Raumtemperatur, wenn notwendig unter Kühlen oder Erwärmen durchgeführt. Die Hydrazinolyse wird auf konventionelle Weise mit einem substituierten Hydrazin, z. B. mit Phenyl- oder einem Nitrophenylhydrazin, wie 2-Nitrophenylhydrazin, 4-Nitrophenylhydrazin, oder 2,4-Dinitrophenylhydrazin, das bevorzugt in etwa äquimolarer Menge eingesetzt wird, in einem organischen Lösungsmittel, wie einem Äther, z. B. Tetrahydrofuran, Dioxan, Diäthyläther, einem aromatischen Kohlenwasserstoff, wie Benzol oder Toluol, einem halogenierten Kohlenwasserstoff, wie Methylenchlorid, Chlorbenzol oder Dichlorbenzol, einem Ester, wie Äthylacetat, und dergleichen, bei Temperaturen zwischen etwa Raumtemperatur und etwa 65 °C durchgeführt. Die α-Ketoverbindung der Formel XII braucht nicht notwendigerweise isoliert zu werden. Führt man z. B. die Spaltung des Ozonids in Gegenwart eines Solvolysemittels, wie z. B. Wasser, durch, so kann direkt eine Verbindung der Formel IVa erhalten werden.

Optisch aktive cis-, trans- und cis,trans-Verbindungen der Formel IIIa können auch nach dem folgenden Reaktionsschema erhalten werden:

Reaktionsschema 3

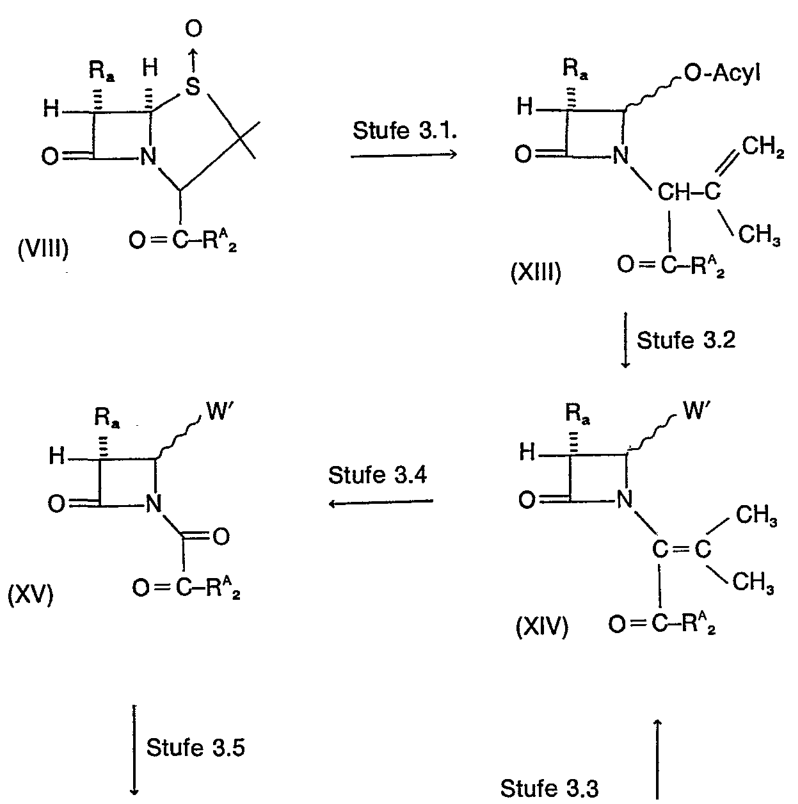

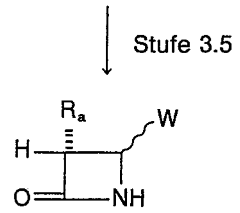


(IIIa)

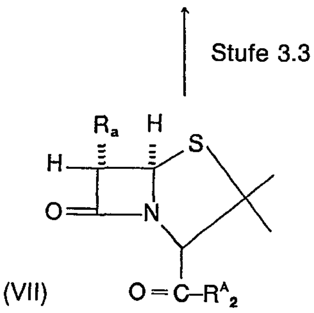


(VII)

W' = Acyloxy oder Halogen

Stufe 3.1: Eine 3-Methylenbuttersäureverbindung der Formel XIII wird erhalten, indem man ein 1-Oxid einer Penicillansäureverbindung der Formel VIII in Gegenwart eines Triniederalkylphosphits mit einer organischen Carbonsäure Acyl-OH behandelt, gewünschtenfalls in einer erhaltenen Verbindung eine Gruppe $R_a$ in eine andere Gruppe $R_a$ umwandelt, und/oder aus einer erhaltenen cis,trans-Verbindung die cis- und/oder die trans-Verbindung isoliert.

Als Triniederalkylphosphit ist beispielsweise Trimethylphosphit geeignet. Als organische Carbonsäure Acyl-OH kommt beispielsweise eine Carbonsäure $R_1$–COOH in Frage, worin $R_1$ Wasserstoff oder einen der genannten, über ein Kohlenstoffatom gebundenen organischen Reste, z.B. einen der genannten Niederalkyl-, Cycloalkyl-, Cycloalkylniederalkyl-, Phenyl- oder Phenylniederalkylreste, darstellt. Bevorzugt sind Niederalkancarbonsäuren, inklusive Ameisensäure, insbesondere Essigsäure.

Die Reaktion findet, analog A. Suarato et. al., Tetrahedron Letters, 42, 4059–4062, 1978, in einem inerten organischen Lösungsmittel, z.B. einem Kohlenwasserstoff, wie Benzol, Toluol oder Xylol, oder einem ätherartigen Lösungsmittel, wie Dioxan oder Tetrahydrofuran, oder einem Lösungsmittelgemisch, bei erhöhter Temperatur, etwa bis zur Rückflusstemperatur des verwendeten Lösungsmittels, etwa bei 50 bis 150 °C, bevorzugt bei etwa 80° bis etwa 100 °C, statt.

Bei der Reaktion entsteht ein Gemisch der cis- und trans-Verbindungen. Durch übliche Trennmethoden, wie Kristallisieren oder Chromatographieren, kann die cis- und/oder die trans-Verbindung in reiner Form erhalten werden.

Stufe 3.2: Eine 3-Methylcrotonsäureverbindung der Formel XIV, worin W' Acyloxy bedeutet, wird erhalten, indem man eine 3-Methylenbuttersäureverbindung der Formel XIII durch Behandeln mit einem geeigneten basischen Mittel isomerisiert, und gewünschtenfalls in einer erhaltenen Verbindung eine Gruppe $R_a$ in eine andere Gruppe $R_a$ umwandelt, und/oder gewünschtenfalls aus einer erhaltenen cis, trans-Verbindung die cis- und/oder die trans-Verbindung isoliert.

Die basische Isomerisierung wird wie in der Stufe 2.3 beschrieben durchgeführt. Die gewünschtenfalls durchzuführende nachträgliche Auftrennung in reine Verbindungen wird wie unter Stufe 3.1 beschrieben durchgeführt.

Stufe 3.3: Eine Verbindung der Formel XIV, worin W' Halogen bedeutet, wird erhalten, indem man eine Penicillansäureverbindung der Formel VII mit einem positive Halogenionen liefernden Halogenierungsmittel und, falls erforderlich, ein gegebenenfalls erhaltenes Zwischenprodukt mit einer Base behandelt, und gewünschtenfalls in einer erhaltenen Verbindung der Formel XIV eine Gruppe $R_a$ in eine andere Gruppe $R_a$ überführt und/oder aus einer erhaltenen cis,trans-Verbindung die cis- und/oder die trans-Verbindung isoliert.

Positive Halogenionen liefernde Halogenierungsmittel sind beispielsweise elementare Halogene, wie Chlor, Brom oder Jod, gemischte Halogene wie BrCl, Cl J oder Br J, Sulfurylhalogenide, wie Sulfurylchlorid oder Sulfurylbromid, N-Halogenamide oder N-Halogenimide, wie N-Chloracetamid, N-Bromacetamid, N-Chlorsuccinimid, N-Bromsuccinimid oder N,N'-Dibromhydantoine, oder organische Hypohalogenite, insbesondere Niederalkanoylhypohalogenite, wie Acetylhypochlorit, Propionylhypochlorit, Butyrylhypochlorit, Acetylhypobromit, Propionylhypobromit, Butylrylhypobromit, und dergleichen.

Die Reaktion wird analog USP 3.920.696 oder St. Kukolja, Journ. Am. Chem. Soc. 93, 6267 (1971), in einem inerten, aprotischen Lösungsmittel, insbesondere in einem halogenierten Kohlenwasserstoff, wie Methylenchlorid oder Tetrachlorkohlenstoff, bei Temperaturen zwischen etwa −80° bis etwa +80 °C, bevorzugt bei etwa −76 °C bis etwa Raumtemperatur, durchgeführt.

Das Molverhältnis von Halogenierungsmittel zur Verbindung der Formel VII liegt zwischen 1:1 bis 3:1 oder auch höher. Wenn das Molverhältnis etwa 1:1 ist, wird als Zwischenprodukt eine Verbindung der Formel

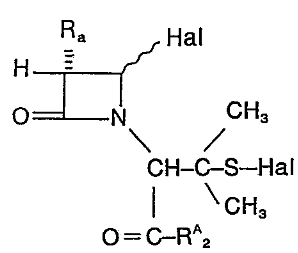

(XIV')

erhalten, die durch Behandeln mit einer Base, wie einem tertiären Amin, z.B. Triäthylamin, in die Verbindung der Formel XIV übergeführt werden kann. Bei Verwendung von mindestens 2 Mol Halogenierungsmittel oder mehr pro Mol Penicillansäureverbindung wird die gewünschte Verbindung der Formel XIV auch ohne nachträgliche Behandlung mit Base erhalten.

Bei der Ringöffnungsreaktion entsteht ein Gemisch der cis- und trans-Verbindung, wobei die trans-Verbindung bevorzugt gebildet wird. Durch übliche Trennmethoden, wie Kristallisieren oder Chromatographieren, können die cis- und/oder die trans-Verbindungen in reiner Form erhalten werden.

Stufe 3.4: Eine 2-Oxoessigsäureverbindung der Formel XV, worin W' Acyloxy oder Halogen bedeutet, wird erhalten, indem man eine Verbindung der Formel XIV ozonisiert und das gebildete Ozonid reduktiv zur Oxogruppe spaltet, und gewünschtenfalls in einer erhaltenen Verbindung eine Gruppe $R_a$ in eine andere Gruppe $R_a$ umwandelt, und/oder gewünschtenfalls aus einer erhaltenen cis, trans-Verbindung die cis- und/oder die trans-Verbindung isoliert.

Die Ozonisierung und die Reduktion des gebildeten Ozonids wird wie in der Stufe 2.5 beschrieben durchgeführt. Die gewünschtenfalls nachträgliche Auftrennung in reine Verbindungen wird wie unter Stufe 3.1 beschrieben durchgeführt.

Stufe 3.5: Eine Verbindung der Formel IIIa, worin W die unter Formel III angegebene Bedeutung hat, wird erhalten, indem man eine Verbindung der Formel XV, worin W' Acyloxy oder Halogen bedeutet, solvolysiert, und gewünschtenfalls in einer erhaltenen Verbindung eine Gruppe $R_a$ in eine andere Gruppe $R_a$ umwandelt, und/oder gewünschtenfalls eine Gruppe W' in eine andere Gruppe W' oder W überführt, und/oder gewünschtenfalls eine erhaltene cis-Verbindung zur entsprechenden trans-Verbindung isomerisiert, und/oder aus einer erhaltenen cis,trans-Verbindung die cis- und/oder die trans-Verbindung isoliert.

Die Solvolyse wird wie in Stufe 2.6 beschrieben durchgeführt. Wenn W Halogen ist, wird bevorzugt die Hydrazinolyse angewendet. Auch hier ist es nicht nötig das Zwischenprodukt der Formel XV nach der Ozonierungs- und Reduktionsreaktion zu isolieren, sondern man kann es in situ herstellen und unmittelbar solvolysieren.

In einer erhaltenen Verbindung der Formel IIIa, worin W Acyloxy oder Halogen bedeutet, kann diese Gruppe durch nucleophilen Austausch in eine andere Gruppe W übergeführt werden, wobei die eintretende Gruppe W nucleophiler sein muss als die austretende. Dieser Austausch kann analog der Stufe 1.1, z.B. durch Behandeln mit einem Alkalimetallsalz, wie einem Natrium- oder Kaliumsalz einer Säure H–W, durchgeführt werden.

Bei diesem Austausch von W gegen ein anderes W, wie auch bei dem nachfolgenden Austausch gegen eine Gruppe $R_1$–C(=Z')–S– gemäss Stufe 1.1, werden die optisch aktiven trans-Verbindungen der Formel IIIa bzw. IVa im Überschuss erhalten, gleichgültig, ob man von einer cis- oder trans-Verbindung ausgeht.

Durch Isomerisierung, z.B. durch Behandeln mit einer milden Lewissäure in katalytischen Mengen, kann eine erhaltene cis- in eine trans-Verbindung übergeführt werden. Die Isomerisierung mit einer Lewissäure findet in einem inerten Lösungsmittel bei erhöhter Temperatur, etwa bei 50° bis 150 °C, z.B. unter Rückfluss in Benzol, statt.

In den Verbindungen IV (incl. IVa) bis VI und II kann eine gegebenenfalls substituierte Methylidengruppe Z' durch Ozonisierung und anschliessende Reduktion des gebildeten Ozonids, gemäss dem in der Stufe 2.5 beschriebenen Verfahren, in eine Oxogruppe Z übergeführt werden.

Die Erfindung umfasst ebenfalls die neuen Zwischenprodukte, wie diejenigen der Formeln IIIa und IV (incl. IVa) bis XV und insbesondere der Formel II, sowie die Verfahren zu ihrer Herstellung.

Im Zusammenhang mit der vorliegenden Beschreibung enthalten mit «nieder» bezeichnete organische Reste, sofern nicht ausdrücklich definiert, bis zu 7, vorzugsweise bis zu 4 Kohlenstoffatome; Acylreste enthalten bis zu 20, vorzugsweise bis zu 12 und in erster Linie bis zu 7 Kohlenstoffatome.

Die folgenden Beispiele dienen zur Illustration der Erfindung; Temperaturen werden in Celsiusgraden angegeben. Folgende Abkürzung wird benutzt: DC = Dünnschichtchromatogramm auf Silicagel.

Beispiel 1:
4-Acetylthio-3-methyl-2-oxo-azetidin
(racemische cis- und trans-Verbindung)

Eine Lösung von 438 mg (3,06 mM) 4-Acetoxy-3-methyl-azetidin-2-on (hergestellt gemäss K. Clauss et al., Lieb. Ann. Chem., 1974, 539; racemische Mischung von cis- und trans-Isomer im Verhältnis 3:1; F. 53–65°) in 1,13 ml Wasser und 0,27 ml Aceton wird bei Raumtemperatur unter Stickstoffatmosphäre tropfenweise mit einer Lösung von 0,33 ml Thioessigsäure in 4,5 ml 1N Natriumhydroxidlösung versetzt und bei der gleichen Temperatur 3 Stunden gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an Silicagel mit Toluol-Äthyl-acetat 4:1 bis 3:2 chromatographiert und ergibt zunächst die reine trans-Verbindung, dann eine Mischung der cis- und trans-Isomeren der Titelverbindung und anschliessend die reine cis-Verbindung. DC: Rf = 0,31 (bis-Isomer); 0,36 (trans-Isomer) (Toluol-Äthylacetat 2:3); IR-Spektrum ($CH_2Cl_2$): Absorptionsbanden bei 2,95; 5,6; 5,87; 8,65; 8,85; 10,45 μ. NMR-Spektrum (in $CDCl_3$/100 Mc; in ppm): cis-Verbindung: 6,2, 1H, breit (Austausch mit $D_2O$); 5,45, 1H, d(J = 5,5 Hz; 3,5–3,9, 1H, m; 2,4, 3H, s; 1,3, 3H, d; trans-Verbindung: 6,5, 1H, breit (Austausch mit $D_2O$); 4,93; 1H, d (J ~ 2,5 Hz); 3,0–3,4, 1H, m; 2,4, 3H, s; 1,42, 3H, d.

Beispiel 2:
2-(4-Acetylthio-3-methyl-2-oxo-1-azetidinyl)-2-hydroxyessigsäure-p-nitrobenzylester (racemisches cis, trans-Gemisch)

Bei Raumtemperatur werden 129 mg (0,81 mM) 4-Acetylthio-3-methyl-2-oxo-azetidin (racemisches cis,trans-Gemisch) mit einer Lösung von 500 mg 2-Äthoxy-2-hydroxy-essigsäure-nitrobenzylester in einer Mischung von 10 ml Toluol und 2,5 ml Dimethylformamid versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an Silikagel mit Toluol-Äthylacetat 9:1 bis 8:2 chromatographiert. Nach Elution von nicht umgesetztem 2-Äthoxy-2-hydroxy-essigsäure-p-nitrobenzylester wird eine Mischung der cis-trans Isomeren der Titelverbindung mit den folgenden physikochemischen Eigenschaften eluiert: DC: Rf = 0,38 (Toluol-Äthylacetat 2:3); IR-Spektrum ($CH_2Cl_2$): Absorptionsbanden bei 2,85; 5,62; 5,7; 5,9; 6,2; 6,55; 7,4 und 8,25 µ.

Beispiel 3:
2-(4-Acetylthio-3-methyl-2-oxo-1-azetidinyl)-2-triphenyl-phosphoranyliden-essigsäure-p-nitrobenzylester (racemisches cis,trans-Gemisch)

a) Eine Lösung von 225 mg 2-(4-Acetylthio-3-methyl-2-oxo-1-azetidinyl)-2-hydroxyessigsäure-p-nitrobenzylester (racemisches cis,trans-Gemisch) in 5 ml absolutem Dioxan wird zu einer bereits 30 Minuten gerührten Lösung von 1 g Poly-Hünigbase in 2,5 ml absolutem Dioxan gegeben. Nach Zugabe einer Lösung von 0,175 ml Thionylchlorid in 1,5 ml absolutem Dioxan wird die Reaktionsmischung 100 Minuten bei Raumtemperatur und unter Stickstoff gerührt. Die Poly-Hünigbase wird abfiltriert, mit Dioxan gewaschen und das Filtrat im Vakuum eingedampft. DC des rohen 2-(4-Acetylthio-3-methyl-2-oxo-1-azetidinyl)-2-chloressigsäure-p-nitrobenzylesters (racemisches cis,trans-Gemisch): Rf = 0,62 (Toluol-Äthylacetat 2:3).

b) Der erhaltene rohe 2-(4-Acetylthio-3-methyl-2-oxo-1-azetidinyl)-2-chloressigsäure-p-nitrobenzylester wird in 12 ml absolutem Dioxan gelöst, mit 1 g Poly-Hünigbase versetzt, 30 Minuten gerührt, dann mit 312 mg Triphenylphosphin versetzt und 15 Stunden bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an Silikagel mit Toluol-Äthylacetat chromatographiert und ergibt ein cis-trans-Gemisch der Titelverbindung mit den folgenden physiko-chemischen Eigenschaften: DC: Rf = 0,28 (Toluol-Äthylacetat 2:3); IR-Spektrum ($CH_2Cl_2$): Absorptionsbanden bei 5,67; 5,9; 6,15; 6,55; 6,95; 7,4; 9,0 und 9,25 µ.

Beispiel 4:
2,6-Dimethyl-2-penem-3-carbonsäure-p-nitrobenzylester (racemisches cis,trans-Gemisch)

Eine Lösung von 118 mg 2-[4-Acetylthio-3-methyl-2-oxo-1-azetidinyl]-2-triphenylphosphoranyliden-essigsäure-p-nitrobenzylester (racemisches cis,trans-Gemisch) in 50 ml absolutem Toluol wird mit einer katalytischen Menge p-Hydroxychinon versetzt und unter Stickstoff 48 Stunden bei 90° gerührt. Das Lösungsmittel wird im Vakuum abgedampft und der Rückstand an Silikagel mit Toluol-Äthylacetat 19:1 chromatographiert. Man erhält ein cis-trans Gemisch (1:4) der Titelverbindung in Form eines gelblichen Öles mit folgenden physikochemischen Eigenschaften: DC: Rf = 0,59 (Toluol-Äthylacetat 2:3); IR-Spektrum ($CH_2Cl_2$): Absorptionsbanden bei 5,6; 5,85; 6,3; 6,55; 7,4; 7,6; 8,3; 9,25 µ; NMR-Spektrum (in $CDCl_3$/100 Mc; in ppm): 8,4–8,2, 2H, 7,75–7,76, 2H, m; 5,7–5,2, 3H, m; 4,1–3,6, 1H, m; 2,4, 2,43, 3H, 2s; 1,6–1,4, 3H, 2d.

Beispiel 5:
2,6-Dimethyl-2-penem-3-carbonsäure (racemisches cis,trans-Gemisch)

Eine Lösung von 47 mg (0,14 mM) 2,6-Dimethyl-2-penem-3-carbonsäure-p-nitrobenzylester (racemisches cis,trans-Gemisch 1:4) in 3 ml absolutem Äthylacetat wird mit 2 ml 0,2 M wässriger Natriumhydrogencarbonatlösung und 100 mg 10%-igem Palladium/Kohle Katalysator versetzt und bei Normaldruck während 40 Minuten unter Wasserstoff gerührt. Die Hydriermischung wird über Diatomeenerde vom Katalysator abfiltriert, mit 0,2 M Natriumhydrogencarbonatlösung und mehrere Male mit Äthylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung (cis,trans-Gemisch ~ 1:4) hat folgende physikochemischen Eigenschaften: DC: Rf = 0,28 (Toluol-Äthylacetat-Essigsäure 60:40:5; IR-Spektrum ($CH_2Cl_2$): Absorptionsbanden bei 3,5, 5,6, 5,95, 6,3 µ; NMR-Spektrum (DMSO d6/100 Mc; in ppm): 5,65, 1H, q; 3,3–3,9, 2H, m (+ $H_2O$); 2,28, 3H, s. Schmelzpunkt 119°.

Beispiel 6:
2,6-Dimethyl-2-penem-3-carbonsäure Natriumsalz (racemisches cis,trans-Gemisch)

Eine Lösung von 50 mg 2,6-Dimethyl-2-penem-3-carbonsäure in der äquivalenten Menge wässriger Natriumhydrogencarbonatlösung wird im Vakuum eingedampft und am Hochvakuum getrocknet.

Beispiel 7:
4-Acetylthio-3-methyl-2-oxo-azetidin (racemische trans-Verbindung)

Eine Lösung von 2 g 4-Acetoxy-3-methyl-azetidin-2-on (hergestellt gemäss K. Clauss et al., Lieb.

Ann. Chem., 1974, 539; racemische Mischung von cis- und trans-Isomer im Verhältnis 3:1; F. 53–65°) in 5,16 ml Wasser und 1,25 ml Aceton wird bei Raumtemperatur unter Stickstoffatmosphäre tropfenweise mit einer Lösung von 1,5 ml Thioessigsäure in 20,5 ml 1N Natriumhydroxidlösung versetzt und bei der gleichen Temperatur 3 Stunden gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an 150 g Silicagel mit Toluol-Äthylacetat 9:1 chromatographiert und ergibt das fast reine trans-Isomere der Titelverbindung mit den folgenden physikochemischen Eigenschaften: DC: 0,38 (Toluol-Äthylacetat 2:3); IR-Spektrum ($CH_2Cl_2$): Absorptionsbanden bei 2,95; 5,6; 5,87; 7,37; 7,45; 8,62; 8,82 μ. NMR-Spektrum (in CD $Cl_3$/100 Mc; in ppm): 6,55, 1H, m (Austausch mit $D_2O$); 4,9, 1H, d, J = 2 Hz, 3,35–3,05, 1H, m; 2,38, 3H, s; 1,4, 3H, d, J = 7 Hz. Nachfolgend wird ein Gemisch des cis- und trans-Isomeren isoliert.

Beispiel 8:
2-(4-Acetylthio-3-methyl-2-oxo-1-azetidinyl)-2-hydroxyessigsäure-p-nitrobenzylester (racemische trans-Verbindung)

Bei Raumtemperatur werden 1,35 g (8,49 mM) 4-Acetylthio-3-methyl-2-oxo-azetidin (racemische trans-Verbindung) mit einer Lösung von 5 g 2-Äthoxy-2-hydroxy-essigsäure-p-nitrobenzylester in einer Mischung von 100 ml Toluol und 25 ml Dimethylformamid versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an 100 g Silikagel mit Toluol-Äthylacetat 9:1 chromatographiert. Nach Elution von nicht umgesetztem 2-Äthoxy-2-hydroxy-essigsäure-p-nitrobenzylester wird die Titelverbindung mit den folgenden physikochemischen Eigenschaften eluiert: DC: Rf = 0,33 (Toluol-Äthylacetat 2:3); IR-Spektrum ($CH_2Cl_2$): Absorptionsbanden bei 2,85; 5,6; 5,7; 5,87; 6,2; 6,52; 7,4; 8,28, 9–9,2 μ.

Beispiel 9:
2-(4-Acetylthio-3-methyl-2-oxo-1-azetidinyl)-2-triphenylphosphoranyliden-essigsäure-p-nitrobenzylester (racemische trans-Verbindung)

a) Eine Lösung von 3 g 2-(4-Acetylthio-3-methyl-2-oxo-1-azetidinyl)-2-hydroxyessigsäure-p-nitrobenzylester (racemische trans-Verbindung) in 75 ml absolutem Dioxan wird zu einer bereits 30 Minuten gerührten Lösung von 13,5 g Poly-Hünigbase in 35 ml absolutem Dioxan gegeben. Nach Zugabe einer Lösung von 2,4 ml Thionylchlorid in 22,4 ml absolutem Dioxan wird die Reaktionsmischung 100 Minuten bei Raumtemperatur und unter Stickstoff gerührt. Die Poly-Hünigbase wird abfiltriert, mit Dioxan gewaschen und das Filtrat im Vakuum eingedampft. DC des rohen 2-(4-Acetylthio-3-methyl-2-oxo-1-azetidinyl)-2-chloressigsäure-p-nitrobenzylesters (racemische trans-Verbindung): Rf = 0,59 (Toluol-Äthylacetat 2:3).

b) Der erhaltene rohe 2-(4-Acetylthio-3-methyl-2-oxo-1-azetidinyl)-2-chloressigsäure-p-nitrobenzylester wird in 175 ml absolutem Dioxan gelöst, mit 13,5 g Poly-Hünigbase versetzt, dann mit 4,2 mg Triphenylphosphin versetzt und 15 Stunden bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an Silikagel mit Toluol-Äthylacetat chromatographiert und ergibt die trans-Titelverbindung mit den folgenden physiko-chemischen Eigenschaften: DC: Rf = 0,24 (Toluol-Äthylacetat 2:3); IR-Spektrum ($CH_2Cl_2$): Absorptionsbanden bei 5,67; 5,9; 6,15; 6,55; 7,4; 9,0 μ.

Beispiel 10:
2,6-Dimethyl-2-penem-3-carbonsäure-p-nitrobenzylester (racemische trans-Verbindung)

Eine Lösung von 363 mg 2-[4-Acetylthio-3-methyl-2-oxo-1-azetidinyl]-3-triphenylphosphoranyliden-essigsäure-p-nitrobenzylester (racemische trans-Verbindung) in 180 ml absolutem Toluol wird mit einer katalytischen Menge p-Hydroxychinon versetzt und unter Stickstoff 48 Stunden bei 90° gerührt. Das Lösungsmittel wird im Vakuum abgedampft und der Rückstand an 20 g Silikagel mit Toluol-Äthylacetat 19:1 chromatographiert. Man erhält die trans-Titelverbindung in Form gelblicher Kristalle vom Schmelzpunkt 141–143° und mit folgenden physikochemischen Eigenschaften: DC: Rf = 0,6 (Toluol-Äthylacetat 2:3); IR-Spektrum ($CH_2Cl_2$): Absorptionsbanden bei 3,4; 5,57; 5,82; 6,27; 6,55; 7,4; 7,6; 8,3; 9,22 μ; NMR-Spektrum (in $CDCl_3$/100 Mc; in ppm): 8,25–8,15, 2H, m; 7,65–7,56, 2H, m; 5,55–5,12, 3H, m + d (J = 1,5 Hz); 3,9–3,6, 1H, m; 2,36, 3H, s; 1,5, 3H, d.

Beispiel 11:
2,6-Dimethyl-2-penem-3-carbonsäure (racemische trans-Verbindung)

Eine Lösung von 80 mg (0,24 mM) 2,3-Dimethyl-2-penem-3-carbonsäure-p-nitrobenzylester (racemische trans-Verbindung) in 5 ml absolutem Äthylacetat wird mit 3 ml 0,2 N wässriger Natriumhydrogencarbonatlösung und 150 mg 10%-igem Palladium/Kohle Katalysator versetzt und bei Normaldruck während 50 Minuten unter Wasserstoff gerührt. Die Hydriermischung wird über Diatomeenerde vom Katalysator abfiltriert, mit 0,2 N Natriumhydrogencarbonatlösung und mehrere Male mit Äthylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: Schmelzpunkt 119° (Zersetzung); DC: RF = 0,3 (Toluol-Äthylacetat-Essigsäure 60:40:5); IR-Spektrum (KBr): Absorptionsbanden bei 3,3–3,5; 5,62; 6,0; 6,35; 6,95; 7,55; 7,85 μ; NMR-Spektrum (DMSO d6/100 Mc; in ppm): 5,38, 1H, d, (J = 1,5 Hz); 3,7, 1H, m; 3,4, 1H, m (Austausch mit $D_2O$); 2,28, 3H, s; 1,34, 3H, d.

Beispiel 12:
4-Acetylthio-3-isopropyl-2-oxo-azetidin
(racemische trans-Verbindung)

Eine Lösung von 750 mg (4,38 mM) 4-Acetoxy-3-isopropyl-azetidin-2-on (racemische Mischung von cis- und trans-Isomer im Verhältnis 1:3) in 3,6 ml Wasser und 9,9 ml Aceton wird bei Raumtemperatur unter Stickstoffatmosphäre tropfenweise mit einer Lösung von 0,52 ml Thioessigsäure in 7 ml 1N Natriumhydroxidlösung versetzt und bei der gleichen Temperatur 75 Minuten gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an 40 g Silicagel mit Toluol-Äthylacetat 4:1 chromatographiert und ergibt die trans-Titelverbindung. DC: Rf = 0,4 (Toluol-Äthylacetat 2:3); IR-Spektrum ($CH_2Cl_2$): Absorptionsbanden bei 2,95; 3,37; 5,62; 5,87; 8,8 μ. NMR-Spektrum (in $CDCl_3$/100 Mc; in ppm): 6,35, 1H, m (Austausch mit $D_2O$); 5,04, 1H, d (J = 2,5 Hz); 3,0, 1H, m; 2,37, 3H, s; 2,1, 1H, m; 1,05, 3H, m.

Das Ausgangsmaterial wird wie folgt hergestellt:

a) Eine Mischung von 172,28 g (216,5 ml; 2 M) Isovaleraldehyd, 306 g (283 ml) Acetanhydrid und 24 g frisch geschmolzenes Kaliumacetat wird während 17 Stunden unter Rückfluss gekocht. Die abgekühlte Mischung wird mit 5%-iger Natriumcarbonatlösung gewaschen, bis die organische Phase neutral reagiert. Nach Waschen mit Wasser und Trocknen über Magnesiumsulfat wird das erhaltene Öl destilliert. Man erhält das 3-Methyl-1-butenylacetat (cis,trans-Gemisch 1:4) vom Siedepunkt 135–140°/760 mmHg.

b) Eine Lösung von 12,8 g (0,1 M) 3-Methyl-1-butenyl-acetate (cis,trans-Gemisch 1:4) in 40 ml absolutem Methylenchlorid wird bei Raumtemperatur und unter Stickstoff tropfenweise mit einer Lösung von 8,72 ml N-Chlorsulfonyl-isocyanat in 10 ml absolutem Methylenchlorid versetzt. Nach 4 Stunden wird die Reaktionsmischung langsam auf eine Mischung von 10 ml Wasser, 45 g Eis, 24 g Natriumbicarbonat und 8,3 g Natriumsulfit gegossen, wobei die Temperatur durch gelegentliche Zugabe von Eis zwischen 0° und 5° gehalten wird. Nach etwa 30 Minuten reagiert die organische Phase neutral, worauf sie abgetrennt wird. Die wässrige Phase wird mit Methylenchlorid extrahiert. Die organischen Phasen werden vereinigt, über Natriumsulfat getrocknet und im Vakuum abgedampft. Der Rückstand wird an Silikagel mit Toluol/Äthylacetat chromatographiert und ergibt ein cis,trans-Gemisch von 4-Acetoxy-3-isopropyl-azetidin-2-on im Verhältnis von ca. 1:3. DC: Rf = 0,3 (Toluol-Äthylacetat 2:3); IR-Spektrum (in Methylenchlorid): Absorptionsbanden bei 2,95; 3,37; 5,6; 5,72; 7,32; 8,1; 9,7; 10,2 μ; NMR-Spektrum ($CDCl_3$/100 Mc; in ppm): 6,75, 1H, m (Austausch mit $D_2O$); 5,85, d, J = 4,5 Hz (cis) und 5,6, d, J = 1,5 Hz (trans), 1H; 3,03, 1H, m; 2,1, 3H, 2s; 2,3–1,8, 1H, m; 1,1, 6H, m.

Beispiel 13:
2-(4-Acetylthio-3-isopropyl-2-oxo-1-azetidinyl)-2-hydroxy-essigsäure-p-nitrobenzylester
(racemische trans-Verbindung)

Bei Raumtemperatur wird eine Lösung von 616 mg (3,3 mM) 4-Acetylthio-3-isopropyl-2-oxo-azetidin (racemische trans-Verbindung) in 48 ml Toluol und 10,5 ml Dimethylformamid mit 1,9 g 2-Äthoxy-2-hydroxy-essigsäure-p-nitrobenzylester versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an 60 g Silikagel mit Toluol-Äthylacetat 9:1 chromatographiert. Man erhält die beiden mit nicht umgesetztem 2-Äthoxy-2-hydroxy-essigsäure-p-nitrobenzylester etwas verunreinigten trans-Isomeren der Titelverbindung mit den folgenden physikochemischen Eigenschaften: DC: Rf = 0,4 und 0,37 (Toluol-Äthylacetat 2:3); IR-Spektrum ($CH_2Cl_2$): Absorptionsbanden bei 5,62; 5,68; 6,55; 7,42 μ.

Beispiel 14:
2-(4-Acetylthio-3-isopropyl-2-oxo-1-azetidinyl)-2-triphenylphosphoranyliden-essigsäure-p-nitrobenzylester
(racemische trans-Verbindung)

a) Eine Lösung von 1,175 g 2-(4-Acetylthio-3-isopropyl-2-oxo-1-azetidinyl)-2-hydroxyessigsäure-p-nitrobenzylester (racemische trans-Verbindung) in 21 ml absolutem Dioxan wird zu einer bereits 30 Minuten gerührten Lösung von 3,8 g Poly-Hünigbase in 10 ml absolutem Dioxan gegeben. Nach tropfenweiser Zugabe einer Lösung von 0,67 ml Thionylchlorid in 6,3 ml absolutem Dioxan wird die Reaktionsmischung 90 Minuten bei Raumtemperatur und unter Stickstoff gerührt. Die Poly-Hünigbase wird abfiltriert, mit Dioxan gewaschen und das Filtrat im Vakuum eingedampft. Der erhaltene rohe 2-(4-Acetylthio-3-isopropyl-2-oxo-1-azetidinyl)-2-chloressigsäure-p-nitrobenzylester (racemische, trans-Verbindung) kann ohne weitere Reinigung in die nächste Stufe eingesetzt werden.

b) Der erhaltene rohe 2-(4-Acetylthio-3-isopropyl-2-oxo-1-azetidinyl)-2-chloressigsäure-p-nitrobenzylester wird in 50 ml absolutem Dioxan gelöst, mit 3,8 g Poly-Hünigbase versetzt, 30 Minuten gerührt, dann mit 1,18 g Triphenylphosphin versetzt und 15 Stunden bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an 60 g Silikagel mit Toluol-Äthylacetat 7:3 chromatographiert und ergibt die trans-Titelverbindung mit den folgenden physiko-chemischen Eigenschaften: DC: Rf = 0,25 (Toluol-Äthylacetat 2:3); IR-Spektrum ($CH_2Cl_2$): Absorptionsbanden bei 5,7; 5,9; 6,17; 6,55; 7,42; 9,05 μ.

Beispiel 15:
2-Methyl-6-isopropyl-2-penem-3-carbonsäure-p-nitrobenzylester
(racemische trans-Verbindung)

Eine Lösung von 660 mg 2-[4-Acetylthio-3-isopropyl-2-oxo-1-azetidinyl]-2-triphenylphosphoranylidenessigsäure-p-nitrobenzylester (racemische trans-Verbindung) in 300 ml absolutem Toluol wird mit einer katalytischen Menge p-Hydroxychinon versetzt und unter Stickstoff 48 Stunden bei 90° gerührt. Das Lösungsmittel wird im Vakuum abgedampft und der Rückstand an 30 g Silikagel mit Toluol-Äthylacetat 19:1 chromatographiert. Man erhält die trans-Titelverbindung nach Kristallisation aus Diäthyläther-Methylenchlorid in Form farbloser Kristalle mit folgenden physikochemischen Eigenschaften: Schmelzpunkt: 138–139°; DC: Rf = 0,59 (Toluol-Äthylacetat 2:3): IR-Spektrum ($CH_2Cl_2$): Absorptionsbanden bei 5,57; 5,82; 6,27; 6,55; 7,4; 7,6 μ; NMR-Spektrum (in $CDCl_3$/100 Mc; in ppm): 8,3–8,2, 2H, m; 7,5–7,4, 2H, m; 5,75–5,1, 3H, m; 3,6–3,5, 1H, dd, J = 8 und 1,5 Hz; 2,35, 3H, s; 1,07, 6H, m.

Beispiel 16:
2-Methyl-6-isopropyl-2-penem-3-carbonsäure
(racemische trans-Verbindung)

Eine Lösung von 100 mg 2-Methyl-6-isopropyl-2-penem-3-carbonsäure-p-nitrobenzylester (racemische trans-Verbindung) in 7 ml absolutem Äthylacetat wird mit 4 ml 0,2 N wässriger Natriumhydrogencarbonatlösung und 50 mg 10%-igem Palladium/Kohle Katalysator versetzt und bei Normaldruck während 30 Minuten unter Wasserstoff gerührt. Die Hydriermischung wird über Diatomeenerde vom Katalysator abfiltriert, mit 0,2 N Natriumhydrogencarbonatlösung und mehrere Male mit Äthylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: Schmelzpunkt 140–143° (Zersetzung); DC: Rf = 0,37 (Toluol-Äthylacetat-Essigsäure 60:40:5; IR-Spektrum (KBr): Absorptionsbanden bei 3,5; 5,62; 6,0; 6,35; 6,9; 7,52; 7,8; 8,0 μ; NMR-Spektrum (DMSO d6/100 Mc; in ppm): 5,52, 1H, d, J = 1,5 Hz; 3,56, 1H + 2 $H_2O$, dd, J = 1,5 und 7,5 Hz, 2,26, 3H, s; 2,04, 1H, m; 1–0,9, 6H, m.

Beispiel 17:
4-Acetylthio-3-benzyl-2-oxo-azetidin
(racemische trans-Verbindung)

Eine Lösung von 2,19 g (10 mM) 4-Acetoxy-3-benzyl-azetidin-2-on (racemische Mischung von cis- und trans-Isomer im Verhältnis 9:13) in 10 ml Dioxan wird bei Raumtemperatur unter Stickstoffatmosphäre tropfenweise mit einer Lösung von 0,76 g (10 mM) Thioessigsäure in 10 ml 1N Natriumhydroxidlösung versetzt und bei der gleichen Temperatur 3 Stunden gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an Silicagel mit Toluol-Äthylacetat 9:1 chromatographiert und ergibt ein cis-trans-Gemisch der Titelverbindung im Verhältnis 2:10. Durch Umkristallisation aus Methylenchlorid/Hexan bei −10° erhält man die reine trans-Verbindung vom Schmelzpunkt 42–43°. DC: Rf = 0,52 (Toluol-Äthylacetat 1:1); IR-Spektrum ($CH_2Cl_2$): Absorptionsbanden bei 2,95; 5,65; 5,95; 7,40; 8,8; 10,5 μ. NMR-Spektrum (in $CDCl_3$/100 Mc; in ppm): 7,24, H, m; 6,60, 1H, b; 4,99, 1H, d, J = 2 Hz; 3,45, 1H, dq, $J_B$ = 8 Hz; $J_C$ = 6 Hz, $J_D$ = 2 Hz; 3,18, 1H, q, $J_A$ = 15 Hz, $J_C$ = 6 Hz; 3,00, 1H, q, $J_A$ = 15 Hz, $J_B$ = 8 Hz; 2,30, 3H, s.

Das Ausgangsmaterial wird wie folgt hergestellt:

a) Eine Mischung von 25 g (0,186 M) 3-Phenylpropionaldehyd, 50 ml Acetanhydrid und 50 ml Pyridin wird während 15 Stunden bei 100° gerührt und dann im Wasserstrahlvakuum eingedampft. Der Rückstand wird im Methylenchlorid gelöst, mit 5%-iger wässriger Natriumhydrogencarbonatlösung und Zitronensäurelösung gewaschen, über Natriumsulfat getrocknet und im Vakuum vom Lösungsmittel befreit. Der Rückstand wird im Vakuum destilliert. Man erhält das 3-Phenyl-1-propenylacetat (cis,trans-Gemisch 1:1) vom Siedepunkt 61–65°/1 mm Hg.

b) Eine bei 0° bereitete Mischung von 17,6 g (0,1 M) 3-Phenyl-1-propenylacetate (cis,trans-Gemisch 1:1) und 14,15 g (0,1 mM) N-Chlorsulfonyl-isocyanat wird 6 Stunden bei 10–15° gerührt. Die Reaktionsmischung wird mit 100 ml kaltem Methylenchlorid verdünnt und langsam auf eine Mischung von 10 ml Wasser, 45 g Eis, 24 g Natriumbicarbonat und 17 g Natriumsulfit gegossen. Nach Filtration wird die organische Phase abgetrennt. Die wässrige Phase wird mit Methylenchlorid extrahiert. Die organischen Phasen werden vereinigt, über Natriumsulfat getrocknet und im Vakuum eingedampft. Der Rückstand wird an Silikagel mit Toluol/Äthylacetat 9:1 bis 8:2 chromatographiert und ergibt ein cis-trans-Gemisch von 4-Acetoxy-3-benzyl-azetidin-2-on im Verhältnis von 9:13. DC: Rf = 0,5 (Toluol-Äthylacetat 1:1); IR-Spektrum (in Methylenchlorid): Absorptionsbanden bei 2,95; 5,6; 5,75; 7,35; 8,15; 8,65; 9,6; 10,25 μ: NMR-Spektrum ($CDCl_3$/100 Mc; in ppm): 2,04, s, und 2,08, s, 3H; 2,95–3,15, 2H, m; 3,35–3,8, 1H, m; 5,50, 0,6 H, d, J = 2 Hz (trans); 5,86, 0,4 H, d, J = 4 Hz (cis); weitere Signale bei 6,80–7,45.

Beispiel 18:
2-(4-Acetylthio-3-benzyl-2-oxo-1-azetidinyl)-2-hydroxy-essigsäure-p-nitrobenzylester
(racemische trans-Verbindung)

Bei Raumtemperatur wird eine Lösung von 0,73 g (3,1 mM) 4-Acetylthio-3-benzyl-3-oxo-azetidin (racemische trans-Verbindung) in 50 ml Toluol

und 20 ml Dimethylformamid mit 2 g 2-Äthoxy-2-hydroxy-essigsäure-p-nitrobenzylester versetzt. Nach Zugabe von frisch getrockneten Molekularsieben wird die Mischung unter Stickstoff über Nacht bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an Silikagel mit Toluol-Äthylacetat 9:1 bis 4:1 chromatographiert. Man erhält die beiden mit nicht umgesetztem 2-Äthoxy-hydroxy-essigsäure-p-nitrobenzylester etwas verunreinigten trans-Isomeren der Titelverbindung mit den folgenden physikochemischen Eigenschaften: DC: Rf = 0,57 (Toluol-Äthylacetat 1:1); IR-Spektrum ($CH_2Cl_2$): Absorptionsbanden bei 2,85; 5,60; 5,70; 6,00; 6,20; 6,55; 7,40; 8,25; 9,00; 11,75 μ.

Beispiel 19:
2-(4-Acetylthio-3-benzyl-2-oxo-1-azetidinyl)-2-triphenyl-phosphoranyliden-essigsäure-p-nitrobenzylester
(racemische trans-Verbindung)

a) Eine Lösung von 1,5 g 2-(4-Acetylthio-3-benzyl-2-oxo-1-azetidinyl)-2-hydroxyessigsäure-p-nitrobenzylester (racemische trans-Verbindung) in 20 ml trockenem Dioxan wird mit 6 g Poly-Hünigbase versetzt. Nach tropfenweiser Zugabe einer Lösung von 1,5 ml Thionylchlorid in 10 ml Dioxan wird die Reaktionsmischung 60 Minuten bei Raumtemperatur unter Stickstoff gerührt. Die Poly-Hünigbase wird abfiltriert, mit Dioxan gewaschen und das Filtrat im Vakuum eingedampft. Der erhaltene rohe 2-(4-Acetylthio-3-benzyl-2-oxo-1-azetidinyl)-2-chloressigsäure-p-nitrobenzylester (racemische, trans-Verbindung) kann ohne weitere Reinigung in die nächste Stufe eingesetzt werden.

b) Der erhaltene rohe 2-(4-Acetylthio-3-isopropyl-2-oxo-1-azetidinyl)-2-chloressigsäure-p-nitrobenzylester wird in 20 ml trockenem Dioxan gelöst, mit 6 g Poly-Hünigbase versetzt, 30 Minuten gerührt, dann mit 1,5 g Triphenylphosphin versetzt und über Nacht bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an Silikagel mit Toluol-Äthylacetat 9:1 bis 1:1 chromatographiert und ergibt die trans-Titelverbindung mit den folgenden physikochemischen Eigenschaften: DC: Rf = 0,50 (Toluol-Äthylacetat 1:1); IR-Spektrum ($CH_2Cl_2$): Absorptionsbanden bei 5,7; 5,9; 6,2; 6,55; 7,00; 7,42; 9,05; 11,75 μ.

Beispiel 20:
2-Methyl-6-benzyl-2-penem-3-carbonsäure-p-nitrobenzylester (racemische trans-Verbindung)

Eine Lösung von 0,90 g (1,3 mM) 2-[4-Acetylthio-3-benzyl-2-oxo-1-azetidinyl]-2-triphenylphosphoranyliden-essigsäure-p-nitrobenzylester (racemische trans-Verbindung) in 50 ml trockenem Toluol wird mit einer katalytischen Menge p-Hydroxychinon versetzt und unter Stickstoff zwei Tage bei 90° gerührt. Das Lösungsmittel wird im Vakuum abgedampft und der Rückstand an Silikagel mit Toluol-Ätyhlacetat 9:1 chromatographiert. Man erhält die trans-Titelverbindung nach Kristallisation aus Methylenchlorid-Diäthyläther mit folgenden physikochemischen Eigenschaften: Schmelzpunkt: 182–183°; DC: Rf = 0,85 (Toluol-Äthylacetat 1:1): IR-Spektrum ($CH_2Cl_2$): Absorptionsbanden bei 5,60; 5,85; 6,30; 6,55; 7,4; 7,6; 8,25; 8,55; 9,25; 11,70 μ; NMR-Spektrum (in $CDCl_3$/100 Mc; in ppm): 2,36, 3H, s; 3,12; 1H, dd, $J_A = 14$ Hz, $J_B = 9$ Hz; 3,34, 1H, dd, $J_A = 14$ Hz, $J_C = 6$ Hz; 4,03, 1H, dq, $J_B = 9$ Hz, $J_C = 6$ Hz, $J_D = 2$ Hz; 5,40, 1H, d, $J_D = 2$ Hz; 5,25, 1H, d, J = 14 Hz; 5,45, 1H, d, J = 14 Hz; 7,30, 5H, m; 7,66, 2H, d, J = 9 Hz ; 8,27, 2H, d, J = 9 Hz.

Beispiel 21:
2-Methyl-6-benzyl-2-penem-3-carbonsäure
(racemische trans-Verbindung)

Eine Lösung von 200 mg 2-Methyl-6-benzyl-2-penem-3-carbonsäure-p-nitrobenzylester (racemische trans-Verbindung) in 12 ml absolutem Äthylacetat wird mit 8 ml 0,2 M wässriger Natriumhydrogencarbonatlösung und 400 mg 10%-igem Palladium/Kohle Katalysator versetzt und bei Normaldruck während 60 Minuten unter Wasserstoff gerührt. Die Hydriermischung wird über Diatomeenerde vom Katalysator abfiltriert. Die wässrige Phase wird abgetrennt, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: DC: Rf = 0,31 (Toluol-Äthylacetat-Essigsäure 60:40:5;); IR-Spektrum (KBr): Absorptionsbanden bei 3,20–4,30 b; 5,65; 6,0; 6,35; 6,9; 7,5; 7,9; 8,2 μ.

Beispiel 22:
4-Äthylthio-thiocarbonylthio-3-isopropyl-2-oxoazetidin (racemische trans-Verbindung)

Eine Lösung von 195 mg (1.14 mM) 4-Acetoxy-3-isopropyl-azetidin-2-on (racemische Mischung von cis- und trans-Isomer im Verhältnis 1:3) in 1 ml Wasser und 0,2 ml Aceton wird bei Raumtemperatur unter Stickstoffatmosphäre tropfenweise mit einer Lösung von 230 mg Kaliumäthyltrithiocarbonat in 1,5 ml Wasser versetzt und bei der gleichen Temperatur 120 Minuten gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an 12 g Silicagel mit Toluol-Äthylacetat 9:1 chromatographiert und ergibt die trans-Titelverbindung. Schmelzpunkt: 65–66°. DC: Rf = 0,5 (Toluol-Äthylacetat 2:3); IR-Spektrum ($CH_2Cl_2$): Absorptionsbanden bei 2,95; 3,37; 5,62; 9,25 μ. NMR-Spektrum (in $CDCl_3$/100 Mc; in ppm): 6,65, 1H, m (Austausch mit $D_2O$); 5,4, 1H, d (J = 2,5 Hz); 3,39, 2H, q; 3,05, 1H, m; 2,15, 1H, m; 1,38, 3H, t; 1,1, 6H, m.

Beispiel 23:
2-(4-Äthylthio-thiocarbonylthio-3-ispropyl-2-oxo-1-azetidinyl)-2-hydroxy-essigsäure-p-nitrobenzylester
(racemische trans-Verbindung)

Bei Raumtemperatur wird eine Lösung von 137 mg (0,55 mM) 4-Äthylthiothiocarbonylthio-3-isopropyl-2-oxoazetidin (racemische trans-Verbindung) in 8 ml Toluol und 2 ml Dimethylformamid mit 311 mg 2-Äthoxy-2-hydroxy-essigsäure-p-nitrobenzylester versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an 80 g Silikagel mit Toluol-Äthylacetat 9:1 chromatographiert. Man erhält die beiden mit nicht umgesetztem 2-Äthoxy-2-hydroxy-essigsäure-p-nitrobenzylester etwas verunreinigten trans-Isomeren der Titelverbindung mit den folgenden physikochemischen Eigenschaften: DC: Rf = 0,4 (Toluol-Äthylacetat 2:3); IR-Spektrum ($CH_2Cl_2$): Absorptionsbanden bei 5,62; 5,7; 6,55; 7,42; 8,2; 9,2 μ.

Beispiel 24:
2-(4-Äthylthiothiocarbonylthio-3-isopropyl-2-oxo-1-azetidinyl)-2-triphenylphosphoranyliden-essigsäure-p-nitrobenzylester
(racemische trans-Verbindung)

Eine Lösung von 606 mg 2-(4-Äthylthio-thiocarbonylthio-3-isopropyl-2-oxo-1-azetidinyl)-2-hydroxy-essigsäure-p-nitrobenzylester (racemische trans-Verbindung) in 6 ml absolutem Tetrahydrofuran wird auf −15° gekühlt, unter Rühren mit 0,16 ml (2,23 mM) Thionylchlorid und anschliessend langsam mit einer Lösung von 0,31 ml Triäthylamin in 0,3 ml absolutem Tetrahydrofuran versetzt. Das Reaktionsgemisch wird eine Stunde bei 0° gerührt, mit 30 ml kaltem Methylenchlorid versetzt und mit eiskalter 2N Salzsäure gewaschen. Die organische Phase wird mit Wasser bis zur neutralen Reaktion gewaschen, mit Natriumsulfat getrocknet und im Vakuum eingedampft. Der erhaltene rohe 2-(4-Äthylthiothiocarbonylthio-3-isopropyl-2-oxo-1-azetidinyl)-2-chlor-essigsäure-p-nitrobenzylester wird in 1,5 ml trockenem Tetrahydrofuran gelöst, mit 0,71 g Triphenylphosphin versetzt und in einer Stickstoffatmosphäre bei Raumtemperatur über Nacht gerührt. Die Reaktionsmischung wird mit Methylenchlorid verdünnt, nacheinander mit gesättigter wässriger Natriumhydrogencarbonatlösung und Wasser gewaschen, über Natriumsulfat getrocknet und im Vakuum eingedampft. Der Rückstand ergibt nach Chromatographie an Silikagel mit Toluol-Äthylacetat 9:1 die Titelverbindung. DC: Rf = 0,5 (Toluol-Äthylacetat 2:3); IR-Spektrum ($CH_2Cl_2$): Absorptionsbanden bei 3,4; 5,7; 6,15; 6,55; 7,45; 9,05; 9,25 μ.

Beispiel 25:
2-Äthylthio-6-isoproyl-2-penem-3carbonsäure-p-nitrobenzylester
(racemische trans-Verbindung)

Eine Lösung von 600 mg (0,855 mM) 2-[4-Äthylthiothiocarbonylthio-3-isopropyl-2-oxo-1-azetidinyl]-2-triphenylphosphoranyliden-essigsäure-p-nitrobenzylester (racemische trans-Verbindung) in 250 ml absolutem o-Xylol wird mit einer katalytischen Menge p-Hydroxychinon versetzt und unter Stickstoff 48 Stunden unter Rückfluss gerührt. Das Lösungsmittel wird im Vakuum abgedampft und der Rückstand an 35 g Silikagel mit Toluol-Äthylacetat 19:1 chromatographiert. Man erhält die trans-Titelverbindung nach Kristallisation aus Diäthyläther-Methylenchlorid in Form farbloser Kristalle; DC: Rf = 0,62 (Toluol-Äthylacetat 2:3): IR-Spektrum ($CH_2Cl_2$): Absorptionsbanden bei 5,57; 5,9; 6,55; 7,4; 7,52 μ.

Beispiel 26:
2-Äthylthio-6-isopropyl-2-penem-3-carbonsäure (racemische trans-Verbindung)

Eine Lösung von 100 mg 2-Äthylthio-6-isopropyl-2-penem-3-carbonsäure-p-nitrobenzylester (racemische trans-Verbindung) in 6 ml absolutem Äthylacetat wird mit 4 ml 0,2 N wässriger Natriumhydrogencarbonatlösung und 150 mg 10%-igem Palladium/Kohle Katalysator versetzt und bei Normaldruck während 240 Minuten unter Wasserstoff gerührt. Die Hydriermischung wird über Diatomeenerde vom Katalysator abfiltriert, mit 0,2 N Natriumhydrogencarbonatlösung und mehrere Male mit Äthylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: D: Rf = 0,35 (Toluol-Äthylacetat-Essigsäure 60:40:5); IR-Spektrum (KBr): Absorptionsbanden bei 3,5, 5,62, 6,0, 6,75, 6,9, 7,52, 7,9, 8,15, 8,9 μ.

Beispiel 27:
6-Diazo-penicillansäure-methylester

Analog der Deutschen Offenlegungsschrift Nr. 2.305.972 werden 1,01 g roher 6β-(N-Nitroso)-phenoxyacetamido-penicillansäure-methylester (hergestellt gemäss USP 3.880.873) bei Zimmertemperatur in 75 ml absolutem Chloroform gelöst und nach Zugabe von 200 ml gesättigter wässriger Natriumbicarbonatlösung während 9 Stunden zwischen 10 und 20° gerührt. Die Chloroformlösung wird abgetrennt, mit Wasser gewaschen und über Natriumsulfat getrocknet. Nach Abdampfen des Lösungsmittels im Vakuum bei Raumtemperatur wird die rohe Diazoverbindung in Form eines Öles erhalten. Sie kann ohne weitere Reinigung in die nächste Reaktion eingesetzt werden. IR-Spektrum (in Methylenchlorid: charakteristische Absorptionsbanden bei 3,40; 4,80; 5,55; 5,70; 6,23; 6,50; 6,68; 7,75; 8,22; 8,85; 10,6; 11,42 μ.

Beispiel 28:

6α-Methoxy-penicillansäure-methylester

Eine Lösung von 2 g rohem 6-Diazo-penicillansäure-methylester in 15 ml absolutem Methylenchlorid wird mit 5 ml Methanol und wenigen Tropfen 30%-iger wässriger Perchlorsäure versetzt und 15 Minuten während Raumtemperatur gerührt. Die Reaktionsmischung wird mit 30 ml Methylenchlorid verdünnt und nacheinander mit wässriger Natriumbicarbonatlösung, Wasser und Kochsalzlösung gewaschen, über Natriumsulfat getrocknet und im Vakuum eingedampft. Der Rückstand wird an Silicagel mit Toluol-Äthylacetat 9:1 und 4:1 chromatographiert und ergibt die leicht verunreinigte Titelverbindung. IR-Spektrum (in Methylenchlorid): charakteristische Banden bei 3,40; 5,63; 5,70; 6,90; 7,30; 7,68; 8,25; 8,47; 8,90; 9,15; 9,70; 9,86 μ.

Beispiel 29:

6α-Methoxy-penicillansäure-methylester-1-oxid

Eine Lösung von 473 mg 6α-Methoxy-penicillansäure-methylester in 10 ml Methylenchlorid wird auf 0° gekühlt, mit 334 mg m-Chlorperbenzoesäure versetzt und die erhaltene Suspension während 1 Stunde bei der gleichen Temperatur weitergerührt. Die Reaktionsmischung wird mit 50 ml Methylenchlorid verdünnt, zweimal mit wässriger Natriumbicarbonatlösung und mit Wasser gewaschen und über Natriumsulfat getrocknet. Das Lösungsmittel wird im Vakuum abgedampft und der Rückstand an Silicagel chromatographiert. Mit Toluol-Äthylacetat 9:1 und 4:1 wird die Titelverbindung in Form eines weissen Pulvers gewonnen. Eine analytische Probe wird aus Methylenchlorid-Diäthyläther-Pentan umkristallisiert und hat folgende physiko-chemischen Eigenschaften: F. 121°; $\alpha_D = +281° \pm 1°$; IR-Spektrum (in Methylenchlorid): charakteristische Absorptionsbanden bei 3,40; 5,58; 5,70; 6,85; 6,97; 7,75; 8,20; 8,90; 9,45 μ.

Beispiel 30:

2-[(3S,4R)-4-(Benzthiazol-2-yldithio)-3-methoxy-2-oxazetidin-1-yl]-3-methylenbuttersäure-methylester

Eine Lösung von 307 mg 6α-Methoxy-penicillansäure-methylester-1-oxid wird in 10 ml Toluol gelöst, mit 196,57 mg 2-Mercaptobenzthiazol versetzt und während 90 Minuten am Rückfluss erhitzt. Das Lösungsmittel wird im Vakuum abdestilliert und der Rückstand an Silicagel chromatographiert. Durch Elution mit Toluol-Äthylacetat 9:1 wird die Titelverbindung in Form eines farblosen Öles gewonnen. IR-Spektrum (in Methylenchlorid): charakteristische Banden bei 3,40; 5,62; 5,72; 6,68; 6,85; 7,02; 7,25; 7,50; 8,10; 8,20; 8,60; 8,95; 9,55; 9,92; 10,92 μ.

Beispiel 31:

2-[(3S,4R)-4-(Benzthiazol-2-yldithio)-3-methoxy-2-oxoazetidin-1-yl]-3-methylcrotonsäure-methylester

Eine Lösung von 432 mg 2-[(3S,4R)-4-(Benzthiazol-2-yldithio)-3-methoxy-2-oxoazetidin-1-yl]-3-methylenbuttersäure-methylester in 25 ml Methylenchlorid wird mit 0,1 ml Triäthylamin versetzt und bei Raumtemperatur 100 Minuten gerührt. Die Reaktionsmischung wird mit Methylenchlorid verdünnt, zweimal mit wässriger Zitronensäurelösung und Wasser gewaschen, über Natriumsulfat getrocknet und im Vakuum vom Lösungsmittel befreit. Der Rückstand wird durch Chromatographie an Silicagel mit Toluol-Äthylacetat 9:1 und 4:1 gereinigt und ergibt die Titelverbindung in Form eines Öles. IR-Spektrum (in Methylenchlorid): charakteristische Absorptionsbanden bei 3,40; 5,63; 5,78; 6,85; 7,03; 7,23; 7,32; 7,0; 8,15; 8,87; 9,00; 9,25; 9,92 μ.

Beispiel 32:

2-[(3S,4R)-4-Acetylthio-3-methoxy-2-oxo-azetidin-1-yl]-3-methylcrotonsäure-methylester

Eine Lösung von 372 mg 2-[(3S,4R)-4-(Benzthiazol-2-yldithio)-3-methoxy-2-oxoazetidin-1-yl]-3-methylcrotonsäure-methylester in 10 ml Dimethylformamid wird auf −20° gekühlt, mit 10 ml einer Lösung von 2 g Natriumborhydrid in 200 ml Dimethylformamid versetzt und bei der gleichen Temperatur 30 Minuten gerührt. Die Reaktionsmischung wird mit 5 ml frisch destilliertem Acetylbromid versetzt und bei 0° während 110 Minuten weiter gerührt. Nach Zugabe von 150 ml Benzol wird die Reaktionsmischung nacheinander mit Natriumcarbonatlösung und Wasser gewaschen, über Natriumsulfat getrocknet und im Vakuum eingedampft. Der Rückstand ergibt nach Chromatographie an Silicagel mit Toluol-Äthylacetat 9:1 die Titelverbindung in Form eines leicht gelblichen Öles. IR-Spektrum (in Methylenchlorid): charakteristische Absorptionsbanden bei 3,40; 5,63; 5,77; 5,83; 6,10; 6,95; 7,20; 7,30; 7,70; 8,12; 8,90; 9,20; 9,90; 10,20; 10,50; 11,83 μ.

Beispiel 33:

2-[(3S,4R)-4-Acetylthio-3-methoxy-2-oxo-azetidin-1-yl]-2-oxoessigsäure-methylester

Durch eine auf −30° gekühlte Lösung von 87 mg (0,31 mM) 2-[(3S,4R)-4-Acetylthio-3-methoxy-2-oxoazetidin-1-yl]-3-methylcrotonsäure-methylester in 5 ml Äthylacetat werden 3 Äquivalente Ozon geleitet. Die Reaktionsmischung wird mit 30 ml Methylenchlorid verdünnt und während 2 Minuten mit einer 10%-igen wässrigen Natriumbisulfitlösung geschüttelt. Die organische Phase wird abgetrennt, mit gesättigter Natriumchloridlösung gewaschen, über Natriumsulfat getrocknet und im Vakuum eingedampft. IR-Spektrum der erhaltenen öligen Titelverbindung (in Methylenchlorid): charakteristische Banden bei 3,40; 5,47; 5,67; 5,82; 6,97; 7,33; 8,10; 8,92; 9,88; 10,40μ. Das erhaltene Produkt kann ohne weitere Reinigung in die nächste Stufe eingesetzt werden.

Beispiel 34:
(3S,4R)-4-Acetylthio-3-methoxy-2-oxoazetidin

Eine Lösung von
71,20 mg 2-[(3S,4R)-4-Acetylthio-3-methoxy-2-oxoazetidin-1-yl]-2-oxoessigsäure-methylester
(Rohprodukt) in 10 ml 1%-igem wässrigem Methanol wird über Nacht bei Raumtemperatur gerührt. Die Reaktionsmischung wird mit Methylenchlorid verdünnt, mit Wasser gewaschen, über Natriumsulfat getrocknet und im Vakuum eingedampft. Der Rückstand wird an Silicagel mit Toluol-Äthylacetat 9:1 chromatographiert und ergibt die Titelverbindung. IR-Spektrum (in Methylenchlorid): charakteristische Absorptionsbanden bei 2,95; 3,40; 5,60; 5,88; 7,00; 7,37; 7,52; 8,25; 8,70; 8,85; 10,5; 12,15 μ.

Beispiel 35:
2-[(3S,4R)-4-Acetylthio-3-methoxy-2-oxo-azetidin-1-yl]-2-hydroxyessigsäure-p-nitrobenzylester

Eine Lösung von 245 mg (3S,4R)-4-Acetylthio-3-methoxy-2-oxoazetidin in einer Mischung von 8 ml Toluol und 2 ml Dimethylformamid wird mit 714 mg 2-Äthoxy-2-hydroxy-essigsäure-p-nitrobenzylester und 4 g Molekularsieb A4 versetzt und bei Raumtemperatur über Nacht gerührt. Von den Molekularsieben wird abfiltriert und das Filtrat im Vakuum eingedampft. Der Rückstand wird an Silicagel chromatographiert, wobei durch Elution mit Toluol-Äthylacetat 9:1 und 4:1 die Titelverbindung, verunreinigt mit etwas Glyoxylat, erhalten wird.

Beispiel 36:
2-[(3S,4R)-4-Acetylthio-3-methoxy-2-oxo-azetidin-1-yl]-2-triphenylphosphoranyliden-essigsäure-p-nitrobenzylester

a) Eine Supsension von 2 g Poly-Hünigbase in 8 ml Dioxan wird 30 Minuten bei Raumtemperatur gerührt, mit
832 mg 2-[(3S, 4R)-4-Acetylthio-3-methoxy-2-oxoazetidin-1-yl]-2-hydroxyessigsäure-p-nitrobenzylester gelöst in 12 ml Dioxan und anschliessend langsam mit einer Lösung von 0,54 ml Thionylchlorid in 10 ml Dioxan versetzt. Die Mischung wird 2 Stunden bei Raumtemperatur gerührt, von der Poly-Hünigbase abfiltriert und das Filtrat im Vakuum eingedampft. Der Rückstand wird durch Chromatographie an Silicagel mit Toluol-Äthylacetat 1:1 gereinigt und ergibt den 2-[(3S, 4R)-4-Acetylthio-3-methoxy-2-oxoazetidin-1-yl]-2-chloressigsäure-p-nitrobenzylester in roher Form.

b) Eine Lösung von
833 mg 2-[(3S,4R)-4-Acetylthio-3-methoxy-2-oxo-azetidin-1-yl]-2-chloressigsäure-p-nitrobenzylester
in 50 ml Dioxan wird mit 812 mg Triphenylphosphin und 3 g Poly-Hünigbase versetzt und über Nacht bei 50° gerührt. Die Poly-Hünigbase wird durch Filtration entfernt und das Filtrat im Vakuum eingedampft. Der Rückstand wird an Silicagel mit Toluol-Äthylacetat 9:1, 4:1 und 1:1, chromatographiert und ergibt die Titelverbindung. IR-Spektrum (in Methylenchlorid): charakteristische Absorptionsbanden bei 3,40; 5,67; 5,85; 6,15; 6,55; 6,97; 7,42; 8,0; 9,03 μ.

Beispiel 37:
(5R,6S)-2-Methyl-6-methoxy-2-penem-3-carbonsäure-p-nitrobenzylester

Eine Lösung von 244 mg 2-[(3S,4R)-4-Acetylthio-3-methoxy-2-oxoazetidin-1-yl]-2-triphenylphosphoranylidenessigsäure-p-nitrobenzylester in 100 ml absolutem Toluol wird mit einer katalytischen Menge Hydrochinon versetzt und 32 Stunden bei 90° unter Stickstoff gerührt. Das Toluol wird im Vakuum abgedampft und der Rückstand an Silicagel mit Toluol-Äthylacetat 19:1 chromatographiert. Die Titelverbindung wird in Form einer weissen festen Substanz erhalten. IR-Spektrum (in Methylenchlorid): charakteristische Absorptionsbanden bei 3,40; 5,55; 6,30; 6,55; 7,03; 7,42; 7,60; 8,20; 8,42; 8,60; 8,90; 9,20; 9,60; 11,7 μ; NMR-Spektrum ($CDCl_3$/100 Mc; in ppm): 8,22, 2H, d, J = 8 Hz; 7,64, 2H, d, J = 8 Hz; 1H, d, J = 2 Hz; 5,35, 2H, AB; 4,91, 1H, d, J = 2 Hz; 3,57, 3H, s; 2,37, 3H, s.

Beispiel 38:
(5R,6S)-2-Methyl-6-methoxy-2-penem-3-carbonsäure

Eine Lösung von 34 mg (5R,6S)-2-Methyl-6-methoxy-2-penem-3-carbonsäure-p-nitrobenzylester in einer Mischung von 2 ml Äthylacetat und 2 ml 2M-Natriumbicarbonatlösung wird mit 75 mg 10% Palladium/Kohle-Katalysator versetzt und unter Atmosphärendruck während 1,5 Stunden bei Raumtemperatur hydriert. Die Hydriermischung wird durch Diatomeenerde filtriert, der Filterrückstand mit 1 ml 2 M wässriger Natriumbicarbonatlösung und Äthylacetat gewaschen. Vom Filtrat wird die wässrige Phase abgetrennt, mit 0,1 M wässriger Zitronensäure angesäuert und mehrere Male mit Methylenchlorid extrahiert. Die vereinigten Methylenchloridextrakte werden über Natriumsulfat getrocknet und im Vakuum eingedampft. IR-Spektrum (in Methylenchlorid) der erhaltenen rohen Titelverbindung: charakteristische Absorptionsbanden bei 3,40; 5,57; 5,80; 5,95; 6,30; 7,00; 8,20; 9,90 μ.

Beispiel 39:
6α-Phenoxyacetoxy-penicillansäure-methylester

Analog D. Hauser und H.P. Sigg, Helv. Chim. Acta 50, 1327 (1967) wird eine Lösung von 7,4 g (20,3 mM) 6β-(N-Nitroso)-phenoxyacetamido-penicillansäure-methylester (Rohprodukt gemäss USP 3.880.837) in 100 ml Benzol 3 Stunden bei 50° unter Stickstoffatmosphäre gerührt. Das Lösungsmittel wird im Vakuum abgedampft und der Rückstand an Silicagel mit Toluol-Äthylacetat 9:1 chromatographiert. Das erhaltene ölige Produkt wird aus Diäthyläther-Hexan umkristallisiert und ergibt die Titelverbindung vom Schmelzpunkt 71°; $\alpha_D = 114 \pm 1°$ ($CHCl_3$); IR-Spektrum (in Methylenchlorid): charakteristische Absorptionsbanden bei 3,4; 5,6; 5,7; 6,25; 6,69; 7,17; 8,26; 8,55; 9,05; 9,18 μ.

Beispiel 40:
6α-Phenoxyacetoxy-penicillansäure-methylester-1-oxid

Eine Lösung von 1,16 g (3,18 mM) 6α-Phenoxyacetoxy-penicillansäure-methylester in 30 ml absolutem Methylenchlorid wird bei 0° portionenweise mit 1,1 g (1 Äquivalent) 50%-iger m-Chlorperbenzoesäure versetzt. Nach Beendigung der Zugabe wird die Reaktionsmischung 30 Minuten bei 0° gerührt, dann mit Methylenchlorid verdünnt, nacheinander mit wässriger Natriumbicarbonatlösung, Wasser und Kochsalzlösung gewaschen und über Natriumsulfat getrocknet. Nach Abdampfen des Lösungsmittels wird der Rückstand an Silicagel mit Toluol-Äthylacetat 4:1 chromatographiert. Man erhält die Titelverbindung in Form eines Schaumes; DC: Rf = 0,24 (Toluol-Äthylacetat 1:1), IR-Spektrum (in Methylenchlorid): charakteristische Absorptionsbanden bei 3,33; 3,41; 5,57; 5,72; 6,27; 6,72; 7,0; 8,25; 8,6; 9,21; 9,46 μ.

Beispiel 41:
2-[(3S,4R)-4-(Benzthiazol-2-yldithio)-3-phenoxyacetoxy-2-oxoazetidin-1-yl]-3-methylenbuttersäure-methylester

Eine Lösung von 1,01 g (2,65 mM) 6α-Phenoxyacetoxy-penicillansäure-methylester-1-oxid wird in 30 ml Toluol gelöst, mit 445 mg (2,65 mM) 2-Mercaptobenzthiazol versetzt und während 60 Minuten in einer Stickstoffatmosphäre am Rückfluss erhitzt. Das Lösungsmittel wird im Vakuum abdestilliert und der Rückstand an Silicagel chromatographiert. Durch Elution mit Toluol-Äthylacetat 19:1 wird die Titelverbindung in Form eines schwach-braunen Öles gewonnen. IR-Spektrum (in Methylenchlorid): charakteristische Banden bei 3,45; 5,62; 5,75; 6,27; 6,71; 6,89; 7,05; 7,30; 7,54; 7,68; 8,15; 8,55; 9,15; 9,35; 9,95 μ; DC: Rf = 0,63 (Toluol-Äthylacetat 1:1).

Beispiel 42:
2-[(3S,4R)-4-(Benzthiazol-2-yldithio-3-phenoxyacetoxy-2-oxoazetidin-1-yl]-3-methylcrotonsäure-methylester

Eine Lösung von 1,28 g (2,41 mM) 2-[(3S,4R)-4-(Benzthiazol-2-yldithio)-3-phenoxyacetoxy-2-oxoazetidin-1-yl]-3-methylenbuttersäure-methylester in 30 ml Methylenchlorid wird mit 0,4 ml Triäthylamin versetzt und bei Raumtemperatur 30 Minuten gerührt. Die Reaktionsmischung wird mit 50 ml Methylenchlorid verdünnt, nacheinander mit 2N Salzsäure, Wasser und Kochsalzlösung gewaschen, über Natriumsulfat getrocknet und im Vakuum vom Lösungsmittel befreit. Der Rückstand wird durch Chromatographie an Silicagel mit Toluol-Äthylacetat 19:1 gereinigt und ergibt die Titelverbindung in Form eines schwachgelben Öles. IR-Spektrum (in Methylenchlorid): charakteristische Absorptionsbanden bei 3,46; 5,69; 5,82; 5,90; 6,28; 6,73; 6,90; 7,06; 7,28; 7,38; 7,75; 8,20; 8,60; 9,27; 9,96 μ; DC: Rf = 0,61 (Toluol-Äthylacetat 1:1).

Beispiel 43:
2-[(3S,4R)-4-Acetylthio-3-phenoxyacetoxy-2-oxoazetidin-1-yl]-3-methylcrotonsäure-methylester

Eine Lösung von 687 mg (1,29 mM) 2-[(3S,4R)-4-(Benzthiazol-2-yldithio)-3-phenoxyacetoxy-2-oxoazetidin-1-yl]-3-methylcrotonsäure-methylester in 14 ml Dimethylformamid wird zu einer auf −20° gekühlten Lösung von 76 mg (2 mM) Natriumborhydrid in 10 ml Dimethylformamid gegeben und bei der gleichen Temperatur 10 Minuten gerührt. Die Reaktionsmischung wird mit 7 ml frisch destilliertem Acetylbromid versetzt und bei 0° während 40 Minuten weiter gerührt. Nach Zugabe von 400 ml Benzol wird die Reaktionsmischung nacheinander mit wässriger Natriumbicarbonatlösung, Wasser und Kochsalzlösung gewaschen, über Natriumsulfat getrocknet und im Vakuum eingedampft. Der Rückstand ergibt nach Chromatographie an Silicagel mit Toluol-Äthylacetat 19:1 die Titelverbindung in Form eines Öles, das auf Silicagelplatten mit Toluol-Äthylacetat 4:1 weiter gereinigt wird. Man erhält die Titelverbindung in öliger Form; IR-Spektrum (in Methylenchlorid): charakteristische Absorptionsbanden bei 3,45; 5,63; 5,83; 6,27; 6,70; 7,00; 7,25; 7,35; 8,15; 8,58; 8,93; 9,20 μ; DC: Rf = 0,54 (Toluol-Äthylacetat 1:1).

Beispiel 44:
2-[(3S,4R)-4-Acetylthio-3-phenoxyacetoxy-2-oxoazetidin-1-yl]-2-oxoessigsäure-methylester

Durch eine auf −20° gekühlte Lösung von 170 mg (0,42 mM) 2-[(3S,4R)-4-Acetylthio-3-phenoxyacetoxy-2-oxoazetidin-1-yl]-3-methylcrotonsäure-methylester in 5 ml Äthylacetat werden 4 Äquivalente Ozon geleitet. Die Reaktionsmischung wird mit 5 ml Äthylacetat verdünnt und intensiv mit einer 10%-igen wässrigen Natriumsulfitlösung geschüttelt. Die organische Phase wird abgetrennt, mit Wasser und gesättigter Natriumcloridlösung gewaschen, über Natriumsulfat getrocknet und im Vakuum eingedampft. IR-Spektrum der erhaltenen öligen Titelverbindung (in Methylenchlorid): charakteristische Banden bei 3,38; 5,48; 5,63; 5,70; 5,83; 6,27; 6,70; 7,00; 7,40; 8,07; 8,25; 8,63; 8,95 μ. Das erhaltene Produkt kann ohne weitere Reinigung in die nächste Stufe eingesetzt werden.

Beispiel 45:
(3S,4R)-4-Acetylthio-3-phenoxyacetoxy-2-oxoazetidin

Eine Lösung von 129 mg (0,34 mM) (3S,4R)-2-(4-Acetylthio-3-phenoxyacetoxy-2-oxoazetidin-1-yl)-2-oxoessigsäure-methylester (Rohprodukt) in 10 ml 1%-igem wässrigem Methanol wird 4 Stunden bei Raumtemperatur gerührt. Die Reaktionsmischung wird mit 50 ml Methylenchlorid verdünnt, nacheinander mit Wasser und gesättigter Kochsalzlösung gewaschen, über Natriumsulfat getrocknet und im Vakuum eingedampft. Der Rückstand wird an Silicagel mit Toluol-Äthylacetat 9:1 chromatographiert und ergibt die Titelverbindung. IR-Spektrum (in Methylenchlorid): charakteristische Banden bei 2,95; 3,45; 5,55; 5,60; 5,88; 6,25; 6,68; 8,33; 8,85 μ; DC: Rf = 0,36 (Toluol-Äthylacetat 1:1).

Beispiel 46:
2-[(3S,4R)-4-Acetylthio-3-phenoxyacetoxy-2-oxoazetidin-1-yl]-2-hydroxyessigsäure-p-nitrobenzylester

Eine Lösung von 283 mg (3S,4R)-4-Acetylthio-3-methoxy-2-oxoazetidin in einer Mischung von 8 ml Toluol und 2 ml Dimethylformamid wird mit 760 mg 2-Äthoxy-2-hydroxy-essigsäure-p-nitrobenzylester und 4 g Molekularsieb A4 versetzt und bei Raumtemperatur über Nacht gerührt. Von den Molekularsieben wird abfiltriert und das Filtrat im Vakuum eingedampft. Der Rückstand wird an Silicagel chromatographiert, wobei durch Elution mit Toluol-Äthylacetat 9:1 und 4:1 die Titelverbindung verunreinigt mit etwas Glyoxylat, erhalten wird.

Beispiel 47:
2-[(3S,4R)-4-Acetylthio-3-phenoxyacetoxy-2-oxoazetidin-1-yl]-2-triphenylphosphoranyliden-essigsäure-p-nitrobenzylester

a) Eine Suspension von 2 g Poly-Hünigbase in 8 ml Dioxan wird 30 Minuten bei Raumtemperatur gerührt, mit 962 mg 2-[(3S,4R)-4-Acetylthio-3-phenoxyacetoxy-2-oxoazetidin-1-yl]-2-hydroxyessigsäure-p-nitrobenzylester gelöst in 10 ml Dioxan und anschliessend langsam mit einer Lösung von 0,38 ml Thionylchlorid in 8 ml Dioxan versetzt. Die Mischung wird 2 Stunden bei Raumtemperatur gerührt, von der Poly-Hünigbase abfiltriert und das Filtrat im Vakuum eingedampft. Der Rückstand wird durch Chromatographie an Silicagel mit Toluol-Äthylacetat 1:1 gereinigt und ergibt den 2-[(3S,4R)-4-Acetylthio-3-methoxy-2-oxoazetidin-1-yl]-2-chloressigsäure-p-nitrobenzylester in roher Form.

b) Eine Lösung von
960 mg 2-[(3S,4R)-4-Acetylthio-3-phenoxyacetoxy-2-oxoazetidin-1-yl]-2-chlor-essigsäure-p-nitrobenzylester
in 40 ml Dioxan wird mit 786 mg Triphenylphosphin und 3 g Poly-Hünigbase versetzt und über Nacht bei 50° unter Stickstoff gerührt. Die Poly-Hünigbase wird durch Filtration entfernt und das Filtrat im Vakuum eingedampft. Der Rückstand wird an Silicagel mit Toluol-Äthylacetat 9:1, 4:1 und 1:1, chromatographiert und ergibt die Titelverbindung. IR-Spektrum (in Methylenchlorid): charakteristische Absorptionsbanden bei 5,7; 5,9; 6,17; 6,55; 7,45 µ.

Beispiel 48:
(5R,6S)-2-Methyl-6-phenoxyacetoxy-2-penem-3-carbonsäure-p-nitrobenzylester

Eine Lösung von
285 mg 2-[(3S,4R)-4-Acetylthio-3-phenoxyacetoxy-2-oxoazetidin-1-yl]-2-triphenylphosphoranylidenessigsäure-p-nitrobenzylester
in 100 ml absolutem Toluol wird mit einer katalytischen Menge Hydrochinon versetzt und 35 Stunden bei 90° unter Stickstoff gerührt. Das Toluol wird im Vakuum abgedampft und der Rückstand an Silicagel mit Toluol-Äthylacetat 19:1 chromatographiert. Die Titelverbindung wird in Form eines Öles erhalten. IR-Spektrum (in Methylenchlorid): charakteristische Absorptionsbanden bei 5,55; 6,30; 6,55; 7,42 µ.

Beispiel 49:
(5R,6S)-2-Methyl-6-phenoxyacetoxy-2-penem-3-carbonsäure

Eine Lösung von
45 mg (5R,6S)-2-Methyl-6-phenoxyacetoxy-2-penem-3-carbonsäure-p-nitrobenzylester
in einer Mischung von 2 ml Äthylacetat und 2 ml 2M-Natriumbicarbonatlösung wird mit 75 mg 10% Palladium/Kohle-Katalysator versetzt und unter Atmosphärendruck während 1,5 Stunden bei Raumtemperatur hydriert. Die Hydriermischung wird durch Diatomeenerde filtriert, der Filterrückstand mit 1 ml 2M wässriger Natriumbicarbonatlösung und Methylacetat gewaschen. Vom Filtrat wird die wässrige Phase abgetrennt, mit 0,1 M wässriger Zitronensäure angesäuert und mehrere Male mit Methylenchlorid extrahiert. Die vereinigten Methylenchloridextrakte werden über Natriumsulfat getrocknet und im Vakuum eingedampft. IR-Spektrum (in Äthanol) der erhaltenen rohen Titelverbindung: charakteristische Absorptionsbande bei 5,6 µ; UV-Spektrum (in Äthanol): $\lambda_{max} = 305$ nm.

Beispiel 50:
6α-Methoxy-penicillansäure-2,2,2-trichloräthylester-1-oxid

Eine Lösung von 2 g 6α-Methoxy-penicillansäure-2,2,2-trichloräthylester (hergestellt nach P.J. Giddins, D.I. Johns, E.J. Thomas; T.L. 11, 995, 1978) in 100 ml Methylenchlorid und 0,3 ml Aceton wird auf −15 °C gekühlt, innert 5 Minuten mit 1 ml 40 prozentiger Peressigsäure versetzt und 15 Minuten bei der gleichen Temperatur gerührt. Anschliessend wird die Reaktionsmischung mit 15 ml einer 0,1 N Natriumthiosulfatlösung versetzt. Die organische Lösung wird abgetrennt und zweimal mit Eiswasser gewaschen. Das Lösungsmittel wird nach Trocknen über Natriumsulfat im Vakuum eingedampft und der Rückstand aus Äther-Petroläther umkristallisiert. Die erhaltende Verbindung hat folgende physiko-chemischen Eigenschaften: F = 127–128°. IR-Spektrum (in Methylenchlorid): charakteristische Absorptionsbanden bei 3,41; 5,58; 5,65; 8,33; 8,47; 8,70; 9,48 µ.

Beispiel 51:
2-[(3S,4R)-4-(Benzthiazol-2-yldithio)-3-methoxy-2-oxo-azetidin-1-yl]-3-methylenbuttersäure-2,2,2-trichloräthylester

Eine Lösung von 3 g 6α-Methoxy-penicillansäure-2,2,2-trichloräthylester-1-oxid in 40 ml absolutem Toluol wird mit 1,39 g 2-Mercaptobenzthiazol versetzt und während 105 Minuten unter Stickstoff am Rückfluss erhitzt. Das Lösungsmittel wird im Vakuum abdestilliert und liefert die Titelverbindung in Form eines gelblichen Öles. IR-Spektrum (in Methylenchlorid): charakteristische Absorptionsbanden bei 3,39; 5,60; 5,65; 6,85; 8,20; 8,62; 8,97; 9,85; 9,95 µ. Das erhaltene Produkt kann ohne weitere Reinigung in die nächste Stufe eingesetzt werden.

Beispiel 52:
2-[(3S,4R)-4-(Benzthiazol-2-yldithio)-3-methoxy-2-oxoazetidin-1-yl]-3-methylcrotonsäure-2,2,2-trichloräthylester

Eine Lösung von 4,17 g 2-[(3S,4R)-4-(Benzthiazol-2-yldithio)-3-methoxy-2-oxoazetidin-1-yl]-3-methylen-buttersäure-2,2,2-trichloräthylester in 75 ml absolutem Methylenchlorid wird bei 0° mit 0,78 ml Triäthylamin versetzt und bei dieser Temperatur 15 Minuten gerührt. Das Reaktionsgemisch wird nacheinander mit 4N Phosphorsäure, gesättigter wässriger Natriumbicarbonatlösung und Sole gewaschen und über Natriumsulfat getrocknet. Das Lösungsmittel wird abgedampft und der Rückstand durch Chromatographie an Silicagel mit Toluol und Toluol-Äthylacetat 19:1 gereinigt. Die Titelverbindung wird in Form eines Öles erhalten. IR-Spektrum (in Methylenchlorid): charakteristische Absorptionsbanden bei 3,39; 5,62; 5,76; 6,85; 7,04; 7,25; 6,85; 9,01; 9,48; 9,85; 9,95 μ.

Beispiel 53:
2-[(3S,4R)-4-Acetylthio-3-methoxy-2-oxoazetidin-1-yl]-3-methylcrotonsäure-2,2,2-trichloräthylester

Eine Lösung von 3,26 g 2-[(3S,4R)-4-(Benzthiazol-2-yldithio)-3-methoxy-2-oxoazetidin-1-yl]-3-methylcrotonsäure-2,2,2-trichloräthylester in 36,3 ml Acetanhydrid und 12,4 ml Essigsäure wird auf −15° gekühlt und mit 1,7 g Triphenylphosphin versetzt. Nach 75 Minuten Rühren unter Stickstoff bei der gleichen Temperatur gibt man dem Gemisch 24,8 ml Pyridin zu. Nach weiteren 3 Stunden Rühren bei 0° wird das Reaktionsgemisch unter vermindertem Druck eingedampft und der erhaltene Rückstand durch Chromatographie an Silicagel mit Toluol und Toluol-Äthylacetat 19:1 gereinigt. IR-Spektrum (in Methylenchlorid): charakteristische Absorptionsbanden bei 3,40; 5,63; 5,77; 5,80; 6,13; 7,25; 7,35; 8,26; 9,0; 9,52; 11,90 μ.

Beispiel 54:
2-[(3S,4R)-4-Acetylthio-3-methoxy-2-oxoazetidin-1-yl]-2-oxoessigsäure-2,2,2-trichloräthylester

Durch eine auf −30° gekühlte Lösung von 8,4 g 2-[(3S,4R)-4-Acetylthio-3-methoxy-2-oxoazetidin-1-yl]-3-methylcrotonsäure-2,2,2-trichloräthylester in 765 ml Methylacetat werden 3 Äquivalente Ozon geleitet. Nach der Ozon-Behandlung wird das Reaktionsgemisch 15 Minuten bei gleicher Temperatur stehengelassen und anschliessend das überschüssige Ozon durch einen Stickstoffstrom entfernt. Das Reaktionsgemisch wird bei 0° mit einer 10%-igen wässrigen Natriumbisulfitlösung und danach mit Sole gewaschen. Nach Abtrennen werden die vereinten wässrigen Phasen noch 4 mal mit Methylacetat extrahiert. Die vereinigten Methylesterlösungen werden über Natriumsulfat getrocknet und im Vakuum eingedampft. IR-Spektrum der erhaltenen öligen Titelverbindung (in Methylenchlorid): charakteristische Absorptionsbanden bei 3,39; 5,48; 5,63; 6,09; 6,94; 7,25; 7,38; 7,46; 8,23; 8,93; 9,90; 11,83 μ.

Beispiel 55:
(3S,4R)-4-Acetylthio-3-methoxy-2-oxoazetidin

a) Eine Lösung von 1,52 g 2-[(3S,4R)-4-Acetylthio-3-methoxy-2-oxoazetidin-1-yl]-2-oxoessigsäure-2,2,2-trichloräthylester (Rohprodukt) in 290 ml Methanol, 40 ml Methylacetat und 5,9 ml Wasser wird während 20 Minuten unter Stickstoff am Rückfluss erhitzt. Das Lösungsmittel wird im Vakuum eingedampft. Der Rückstand ergibt nach Chromatographie an Silicagel mit Toluol/Äthylacetat 3:1 die Titelverbindung. IR-Spektrum (in Methylenchlorid): charakteristische Absorptionsbanden bei 2,95; 3,40; 5,60; 5,88; 7,37; 7,52; 8,25; 8,70; 8,85; 10,5; 12,12 μ.

Die gleiche Verbindung kann auch wie folgt erhalten werden:

b) Eine Lösung von 40 mg (3S,4S)-4-Acetoxy-3-methoxy-2-oxoazetidin (Herstellung siehe unten) in 1,5 ml Phosphatpuffer pH 7 und 0,1 ml Dioxan wird mit 1,5 Äquivalenten einer wässrigen Natriumthioacetat-Lösung versetzt und bei Raumtemperatur 30 Minuten gerührt. Das Reaktionsgemisch wird mit Methylenchlorid extrahiert und die abgetrennte organische Lösung wird anschliessend über Natriumsulfat getrocknet. Das Lösungsmittel wird im Vakuum eingedampft und der Rückstand durch Chromatographie an Silicagel mit Toluol/Äthylacetat 3:1 gereinigt. Das IR-Spektrum der so erhaltenen Titelverbindung (in Methylenchlorid) ist identisch mit demjenigen des gemäss a) erhaltenen Produktes.

Beispiel 56:
2-[(3S,4R)-4-Acetylthio-3-methoxy-2-oxoazetidin-1-yl]-2-hydroxyessigsäure-p-nitrobenzylester

Eine Lösung von 350 mg (3S,4R)-4-Acetylthio-3-methoxy-2-oxoazetidin in einer Mischung von 24 ml absolutem Toluol und 6 ml absolutem Dimethylformamid wird mit 1,15 g 2-Äthoxy-2-hydroxyessigsäure-p-nitrobenzylester und 4 g Molekularsieben A4 versetzt und bei Raumtemperatur unter Stickstoff über Nacht gerührt. Von den Molekularsieben wird abfiltriert und das Filtrat im Vakuum eingedampft. Der Rückstand wird an Silicagel chromatographiert wobei durch Elution mit Toluol und Toluol-Äthylacetat 19:1 die Titelverbindung erhalten wird. IR-Spektrum (in Methylenchlorid): charakteristische Absorptionsbanden bei 2,86; 3,39; 5,60; 5,68; 5,88; 6,21; 6,56; 7,41; 8,26; 9,01; 11,76 μ.

Beispiel 57:
2-[(3S,4R)-4-Acetylthio-3-methoxy-2-oxoazetidin-1-yl]-2-chloressigsäure-p-nitrobenzylester

Eine Lösung von 0,6 g 2-[(3S,4R)-4-Acetylthio-3-methoxy-2-oxoazetidin-1-yl]-2-hydroxyessigsäure-p-nitrobenzylester in 7 ml trockenem Tetrahydrofuran wird auf –15° gekühlt und mit 0,19 ml Thionylchlorid versetzt. Danach gibt man 0,37 ml Triäthylamin in 0,4 ml trockenem Tetrahydrofuran bei gleicher Temperatur tropfenweise dazu. Das Rekationsgemisch wird eine Stunde bei 0° gerührt, mit kaltem Methylenchlorid verdünnt und mit einer eiskalten 2N HCl-Lösung gewaschen. Nach mehrmaligen Ausschütteln mit Wasser wird die Methylenchloridlösung über Natriumsulfat getrocknet und eingedampft. IR-Spektrum (in Methylenchlorid): charakteristische Absorptionsbanden bei 3,41; 5,59; 5,65; 5,88; 6,21; 6,56; 7,41; 8,23; 8,55; 9,05; 10,5; 11,76 µ.

Beispiel 58:
2-[(3S,4R)-4-Acetylthio-3-methoxy-2-oxo-azetidin-1-yl]-2-triphenylphosphoranyliden-essigsäure-p-nitrobenzylester

Eine Lösung von 0,63 g 2–[(3S,4R)-4-Acetylthio-3-methoxy-2-oxoazetidin-1-yl]-2-chloressigsäure-p-nitrobenzylester in 1,8 ml trockenem Tetrahydrofuran wird mit 0,84 g Triphenylphosphin versetzt und über Nacht unter Stickstoff bei Raumtemperatur gerührt. Das Gemisch wird mit Methylenchlorid verdünnt und mit einer kalten, gesättigten, wässrigen Natriumbicarbonatlösung gewaschen. Zusätzliches Waschen mit Wasser, Trocknen über Natriumsulfat und Eindampfen im Vakuum gibt die rohe Titelverbindung die durch Chromatographie an Silicagel mit Toluol-Äthylacetat 19:1 bis 3:1 gereinigt wird. IR-Spektrum (in Methylenchlorid): charakteristische Absorptionsbanden bei 3,40; 5,67; 5,90; 6,20; 6,58; 7,46; 9,05 µ.

Beispiel 59:
(5R,6S)-2-Methyl-6-methoxy-2-penem-3-carbonsäure-p-nitrobenzylester

Eine Lösung von
74 mg 2-[(3S,4R)-4-Acetylthio-3-methoxy-2-oxoazetidin-1-yl]-2-triphenylphosphoranyliden-essigsäure-p-nitrobenzylester
in 30 ml absolutem Toluol wird mit einer katalytischen Menge 3,5-Di-tert.butyl-4-hydroxytoluol versetzt und 3 Stunden unter Stickstoff am Rückfluss erhitzt. Das Toluol wird im Vakuum abgedampft und der Rückstand an Silicagel mit Toluol-Äthylacetat 19:1 chromatographiert. Die Titelverbindung wird in festem Zustand erhalten. IR-Spektrum (in Methylenchlorid): charakteristische Absorptionsbanden bei 3,41; 5,60; 5,85; 6,63; 6,58; 7,41; 7,60; 8,23; 9,26; 11,76 µ.

Beispiel 60:
3-Äthyl-4-(2-acetylaminoäthylthio-thiocarbonylthio)-2-oxoazetidin
(racemische cis,trans-Verbindung)

a) Eine Lösung von 0,78 g (5 mM) 4-Acetoxy-3-äthylazetidin-2-on (racemische Mischung von cis- und trans-Isomer im Verhältnis 6:4) in 2 ml Dioxan wird unter Stickstoffatmosphäre tropfenweise zu einer Lösung von 1,175 g Kalium-(2-acetylaminoäthyl)-trithiocarbonat in 20 ml vorgekühltem pH 7-Phosphatpuffer gegeben und 60 Minuten gerührt. Die Reaktionsmischung wird zentrifugiert, die überstehende klare Lösung abdekantiert und der ölige Rückstand in Methylenchlorid aufgenommen. Die organische Phase wird über Natriumsulfat getrocknet und im Vakuum eingedampft. Der Rückstand wird einmal mit Diäthyläther trituriert und in dieser Form weiterverarbeitet. DC: Rf = 0,16 (Äthylacetat); IR-Spektrum ($CH_2Cl_2$): Absorptionsbanden bei 2,92; 2,97; 5,63; 5,97; 6,62; 9,35 und 12,34 µ.

Die beiden Ausgangsmaterialien können wie folgt hergestellt werden:

b) Zu einer gerührten Lösung von 34,5 g (0,302 Mol) 1-Butenylacetat in 35 ml trockenem Methylenchlorid werden bei −10° innerhalb 30 Minuten 42,7 g (26,3 ml; 0,302 Mol) N-Chlorsulfonylisocyanat getropft. Nach weiteren 4 Stunden Rühren bei 0° wird die Reaktionsmischung mit 50 ml vorgekühltem Methylenchlorid verdünnt und zu einer hydrolysierenden Mischung von 32 ml Wasser, 144 g Eis, 113 g Natriumhydrogencarbonat und 38,2 g wasserfreiem Natriumsulfit getropft. Während der Hydrolyse wird die Temperatur durch äussere Kühlung bei 0° gehalten. Wenn die organische Phase nicht mehr sauer reagiert, wird die Reaktionsmischung mit 100 ml Diäthyläther verdünnt und durch Celite filtriert. Die organische Phase wird abgetrennt, die wässrige Phase mit dreimal 400 ml Diäthyläther extrahiert, die organischen Phasen vereinigt, getrocknet und im Vakuum eingedampft. Der Rückstand wird an Silicagel mit Toluol/Äthylacetat 2:1 chromatographiert und ergibt eine racemische Mischung von cis- und trans-4-Acetoxy-3-äthylazetidin-2-on im Verhältnis 6:4 in öliger Form. IR-Spektrum ($CH_2Cl_2$): Absorptionsbanden bei 2,94; 5,60; 5,75; 7,35; 8,06 und 8,85 µ.

c) Eine Lösung von 1,708 g (14,35 mM) 2-Acetylaminoäthylmercaptan in 2 ml absolutem Äthanol wird während 0,5 Stunden unter Rühren und Kühlen auf 10–15° zu einer Lösung von 0,80 g (14,35 mM) Kaliumhydroxid in 5 ml absolutem Äthanol getropft. Nach einer weiteren halben Stunde wird eine Lösung von 1,09 g (14,35 mM) Schwefelkohlenstoff in 3 ml absolutem Äthanol zugefügt, wobei die Temperatur bei 10–15° gehalten wird. Die Reaktionsmischung wird während 3 Stunden bei Raumtemperatur weitergerührt und während 20 Minuten im Eisbad gekühlt. Der gelbe kristalline Niederschlag wird abfiltriert, einmal mit absolutem Äthanol gewaschen und ergibt das Kalium-(2-acetylaminoäthyl)-trithiocarbonat vom Schmelzpunkt 171–174°. IR-Spektrum (KBr): Absorptionsbanden bei 2,95; 6,18; 6,50; 7,00; 7,32; 7,43; 7,79; 8,33; 9,09 und 11,83 µ.

Beispiel 61:
2-[3-Äthyl-4-(2-acetylaminoäthylthio-thiocarbonylthio)-2-oxo-1-azetidinyl]-2-hydroxy-essigsäure-p-nitrobenzylester
(racemische cis,trans-Verbindung)

Bei Raumtemperatur wird eine Lösung von 3,30 g (11 mM) 3-Äthyl-4-(2-acetylaminoäthylthio-thiocarbonylthio-2-oxo-azetidin (racemische cis, trans-Verbindung) in 120 ml Toluol und 32 ml Dimethylformamid mit 4,20 g (16,5 mM) 2-Äthoxy-2-hydroxy-essigsäure-p-nitrobenzylester versetzt. Nach Zugabe von frisch getrockneten Molekularsieben wird die Mischung unter Stickstoff 3 Stunden bei Raumtemperatur gerührt. Die Molekularsiebe werden abfiltriert, mit 20 ml Toluol gewaschen und Filtrat und Waschflüssigkeit zusammen im Vakuum eingedampft. Der Rückstand wird im Hochvakuum getrocknet und dann mit Diäthyläther trituriert zur Entfernung von nicht umgesetztem 2-Äthoxy-2-hydroxy-essigsäure-p-nitrobenzylester. Man erhält die Titelverbindung mit den folgenden physikochemischen Eigenschaften: DC: Rf = 0,16 (Äthylacetat); IR-Spektrum ($CH_2Cl_2$): Absorptionsbanden bei 2,86; 2,92; 3,03; 5,65; 5,71; 5,97; 6,58; 7,41 und 8,37 μ.

Beispiel 62:
2-[3-Äthyl-4-(2-acetylaminoäthylthio-thiocarbonylthio)-2-oxo-1-azetidinyl]-2-triphenylphosphoranyliden-essigsäure-p-nitrobenzylester
(racemische cis,trans-Verbindung)

Eine Lösung von 5,52 g (11 mM) 2-[3-Äthyl-4-(2-acetylaminoäthylthio-thiocarbonylthio)-2-oxo-1-azetidinyl]-2-hydroxyessigsäure-p-nitrobenzylester (racemische cis,trans-Verbindung) in 30 ml absolutem Tetrahydrofuran wird auf −15° gekühlt, unter Rühren mit 1,02 ml (14 mM) Thionylchlorid und anschliessend langsam mit 1,95 ml (14 mM) Triäthylamin versetzt. Das Reaktionsgemisch wird 20 Minuten bei 0° gerührt, mit 150 ml Methylenchlorid versetzt und mit eiskalter 1N Salzsäure gewaschen. Die organische Phase wird mit Natriumsulfat getrocknet und im Vakuum eingedampft. Der erhaltene rohe 2-[3-Äthyl-4-(2-acetylaminoäthylthiothiocarbonylthio)-2-oxo-1-azetidinyl)-2-chlor-essigsäure-p-nitrobenzylester wird in 3 ml trockenem Tetrahydrofuran gelöst, mit 6 g Triphenylphosphin versetzt und 24 Stunden bei Raumtemperatur gerührt. Die Reaktionsmischung wird mit 200 ml Methylenchlorid verdünnt, mit gesättigter wässriger Natriumhydrogencarbonatlösung gewaschen, über Natriumsulfat getrocknet und im Vakuum eingedampft. Der Rückstand ergibt nach Chromatographie an Silikagel mit Äthylacetat die Titelverbindung. DC: Rf = 0,19 (Äthylacetat); IR-Spektrum ($CH_2Cl_2$): Absorptionsbanden bei 2,93; 5,70; 5,97; 6,17; 6,58; 6,99; 7,00; 8,07; 8,33 und 9,39 μ.

Beispiel 63:
2-(2-Acetylaminoäthylthio)-6-äthyl-2-penem-3-carbonsäure-p-nitrobenzylester
(racemische cis- und trans-Verbindung)

Eine Lösung von 1,75 g (2,34 mM) 2-[3-Äthyl-4-(2-acetylaminoäthylthio-thiocarbonylthio)-2-oxo-1-azetidinyl]-2-triphenylphosphoranyliden-essigsäure-p-nitrobenzylester (racemische cis,trans-Verbindung) in 1500 ml trockenem o-Xylol wird mit einer katalytischen Menge Hydrochinon versetzt und unter Stickstoff 7 Stunden unter Rückfluss gerührt. Das Lösungsmittel wird im Vakuum abgedampft und der Rückstand an Silikagel mit Äthylacetat chromatographiert. Man erhält ein Gemisch der cis- und trans-Titelverbindung. Durch eine Kombination von präparativer Dünnschichtchromatographie (Silicagel mit Methyl-Isobutylketon) und Säulenchromatographie (Silikagel mit Äthylacetat) können die cis- und die trans-Titelverbindung erhalten werden.

cis-Verbindung: Schmelzpunkt 141–142° (nach Kristallisation aus Methylenchlorid/Diäthyläther); DC: Rf = 0,62 (Methyl-isobutylketon); IR-Spektrum ($CH_2Cl_2$): Absorptionsbanden bei 2,93; 5,62; 5,98; 6,60; 7,46; 7,57; 8,44 und 9,09 μ.

trans-Verbindung: Schmelzpunkt 132–133° (nach Kristallisation aus Methylenchlorid/Diäthyläther); DC: Rf = 0,56 (Methyl-isobutylketon); IR-Spektrum ($CH_2Cl_2$): Absorptionsbanden bei 2,92; 5,62; 5,96; 6,58; 7,44; 7,58; 8,40 und 9,01 μ.

Beispiel 64:
2-(2-Acetylaminoäthylthio)-6-äthyl-2-penem-3-carbonsäure
(racemische cis- und trans-Verbindung)

a) Eine Lösung von 100 mg (0,22 mM) 2-(2-Acetylaminoäthylthio)-6-äthyl-2-penem-3-carbonsäure-p-nitrobenzylester (racemische cis-Verbindung) in 6 ml absolutem Äthylacetat wird mit 4 ml 0,2 N wässriger Natriumhydrogencarbonatlösung und 200 mg 10%-igem Palladium/Kohle Katalysator versetzt und bei Normaldruck während 60 Minuten unter Wasserstoff gerührt. Die Hydriermischung wird über Diatomeenerde vom Katalysator abfiltriert. Die wässrige Phase wird abgetrennt, mit Diäthyläther gewaschen, mit 5%-iger wässriger Zitronensäurelösung angesäuert und mit Methylenchlorid erschöpfend extrahiert. Die vereinigten Methylenchlorid-Phasen werden über Natriumsulfat getrocknet, filtriert, im Vakuum eingedampft und im Hochvakuum getrocknet. Die erhaltene cis-Titelverbindung hat folgende physikochemischen Eigenschaften: Schmelzpunkt 153–154° (Methylenchlorid/Diäthyläther); IR-Spektrum (KBr): Absorptionsbanden bei 3,08; 3,22; 3,39; 3,42; 3,50; 3,77; 4,08; 5,67; 6,06; 6,21; 6,35; 6,75; 7,04; 7,69; 7,93; 8,27; 9,01; 9,62 und 14,38 μ.

b) Auf die gleiche Weise wird ausgehend von 100 mg 2-(2-Acetylaminoäthylthio)-6-äthyl-2-penem-3-carbonsäure-p-nitrobenzylester (racemische trans-Verbindung) die trans-Titelverbindung erhalten. IR-Spektrum (KBr): Absorptionsbanden bei 3,01; 3,39; 3,44; 5,68; 6,10; 6,85; 7,75; 8,16; 8,47 und 8,93 μ.

Beispiel 65:
3-Äthyl-4-(4-p-nitrobenzyloxycarbonylaminobutyrylthio)-2-oxo-azetidin
(racemische cis,trans-Verbindung
im Verhältnis 1:4)

Eine vorgekühlte Lösung von 3,24 g (20 mM) 3-Äthyl-4-acetoxyazetidin-2-on (racemische cis,-trans-Verbindung im Verhältnis 6:4) in 50 ml Dioxan wird tropfenweise mit einer in der Kälte bereiteten Lösung von 7,95 g (26,7 mM) 4-p-Nitrobenzyloxycarbonylamino-thiobuttersäure in 26,7 ml 1N Natriumhydroxidlösung versetzt und bei Raumtemperatur 2 Stunden gerührt. Die Reaktionsmischung wird erschöpfend mit Methylenchlorid extrahiert. Die vereinigten organischen Phasen werden über Natriumsulfat getrocknet und im Vakuum eingedampft. Der Rückstand wird an Silicagel mit Toluol-Äthylacetat 9:1, 4:1 und 1:1 chromatographiert und ergibt die Titelverbindung mit den folgenden physikochemischen Eigenschaften. DC: Rf = 0,10 (Toluol-Äthylacetat 1:1); IR-Spektrum ($CH_2Cl_2$): Absorptionsbanden bei 2,81; 2,92; 5,66; 5,81; 5,94; 6,58; 7,52 und 8,20 μ.

Die als Ausgangsmaterial verwendete Thiocarbonsäure wird wie folgt erhalten:

a) Eine Lösung von 10,30 g (0,1 mM) 4-Aminobuttersäure wird in 300 ml 1N Natriumhydroxidlösung in einem Eisbad tropfenweise innerhalb 20 Minuten mit einer Lösung von 25,87 g (0,12 mM) p-Nitrobenzyl-chloroformat in 100 ml trockenem Dioxan versetzt. Die Reaktionsmischung wird 3 Stunden bei Raumtemperatur gerührt, mit Äthylacetat gewaschen und mit 2N Salzsäure angesäuert. Die ausfallende 4-p-Nitrobenzyloxycarbonylaminobuttersäure wird abfiltriert und aus Äthylacetat umkristallisiert; Schmelzpunkt 145–146°.

b) Zu einer auf −10° gekühlten Lösung von 2,82 g (10 mM) 4-p-Nitrobenzyloxycarbonylaminobuttersäure in 50 ml trockenem Methylenchlorid werden nacheinander 2,2 g (20 mM) Triäthylamin und eine Lösung von 1,4 ml (10 mM) Isobutylchloroformat in 20 ml trockenem Methylenchlorid zugetropft. Die Reaktionsmischung wird eine Stunde gerührt und anschliessend während 2 Stunden ein starker Schwefelwasserstoffstrom durchgeleitet. Nach Ansäuern mit 2N Schwefelsäure wird die organische Phase abgetrennt, getrocknet und im Vakuum eingedampft. Die erhaltene 4-p-Nitrobenzyloxycarbonylamino-thiobuttersäure kann ohne weitere Reinigung weiterverarbeitet werden.

Beispiel 66:
2-[3-Äthyl-4-(4-p-Nitrobenzyloxycarbonylaminobutyrylthio)-2-oxo-1-azetidinyl]-2-hydroxyessigsäure-p-nitrobenzylester
(racemische cis,trans-Verbindung)

Bei Raumtemperatur werden 6,50 g (16,45 mM) 3-Äthyl-4-(4-p-Nitrobenzyloxycarbonylaminobutyrylthio)-2-oxo-azetidin (racemische cis,trans-Verbindung) und 8,41 g 2-Äthoxy-2-hydroxy-essigsäure-p-nitrobenzylester in 160 ml Toluol und 40 ml Dimethylformamid gelöst. Nach Zugabe von etwa 15 g frisch getrockneten Molekularsieben wird die Mischung unter Stickstoff 3 Stunden bei Raumtemperatur gerührt. Die Molekularsiebe werden abfiltriert und mit Dimethylformamid/Toluol (1:4) gewaschen. Das Filtrat wird im Vakuum eingedampft, am Hochvakuum getrocknet und der Rückstand mit Diäthyläther zur Entfernung von nicht umgesetztem 2-Äthoxy-2-hydroxyessigsäure-p-nitrobenzylester digeriert. Die rohe Titelverbindung hat folgende physikochemischen Eigenschaften: DC: Rf = 0,1 (Toluol-Äthylacetat 1:1); IR-Spektrum ($CH_2Cl_2$): Absorptionsbanden bei 2,83; 2,90; 5,67; 5,73; 5,80; 5,99; 6,58; 7,52; 8,26 und 9,52 μ.

Beispiel 67:
2-[3-Äthyl-4-(4-p-nitrobenzyloxycarbonylaminobutyrylthio)-2-oxo-1-azetidinyl]-2-triphenylphosphoranyliden-essigsäure-p-nitrobenzylester
(racemische cis,trans-Verbindung)

a) Zu einer Mischung von 10,40 g (17,2 mM) 2–[3-Äthyl-4-(4-p-nitrobenzyloxycarbonylaminobutyrylthio)-2-oxo-1-azetidinyl]-2-hydroxyessigsäure-p-nitrobenzylester in 40 ml absolutem Dioxan werden bei −15° nacheinander 3,06 ml (42 mM) Thionylchlorid und 5,85 ml (42 mM) Triäthylamin getropft. Die Reaktionsmischung wird 20 Minuten bei 0° und unter Stickstoff gerührt, mit 200 ml Methylenchlorid verdünnt und mit gekühlter 1N Salzsäure gewaschen. Die organische Phase wird getrocknet und im Vakuum eingedampft.

b) Der erhaltene rohe
2-[3-Äthyl-4-(4-p-nitrobenzyloxycarbonylaminobutyrylthio)-2-oxo-1-azetidinyl]-2-chloressigsäure-p-nitrobenzylester
wird in der minimalen Menge Tetrahydrofuran gelöst, mit 9 g Triphenylphosphin versetzt und über Nacht bei Raumtemperatur unter Stickstoff gerührt. Die Reaktionsmischung wird mit 250 ml Methylenchlorid verdünnt, mit gesättigter wässriger Natriumhydrogencarbonatlösung gewaschen, getrocknet und im Vakuum eingedampft. Der Rückstand wird an Silikagel mit Toluol-Äthylacetat 1:1 chromatographiert und ergibt die Titelverbindung mit den folgenden physiko-chemischen Eigenschaften: DC: Rf 0,05 (Toluol:Äthylacetat 1:1), IR-Spektrum ($CH_2Cl_2$): Absorptionsbanden bei 2,90; 5,73; 5,80; 5,94; 6,58; 7,52; 8,20 und 9,35 μ.

Beispiel 68:
6-Äthyl-2-(3-p-nitrobenzyloxycarbonylaminopropyl)-2-penem-3-carbonsäure-p-nitrobenzylester
(racemische cis,trans-Verbindung)

Eine Lösung von
5,40 g (6,36 mM) 2–[3-Äthyl-4-(4-p-nitrobenzyloxycarbonylaminobutyrylthio)-2-oxo-1-azetidinyl]-2-triphenylphosphoranyliden-essigsäure-p-nitrobenzylester
(racemische cis,trans-Verbindung) in 1500 ml trockenem Toluol wird mit einer katalytischen Menge Hydrochinon versetzt und unter Stickstoff 20 Stunden bei 100° gerührt. Das Lösungsmittel wird im Vakuum abgedampft und der Rückstand an Silikagel mit Toluol-Äthylacetat 4:1 chromatographiert. Man erhält ein Gemisch der cis- und trans-Titelverbindung im Verhältnis 1:10 mit folgenden physikochemischen Eigenschaften: DC: Rf = 0,22 (Toluol-Äthylacetat 1:1); IR-Spektrum ($CH_2Cl_2$): Absorptionsbanden bei 2,90; 5,62; 5,81; 5,85; 6,58; 7,52; 7,87 und 8,20 μ. Durch wiederholte Chromatographie können die cis- und die trans-Verbindung in reiner Form erhalten werden.

Beispiel 69:
6-Äthyl-2-(3-aminopropyl)-2-penem-3-carbonsäure (racemische cis,trans-Verbindung)

Eine Lösung von 2 g (3,5 mM) 6-Äthyl-2-(3-p-nitrobenzyloxycarbonylaminopropyl)-2-penem-3-carbonsäure-p-nitrobenzylester (racemische cis,-trans-Verbindung) in 600 ml Dioxan, 330 ml Äthanol und 600 ml Wasser wird mit 2 g Dinatriumhydrogenphosphat und 4 g 10%-igem Palladium/Kohle Katalysator versetzt und bei Normaldruck während einer Stunde unter Wasserstoff gerührt. Die Hydriermischung wird über Diatomeenerde vom Katalysator abfiltriert. Das Filtrat wird mit 3 ml 1500 ml Äthylacetat gewaschen und lyophilisiert. Das Lyophilisat wird zweimal an silyliertem Silikagel (Dünnschichtplatten ANTEC-GEL, UP–$C_{12}$) mit Wasser-Acetonitril 9:1 chromatographiert und ergibt die Titelverbindung (cis:trans etwa 1:10) mit den folgenden physikochemischen Eigenschaften: DC (ANTEC-GEL, UP–$C_{12}$): Rf 0,55 (Wasser-Acetonitril 9:1); IR-Spektrum (KBr): Absorptionsbanden bei 2,94; 3,39; 5,68; 6,41; 7,33; 7,81; 8,93; 12,82 und 14,28 μ. Ausgehend von der reinen cis- oder trans-Vorstufe können die reinen cis- und trans-Titelverbindungen erhalten werden.

Beispiel 70:
2–[(3S,4S)- und (3S,4R)-4-Acetoxy-3-methoxy-2-oxoazetidin-1-yl]-3-methylenbuttersäure-β,β,β-trichloräthylester

200mg (6S)-6-Methoxy-penicillansäure-β,β,β-trichloräthylester-1-oxid werden in 13 ml absolutem Benzol mit 0,114 ml Eisessig und 0,35 ml Trimethylphosphit versetzt und 7 Stunden am Rückfluss erhitzt. Das Lösungsmittel wird am Vakuum abgedampft und der Rückstand durch Chromatographie an Silicagel mit Toluol-Äthylacetat 19:1 und 9:1 gereinigt. Die Titelverbindungen können so getrennt werden. IR-Spektrum (in Methylenchlorid): charakteristische Absorptionsbanden für (3S,4R)-Isomer (trans-Verbindung): 3,42; 5,62; 5,68; 7,25; 7,35; 8,26; 9,01; 10,93; 11,83 μ; (3S,4S)-Isomer (cis-Verbindung): 3,42; 5,61; 5,7; 7,25; 7,35; 8,21; 9,61; 10,93 μ. Das Verhältnis von cis-:trans-Verbindung ist etwa 1:1.

Beispiel 71:
2-[(3S,4S)- und (3S,4R)-4-Acetoxy-3-methoxy-2-oxoazetidin-1-yl]-3-methylcrotonsäure-β,β,β-trichloräthylester

Eine Lösung von
0,93 g 2-[(3S,4S)- und (3S,4R)-4-Acetoxy-3-methoxy-2-oxo-azetidin-1-yl]-3-methylenbuttersäure-β,β,β-trichloräthylester
in 60 ml absolutem Methylenchlorid wird auf 0° gekühlt und 10 Minuten mit 0,33 ml Triäthylamin gerührt. Das Reaktionsgemisch wird dann nacheinander mit 4N Phosphorsäure, gesättigter wässriger Natriumbicarbonatlösung und Wasser gewaschen und über Natriumsulfat getrocknet. Das Lösungsmittel wird im Vakuum abgedampft und der Rückstand durch Chromatographie an Silicagel gereinigt. IR-Spektrum (in Methylenchlorid): charakteristische Absorptionsbanden bei: 3,41; 5,60; 5,73; 6,13; 7,19; 7,33; 8,26; 9,09; 9,57; 10,64; 10,87; 12,19 μ.

Beispiel 72:
2-[(3S,4S)- und (3S,4R)-4-Acetoxy-3-methoxy-2-oxoazetidin-1-yl]-2-oxoessigsäure-β,β,β-trichloräthylester

Durch eine auf −30° gekühlte Lösung von 0,91 g 2-[(3S,4S)- und (3S,4R)-4-Acetoxy-3-methoxy-2-oxoazetidin-1-yl]-3-methylcrotonsäure-β,β,β-trichloräthylester in 130 ml Essigsäuremethylester werden 3 Äquivalente Ozon geleitet. Nach der Ozon-Behandlung wird das Gemisch 15 Minuten bei gleicher Temperatur stehengelassen und anschliessend das überschüssige Ozon durch einen Stickstoffstrom entfernt. Das Reaktionsgemisch wird bei 0° mit einer 10%igen wässrigen Natriumsulfitlösung und danach mit Sole gewaschen. Die vereinten wässrigen Lösungen werden noch dreimal mit Essigsäuremethylester rückextrahiert. Die vereinten organischen Extrakte werden über Natriumsulfat getrocknet und im Vakuum eingedampft. IR-Spektrum (in Methylenchlorid): charakteristische Absorptionsbanden bei 3,41; 5,46; 5,68; 5,81; 7,27; 7,43; 8,23; 8,40; 9,52; 9,90 μ.

Beispiel 73:
(3S,4S)- und (3S,4R)-4-Acetoxy-3-methoxy-2-oxoazetidin

Eine Lösung von 120 mg 2-[(3S,4S)- und (3S,4R)-4-Acetoxy-3-methoxy-2-oxoazetidin-1-yl)-2-oxoessigsäure-β,β,β-trichloräthylester in 25 ml Methanol, 3,5 ml Essigsäuremethylester und 0,5 ml Wasser wird während 20 Minuten am Rückfluss erhitzt. Das Lösungsmittel wird im Vakuum abgedampft und der Rückstand ergab nach Chromatographie an Silicagel mit Toluol/Äthylacetat 9:1 das reine (3S,4R)-4-Acetoxy-3-methoxy-2-oxoazetidin, IR-Spektrum (in Methylenchlorid): charakteristische Absorptionsbanden bei 2,96; 3,42; 5,57; 5,73; 7,30; 8,23; 8,70; 8,85; 9,62; 10,0; 10,20 μ, und bei weiterem Eluieren das reine (3S,4S)-4-Acetoxy-3-methoxy-2-oxoazetidin, IR-Spektrum (in Methylenchlorid): charakteristische Absorptionsbanden bei 2,94; 3,41; 5,56; 5,73; 7,35; 7,49; 8,20; 9,52 μ.

Beispiel 74:
(3S,4R)-4-(2-p-Nitrobenzyloxycarbonylaminoäthylthio-thiocarbonylthio)-3-methoxy-2-oxoazetidin

Eine Lösung von 159 mg (1 mM) (3S,4S)-4-Acetoxy-3-methoxy-2-oxoazetidin in 3 ml Phosphatpuffer pH 7 und 0,2 ml Dioxan wird bei Raumtemperatur unter Stickstoffatmosphäre tropfenweise mit einer Lösung von 422 mg Kalium-2-p-nitrobenzyloxycarbonylaminoäthyl-trithiocarbonat in 1 ml Wasser versetzt und bei der gleichen Temperatur 30 Minuten gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an Silicagel chromatographiert und liefert die Titelverbindung mit folgendem IR-Spektrum (in Methylenchlorid): charakteristische Absorptionsbanden bei 2,95; 5,62; 5,78; 6,21; 6,56; 7,41; 8,26; 9,25 μ.

Beispiel 75:
2-[(3S,4R)-4-(2-p-Nitrobenzyloxycarbonylaminoäthylthio-thiocarbonylthio)-3-methoxy-2-oxo-1-azetidinyl]-2-hydroxy-essigsäure-p-nitrobenzylester

Analog Beispiel 23 werden
646 mg (1,5 mM) (3S,4R)-4-(2-p-Nitrobenzyloxycarbonylaminoäthylthio-thiocarbonylthio)-3-methoxy-2-oxoazetidin
in 22 ml absolutem Toluol und 5,5 ml absolutem Dimethylformamid mit 848 mg 2-Äthoxy-2-hydroxy-essigsäure-p-nitrobenzylester in Gegenwart von frisch getrockneten Molekularsieben umgesetzt. Nach Aufarbeiten und Chromatographie an Silikagel erhält man die Titelverbindung. IR-Spektrum (in Methylenchlorid): charakteristische Absorptionsbanden bei 5,62; 5,7; 5,78; 6,56; 7,41; 8,26 μ.

Beispiel 76:
2-[(3S,4R)-4-(2-p-Nitrobenzyloxycarbonylaminoäthylthio-thiocarbonylthio)-3-methoxy-2-oxo-1-azetidinyl]-2-triphenylphosphoranyliden-essigsäure-p-nitrobenzylester

Analog Beispiel 24 wird eine Lösung von 640 mg 2-[(3S,4R)-4-(2-p-Nitrobenzyloxycarbonylaminoäthylthio-thiocarbonylthio)-3-methoxy-2-oxo-1-azetidinyl]-2-hydroxyessigsäure-p-nitrobenzylester in 4,5 ml absolutem Tetrahydrofuran mit 0,12 ml Thionylchlorid und 0,23 ml Triäthylamin in 0,23 ml absolutem Tetrahydrofuran versetzt. Nach Reaktion und Aufarbeiten wird der als Zwischenprodukt erhaltene rohe 2-[(3S,4R)-4-(2-p-Nitrobenzyloxycarbonylaminoäthylthio-thiocarbonylthio)-3-methoxy-2-oxo-1-azetidinyl]-2-chlor-essigsäure-p-nitrobenzylester in 1,15 ml absolutem Tetrahydrofuran mit 0,54 g Triphenylphosphin versetzt. Nach Aufarbeiten und Chromatographie an Silikagel erhält man die Titelverbindung. IR-Spektrum (in Methylenchlorid): charakteristische Absorptionsbanden bei 3,4; 5,7; 5,78; 6,15; 6,55; 7,45; 8,26 μ.

Beispiel 77:
(6S,5R)-2-(2-p-Nitrobenzyloxycarbonylaminoäthylthio)-6-methoxy-2-penem-3-carbonsäure-p-nitrobenzylester

Analog Beispiel 25 wird eine Lösung von 500 mg 2-[(3S,4R)-4-(2-p-Nitrobenzyloxycarbonylaminoäthylthio-thiocarbonylthio)-3-methoxy-2-oxo-1-azetidinyl]-2-triphenylphosphoranyliden-essigsäure-p-nitrobenzylester in 165 ml absolutem o-Xylol am Rückfluss gerührt. Nach Aufarbeiten und Chromatographie an Silikagel mit Toluol-Äthylacetat 19:1 bis 9:1 erhält man die Titelverbindung. IR-Spektrum ($CH_2Cl_2$): Absorptionsbanden bei 5,57; 5,78; 5,9; 6,55; 7,45 und 8,26 μ.

Beispiel 78:
(6S,5R)-2-(2-Aminoäthylthio)-6-methoxy-2-penem-3-carbonsäure

Analog Beispiel 69 wird eine Lösung von 295 mg (6S,5R)-2-(2-p-Nitrobenzyloxycarbonylaminoäthylthio)-6-methoxy-2-penem-3-carbonsäure-p-nitrobenzylester in 85 ml Dioxan, 47 ml Äthanol und 85 ml Wasser mit 286 mg Dinatriumhydrogenphosphat und 570 mg eines 10%-igen Palladium-auf-Kohle-Katalysators versetzt und bei Normaldruck unter Wasserstoff gerührt. Nach Reaktion und Aufarbeiten erhält man die Titelverbindung mit folgendem IR-Spektrum (KBr): Absorptionsbanden bei 2,8–4,16; 5,68; 6,41 und 8,26 μ.

Beispiel 79:
(3S,4R)-4-(4-p-Nitrobenzyloxycarbonylaminobutyrylthio)-3-methoxy-2-oxoazetidin

Analog Beispiel 55b) werden 159 mg (3S,4S)-4-Acetoxy-3-methoxy-2-oxoazetidin in 6 ml Phosphatpuffer pH 7 und 0,4 ml Dioxan mit einer wässrigen Lösung von 480 mg 4-p-Nitrobenzyloxycarbonylamino-thiobuttersäure-natriumsalz versetzt. Nach Aufarbeiten und Chromatographie an Silikagel erhält man die Titelverbindung mit folgendem IR-Spektrum ($CH_2Cl_2$): charakteristische Absorptionsbanden bei 2,95; 5,6; 5,78; 5,87; 6,56; 7,41 und 8,26 μ.

Beispiel 80:
2-[(3S,4R)-4-(4-p-Nitrobenzyloxycarbonylaminobutyrylthio)-3-methoxy-2-oxo-1-azetidinyl]-2-hydroxy-essigsäure-p-nitrobenzylester

Analog Beispiel 23 werden 400 mg (3S,4R)-4-(4-p-Nitrobenzyloxycarbonylaminobutyrylthio)-3-methoxy-2-oxoazetidin in 15 ml absolutem Toluol und 3,7 ml absolutem Dimethylformamid mit 565 mg 2-Äthoxy-2-hydroxy-essigsäure-p-nitrobenzylester in Gegenwart von frisch getrockneten Molekularsieben umgesetzt. Nach Aufarbeiten und Chromatographie an Silikagel erhält man die Titelverbindung mit folgendem IR-Spektrum ($CH_2Cl_2$): charakteristische Absorptionsbanden bei 5,6; 5,7; 5,78; 5,87; 6,56; 7,41 und 8,26 μ.

Beispiel 81:
2-[(3S,4R)-4-(4-p-Nitrobenzyloxycarbonylaminobutyrylthio)-3-methoxy-2-oxo-1-azetidinyl]-2-triphenylphosphoranyliden-essigsäure-p-nitrobenzylester

Analog zu Beispiel 24 wird eine Lösung von 606 mg 2-[(3S,4R)-4-p-Nitrobenzyloxycarbonylaminobutyrylthio)-3-methoxy-2-oxo-1-azetidinyl]-2-hydroxy-essigsäure-p-nitrobenzylester in 4,5 ml absolutem Tetrahydrofuran mit 0,12 ml Thionylchlorid und anschliessend 0,23 ml Triäthylamin in 0,23 ml absolutem Tetrahydrofuran versetzt. Nach Reaktion und Aufarbeiten wird der erhaltene rohe 2–[(3S,4R)-4-(4-p-Nitrobenzyloxycarbonylamino-butyrylthio)-3-methoxy-2-oxo-1-azetidinyl]-2-chlor-essigsäure-p-nitrobenzylester in 1,15 ml absolutem Tetrahydrofuran mit 0,54 g Triphenylphosphin versetzt. Aufarbeiten und Chromatographie an Silikagel geben die Titelverbindung mit folgendem IR-Spektrum ($CH_2Cl_2$): charakteristische Absorptionsbanden bei 5,7; 5,78; 5,9; 6,15; 6,55; 7,45 und 8,26 μ.

Beispiel 82:
(6S,5R)-2-(3-p-Nitrobenzyloxycarbonylaminopropyl)-6-methoxy-2-penem-3-carbonsäure-p-nitrobenzylester

Analog Beispiel 68 wird eine Lösung von 400 mg 2-[3S,4R)-4-(4-p-Nitrobenzyloxycarbonylaminobutyrylthio-3-methoxy-2-oxo-1-azetidinyl]-2-triphenylphosphoranylidenessigsäure-p-nitrobenzylester in 160 ml absolutem Toluol am Rückfluss gerührt. Nach Aufarbeiten und Chromatographie an Silikagel mit Toluol/Äthylacetat von 19:1 bis 9:1 erhält man die Titelverbindung. IR-Spektrum ($CH_2Cl_2$): charakteristische Absorptionsbanden bei 5,57; 5,78; 5,85; 6,55; 7,45 und 8,26 μ.

Beispiel 83:
(6S,5R)-2-(3-Aminopropyl)-6-methoxy-2-penem-3-carbonsäure

Analog Beispiel 69 wird eine Lösung von

572 mg (6S,5R)-2-(3-p-Nitrobenzyloxycarbonyl-aminopropyl-6-methoxy-2-penem-3-carbonsäure-p-nitrobenzylester
in 170 ml Dioxan, 94 ml Äthanol und 170 ml Wasser und mit 571 mg Dinatriumhydrogenphosphat und 1,14 g eines 10%-igen Palladium-auf-Kohle-Katalysators versetzt und bei Normaldruck unter Wasserstoff gerührt. Nach Reaktion und Aufarbeiten erhält man die Titelverbindung mit folgendem IR-Spektrum (KBr): Absorptionsbanden bei 2,75–4,15; 5,67; 6,42 und 8,25 μ.

Beispiel 84:
2-[(3S,4S)- und (3S,4R)-4-Chlor-3-methoxy-2-oxoazetidin-1-yl]-3-methylcrotonsäure-β,β,β-trichloräthylester

612 mg (6S)-6-Methoxy-penicillansäure-β,β,β-trichloräthylester werden in 9 ml absolutem Methylenchlorid bei −80° tropfenweise mit 3,25 ml einer in $CCl_4$ 1,1 M Chlorlösung versetzt. Nach 2 Stunden Rühren bei −80° lässt man das Reaktionsgemisch innert einer Stunde auf Raumtemperatur anwärmen. Das Lösungsmittel wird im Vakuum eingedampft und der Rückstand an Silikagel 10% Wasser chromatographiert. Die Titelverbindungen haben folgendes IR-Spektrum (in $CH_2Cl_2$): charakteristische Absorptionsbanden bei 3,41; 5,60; 5,76; 6,15; 7,22; 7,35; 8,33; 9,09; 9,52 und 12,20 μ. In dem erhaltenen Gemisch ist das Verhältnis (3S,4S)-:(3S,4R)-Verbindung 1:10.

Beispiel 85:
2-[(3S,4S)- und (3S,4R)-4-Chlor-3-methoxy-2-oxoazetidin-1-yl]-2-oxoessigsäure-β,β,β-trichloräthylester

Durch eine auf −35° gekühlte Lösung von
210 mg 2-[(3S,4S)- und (3S,4R)-4-Chlor-3-methoxy-2-oxoazetidin-1-yl]-3-methylcrotonsäure-β,β,β-trichloräthylester
in 30 ml Essigsäuremethylester werden 3 Äquivalente Ozon geleitet. Nach der Ozon-Behandlung wird das Gemisch 15 Minuten bei gleicher Temperatur stehengelassen und anschliessend das überschüssige Ozon durch einen Stickstoffstrom entfernt. Das Reaktionsgemisch wird bei 0° mit einer 10%-igen wässrigen Natriumbisulfitlösung und danach mit Sole gewaschen. Die vereinten wässrigen Lösungen werden noch dreimal mit Essigsäuremethylester rückextrahiert. Die organischen Extrakte werden über Natriumsulfat getrocknet und im Vakuum eingedampft. Die rohe Titelverbindung hat folgendes IR-Spektrum (in $CH_2Cl_2$): charakteristische Absorptionsbanden bei 3,41; 5,46; 5,65; 5,80; 7,46; 8,23; 8,47; 8,89; 9,57; 9,95 und 11,90 μ.

Beispiel 86:
(3S,4S)- und (3S,4R)-4-Chlor-3-methoxy-2-oxoazetidin

Eine Lösung von
339 mg 2-[(3S,4S)- und (3S,4R)-4-Chlor-3-methoxy-2-oxoazetidin-1-yl]-2-oxoessigsäure-β,β,β-trichloräthylester
und 197 mg 2,4-Dinitrophenylhydrazin in 9 ml Tetrahydrofuran wird 30 Minuten am Rückfluss erhitzt. Das Lösungsmittel wird abgedampft und der Rückstand an Silikagel chromatographiert. Die Titelverbindung hat folgendes IR-Spektrum ($CH_2Cl_2$): charakteristische Absorptionsbanden bei 2,94; 5,56; 8,26 und 9,09 μ.

Beispiel 87:
(3S,4R)-4-(4-p-Nitrobenzyloxycarbonylamino-butyrylthio)-3-methoxy-2-oxoazetidin

Eine Lösung von 135 mg (3S,4R)-4-Chlor-3-methoxy-2-oxoazetidin in 6 ml Phosphatpuffer pH 7 und 0,4 ml Dioxan wird in Gegenwart von 150 mg Natriumjodid tropfenweise mit einer Lösung von 350 mg 4-p-Nitrobenzyloxycarbonylamino-thiobuttersäure-natriumsalz in 4 ml Wasser versetzt. Nach 30 Minuten Rühren bei Raumtemperatur wird erschöpfend mit Methylenchlorid extrahiert. Nach Abtrennen und Trocknen der organischen Phase über Natriumsulfat wird das Lösungsmittel im Vakuum abgedampft und der Rückstand an Silikagel chromatographiert. Die Titelverbindung hat folgendes IR-Spektrum ($CH_2Cl_2$): Absorptionsbanden bei 2,95; 5,6; 5,78; 5,87; 6,56; 7,41 und 8,26 μ.

Beispiel 88:

Ausgehend von entsprechenden Ausgangsmaterialien und über entsprechende Zwischenprodukte werden analog den voranstehenden Beispielen die folgenden Verbindungen erhalten:
6-Äthyl-2-(2-aminoäthylthio)-2-penem-3-carbonsäure,
6-Hydroxymethyl-2-penem-3-carbonsäure,
6-Hydroxymethyl-2-methyl-2-penem-3-carbonsäure,
6-Hydroxymethyl-2-(3-aminopropyl)-2-penem-3-carbonsäure,
6-Hydroxymethyl-2-(3-acetylaminopropyl)-2-penem-3-carbonsäure,
6-Hydroxymethyl-2-äthylthio-2-penem-3-carbonsäure,
6-Hydroxymethyl-2-(2-aminoäthylthio)-2-penem-3-carbonsäure,
6-Hydroxymethyl-2-(2-acetylaminoäthylthio)-2-penem-3-carbonsäure,
6-(1-Hydroxyäthyl)-2-penem-3-carbonsäure,
6-(1-Hydroxyäthyl)-2-methyl-2-penem-3-carbonsäure,
6-(1-Hydroxyäthyl)-2-(3-aminopropyl)-2-penem-3-carbonsäure,
6-(1-Hydroxyäthyl)-2-(3-acetylaminopropyl)-2-penem-3-carbonsäure,
6-(1-Hydroxyäthyl)-2-äthylthio-2-penem-3-carbonsäure,
6-(1-Hydroxyäthyl)-2-(2-aminoäthylthio)-2-penem-3-carbonsäure,
6-(1-Hydroxyäthyl)-2-(2-acetylaminoäthylthio)-2-penem-3-carbonsäure,
6-Methoxy-2-penem-3-carbonsäure,
6-Methoxy-2-(3-acetylaminopropyl)-2-penem-3-carbonsäure,
6-Methoxy-2-äthylthio-2-penem-3-carbonsäure,
6-Methoxy-2-(2-acetylaminoäthylthio)-2-penem-3-carbonsäure,
6-Methoxy-2-(1,3,4-thiadiazol-2-ylthio)-2-penem-3-carbonsäure,

6-(2-Hydroxyprop-2-yl)-2-penem-3-carbonsäure,
6-(2-Hydroxyprop-2-yl)-2-methyl-2-penem-3-carbonsäure,
6-(2-Hydroxyprop-2-yl)-2-(3-aminopropyl)-2-penem-3-carbonsäure,
6-(2-Hydroxyprop-2-yl)-2-(3-acetylaminopropyl)-2-penem-3-carbonsäure,
6-(2-Hydroxyprop-2-yl)-2-äthylthio-2-penem-3-carbonsäure,
6-(2-Hydroxyprop-2-yl)-2-(2-aminoäthylthio-2-penem-3-carbonsäure,
6-(2-Hydroxyprop-2-yl)-2-(2-acetylaminoäthylthio-2-penem-3-carbonsäure,
sowohl in racemischer als auch in optisch aktiver Form und ihre Salze.

**Patentansprüche für die Vertragsstaaten: BE CH DE FR GB LU NL SE**

1. Verfahren zur Herstellung von Verbindungen der Formel

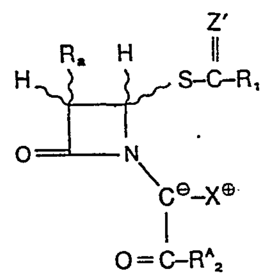

(II),

worin $R_a$ ein gesättigter oder ungesättigter, gegebenenfalls substituierter, aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel,

in welchen Resten $R_a$ die gegebenenfalls vorhandenen Substituenten Hydroxy, Niederalkoxy, Niederalkanoyloxy, in Salzform vorliegendes Hydroxysulfonyloxy, Halogen, Mercapto, Niederalkylmercapto, Carboxyl, Niederalkoxycarbonyl, Carbamoyl, Cyan, Nitro, in Salzform vorliegendes Sulfo, oder gegebenenfalls durch Niederalkyl oder $C_1$–$C_{20}$-Acyl mono- oder disubstituiertes oder durch Niederalkylen disubstituiertes Amino sind,

gegebenenfalls geschütztes Carboxyl,

Hydroxy,

Mercapto,

durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy oder Mercapto, worin

die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$–$C_{20}$-Acyl mono- oder disubstituiertes oder durch Niederalkylen disubstituiertes Amino sind,

durch einen Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen verestertes Hydroxy oder Mercapto, worin

die gegebenenfalls vorhandenen Substituenten Hydroxy, Niederalkoxy, Phenyloxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$–$C_{20}$-Acyl mono- oder disubstituiertes oder durch Niederalkylen disubstituiertes Amino sind, oder Halogen ist,

$R_1$ Wasserstoff, ein gesättigter oder ungesättigter, gegebenenfalls substituierter aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, oder

ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel ist,

in welchen Resten $R_1$ die gegebenenfalls vorhandenen Substituenten Hydroxy, Niederalkoxy, Niederalkanoyloxy, Halogen, Mercapto, Niederalkylmercapto, gegebenenfalls durch Niederalkyl, Hydroxyniederalkyl, Carboxyniederalkyl, Aminoniederalkyl, Diniederalkylaminoniederalkyl, in Salzform vorliegendes Sulfoniederalkyl, Cycloalkyl, Aryl, Arylniederalkyl, Halogen, Amino, Mono- oder Di-niederalkylamino, Nitro, Hydroxy, Niederalkoxy, Carboxyl, Niederalkoxycarbonyl, Carbamoyl, N-Mono- oder N,N-Diniederalkylcarbamoyl, Cyan, Oxo oder Oxido substituiertes Heterocyclylmercapto, Carboxyl, Niederalkoxycarbonyl, Carbamoyl, Cyan, Nitro, Amino, Niederalkylamino, Diniederalkylamino, $C_1$–$C_{20}$-Acylamino, Di-$C_1$–$C_{20}$-acylamino oder Niederalkylenamino sind, oder worin $R_1$ eine durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen, verätherte Mercaptogruppe ist,

worin die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, gegebenenfalls durch Niederalkyl oder $C_1$–$C_{20}$-Acyl mono- oder disubstituiertes oder durch Niederalkylen disubstituiertes Amino sind, oder worin $R_1$ durch Niederalkyl substituiertes Phenylthio oder Phenylniederalkylthio ist, oder worin $R_1$ gegebenenfalls geschütztes Carboxyl ist, oder worin

$R_1$ eine durch einen gegebenenfalls substituierten heterocyclischen Rest verätherte Mercaptogruppe ist, worin der Heterocyclylrest über ein Ringkohlenstoffatom an die Mercaptogruppe gebunden ist und 1 bis 4 Ringstickstoffatome und gegebenenfalls ein weiteres Ringheteroatom der Gruppe Sauerstoff und Schwefel enthält und worin die gegebenenfalls vorhandenen Substituenten Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$–$C_{20}$-Acylamino oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl, Oxazolyl, Halogen, gegebenenfalls durch Niederalkyl mono- oder disubstituiertes Amino, $C_1$–$C_{20}$-Acylamino, Nitro, Hydroxy, Niederalkoxy, Carboxy, Niederalkoxycarbonyl, gegebenenfalls N-mono- oder N,N-diniederalkyliertes Carbamoyl, Cyan, Oxo oder Oxido sind, wobei funktionelle Gruppen in diesen Resten $R_a$ und $R_1$ vorzugsweise in geschützter Form vorliegen und die mit «Nieder» bezeichneten Gruppen bis zu 7, insbesondere bis zu 4 Kohlenstoffatome enthalten, Z′ Sauerstoff, Schwefel oder eine gegebenenfalls einen oder zwei Substituenten tragende Methylidengruppe bedeutet, $X^{\oplus}$ entweder eine dreifach substituierte Phosphoniogruppe oder eine zweifach veresterte Phosphonogruppe zusammen mit einem Kation bedeutet und $R_2^A$ zusammen mit der Carbonylgruppe $-C(=O)-$ eine geschützte Carboxylgruppe darstellt, in racemischer oder optisch aktiver Form, dadurch gekennzeichnet, dass man die Stufen 1.1 bis 1.4 gemäss

Reaktionsschema 1

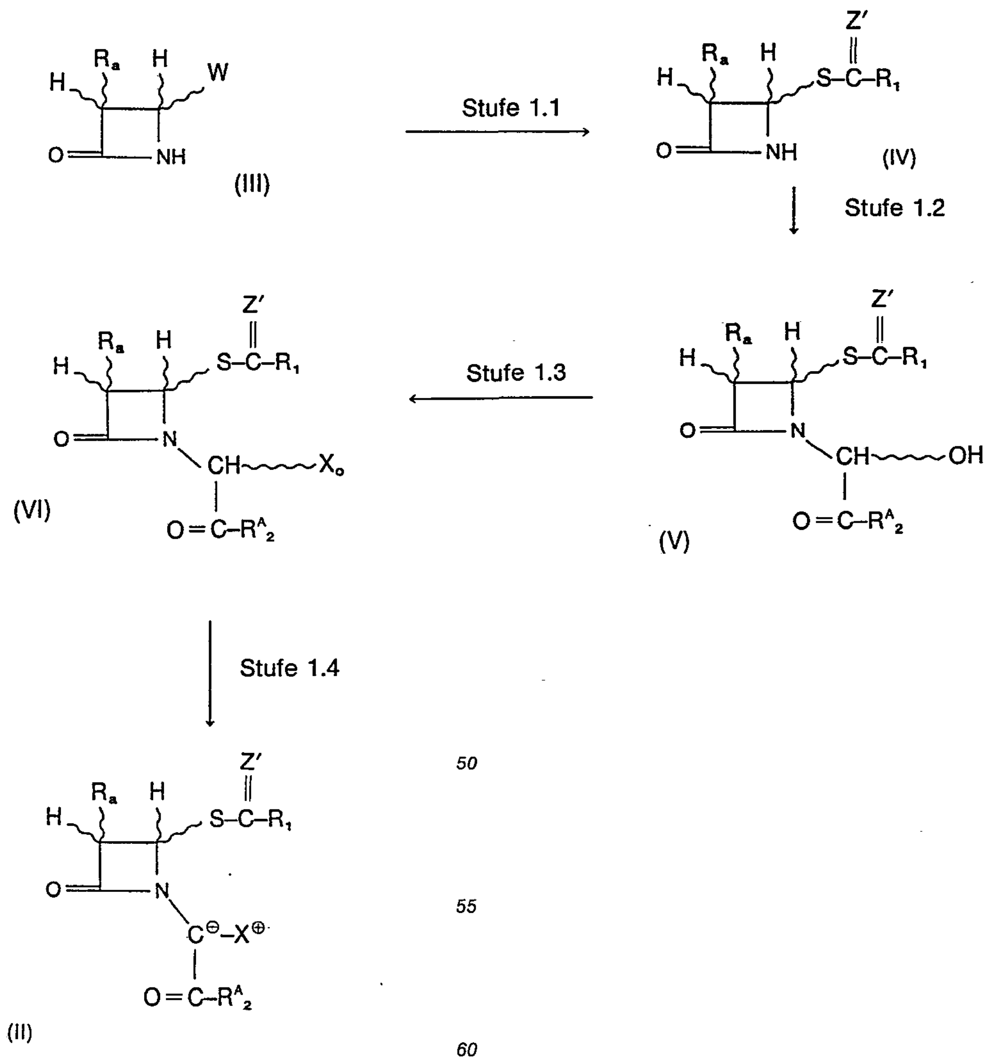


oder die Stufen 2.1 bis 2.6 gemäss

Reaktionsschema 2

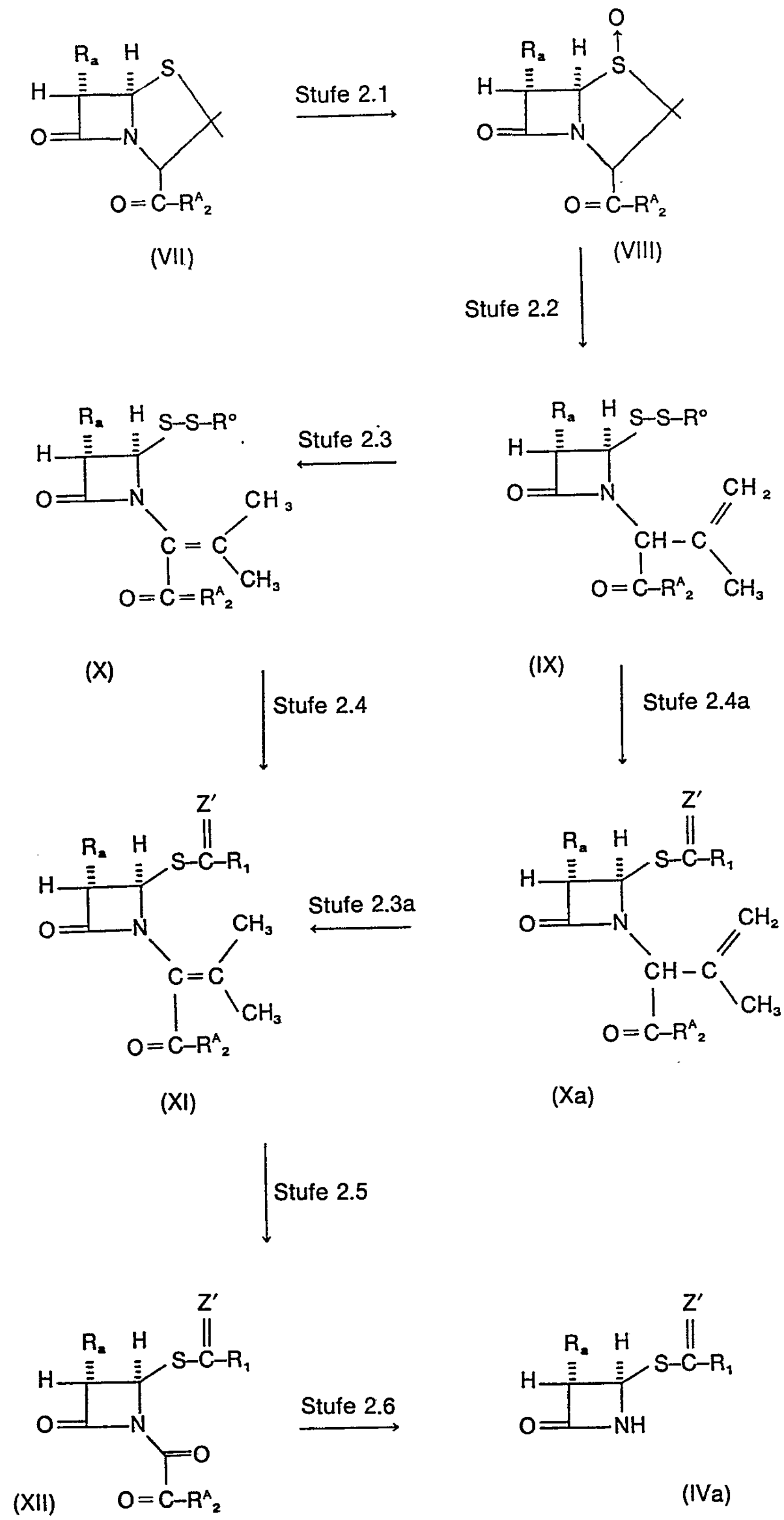


(VII) (VIII) (IX) (X) (Xa) (XI) (XII) (IVa)

gefolgt von Stufen 1.2 bis 1.4, oder die Stufen 3.1 bis 3.5 gemäss

Reaktionsschema 3

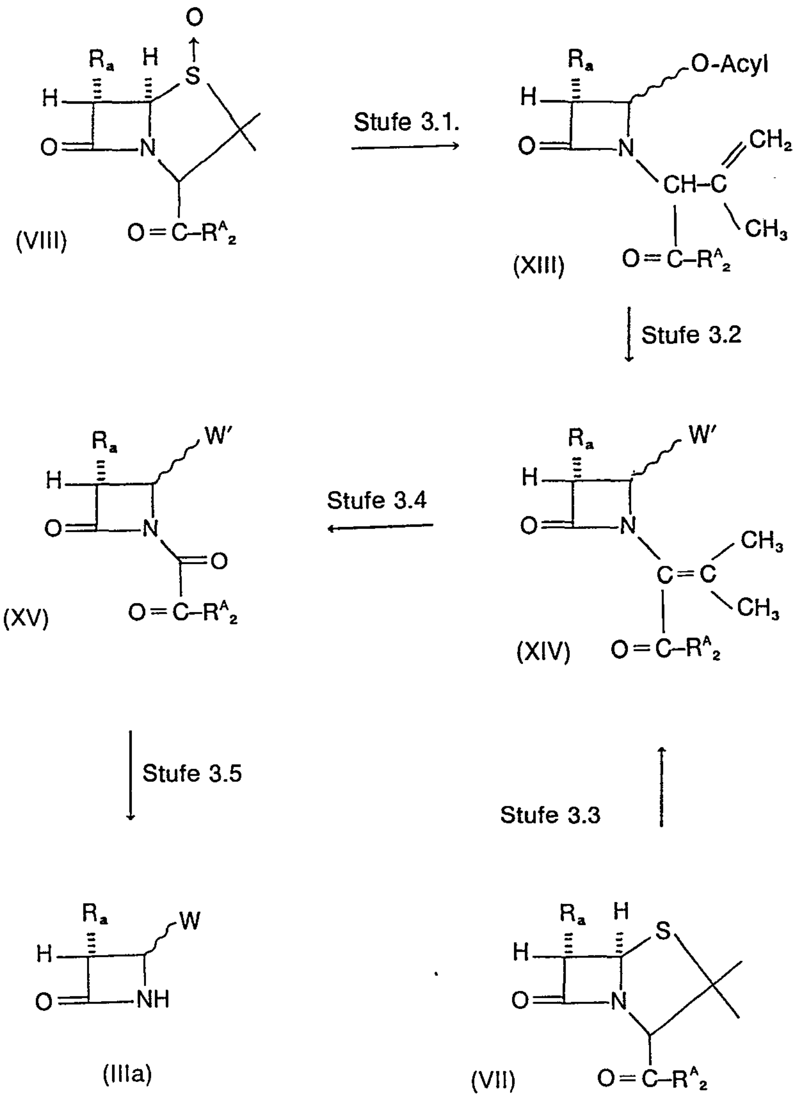


(VIII) (XIII) (XV) (XIV) (IIIa) (VII)

gefolgt von den Stufen 1.1 bis 1.4, durchführt, wobei man gemäss Stufe 1.1 zur Herstellung von Verbindungen der Formel

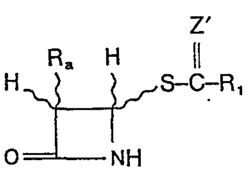

(IV) oder (IVa),

worin $R_a$, $R_1$ und Z' die unter Formel II genannten Bedeutungen haben, in racemischer oder optisch aktiver Form, ein 4–W-Azetidinon der Formel

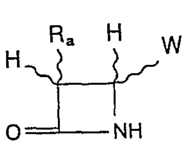

(III)

oder

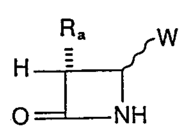

(IIIa),

worin W eine nucleofuge Abgangsgruppe bedeutet, mit einer Mercaptoverbindung $R_1$–C(=Z')–SH oder einem Salz davon behandelt, und gewünschtenfalls ein erhaltenes Isomerengemisch in die einzelnen Isomeren auftrennt, oder, gemäss Stufe 2.6 zur Herstellung von optisch aktiven (3S,4R)-Verbindungen der Formel

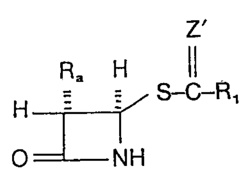

(IVa),

eine Verbindung der Formel

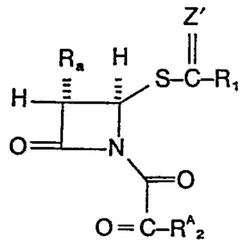

(XII),

worin $R_a$, $R_1$, Z′ und $R_2^A$ die unter Formel II angegebenen Bedeutungen haben, solvolysiert, und wenn erwünscht in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, gemäss Stufe 1.2 zur Herstellung von Verbindungen der Formel

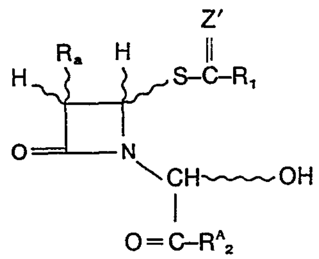

(V),

worin $R_a$, $R_1$, Z′ und $R_2^A$ die unter Formel II gegebenen Bedeutungen haben, in racemischer oder optisch aktiver Form, eine Verbindung der Formel

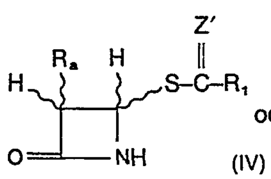

(IV) oder

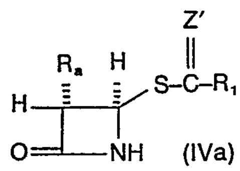

(IVa),

worin $R_a$, $R_1$ und Z′ die oben gegebenen Bedeutungen haben, mit einer Glyoxylsäure-Verbindung der Formel $-OHC-C(=O)-R_2^A$ oder einem geeigneten Derivat davon umsetzt, und gewünschtenfalls ein erhaltenes Isomerengemisch in die einzelnen Isomeren auftrennt, und/oder gewünschtenfalls eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, gemäss Stufe 1.3 zur Herstellung von Verbindungen der Formel

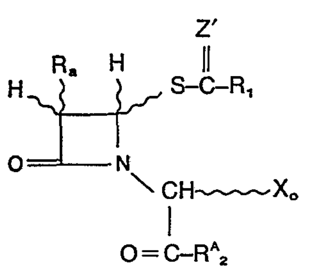

(VI),

worin $R_a$, $R_1$, Z′ und $R_2^A$ die unter Formel II gegebenen Bedeutungen haben und $X_0$ für eine reaktionsfähige veresterte Hydroxygruppe steht, in racemischer oder optisch aktiver Form, in einer Verbindung der Formel

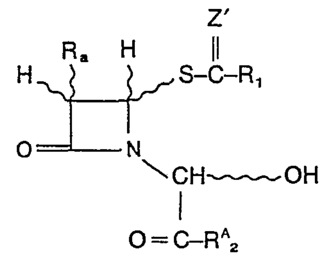

(V),

worin $R_a$, $R_1$, Z′ und $R_2^A$ die oben gegebenen Bedeutungen haben, die sekundäre Hydroxygruppe in eine reaktionsfähige veresterte Hydroxygruppe umwandelt, und gewünschtenfalls ein erhaltenes Isomerengemisch in die einzelnen Isomeren auftrennt, und/oder gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, und gemäss Stufe 1.4, zur Herstellung von Verbindungen der Formel II, eine Verbindung der Formel

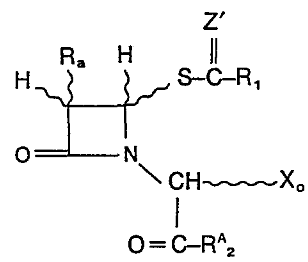

(VI),

worin $X_0$ für eine reaktionsfähige veresterte Hydroxygruppe steht, und $R_a$, $R_1$, Z′ und $R_2^A$ die oben gegebenen Bedeutungen haben, mit einer geeigneten Phosphin-Verbindung oder einer geeigneten Phosphit-Verbindung behandelt, oder eine Verbindung der Formel

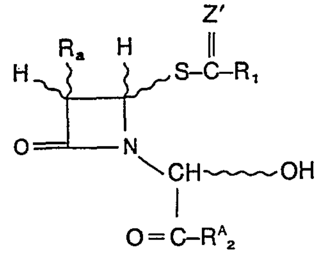

(V),

worin $R_a$, $R_1$, Z′ und $R_2^A$ die oben gegebenen Bedeutungen haben, mit Tetrachlorkohlenstoff

und einem Phosphin behandelt, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt, oder gemäss Stufe 2.1, zur Herstellung von Verbindungen der Formel

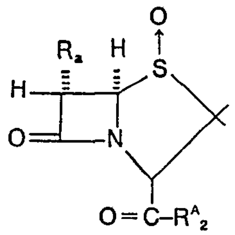

(VIII),

worin $R_a$ und $R_2^A$ die unter Formel II gegebenen Bedeutungen haben, eine Penicillansäureverbindung der Formel

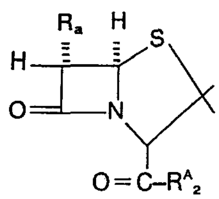

(VII),

in 1-Stellung oxidiert, und gewünschtenfalls in einer erhaltenen Verbindung eine Gruppe $R_a$ in eine andere Gruppe $R_a$ überführt, gemäss Stufe 2.2 zur Herstellung von Verbindungen der Formel

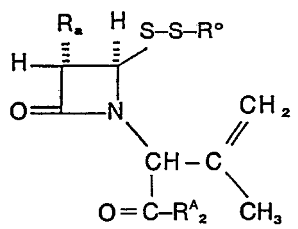

(IX),

worin $R_a$ und $R_2^A$ die unter Formel II gegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist, eine Verbindung der Formel

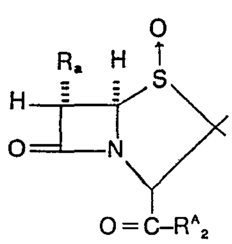

(VIII),

mit einer Mercaptoverbindung $R^0$–SH behandelt, und gewünschtenfalls in einer erhaltenen Verbindung eine Gruppe $R_a$ in eine andere Gruppe $R_a$ umwandelt, gemäss Stufe 2.3 zur Herstellung von Verbindungen der Formel

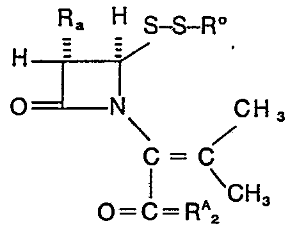

(X),

worin $R_a$ und $R_2^A$ die unter Formel II gegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist, eine 3-Methylenbuttersäureverbindung der Formel

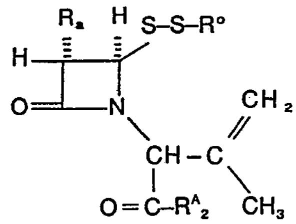

(IX),

durch Behandeln mit einem geeigneten basischen Mittel isomerisiert, und gewünschtenfalls in einer erhaltenen Verbindung eine Gruppe $R_a$ in eine andere Gruppe $R_a$ umwandelt, gemäss Stufe 2.4 zur Herstellung von Verbindungen der Formel

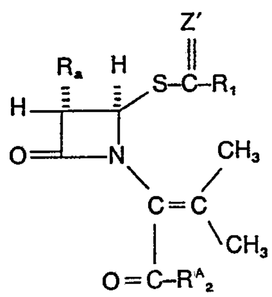

(XI),

worin $R_a$, $R_1$, Z' und $R_2^A$ die unter Formel II gegebenen Bedeutungen haben, eine Verbindung der Formel

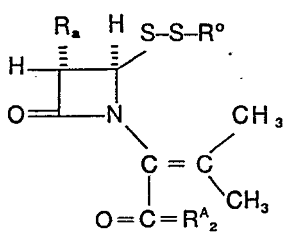

(X),

worin $R^0$ eine gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist, und $R_a$ und $R_2^A$ die oben angegebenen Bedeutungen haben, mit einem geeigneten Reduktionsmittel behandelt und gleichzeitig oder nachträglich mit einem Acylierungsderivat einer Carbonsäure der Formel $R_1$–C(=Z)–OH, oder, wenn Z′ eine gegebenenfalls durch Y substituierte Methylidengruppe bedeutet, mit einem Alkin der Formel $R_1$–C≡C–Y, umsetzt, oder gemäss Stufe 2.3a eine Verbindung der Formel

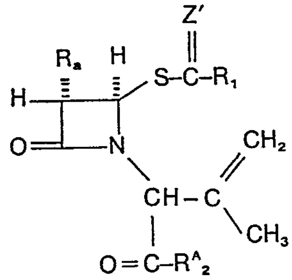

(Xa),

worin $R_a$, $R_1$, Z′ und $R_2^A$ die oben gegebenen Bedeutungen haben, durch Behandeln mit einem geeigneten basischen Mittel isomerisiert, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z′ in eine Oxogruppe Z überführt, gemäss Stufe 2.4a zur Herstellung von Verbindungen der Formel

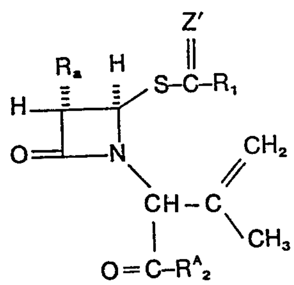

(Xa),

worin $R_a$, $R_1$, Z′ und $R_2^A$ die unter Formel II angegebenen Bedeutungen haben, eine Verbindung der Formel

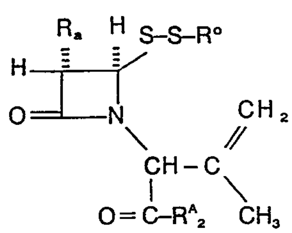

(IX),

worin $R^0$, $R_a$ und $R_2^A$ die oben gegebenen Bedeutungen haben, mit einem geeigneten Reduktionsmittel behandelt und gleichzeitig oder nachträglich mit einem Acylierungsderivat einer Carbonsäure der Formel $R_1$–C(=Z)–OH, oder, wenn Z′ eine gegebenenfalls durch Y substituierte Methylidengruppe bedeutet, mit einem Alkin der Formel $R_1$–C≡C–Y umsetzt und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z′ in eine Oxogruppe Z überführt, gemäss Stufe 2.5 zur Herstellung von Verbindungen der Formel

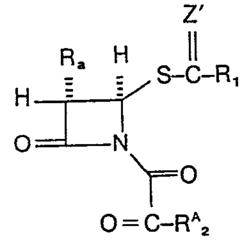

(XII),

worin $R_a$, $R_1$, Z′ und $R_2^A$ die unter Formel II gegebenen Bedeutungen haben, eine Verbindung der Formel

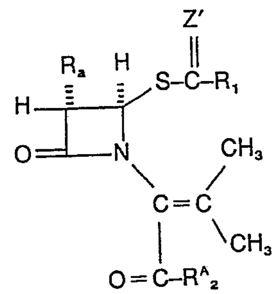

(XI),

worin $R_a$, $R_1$, Z′ und $R_2^A$ die oben gegebenen Bedeutungen haben, ozonisiert und das gebildete Ozonid reduktiv zur Oxogruppe spaltet, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, und anschliessend die Stufen 2.6, und 1.2 bis 1.4 durchführt, oder, gemäss Stufe 3.1 zur Herstellung von Verbindungen der Formel

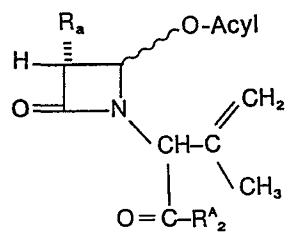

(XIII),

worin $R_a$ und $R_2^A$ die unter Formel II genannten Bedeutungen haben und Acyl der Acylrest einer organischen Carbonsäure ist, in der cis,trans-, der cis- oder der trans-Form, ein 1-Oxid einer Penicillansäureverbindung der Formel

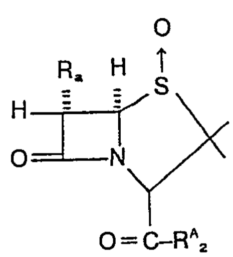

(VIII),

worin $R_a$ und $R_2^A$ die oben angegebenen Bedeutungen haben, in Gegenwart eines Triniederalkylphosphits mit einer organischen Carbonsäure Acyl–OH behandelt, und gewünschtenfalls in einer erhaltenen Verbindung eine Gruppe $R_a$ in eine andere Gruppe $R_a$ umwandelt, und/oder aus einer

erhaltenen cis,trans-Verbindung die cis- und/oder die trans-Verbindung isoliert, gemäss Stufe 3.2 zur Herstellung von Verbindungen der Formel

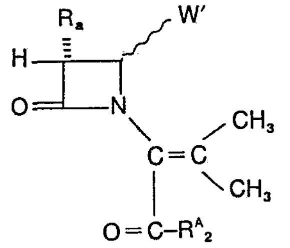

(XIV),

worin $R_a$ und $R_2^A$ die unter Formel II gegebenen Bedeutungen haben und W' für Acyloxy steht, worin Acyl der Acylrest einer organischen Carbonsäure ist, und $R_2^A$ zusammen mit der Carbonylgruppe $-C(=O)-$ eine geschützte Carboxylgruppe darstellt, in der cis,trans-, der cis- oder der trans-Form, eine Verbindung der Formel

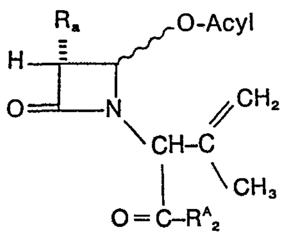

(XIII),

worin $R_a$, Acyl und $R_2^A$ die oben angegebenen Bedeutungen haben, durch Behandeln mit einem geeigneten basischen Mittel isomerisiert, und gewünschtenfalls in einer erhaltenen Verbindung eine Gruppe $R_a$ in eine andere Gruppe $R_a$ umwandelt, und/oder gewünschtenfalls aus einer erhaltenen cis,trans-Verbindung die cis- und/oder die trans-Verbindung isoliert, oder gemäss Stufe 3.3 zur Herstellung von Verbindung der Formel

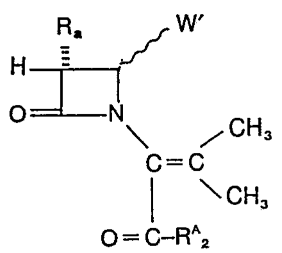

(XIV),

worin $R_a$ und $R_2^A$ die unter Formel II gegebenen Bedeutungen haben und W' Halogen bedeutet, in der cis,trans-, der cis- oder der trans-Form, eine Penicillansäureverbindung der Formel

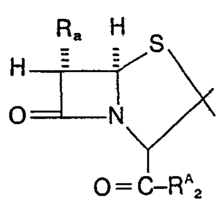

(VII),

worin $R_a$ und $R_2^A$ die oben angegebenen Bedeutungen haben, mit einem positive Halogenionen liefernden Halogenierungsmittel umsetzt und, falls erforderlich, ein gegebenenfalls erhaltenes Zwischenprodukt mit einer Base behandelt, und gewünschtenfalls in einer erhaltenen Verbindung der Formel XIV eine Gruppe $R_a$ in eine andere Gruppe $R_a$ überführt und/oder aus einer erhaltenen cis,trans-Verbindung die cis- und/oder die trans-Verbindung isoliert, gemäss Stufe 3.4 zur Herstellung von Verbindungen der Formel

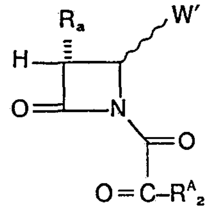

(XV),

worin $R_a$ und $R_2^A$ die unter Formel II gegebenen Bedeutungen haben und W' Halogen oder Acyloxy bedeutet, worin Acyl der Acylrest einer organischen Carbonsäure ist, in der cis,trans-, der cis- oder der trans-Form, eine Verbindung der Formel

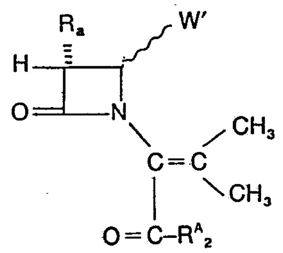

(XIV),

worin $R_a$, W' und $R_2^A$ die oben angegebenen Bedeutungen haben, ozonisiert und das gebildete Ozonid reduktiv zur Oxogruppe spaltet, und gewünschtenfalls in einer erhaltenen Verbindung eine Gruppe $R_a$ in eine andere Gruppe $R_a$ umwandelt, und/oder gewünschtenfalls aus einer erhaltenen cis,trans-Verbindung die cis- und/oder die trans-Verbindung isoliert, gemäss Stufe 3.5 zur Herstellung von Verbindungen der Formel

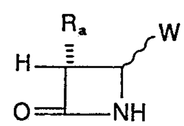

(IIIa),

worin $R_a$ die unter Formel II gegebenen Bedeutungen hat und W eine nucleofuge Abgangsgruppe bedeutet, in der cis,trans-, der cis- oder der trans-Form, eine Verbindung der Formel

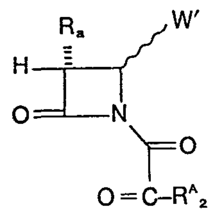

(XV),

worin $R_a$ und $R_2^A$ die oben angegebenen Bedeutungen haben, und W' Acyloxy oder Halogen bedeutet, solvolysiert, und gewünschtenfalls in einer erhaltenen Verbindung eine Gruppe $R_a$ in eine andere Gruppe $R_a$ umwandelt, und/oder gewünschtenfalls eine Gruppe W' in eine andere Gruppe W' oder in W überführt, und/oder gewünschtenfalls eine erhaltende cis-Verbindung

zur entsprechenden trans-Verbindung isomerisiert, und/oder aus einer erhaltenen cis,trans-Verbindung die cis- und/oder die trans-Verbindung isoliert, und anschliessend die Stufen 1.1 bis 1.4 durchführt.

2. Verfahren nach Patentanspruch 1, dadurch gekennzeichnet, dass man Verbindungen der Formel II herstellt, worin $R_a$ Niederalkyl, Hydroxyniederalkyl, Niederalkoxyniederalkyl, Niederalkanoyloxyniederalkyl, in Salzform vorliegendes Hydroxysulfonyloxyniederalkyl, Hydroxy, Niederalkoxy, Niederalkanoyloxy, Niederalkanoyloxy substituiert durch Phenyloxy, Halogen, Amino oder Cyan, Phenylniederalkanoyloxy, oder durch Hydroxy oder Amino substituiertes Phenylniederalkanoyloxy ist, $R_1$ Wasserstoff, Niederalkyl, Hydroxyniederalkyl, Niederalkoxyniederalkyl, Niederalkanoyloxyniederalkyl, Niederalkylthioniederalkyl, Heterocyclylthioniederalkyl, worin Heterocyclyl einen gegebenenfalls durch Niederalkyl, Carboxyniederalkyl, Diniederalkylaminoniederalkyl oder in Salzform vorliegendes Sulfoniederalkyl substituierten fünfgliedrigen aromatischen diaza-, triaza-, tetraza-, thiaza-, thiadiaza-, thiatriaza-, oxaza- oder oxadiazacyclischen Rest darstellt, Aminoniederalkyl, Acylaminoniederalkyl, worin Acyl Niederalkanoyl oder eine als Aminoschutzgruppe gebräuchliche substituierte Oxycarbonylgruppe, z.B. eine tert.-Butyl-, 2,2,2-Trichloräthyl-, Diphenylmethyl- oder p-Nitrobenzyloxycarbonylgruppe, ist, Carboxyniederalkyl, Niederalkoxycarbonylniederalkyl, Phenylniederalkyl, Phenyl, Hydroxyphenyl, Aminophenyl, Furyl, Thienyl, Pyridyl, Niederalkylthio, durch Amino, Mono- oder Diniederalkylamino oder Niederalkanoylamino substituiertes Niederalkylthio oder Niederalkenylthio, oder gegebenenfalls durch Niederalkyl, Sulfoniederalkyl, Carboxyniederalkyl oder Diniederalkylaminoniederalkyl substituiertes Tetrazolylthio oder Thiadiazolylthio darstellt, wobei funktionelle Gruppen in solchen Resten $R_a$ und $R_1$ vorzugsweise in geschützter Form vorliegen, und $Z'$, $X^{\oplus}$ und $R_2^A$ die im Patentanspruch 1 angegebenen Bedeutungen haben, in racemischer oder optisch aktiver Form.

3. Verbindungen der Formel

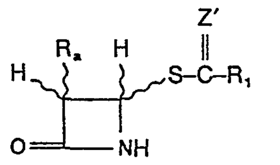

(IV),

worin $R_a$, $R_1$ und $Z'$ die in Anspruch 1 angegebenen Bedeutungen haben, in racemischer und optisch aktiver Form, mit der ersten Massgabe, dass $Z'$ von einer durch eine geschützte Carboxylgruppe substituierten Methylidengruppe verschieden ist, wenn $R_a$ Alkyl, Alkoxyl, Aryl, Aralkyl, Heterocyclyl oder Heterocyclylalkyl ist und $R_1$ Wasserstoff oder Alkyl, Aryl, Aralkyl, Heterocyclyl, Heterocyclylalkyl, Alkylthio, Arylthio, Aralkylthio oder Heterocyclylthio bedeutet, wobei diese Reste $R_a$ und $R_1$ unsubstituiert oder durch Amino, geschütztes Amino, Mono- oder Dialkylamino, Hydroxyl, geschütztes Hydroxyl, Alkoxyl, Mercapto, Alkylthio, Nitro, Chlor, Brom, Fluor, Cyan, Carboxyl oder geschütztes Carboxyl substituiert sind,

und mit der zweiten Massgabe, dass $Z'$ von Sauerstoff verschieden ist, wenn entweder

$R_a$ die unter der 1. Massgabe angegebenen Bedeutungen hat und $R_1$ von Phenyl verschiedenes Aryl, von Phenylniederalkyl verschiedenes Aralkyl, von Pyridyl, Thienyl und Furyl verschiedenes Heterocyclyl, Heterocyclylalkyl, Arylthio, Aralkylthio oder Heterocyclylthio ist, welche Reste unsubstituiert oder durch Amino, geschütztes Amino, Mono- oder Dialkylamino, Hydroxy geschütztes Hydroxy, Alkoxyl, Mercapto, Alkylthio, Nitro, Chlor, Brom, Fluor, Cyan, Carboxyl oder geschütztes Carboxyl substituiert sind; durch Mono- oder Dialkylamino, Mercapto, Nitro, Chlor, Fluor, Brom oder Cyan substituiertes Alkyl mit 1 bis 6 C-Atomen; durch Mono- oder Dialkylamino, Mercapto, Alkylthio, Nitro, Cyan, Carboxyl oder geschütztem Carboxyl substituiertes Phenyl; durch Amino, geschütztes Amino, Mono- oder Dialkylamino, Hydroxy, geschütztes Hydroxy, Alkoxyl, Mercapto, Alkylthio, Nitro, Chlor, Brom, Fluor, Cyan, Carboxyl oder geschütztes Carboxyl substituiertes Phenylniederalkyl, Pyridyl, Thienyl oder Furyl; oder Alkylthio mit 1–6 C-Atomen, welches substituiert ist durch Hydroxy, geschütztes Hydroxy, Mercapto, Alkylthio, Nitro, Chlor, Brom, Fluor, Cyan, Carboxyl oder geschütztes Carboxyl, bedeutet;

oder $R_1$ die unter der 1. Massgabe angegebenen Bedeutungen hat und $R_a$ substituiertes Alkoxyl oder gegebenenfalls substituiertes Aryl, Aralkyl, Heterocyclyl oder Heterocyclylalkyl ist, wobei Substituenten solcher Reste Amino, geschütztes Amino, Mono- oder Dialkylamino, Hydroxyl, geschütztes Hydroxyl, Alkoxyl, Mercapto, Alkylthio, Nitro, Chlor, Brom, Fluor, Cyan, Carboxyl oder geschütztes Carboxyl sind; oder Alkyl mit 1–6 C-Atomen ist, welcher Rest durch Amino, geschütztes Amino, Mono- oder Dialkylamino, Mercapto, Alkylthio, Nitro, Chlor, Brom, Fluor, Cyan, Carboxyl oder geschütztes Carboxyl substituiert ist.

4. Verbindungen der Formel IV nach Patentanspruch 3, worin $R_a$, $R_1$ und $Z'$ die im Patentanspruch 2 angegebenen Bedeutungen haben.

5. 4-Acetylthio-3-methyl-2-oxo-azetidin (racemische cis-Verbindung) nach Patentanspruch 3.

6. 4-Acetylthio-3-methyl-2-oxo-azetidin (racemische trans-Verbindung) nach Patentanspruch 3.

7. 4-Acetylthio-3-isopropyl-2-oxo-azetidin (racemische trans-Verbindung), nach Patentanspruch 3.

8. 4-Acetylthio-3-benzyl-2-oxo-azetidin (racemische trans-Verbindung), nach Patentanspruch 3.

9. 4-Äthylthio-thiocarbonylthio-3-isopropyl-2-oxoazetidin (racemische trans-Verbindung), nach Patentanspruch 3.

10. (3S,4R)-4-Acetylthio-3-methoxy-2-oxo-azetidin,
nach Patentanspruch 3.

11. (3S,4R)-4-Acetylthio-3-phenoxyacetoxy-2-oxo-azetidin,
nach Patentanspruch 3.

12. 3-Äthyl-4-(4-p-nitrobenzyloxycarbonylamino-butyryl-thio)-2-oxo-azetidin (racemische cis-trans-Verbindung) nach Patentanspruch 3.

13. (3S,4R)-4-(2-p-Nitrobenzyloxycarbonylaminoäthyl-thio-thiocarbonylthio)-3-methoxy-2-oxoazetidin
nach Patentanspruch 3.

14. (3S,4R)-4-(4-p-Nitrobenzyloxycarbonylamino-butyrylthio)-3-methoxy-2-oxoazetidin
nach Patentanspruch 3.

15. 3-Äthyl-4-(2-acetylaminoäthylthio-thiocarbonylthio)-2-oxoazetidin
(racemische cis,trans-Verbindung) nach Patentanspruch 3.

16. Verbindungen der Formel

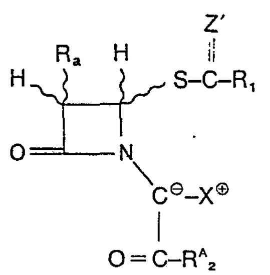

(II),

worin $R_a$, $R_1$, Z', $X^{\oplus}$ und $R_2^A$ die im Patentanspruch 1 angegebenen Bedeutungen haben, in racemischer oder optisch aktiver Form.

17. Verbindungen der Formel II nach Patentanspruch 16, worin $R_a$, $R_1$, Z', $X^{\oplus}$ und $R_2^A$ die im Patentanspruch 2 angegebenen Bedeutungen haben, in racemischer oder optisch aktiver Form.

18. Verbindungen der Formel II, nach Patentanspruch 16 oder 17, worin $R_a$ Methyl, Äthyl, Isopropyl, Benzyl, Methoxy oder Phenoxyacetoxy, $R_1$ Methyl, 3-(p-Nitrobenzyloxycarbonyl-amino)propyl, Äthylthio, 2-Acetylaminoäthylthio oder 2-(p-Nitrobenzyloxycarbonylamino) äthylthio, Z' Sauerstoff oder Schwefel, $R_2^A$ p-Nitrobenzyloxy und $X^{\oplus}$ die Gruppe $P^{\ominus}$ $(Phenyl)_3$ bedeuten.

19. Verbindungen der Formel

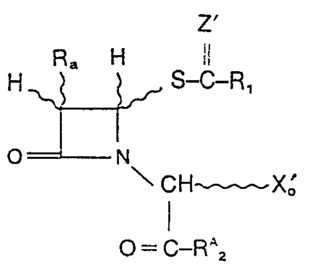

(V) oder (VI),

worin $R_a$, $R_1$, Z' und $R_2^A$ die im Patentanspruch 1 angegebenen Bedeutungen haben und $X_0'$ für Hydroxy oder eine reaktionsfähige veresterte Hydroxygruppe steht, in racemischer oder optisch aktiver Form.

20. Verbindungen der Formel V oder VI nach Patentanspruch 19, worin $R_a$, $R_1$, Z' und $R_2^A$ die im Patentanspruch 2 angegebenen Bedeutungen haben und $X_0'$ die im Patentanspruch 19 angegebenen Bedeutungen hat, in racemischer oder optisch aktiver Form.

21. Verbindungen der Formel V oder VI nach Patentanspruch 19 oder 20, worin $R_a$ Methyl, Äthyl, Isopropyl, Benzyl, Methoxy oder Phenoxyacetoxy, $R_1$ Methyl, 3-(p-Nitrobenzyloxycarbonylamino)-propyl, Äthylthio, 2-Acetylaminoäthylthio oder 2-(p-Nitrobenzyloxycarbonylamino) äthylthio, Z' Sauerstoff oder Schwefel, $R_2^A$ p-Nitrobenzyloxy und $X_0'$ Hydroxy oder Chlor, bedeuten.

22. Verbindungen der Formel

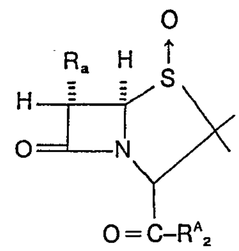

(VIII),

worin $R_a$ und $R_2^A$ die im Patentanspruch 1 genannten Bedeutungen haben.

23. Verbindungen der Formel VIII nach Patentanspruch 22, worin $R_a$ und $R_2^A$ die im Patentanspruch 2 angegebenen Bedeutungen haben.

24. Verbindungen der Formel VIII nach Patentanspruch 22 oder 23, worin $R_a$ Methoxy oder Phenoxyacetoxy und $R_2^A$ Methoxy oder 2,2,2-Trichloräthoxy bedeuten.

25. Verbindungen der Formel

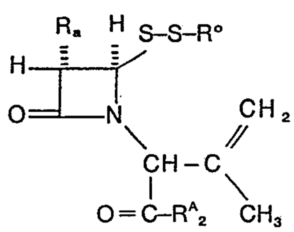

(IX),

worin $R_a$ und $R_2^A$ die im Patentanspruch 1 angegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist.

26. Verbindungen der Formel IX nach Patentanspruch 25, worin $R_a$ und $R_2^A$ die im Patentanspruch 2 angegebenen Bedeutungen haben und $R^0$ die im Patentanspruch 25 angegebenen Bedeutungen hat.

27. Verbindungen der Formel IX, nach Patentanspruch 25 oder 26, worin $R_a$ Methoxy oder Phenoxyacetoxy, $R_2^A$ Methoxy oder 2,2,2-Trichloräthoxy und $R^0$ 2-Benzthiazolyl bedeuten.

28. Verbindungen der Formel

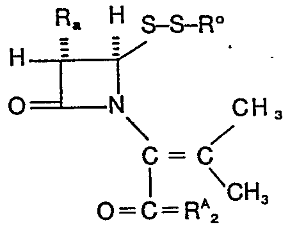

(X),

worin $R_a$ und $R_2^A$ die im Patentanspruch 1 angegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist.

29. Verbindungen der Formel X nach Patentanspruch 28, worin $R_a$ und $R_2^A$ die im Patentanspruch 2 angegebenen Bedeutungen haben und $R^0$ die im Patentanspruch 28 angegebenen Bedeutungen hat.

30. Verbindungen der Formel X, nach Patentanspruch 28 oder 29, worin $R_a$ Methoxy oder Phenoxyacetoxy, $R_2^A$ Methoxy oder 2,2,2-Trichloräthoxy und $R^0$ 2-Benzthiazolyl bedeuten.

31. Verbindungen der Formel

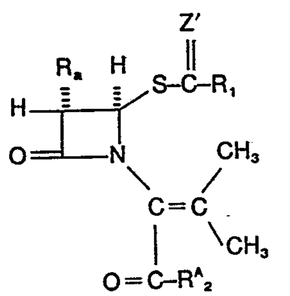

(XI),

worin $R_a$, $R_1$, Z′ und $R_2^A$ die im Patentanspruch 1 angegebenen Bedeutungen haben.

32. Verbindungen der Formel XI nach Patentanspruch 31, worin $R_a$, $R_1$, Z′ und $R_2^A$ die im Patentanspruch 2 angegebenen Bedeutungen haben.

33. Verbindungen der Formel XI, nach Patentanspruch 31 oder 32, worin $R_a$ Methyl, Methoxy oder Phenoxyacetoxy, $R_1$ Methyl, Z′ Sauerstoff und $R_2^A$ Methoxy oder 2,2,2-Trichloräthoxy bedeuten.

34. Verbindungen der Formel

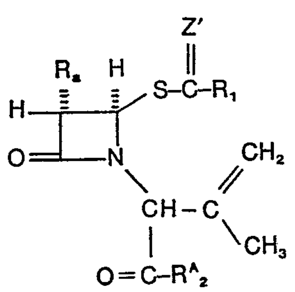

(Xa),

worin $R_a$, $R_1$, Z′ und $R_2^A$ die im Patentanspruch 1 angegebenen Bedeutungen haben.

35. Verbindungen der Formel Xa nach Patentanspruch 34, worin $R_a$, $R_1$, Z′ und $R_2^A$ die im Patentanspruch 2 angegebenen Bedeutungen haben.

36. Verbindungen der Formel

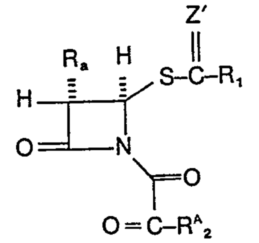

(XII),

worin $R_a$, $R_1$, Z′ und $R_2^A$ die im Patentanspruch 1 angegebenen Bedeutungen haben.

37. Verbindungen der Formel XII nach Patentanspruch 36, worin $R_a$, $R_1$, Z′ und $R_2^A$ die im Patentanspruch 2 angegebenen Bedeutungen haben.

38. Verbindungen der Formel XII, nach Patentanspruch 36 oder 37, worin $R_a$ Methyl, Methoxy oder Phenoxyacetoxy, $R_1$ Methyl, Z′ Sauerstoff und $R_2^A$ Methoxy oder 2,2,2-Trichloräthoxy bedeuten.

39. Verbindungen der Formel

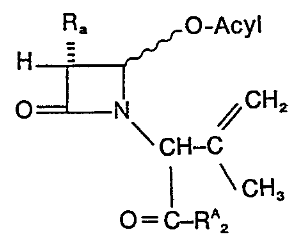

(XIII),

worin $R_a$ und $R_2^A$ die im Patentanspruch 1 angegebenen Bedeutungen haben und Acyl der Acylrest einer organischen Carbonsäure ist in der cis,. trans-, der cis- oder der trans-Form.

40. Verbindungen der Formel XIII, nach Patentanspruch 39, worin $R_a$ Methyl, Methoxy, $R_2^A$ 2,2,2-Trichloräthoxy und Acyl eine Acetylgruppe bedeuten, in der cis,trans-, der cis- oder trans-Form.

41. Verbindungen der Formel

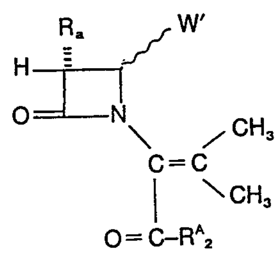

(XIV),

worin $R_a$ und $R_2^A$ die im Patentanspruch 1 angegebenen Bedeutungen haben, W′ Halogen oder Acyloxy bedeutet, worin Acyl der Acylrest einer organischen Carbonsäure ist, in der cis,trans-, der cis- oder trans-Form, mit Ausnahme von Verbindungen der Formel XIV, worin $R_a$ Alkyl, Alkoxyl, Aryl, Aralkyl, Heterocyclyl oder Heterocyclylalkyl ist, wobei diese Reste unsubstituiert oder durch Amino, geschütztes Amino, Mono- oder Dialkylamino, Hydroxy, geschütztes Hydroxy, Alkoxy, Mercapto, Alkylthio, Nitro, Chlor, Brom, Fluor,

Cyan, Carboxyl oder geschütztes Carboxyl substituiert sind, W' Acetyloxy bedeutet und $R_2^A$ zusammen mit der Carbonylgruppe –C(=O)– ein durch eine Carboxylschutzgruppe geschützte Carboxylgruppe darstellt.

42. Verbindungen der Formel XIV, nach Patentanspruch 41, worin $R_a$ Methoxy, $R_2^A$ 2,2,2-Trichloräthoxy und W' Chlor bedeutet, in der cis,trans-, der cis- oder der trans-Form.

43. Verbindungen der Formel

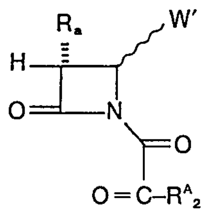

(XV),

worin $R_a$ und $R_2^A$ die im Patentanspruch 1 angegebenen Bedeutungen haben, W' Halogen oder Acyloxy bedeutet, worin Acyl der Acylrest einer organischen Carbonsäure ist, in der cis,trans-, der cis- oder der trans-Form.

44. Verbindungen der Formel XI, nach Patentanspruch 43, worin $R_a$ Methoxy, $R_2^A$ 2,2,2-Trichloräthoxy und W' Acetoxy oder Chlor bedeuten, in der cis,trans-, der cis- oder der trans-Form.

45. Verbindungen der Formel

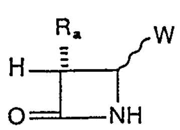

(IIIa),

worin $R_a$ die im Anspruch 1 angegebenen Bedeutungen hat und W eine nucleofuge Abgangsgruppe bedeutet, in der cis,trans-, der cis- oder der trans-Form, mit Ausnahme von Verbindungen der Formel IIIa, worin $R_a$ Alkyl, Alkoxyl, Aryl, Aralkyl, Heterocyclyl oder Heterocyclylalkyl ist, wobei diese Reste unsubstituiert oder durch Amino, geschütztes Amino, Mono- oder Dialkylamino, Hydroxy, geschütztes Hydroxy, Alkoxy, Mercapto, Alkylthio, Nitro, Chlor, Brom, Fluor, Cyan, Carboxyl oder geschütztes Carboxyl substituiert sind und W Acetyloxy bedeutet.

46. Verbindungen der Formel IIIa, nach Patentanspruch 45, worin $R_a$ Methoxy und W Chlor bedeutet, in der cis,trans-, der cis- oder der trans-Form.

47. Die einzelnen Verfahrensstufen nach Patentanspruch 1, mit der Massgabe, dass man gemäss Stufe 1.1 Verbindungen der Formel IV herstellt, worin $R_a$, $R_1$ und Z' die in Anspruch 3 angegebenen Bedeutungen haben.

48. Die einzelnen Verfahrensstufen nach Patentanspruch 1 gemäss dem Reaktionsschema 3.

49. Die einzelnen Verfahrensstufen nach Patentanspruch 1 gemäss den Reaktionsschemata 1 und 2, mit der Massgabe, dass man gemäss Stufe 1.1 Verbindungen der Formel IV herstellt, wobei $R_a$, $R_1$ und Z' die im Anspruch 3 angegebenen Bedeutungen haben.

**Patentansprüche für den Vertragsstaat: IT**

1. Verfahren zur Herstellung von Verbindungen der Formel

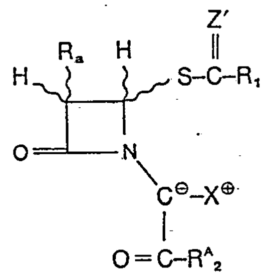

(II),

worin $R_a$ ein gesättigter oder ungesättigter, gegebenenfalls substituierter, aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel,

in welchen Resten $R_a$ die gegebenenfalls vorhandenen Substituenten Hydroxy, Niederalkoxy, Niederalkanoyloxy, in Salzform vorliegendes Hydroxysulfonyloxy, Halogen, Mercapto, Niederalkylmercapto, Carboxyl, Niederalkoxycarbonyl, Carbamoyl, Cyan, Nitro, in Salzform vorliegendes Sulfo, oder gegebenenfalls durch Niederalkyl oder $C_1$–$C_{20}$-Acyl mono- oder disubstituiertes oder durch Niederalkylen disubstituiertes Amino sind,

gegebenenfalls geschütztes Carboxyl,

Hydroxy,

Mercapto,

durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy oder Mercapto, worin die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$–$C_{20}$-Acyl mono- oder disubstituiertes oder durch Niederalkylen disubstituiertes Amino sind,

durch einen Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen verestertes Hydroxy oder Mercapto,

worin die gegebenenfalls vorhandenen Substituenten Hydroxy, Niederalkoxy, Phenyloxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$–$C_{20}$-Acyl mono- oder disubstituiertes oder durch Niederalkylen disubstituiertes Amino sind, oder Halogen ist,

$R_1$ Wasserstoff, ein gesättigter oder ungesättigter, gegebenenfalls substituierter aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, oder

ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/ oder Schwefel ist,

in welchen Resten $R_1$ die gegebenenfalls vorhandenen Substituenten Hydroxy, Niederalkoxy, Niederalkanoyloxy, Halogen, Mercapto, Niederalkylmercapto, gegebenenfalls durch Niederalkyl, Hydroxyniederalkyl, Carboxyniederalkyl, Aminoniederalkyl, Diniederalkylaminoniederalkyl, in Salzform vorliegendes Sulfoniederalkyl, Cycloalkyl, Aryl, Arylniederalkyl, Halogen, Amino, Mono- oder Di-niederalkylamino, Nitro, Hydroxy, Niederalkoxy, Carboxyl, Niederalkoxycarbonyl, Carbamoyl, N-Mono- oder N,N-Diniederalkylcarbamoyl, Cyan, Oxo oder Oxido substituiertes Heterocyclylmercapto, Carboxyl, Niederalkoxycarbonyl, Carbamoyl, Cyan, Nitro, Amino, Niederalkylamino, Diniederalkylamino, $C_1$–$C_{20}$-Acylamino, Di-$C_1$–$C_{20}$-acylamino oder Niederalkylenamino sind, oder worin $R_1$ eine durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen, verätherte Mercaptogruppe ist,

worin die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, gegebenenfalls durch Niederalkyl oder $C_1$–$C_{20}$-Acyl mono- oder disubstituiertes oder durch Niederalkylen disubstituiertes Amino sind, oder worin $R_1$ durch Niederalkyl substituiertes Phenylthio oder Phenylniederalkylthio ist oder worin $R_1$ gegebenenfalls geschütztes Carboxyl ist, oder worin

$R_1$ eine durch einen gegebenenfalls substituierten heterocyclischen Rest verätherte Mercaptogruppe ist, worin der Heterocyclylrest über ein Ringkohlenstoffatom an die Mercaptogruppe gebunden ist und 1 bis 4 Ringstickstoffatome und gegebenenfalls ein weiteres Ringheteroatom der Gruppe Sauerstoff und Schwefel enthält und worin die gegebenenfalls vorhandenen Substituenten Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$–$C_{20}$-Acylamino oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl, Oxazolyl, Halogen, gegebenenfalls durch Niederalkyl mono- oder disubstituiertes Amino, $C_1$–$C_{20}$-Acylamino, Nitro, Hydroxy, Niederalkoxy, Carboxy, Niederalkoxycarbonyl, gegebenenfalls N-mono- oder N,N-diniederalkyliertes Carbamoyl, Cyan, Oxo oder Oxido sind, wobei funktionelle Gruppen in diesen Resten $R_a$ und $R_1$ vorzugsweise in geschützter Form vorliegen und die mit «Nieder» bezeichneten Gruppen bis zu 7, insbesondere bis zu 4 Kohlenstoffatome enthalten, Z′ Sauerstoff, Schwefel oder eine gegebenenfalls einen oder zwei Substituenten tragende Methylidengruppe bedeutet, $X^{\oplus}$ entweder eine dreifach substituierte Phosphoniogruppe oder eine zweifach veresterte Phosphonogruppe zusammen mit einem Kation bedeutet und $R_2^A$ zusammen mit der Carbonylgruppe $-C(=O)-$ eine geschützte Carboxylgruppe darstellt, in racemischer oder optisch aktiver Form, dadurch gekennzeichnet, dass man die Stufen 1.1 bis 1.4 gemäss

Reaktionsschema 1

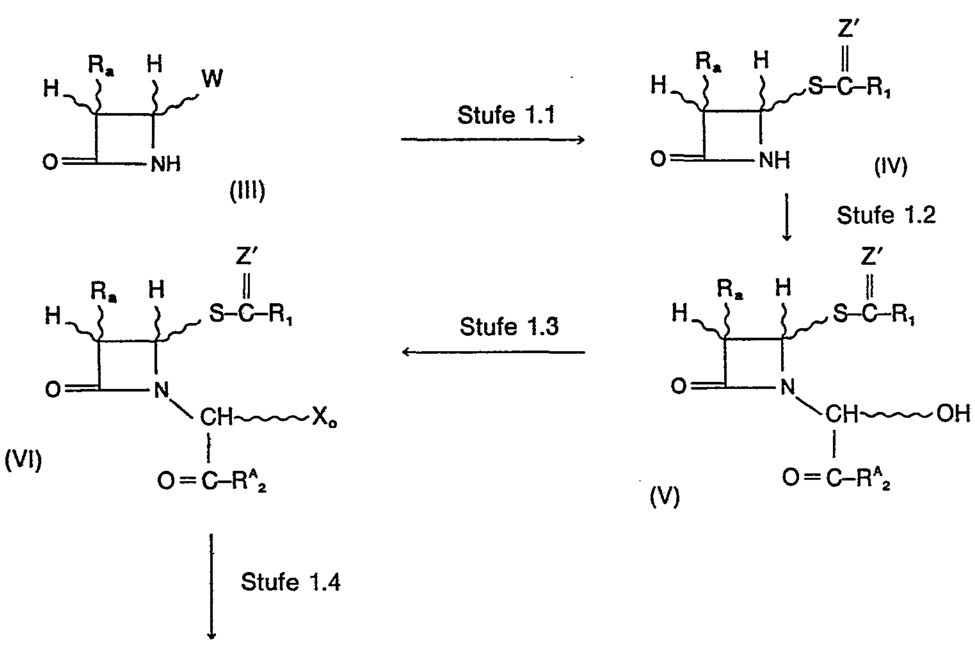

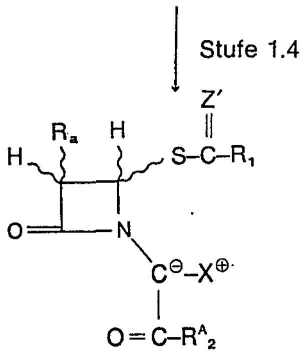


(II)

oder die Stufen 2.1 bis 2.6 gemäss

Reaktionsschema 2

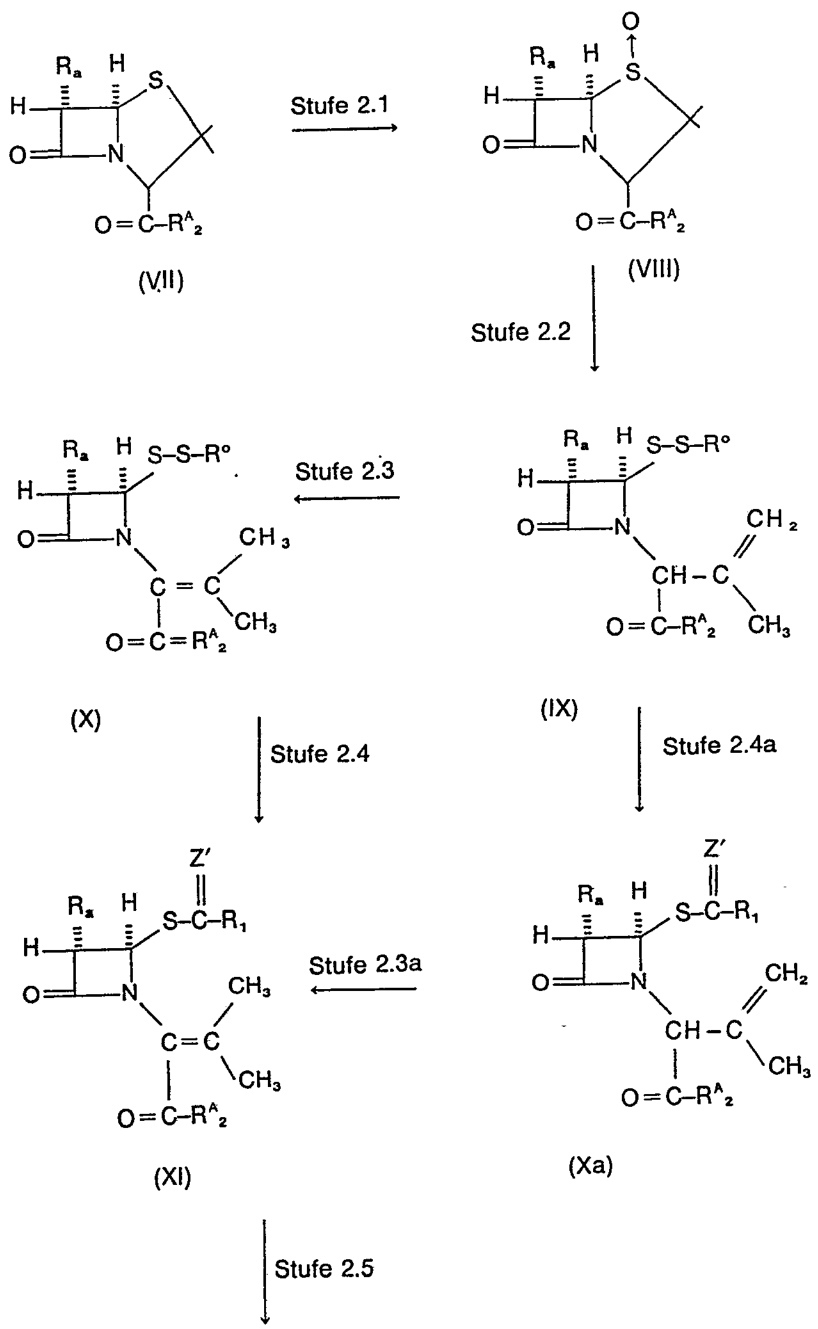


(VII) (VIII) (X) (IX) (XI) (Xa)

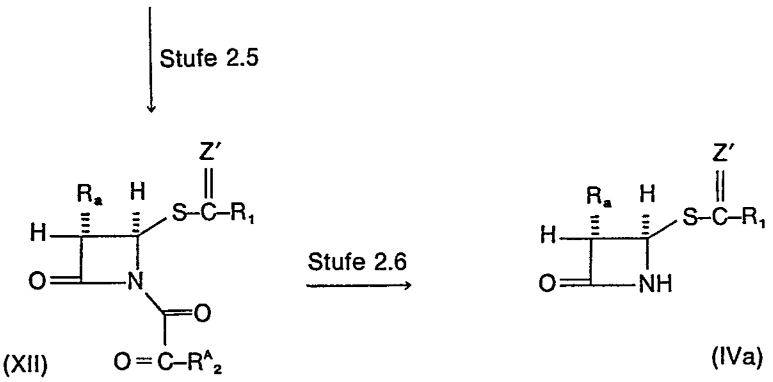


(XII) (IVa)

gefolgt von Stufen 1.2 bis 1.4, oder die Stufen 3.1 bis 3.5 gemäss

Reaktionsschema 3

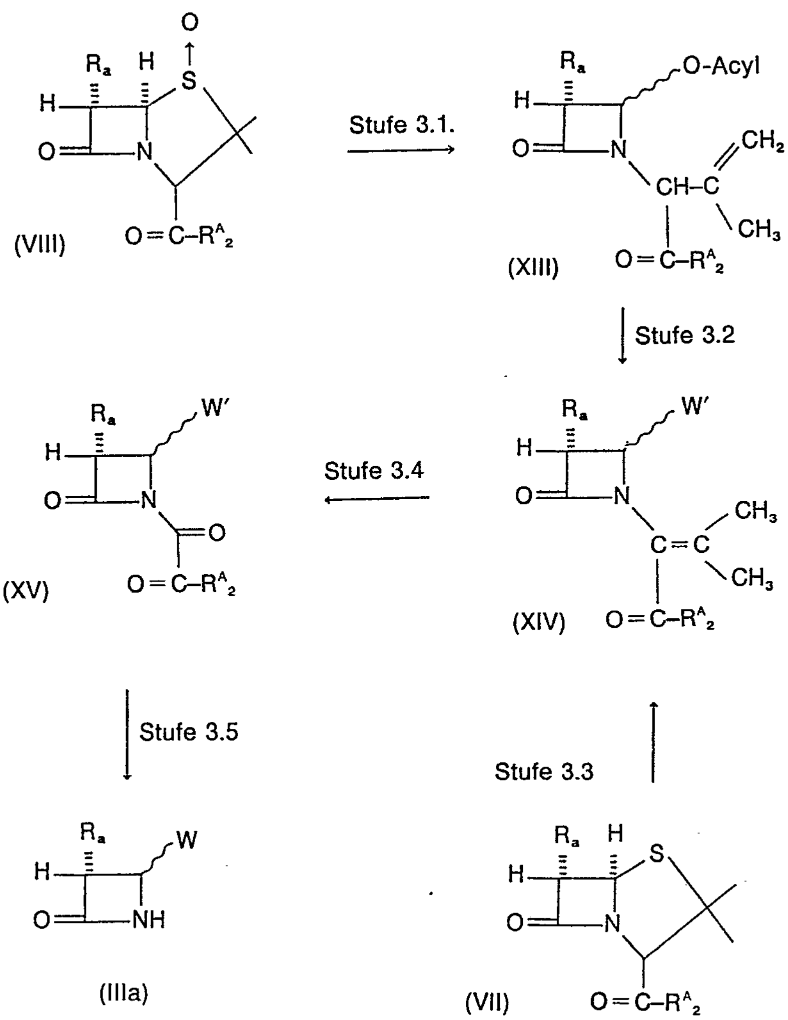


(VIII) (XIII) (XV) (XIV) (IIIa) (VII)

gefolgt von den Stufen 1.1 bis 1.4, durchführt, wobei man gemäss Stufe 1.1 zur Herstellung von Verbindungen der Formel

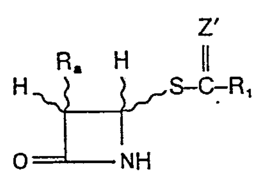

(IV) oder (IVa),

worin $R_a$, $R_1$ und Z′ die unter Formel II genannten Bedeutungen haben, in racemischer oder optisch aktiver Form, ein 4-W-Azetidinon der Formel

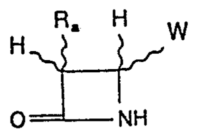

(III)

oder

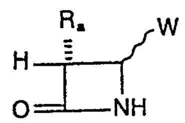

(IIIa),

worin W eine nucleofuge Abgangsgruppe bedeutet, mit einer Mercaptoverbindung $R_1$–C(=Z′)–SH oder einem Salz davon behandelt, und gewünschtenfalls ein erhaltenes Isomerengemisch in die einzelnen Isomeren auftrennt, oder, gemäss Stufe 2.6 zur Herstellung von optisch aktiven (3S,4R)-Verbindungen der Formel

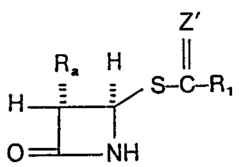

(IVa),

eine Verbindung der Formel

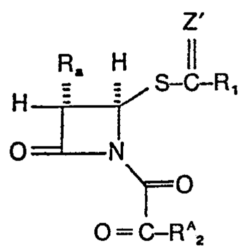

(XII),

worin $R_a$, $R_1$, Z′ und $R_2^A$ die unter Formel II angegebenen Bedeutungen haben, solvolysiert, und wenn erwünscht in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, gemäss Stufe 1.2 zur Herstellung von Verbindungen der Formel

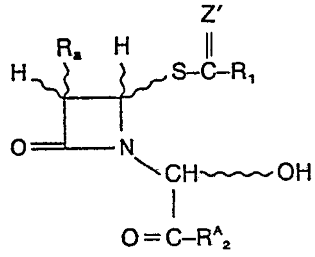

(V)

worin $R_a$, $R_1$, Z′ und $R_2^A$ die unter Formel II gegebenen Bedeutungen haben, in racemischer oder optisch aktiver Form, eine Verbindung der Formel

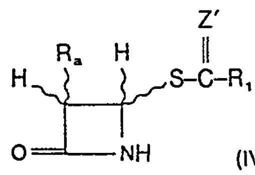

oder

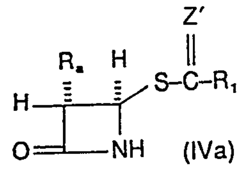

(IVa),

worin $R_a$, $R_1$ und Z′ die oben gegebenen Bedeutungen haben, mit einer Glyoxylsäure-Verbindung der Formel OHC–C(=O)–$R_2^A$ oder einem geeigneten Derivat davon umsetzt, und gewünschtenfalls ein erhaltenes Isomerengemisch in die einzelnen Isomeren auftrennt, und/oder gewünschtenfalls eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, gemäss Stufe 1.3 zur Herstellung von Verbindungen der Formel

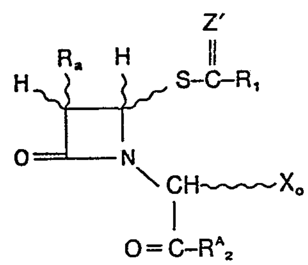

(VI),

worin $R_a$, $R_1$, Z′ und $R_2^A$ die unter Formel II gegebenen Bedeutungen haben und $X_0$ für eine reaktionsfähige veresterte Hydroxygruppe steht, in racemischer oder optisch aktiver Form, in einer Verbindung der Formel

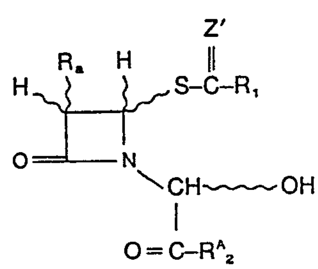

(V),

worin $R_a$, $R_1$, Z′ und $R_2^A$ die oben gegebenen Bedeutungen haben, die sekundäre Hydroxygruppe in eine reaktionsfähige veresterte Hydroxygruppe umwandelt, und gewünschtenfalls ein erhaltenes Isomerengemisch in die einzelnen Isomeren auftrennt, und/oder gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, und gemäss Stufe 1.4, zur Herstellung von Verbindungen der Formel II, eine Verbindung der Formel

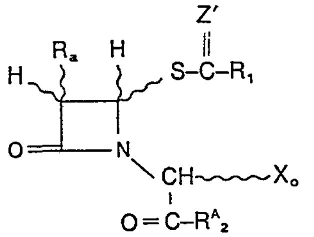

(VI),

worin $X_0$ für eine reaktionsfähige veresterte Hydroxygruppe steht, und $R_a$, $R_1$, Z' und $R_2^A$ die oben gegebenen Bedeutungen haben, mit einer geeigneten Phosphin-Verbindung oder einer geeigneten Phosphit-Verbindung behandelt, oder eine Verbindung der Formel

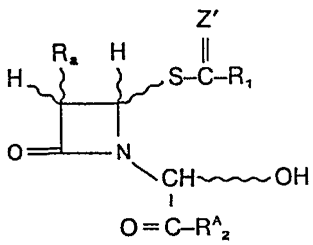

(V),

worin $R_a$, $R_1$, Z' und $R_2^A$ die oben gegebenen Bedeutungen haben, mit Tetrachlorkohlenstoff und einem Phosphin behandelt, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt, oder gemäss Stufe 2.1, zur Herstellung von Verbindungen der Formel

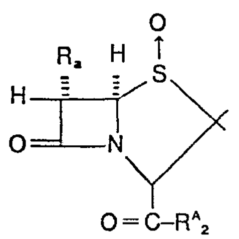

(VIII),

worin $R_a$ und $R_2^A$ die unter Formel II gegebenen Bedeutungen haben, eine Penicillansäureverbindung der Formel

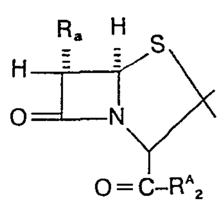

(VII),

in 1-Stellung oxidiert, und gewünschtenfalls in einer erhaltenen Verbindung eine Gruppe $R_a$ in eine andere Gruppe $R_a$ überführt, gemäss Stufe 2.2 zur Herstellung von Verbindungen der Formel

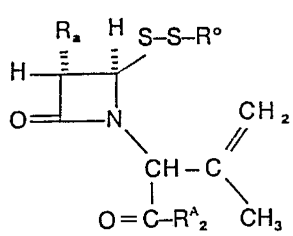

(IX),

worin $R_a$ und $R_2^A$ die unter Formel II gegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist, eine Verbindung der Formel

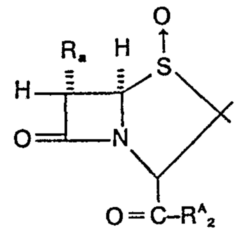

(VIII),

mit einer Mercaptoverbindung $R^0$–SH behandelt, und gewünschtenfalls in einer erhaltenen Verbindung eine Gruppe $R_a$ in eine andere Gruppe $R_a$ umwandelt, gemäss Stufe 2.3 zur Herstellung von Verbindungen der Formel

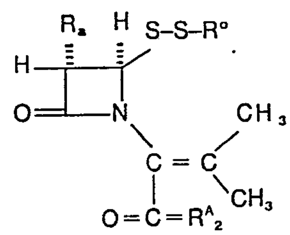

(X),

worin $R_a$ und $R_2^A$ die unter Formel II gegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist, eine 3-Methylenbuttersäureverbindung der Formel

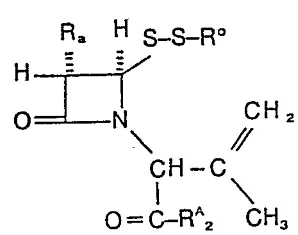

(IX),

durch Behandeln mit einem geeigneten basischen Mittel isomerisiert, und gewünschtenfalls in einer erhaltenen Verbindung eine Gruppe $R_a$ in eine andere Gruppe $R_a$ umwandelt, gemäss Stufe 2.4 zur Herstellung von Verbindungen der Formel

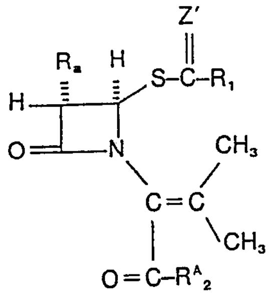

(XI),

worin $R_a$, $R_1$, Z′ und $R_2^A$ die unter Formel II gegebenen Bedeutungen haben, eine Verbindung der Formel

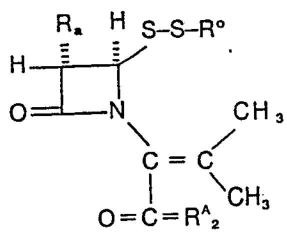

(X),

worin $R^0$ eine gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist, und $R_a$ und $R_2^A$ die oben angegebenen Bedeutungen haben, mit einem geeigneten Reduktionsmittel behandelt und gleichzeitig oder nachträglich mit einem Acylierungsderivat einer Carbonsäure der Formel $R_1$–C(=Z)–OH, oder, wenn Z′ eine gegebenenfalls durch Y substituierte Methylidengruppe bedeutet, mit einem Alkin der Formel $R_1$–C≡C–Y, umsetzt, oder gemäss Stufe 2.3a eine Verbindung der Formel

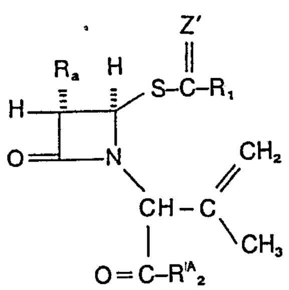

(Xa),

worin $R_a$, $R_1$, Z′ und $R_2^A$ die oben gegebenen Bedeutungen haben, durch Behandeln mit einem geeigneten basischen Mittel isomerisiert, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z′ in eine Oxogruppe Z überführt, gemäss Stufe 2.4a zur Herstellung von Verbindungen der Formel

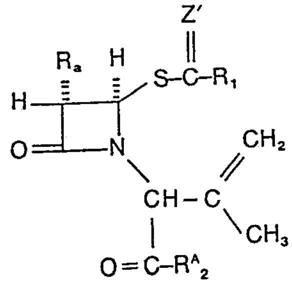

(Xa),

worin $R_a$, $R_1$, Z′ und $R_2^A$ die unter Formel II angegebenen Bedeutungen haben, eine Verbindung der Formel

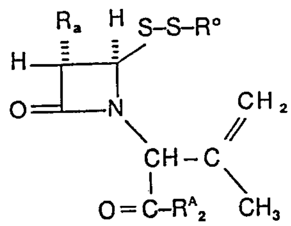

(IX),

worin $R^0$, $R_a$ und $R_2^A$ die oben gegebenen Bedeutungen haben, mit einem geeigneten Reduktionsmittel behandelt und gleichzeitig oder nachträglich mit einem Acylierungsderivat einer Carbonsäure der Formel $R_1$–C(=Z)–OH, oder, wenn Z′ eine gegebenenfalls durch Y substituierte Methylidengruppe bedeutet, mit einem Alkin der Formel $R_1$–C≡C–Y umsetzt und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z′ in eine Oxogruppe Z überführt, gemäss Stufe 2.5 zur Herstellung von Verbindungen der Formel

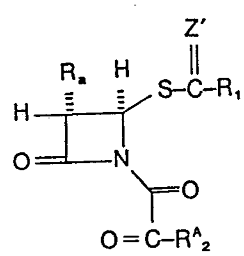

(XII),

worin $R_a$, $R_1$, Z′ und $R_2^A$ die unter Formel II gegebenen Bedeutungen haben, eine Verbindung der Formel

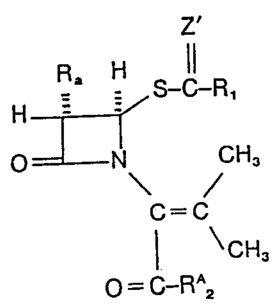

(XI),

worin $R_a$, $R_1$, Z′ und $R_2^A$ die oben gegebenen Bedeutungen haben, ozonisiert und das gebildete

Ozonid reduktiv zur Oxogruppe spaltet, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z′ in eine Oxogruppe Z überführt, und anschliessend die Stufen 2.6, und 1.2 bis 1.4 durchführt, oder, gemäss Stufe 3.1 zur Herstellung von Verbindungen der Formel

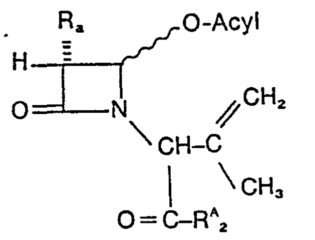
(XIII),

worin $R_a$ und $R_2^A$ die unter Formel II genannten Bedeutungen haben und Acyl der Acylrest einer organischen Carbonsäure ist, in der cis,trans-, der cis- oder der trans-Form, ein 1-Oxid einer Penicillansäureverbindung der Formel

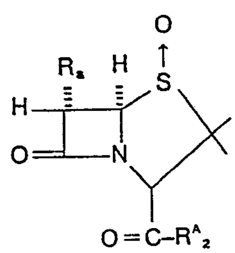
(VIII),

worin $R_a$ und $R_2^A$ die oben angegebenen Bedeutungen haben, in Gegenwart eines Triniederalkylphosphits mit einer organischen Carbonsäure Acyl–OH behandelt, und gewünschtenfalls in einer erhaltenen Verbindung eine Gruppe $R_a$ in eine andere Gruppe $R_a$ umwandelt, und/oder aus einer erhaltenen cis,trans-Verbindung die cis- und/oder die trans-Verbindung isoliert, gemäss Stufe 3.2 zur Herstellung von Verbindungen der Formel

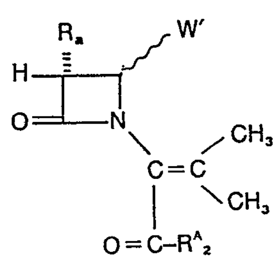
(XIV),

worin $R_a$ und $R_2^A$ die unter Formel II gegebenen Bedeutungen haben und W′ für Acyloxy steht, worin Acyl der Acylrest einer organischen Carbonsäure ist, und $R_2^A$ zusammen mit der Carbonylgruppe –C(=O)– eine geschützte Carboxylgruppe darstellt, in der cis,trans-, der cis- oder der trans-Form, eine Verbindung der Formel

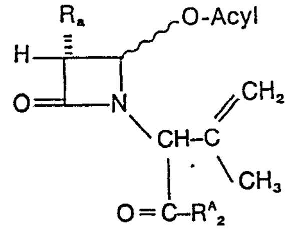
(XIII),

worin $R_a$, Acyl und $R_2^A$ die oben angegebenen Bedeutungen haben, durch Behandeln mit einem geeigneten basischen Mittel isomerisiert, und gewünschtenfalls in einer erhaltenen Verbindung eine Gruppe $R_a$ in eine andere Gruppe $R_a$ umwandelt, und/oder gewünschtenfalls aus einer erhaltenen cis,trans-Verbindung die cis- und/oder die trans-Verbindung isoliert, oder gemäss Stufe 3.3 zur Herstellung von Verbindung der Formel

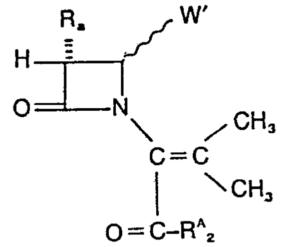
(XIV),

worin $R_a$ und $R_2^A$ die unter Formel II gegebenen Bedeutungen haben und W′ Halogen bedeutet, in der cis,trans-, der cis- oder der trans-Form, eine Penicillansäureverbindung der Formel

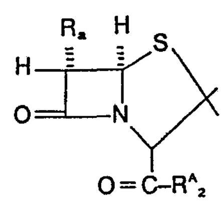
(VII),

worin $R_a$ und $R_2^A$ die oben angegebenen Bedeutungen haben, mit einem positive Halogenionen liefernden Halogenierungsmittel umsetzt und, falls erforderlich, ein gegebenenfalls erhaltenes Zwischenprodukt mit einer Base behandelt, und gewünschtenfalls in einer erhaltenen Verbindung der Formel XIV eine Gruppe $R_a$ in eine andere Gruppe $R_a$ überführt und/oder aus einer erhaltenen cis,trans-Verbindung die cis- und/oder die trans-Verbindung isoliert, gemäss Stufe 3.4 zur Herstellung von Verbindungen der Formel

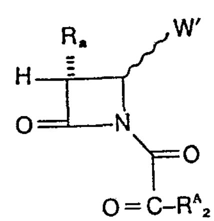
(XV),

worin $R_a$ und $R_2^A$ die unter Formel II gegebenen Bedeutungen haben und W′ Halogen oder Acyloxy bedeutet, worin Acyl der Acylrest einer organischen Carbonsäure ist, in der cis,trans-, der cis- oder der trans-Form, eine Verbindung der Formel

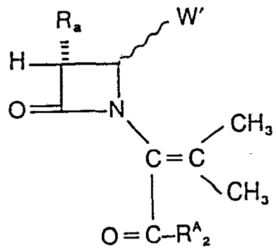

(XIV),

worin $R_a$, W' und $R_2^A$ die oben angegebenen Bedeutungen haben, ozonisiert und das gebildete Ozonid reduktiv zur Oxogruppe spaltet, und gewünschtenfalls in einer erhaltenen Verbindung eine Gruppe $R_a$ in eine andere Gruppe $R_a$ umwandelt, und/oder gewünschtenfalls aus einer erhaltenen cis,trans-Verbindung die cis- und/oder die trans-Verbindung isoliert, gemäss Stufe 3.5 zur Herstellung von Verbindungen der Formel

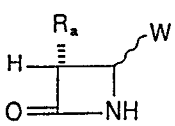

(IIIa),

worin $R_a$ die unter Formel II gegebenen Bedeutungen hat und W eine nucleofuge Abgangsgruppe bedeutet, in der cis,trans-, der cis- oder der trans-Form, eine Verbindung der Formel

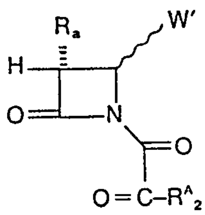

(XV),

worin $R_a$ und $R_2^A$ die oben angegebenen Bedeutungen haben, und W' Acyloxy oder Halogen bedeutet, solvolysiert, und gewünschtenfalls in einer erhaltenen Verbindung eine Gruppe $R_a$ in eine andere Gruppe $R_a$ umwandelt, und/oder gewünschtenfalls eine Gruppe W' in eine andere Gruppe W' oder in W überführt, und/oder gewünschtenfalls eine erhaltene cis-Verbindung zur entsprechenden trans-Verbindung isomerisiert, und/oder aus einer erhaltenen cis,trans-Verbindung die cis- und/oder die trans-Verbindung isoliert, und anschliessend die Stufen 1.1 bis 1.4 durchführt.

2. Verfahren nach Patentanspruch 1, dadurch gekennzeichnet, dass man Verbindungen der Formel II herstellt, worin $R_a$ Niederalkyl, Hydroxyniederalkyl, Niederalkoxyniederalkyl, Niederalkanoyloxyniederalkyl, in Salzform vorliegendes Hydroxysulfonyloxyniederalkyl, Hydroxy, Niederalkoxy, Niederalkanoyloxy, Niederalkanoyloxy substituiert durch Phenyloxy, Halogen, Amino oder Cyan, Phenylniederalkanoyloxy, oder durch Hydroxy oder Amino substituiertes Phenylniederalkanoyloxy ist, $R_1$ Wasserstoff, Niederalkyl, Hydroxyniederalkyl, Niederalkoxyniederalkyl, Niederalkanoyloxyniederalkyl, Niederalkylthioniederalkyl, Heterocyclylthioniederalkyl, worin Heterocyclyl einen gegebenenfalls durch Niederalkyl, Carboxyniederalkyl, Diniederalkylaminoniederalkyl oder in Salzform vorliegendes Sulfoniederalkyl substituierten fünfgliedrigen aromatischen diaza-, triaza-, tetraza-, thiaza-, thiadiaza-, thiatriaza-, oxaza- oder oxadiazacyclischen Rest darstellt, Aminoniederalkyl, Acylaminoniederalkyl, worin Acyl Niederalkanoyl oder eine als Aminoschutzgruppe gebräuchliche substituierte Oxycarbonylgruppe, z.B. eine tert.-Butyl-, 2,2,2-Trichloräthyl-, Diphenylmethyl- oder p-Nitrobenzyloxycarbonylgruppe, ist, Carboxyniederalkyl, Niederalkoxycarbonylniederalkyl, Phenylniederalkyl, Phenyl, Hydroxyphenyl, Aminophenyl, Furyl, Thienyl, Pyridyl, Niederalkylthio, durch Amino, Mono- oder Diniederalkylamino oder Niederalkanoylamino substituiertes Niederalkylthio oder Niederalkenylthio, oder gegebenenfalls durch Niederalkyl, Sulfoniederalkyl, Carboxyniederalkyl oder Diniederalkylaminoniederalkyl substituiertes Tetrazolylthio oder Thiadiazolylthio darstellt, wobei funktionelle Gruppen in solchen Resten $R_a$ und $R_1$ vorzugsweise in geschützter Form vorliegen, und Z', $X^{\oplus}$ und $R_2^A$ die im Patentanspruch 1 angegebenen Bedeutungen haben, in racemischer oder optisch aktiver Form.

3. Verbindungen der Formel

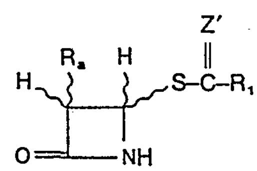

(IV),

worin $R_a$, $R_1$ und Z' die in Anspruch 1 angegebenen Bedeutungen haben in racemischer und optisch aktiver Form.

4. Verbindungen der Formel IV nach Patentanspruch 3, worin $R_a$, $R_1$ und Z' die im Patentanspruch 2 angegebenen Bedeutungen haben.

5. 4-Acetylthio-3-methyl-2-oxo-azetidin (racemische cis-Verbindung) nach Patentanspruch 3.

6. 4-Acetylthio-3-methyl-2-oxo-azetidin (racemische trans-Verbindung) nach Patentanspruch 3.

7. 4-Acetylthio-3-isopropyl-2-oxo-azetidin (racemische trans-Verbindung), nach Patentanspruch 3.

8. 4-Acetylthio-3-benzyl-2-oxo-azetidin (racemische trans-Verbindung), nach Patentanspruch 3.

9. 4-Äthylthio-thiocarbonylthio-3-isopropyl-2-oxoazetidin (racemische trans-Verbindung), nach Patentanspruch 3.

10. (3S,4R)-4-Acetylthio-3-methoxy-2-oxo-azetidin, nach Patentanspruch 3.

11. (3S,4R)-4-Acetylthio-3-phenoxyacetoxy-2-oxo-azetidin, nach Patentanspruch 3.

12. 3-Äthyl-4-(4-p-nitrobenzyloxycarbonyl-amino-butyrylthio)-2-oxo-azetidin (racemische cis-trans-Verbindung) nach Patentanspruch 3.

13. (3S,4R)-4-(2-p-Nitrobenzyloxycarbonyl-aminoäthyl-thio-thiocarbonylthio)-3-methoxy-2-oxoazetidin nach Patentanspruch 3.

14. (3S,4R)-4-(4-p-Nitrobenzyloxycarbonyl-amino-butyrylthio)-3-methoxy-2-oxoazetidin nach Patentanspruch 3.

15. 3-Äthyl-4-(2-acetylaminoäthylthio-thiocarbonylthio)-2-oxoazetidin (racemische cis,trans-Verbindung) nach Patentanspruch 3.

16. Verbindungen der Formel

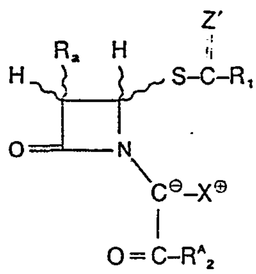

(II),

worin $R_a$, $R_1$, Z', $X^{\oplus}$ und $R_2^A$ die im Patentanspruch 1 angegebenen Bedeutungen haben, in racemischer oder optisch aktiver Form.

17. Verbindungen der Formel II nach Patentanspruch 16, worin $R_a$, $R_1$, Z', $X^{\oplus}$ und $R_2^A$ die im Patentanspruch 2 angegebenen Bedeutungen haben, in racemischer oder optisch aktiver Form.

18. Verbindungen der Formel II, nach Patentanspruch 16 oder 17, worin $R_a$ Methyl, Äthyl, Isopropyl, Benzyl, Methoxy oder Phenoxyacetoxy, $R_1$ Methyl, 3-(p-Nitrobenzyloxycarbonylamino)propyl, Äthylthio, 2-Acetylaminoäthylthio oder 2-(p-Nitrobenzyloxycarbonylamino)äthylthio, Z' Sauerstoff oder Schwefel, $R_2^A$ p-Nitrobenzyloxy und $X^{\oplus}$ die Gruppe $P^{\ominus}$ $(Phenyl)_3$ bedeuten.

19. Verbindungen der Formel

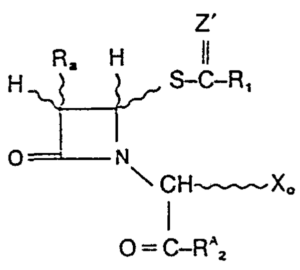

(V) oder (VI),

worin $R_a$, $R_1$, Z' und $R_2^A$ die im Patentanspruch 1 angegebenen Bedeutungen haben und $X_0'$ für Hydroxy oder eine reaktionsfähige veresterte Hydroxygruppe steht, in racemischer oder optisch aktiver Form.

20. Verbindungen der Formel V oder VI nach Patentanspruch 19, worin $R_a$, $R_1$, Z' und $R_2^A$ die im Patentanspruch 2 angegebenen Bedeutungen haben und $X_0'$ die im Patentanspruch 19 angegebenen Bedeutungen hat, in racemischer oder optisch aktiver Form.

21. Verbindungen der Formel V oder VI nach Patentanspruch 19 oder 20, worin $R_a$ Methyl, Äthyl, Isopropyl, Benzyl, Methoxy oder Phenoxyacetoxy, $R_1$ Methyl, 3-(p-Nitrobenzyloxycarbonylamino)-propyl, Äthylthio, 2-Acetylaminoäthylthio oder 2-(p-Nitrobenzyloxycarbonylamino) äthylthio, Z' Sauerstoff oder Schwefel, $R_2^A$ p-Nitrobenzyloxy und $X_0'$ Hydroxy oder Chlor, bedeuten.

22. Verbindungen der Formel

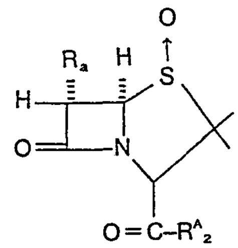

(VIII),

worin $R_a$ und $R_2^A$ die im Patentanspruch 1 genannten Bedeutungen haben.

23. Verbindungen der Formel VIII nach Patentanspruch 22, worin $R_a$ und $R_2^A$ die im Patentanspruch 2 angegebenen Bedeutungen haben.

24. Verbindungen der Formel VIII nach Patentanspruch 22 oder 23, worin $R_a$ Methoxy oder Phenoxyacetoxy und $R_2^A$ Methoxy oder 2,2,2-Trichloräthoxy bedeuten.

25. Verbindungen der Formel

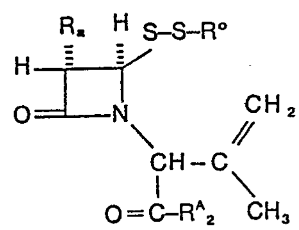

(IX),

worin $R_a$ und $R_2^A$ die im Patentanspruch 1 angegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist.

26. Verbindungen der Formel IX nach Patentanspruch 25, worin $R_a$ und $R_2^A$ die im Patentanspruch 2 angegebenen Bedeutungen haben und $R^0$ die im Patentanspruch 25 angegebenen Bedeutungen hat.

27. Verbindungen der Formel IX, nach Patentanspruch 25 oder 26, worin $R_a$ Methoxy oder Phenoxyacetoxy, $R_2^A$ Methoxy oder 2,2,2-Trichloräthoxy und $R^0$ 2-Benzthiazolyl bedeuten.

28. Verbindungen der Formel

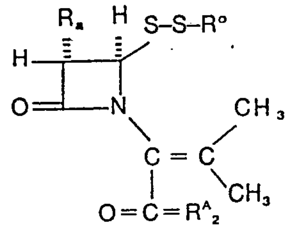
(X),

worin $R_a$ und $R_2^A$ die im Patentanspruch 1 angegebenen Bedeutungen haben und $R^0$ ein gegebenenfalls substituierter aromatischer heterocyclischer Rest mit bis zu 15 Kohlenstoffatomen, mindestens einem Ringstickstoffatom und gegebenenfalls einem weiteren Ringheteroatom ist, welcher Rest mit einem seiner Ringkohlenstoffatome, das mit einem Ringstickstoffatom durch eine Doppelbindung verbunden ist, an die Thiogruppe –S– gebunden ist.

29. Verbindungen der Formel X nach Patentanspruch 28, worin $R_a$ und $R_2^A$ die im Patentanspruch 2 angegebenen Bedeutungen haben und $R^0$ die im Patentanspruch 28 angegebenen Bedeutungen hat.

30. Verbindungen der Formel X, nach Patentanspruch 28 oder 29, worin $R_a$ Methoxy oder Phenoxyacetoxy, $R_2^A$ Methoxy oder 2,2,2-Trichloräthoxy und $R^0$ 2-Benzthiazolyl bedeuten.

31. Verbindungen der Formel

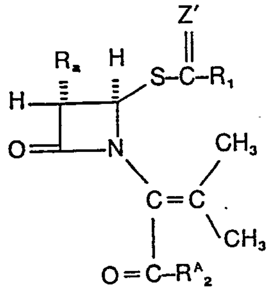
(XI),

worin $R_a$, $R_1$, Z' und $R_2^A$ die im Patentanspruch 1 angegebenen Bedeutungen haben.

32. Verbindungen der Formel XI nach Patentanspruch 31, worin $R_a$, $R_1$, Z' und $R_2^A$ die im Patentanspruch 2 angegebenen Bedeutungen haben.

33. Verbindungen der Formel XI, nach Patentanspruch 31, oder 32, worin $R_a$ Methyl, Methoxy oder Phenoxyacetoxy, $R_1$ Methyl, Z' Sauerstoff und $R_2^A$ Methoxy oder 2,2,2-Trichloräthoxy bedeuten.

34. Verbindungen der Formel

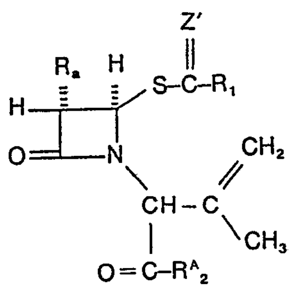
(Xa),

worin $R_a$, $R_1$, Z' und $R_2^A$ die im Patentanspruch 1 angegebenen Bedeutungen haben.

35. Verbindungen der Formel Xa nach Patentanspruch 34, worin $R_a$, $R_1$, Z' und $R_2^A$ die im Patentanspruch 2 angegebenen Bedeutungen haben.

36. Verbindungen der Formel

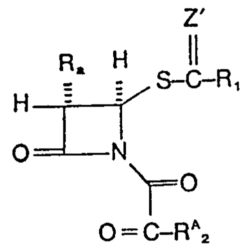
(XII),

worin $R_a$, $R_1$, Z' und $R_2^A$ die im Patentanspruch 1 angegebenen Bedeutungen haben.

37. Verbindungen der Formel XII nach Patentanspruch 36, worin $R_a$, $R_1$, Z' und $R_2^A$ die im Patentanspruch 2 angegebenen Bedeutungen haben.

38. Verbindungen der Formel XII, nach Patentanspruch 36 oder 37, worin $R_a$ Methyl, Methoxy oder Phenoxyacetoxy, $R_1$ Methyl, Z' Sauerstoff und $R_2^A$ Methoxy oder 2,2,2-Trichloräthoxy bedeuten.

39. Verbindungen der Formel

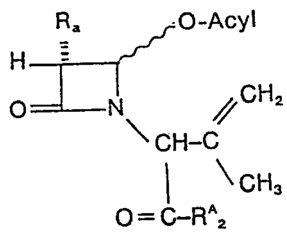
(XIII),

worin $R_a$ und $R_2^A$ die im Patentanspruch 1 angegebenen Bedeutungen haben und Acyl der Acylrest einer organischen Carbonsäure ist in der cis, trans-, der cis- oder der trans-Form.

40. Verbindungen der Formel XIII, nach Patentanspruch 39, worin $R_a$ Methyl, Methoxy, $R_2^A$ 2,2,2-Trichloräthoxy und Acyl eine Acetylgruppe bedeuten, in der cis,trans-, der cis- oder trans-Form.

41. Verbindungen der Formel

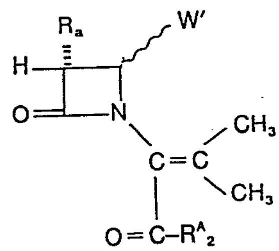
(XIV),

worin $R_a$ und $R_2^A$ die im Patentanspruch 1 angegebenen Bedeutungen haben, W' Halogen oder Acyloxy bedeutet, worin Acyl der Acylrest einer organischen Carbonsäure ist, in der cis,trans-, der cis- oder trans-Form.

42. Verbindungen der Formel XIV, nach Patentanspruch 41, worin $R_a$ Methoxy, $R_2^A$ 2,2,2-Trichloräthoxy und W' Chlor oder Acetoxy bedeuten, in der cis,trans-, der cis- oder der trans-Form.

43. Verbindungen der Formel

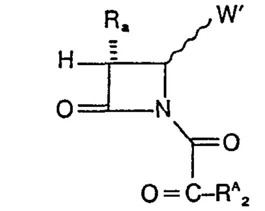

(XV),

worin $R_a$ und $R_2^A$ die im Patentanspruch 1 angegebenen Bedeutungen haben, W' Halogen oder Acyloxy bedeutet, worin Acyl der Acylrest einer organischen Carbonsäure ist, in der cis,trans-, der cis- oder der trans-Form.

44. Verbindungen der Formel XI, nach Patentanspruch 43, worin $R_a$ Methoxy, $R_2^A$ 2,2,2-Trichloräthoxy und W' Acetoxy oder Chlor bedeuten, in der cis,trans-, der cis- oder der trans-Form.

45. Verbindungen der Formel

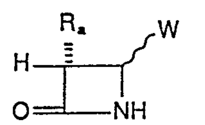

(IIIa),

worin $R_a$ die im Anspruch 1 angegebenen Bedeutungen hat und W eine nucleofuge Abgangsgruppe bedeutet, in der cis,trans-, der cis- oder der trans-Form.

46. Verbindungen der Formel IIIa, nach Patentanspruch 45, worin $R_a$ Methoxy, W Chlor oder Acetoxy bedeuten, in der cis,trans-, der cis- oder der trans-Form.

47. Die einzelnen Verfahrensstufen nach Patentanspruch 1.

48. Die einzelnen Verfahrensstufen nach Patentanspruch 1 gemäss dem Reaktionsschema 3.

49. Die einzelnen Verfahrensstufen nach Patentanspruch 1 gemäss den Reaktionsschemata 1 und 2.

**Claims for the contracting states: BE CH DE FR GB LU NL SE**

1. Process for the manufacture of compounds of the formula

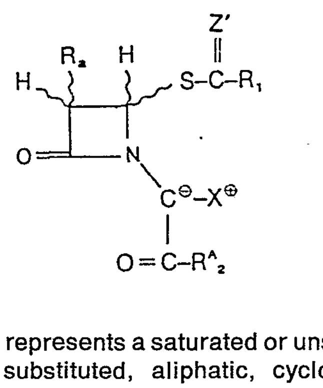

(II),

in which $R_a$ represents a saturated or unsaturated, optionally substituted, aliphatic, cycloaliphatic, cycloaliphatic-aliphatic, aromatic or araliphatic hydrocarbon radical having up to 18, preferably up to 10, carbon atoms,

an optionally substituted heterocyclyl or heterocyclyllower alkyl radical having up to 10 carbon atoms and up to 4 ring hetero atoms selected from the group nitrogen, oxygen and/or sulphur,

in which radicals $R_a$ the substituents optionally present are hydroxy, lower alkoxy, lower alkanoyloxy, hydroxysulphonyloxy present in salt form, halogen, mercapto, lower alkylmercapto, carboxy, lower alkoxycarbonyl, carbamoyl, cyano, nitro, sulpho present in salt form, or amino optionally mono- or di-substituted by lower alkyl or $C_1$–$C_{20}$-acyl or

optionally disubstituted by lower alkylene,

optionally protected carboxy,

hydroxy,

mercapto,

hydroxy or mercapto etherified by an optionally substituted aliphatic, cycloaliphatic, cycloaliphatic-aliphatic, aromatic or araliphatic hydrocarbon radical having up to 18, especially up to 10, carbon atoms, in which the substituents optionally present are hydroxy, lower alkoxy, lower alkanoyloxy, halogen, mercapto, lower alkylmercapto, carboxy, lower alkoxycarbonyl, carbamoyl, cyano, nitro, or amino optionally mono- or di-substituted by lower alkyl or $C_1$–$C_{20}$-acyl or optionally disubstituted by lower alkylene,

hydroxy or mercapto esterified by an acyl radical of an optionally substituted aliphatic, cycloaliphatic, cycloaliphatic-aliphatic, aromatic or araliphatic carboxylic acid having up to 18 carbon atoms, in which the substituents optionally present are hydroxy, lower alkoxy, phenoxy, lower alkanoyloxy, halogen, mercapto, lower alkylmercapto, carboxy, lower alkoxycarbonyl, carbamoyl, cyano, nitro, or amino optionally mono- or disubstituted by lower alkyl or $C_1$–$C_{20}$-acyl or optionally disubstituted by lower alkylene, or halogen,

$R_1$ represents hydrogen, a saturated or unsaturated, optionally substituted aliphatic, cycloaliphatic, cycloaliphatic-aliphatic, aromatic or araliphatic hydrocarbon radical having up to 18, preferably up to 10, carbon atoms, or

an optionally substituted heterocyclyl or heterocyclyllower alkyl radical having up to 10 carbon atoms and up to 4 ring hetero atoms selected from the group nitrogen, oxygen and/or sulphur,

in which radicals $R_1$ the substituents optionally present are hydroxy, lower alkoxy, lower alkanoyloxy, halogen, mercapto, lower alkylmercapto, heterocyclylmercapto optionally substituted by lower alkyl, hydroxylower alkyl, carboxy-lower alkyl, amino-lower alkyl, di-lower alkylamino-lower alkyl, sulpho-lower alkyl in salt form, cycloalkyl, aryl, aryl-lower alkyl, halogen, amino, mono- or di-lower alkylamino, nitro, hydroxy, lower alkoxy, carboxy, lower alkoxycarbonyl, carbamoyl, N-mono- or N,N-di-lower alkylcarbamoyl, cyano, oxo or by oxido, carboxy, lower alkoxycarbonyl, carbamoyl, cyano, nitro, amino, lower alkylamino, di-lower alkylamino, $C_1$–$C_{20}$-acylamino, di-$C_1$–$C_{20}$-acylamino or lower alkyleneamino,

or in which

$R_1$ represents a mercapto group etherified by an optionally substituted aliphatic, cycloaliphatic, cycloaliphatic-aliphatic, aromatic or araliphatic hydrocarbon radical having up to 18, especially up to 10, carbon atoms, in which the substituents optionally present are hydroxy, lower alkoxy, lower alkanoyloxy, halogen, mercapto, lower alkylmercapto, carboxy, lower alkoxycarbonyl, carbamoyl, cyano, nitro, or amino optionally mono- or di-substituted by lower alkyl or $C_1$–$C_{20}$-acyl or optionally disubstituted by lower alkyl or $C_1$–$C_{20}$-acyl or optionally disubstituted by lower alkylene, or in which

$R_1$ represents phenylthio or phenyl-lower alkylthio substituted by lower alkyl or in which

$R_1$ represents optionally protected carboxy, or in which

$R_1$ represents a mercapto group etherified by an optionally substituted heterocyclic radical, in which the heterocyclyl radical is bonded via a ring carbon atom to the mercapto group and contains from 1 to 4 ring nitrogen atoms and optionally one other ring hetero atom selected from the group oxygen and sulphur and in which the substituents optionally present are lower alkyl, lower alkyl substituted by hydroxy, lower alkanoyloxy, halogen, carboxy, lower alkoxycarbonyl, sulpho, amidated sulpho, amino, mono- or di-lower alkylamino, $C_1$–$C_{20}$-acylamino or by carboxy- or halogen-substituted lower alkanoylamino, cycloalkyl, optionally halogen- or nitrosubstituted phenyl, benzyl, furyl, thienyl, oxazolyl, halogen, amino optionally mono- or di-substituted by lower alkyl, $C_1$–$C_{20}$-acylamino, nitro, hydroxy, lower alkoxy, carboxy, lower alkoxycarbonyl, optionally N-mono- or N,N-di-lower alkylated carbamoyl, cyano, oxo or oxido, functional groups in these radicals $R_a$ and $R_1$ preferably being in protected form and the groups designated «lower» containing up to 7, especially up to 4, carbon atoms,

Z′ represents oxygen, sulphur, or a methylidene group optionally carrying one or two substituents,

$X^{\oplus}$ represents either a tri-substituted phosphonio group or a di-esterified phosphono group together with a cation, and

$R_2^A$ together with the carbonyl group –C(=O)– represents a protected carboxy group, in racemic or optically active form, characterised in that stages 1.1 to 1.4 in accordance with

Reaction scheme 1

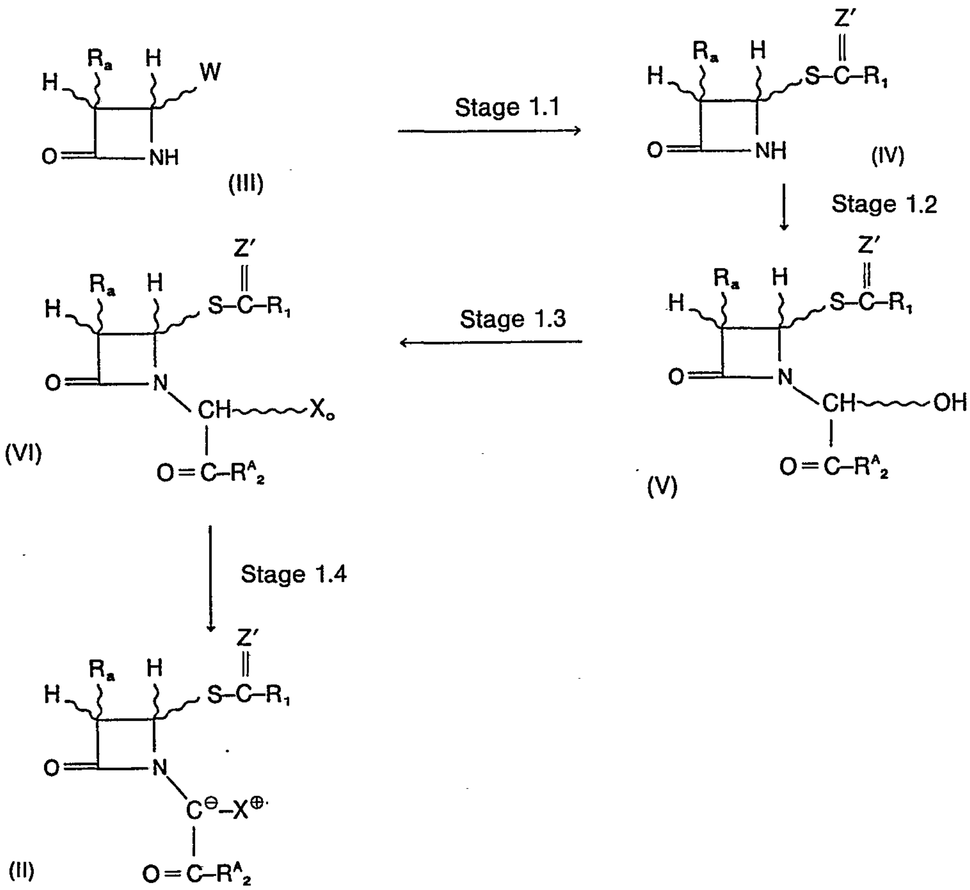


or stages 2.1 to 2.6 in accordance with

Reaction scheme 2

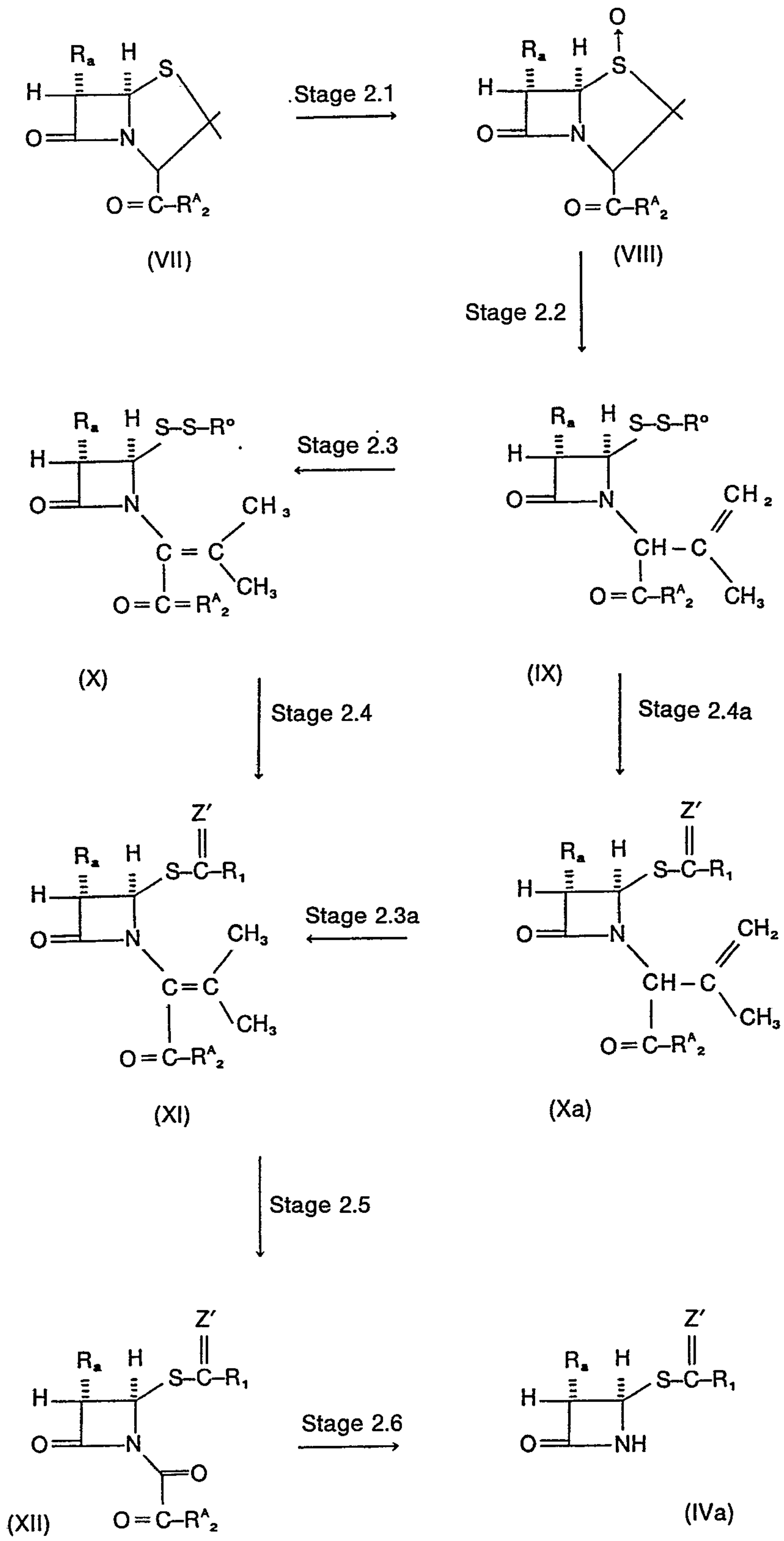


(VII) (VIII) (X) (IX) (XI) (Xa) (XII) (IVa)

followed by stages 1.2 to 1.4, or stages 3.1 to 3.5 in accordance with

Reaction scheme 3

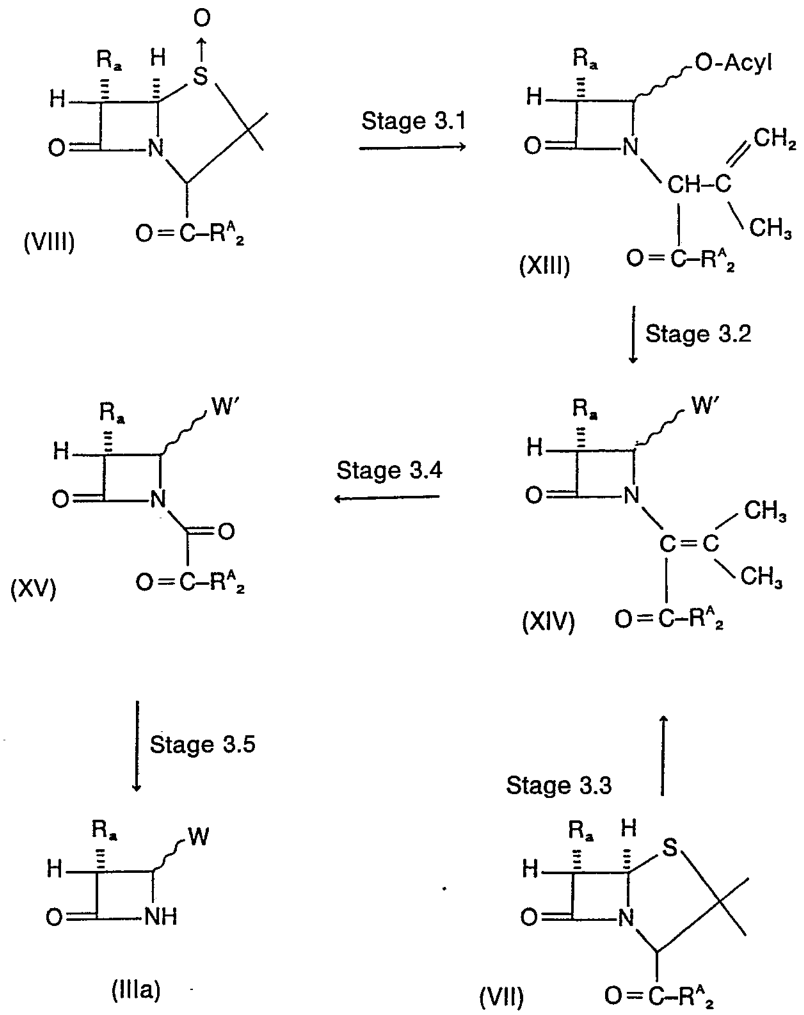


(VIII) (XIII) (XIV) (XV) (IIIa) (VII)

followed by stages 1.1 to 1.4, are carried out; in accordance with stage 1.1 for the manufacture of compounds of the formula

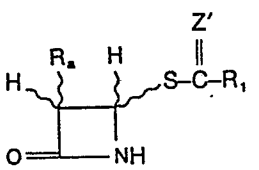

(IV) or (IVa)

in which $R_a$, $R_1$ and Z′ have the meanings given under formula II, in racemic or optically active form, a 4-W-azetidinone of the formula

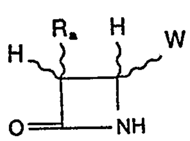

(III)

or

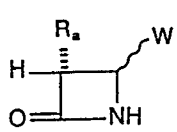

(IIIa)

in which W represents a nucleofugal leaving group, is treated with a mercapto compound $R_1$–C(=Z′)–SH or a salt thereof and, if desired, a resulting isomeric mixture is separated into the individual isomers, or, in accordance with stage 2.6 for the manufacture of optically active (3S, 4R)-compounds of the formula

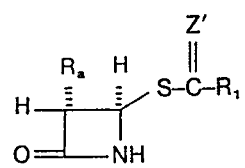

(IVa),

a compound of the formula

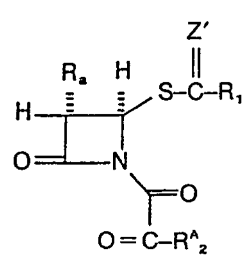

(XII),

in which $R_a$, $R_1$, Z′ and $R_2^A$ have the meanings given under formula II, is solvolysed and, if

desired, a group $R_a$ or $R_1$ in a resulting compound is converted into a different group $R_a$ or $R_1$, respectively, and/or, if desired, an optionally substituted methylidene group Z′ is converted into an oxo group Z,

in accordance with stage 1.2 for the manufacture of compounds of the formula

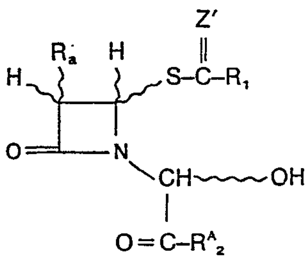

(V),

in which $R_a$, $R_1$, Z′ and $R_2^A$ have the meanings given under formula II, in racemic or optically active form, a compound of the formula

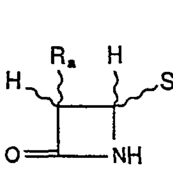

(IV) or

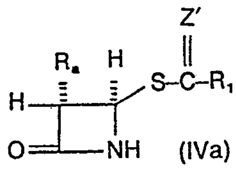

(IVa),

in which $R_a$, $R_1$ and Z′ have the meanings given above, is reacted with a glyoxylic acid compound of the formula $OHC–C(=O)–R_2^A$ or a suitable derivative thereof, and, if desired, a resulting isomeric mixture is separated into the individual isomers, and/or, if desired, a group $R_a$ or $R_1$ is converted into a different group $R_a$ or $R_1$, respectively, and/or, if desired, an optionally substituted methylidene group Z′ is converted into an oxo group Z,

in accordance with stage 1.3 for the manufacture of compounds of the formula

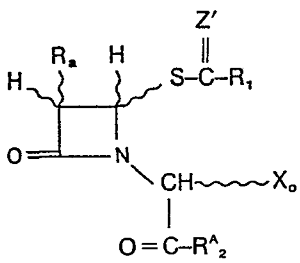

(VI)

in which $R_a$, $R_1$, Z′ and $R_2^A$ have the meanings given under formula II and $X_0$ represents a reactive esterified hydroxy group, in racemic or optically active form, in a compound of the formula

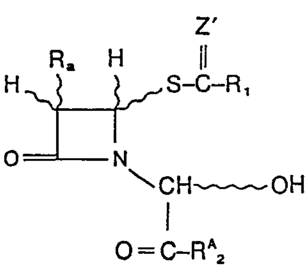

(V),

in which $R_a$, $R_1$, Z′ and $R_2^A$ have the meanings given above, the secondary hydroxy group is converted into a reactive esterified hydroxy group, and, if desired, a resulting isomeric mixture is separated into the individual isomers, and/or, if desired, a group $R_a$ or $R_1$ in a resulting compound is converted into a different group $R_a$ or $R_1$, respectively, and/or, if desired, an optionally substituted methylidene group Z′ is converted into an oxo group Z, and

in accordance with stage 1.4 for the manufacture of compounds of the formula II, a compound of the formula

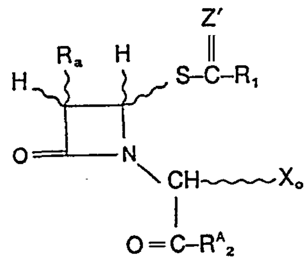

(VI),

in which $X_0$ represents a reactive esterified hydroxy group and $R_a$, $R_1$, Z′ and $R_2^A$ have the meanings given above, is treated with a suitable phosphine compound or a suitable phosphite compound,

or a compound of the formula

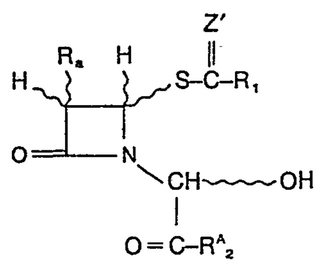

(V)

in which $R_a$, $R_1$, Z′ and $R_2^A$ have the meanings given above is treated with carbon tetrachloride and a phosphine, and, if desired, a group $R_a$ or $R_1$ in a resulting compound is converted into a different group $R_a$ or $R_1$, respectively, and/or, if desired, an optionally substituted methylidene group Z′ is converted into an oxo group Z, or,

in accordance with stage 2.1 for the manufacture of compounds of the formula

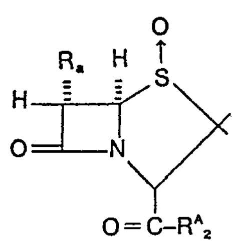

(VIII)

in which $R_a$ and $R_2^A$ have the meanings given under formula II, a penicillanic acid compound of the formula

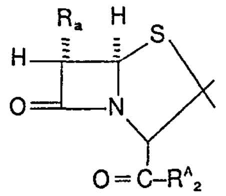

(VII)

is oxidised in the 1-position and, if desired, a group $R_a$ in a resulting compound is converted into a different group $R_a$, in accordance with stage 2.2 for the manufacture of compounds of the formula

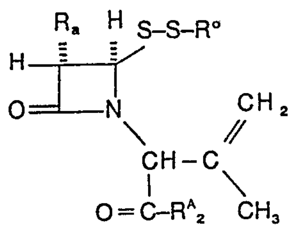

(IX)

in which $R_a$ and $R_2^A$ have the meanings given under formula II and R° is an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–, a compound of the formula

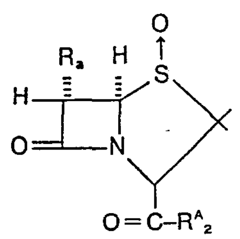

(VIII)

is treated with a mercapto compound R°-SH and, if desired, a group $R_a$ in a resulting compound is converted into a different group $R_a$, in accordance with stage 2.3 for the manufacture of compounds of the formula

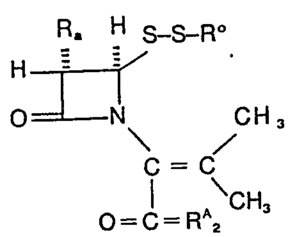

(X)

in which $R_a$ and $R_2^A$ have the meanings given under formula II and R° is an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–, a 3-methylenebutyric acid compound of the formula

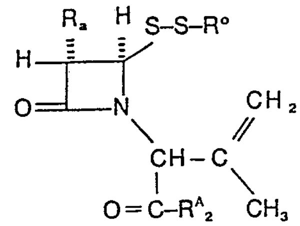

(IX)

is isomerised by treating with a suitable basic agent and, if desired, a group $R_a$ in a resulting compound is converted into a different group $R_a$, in accordance with stage 2.4 for the manufacture of compounds of the formula

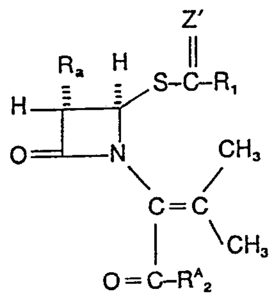

(XI)

in which $R_a$, $R_1$, Z′ and $R_2^A$ have the meanings given under formula II, a compound of the formula

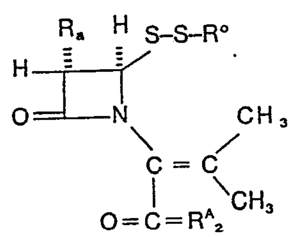

(X)

in which R° represents an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–, and $R_a$ and $R_2^A$ have the meanings given above, is treated with a suitable reducing agent and simultaneously or subsequently reacted with an acylating derivative of a carboxylic acid of the formula $R_1$–C(=Z)–OH or, when Z′ represents a methylidene group optionally substituted by Y, reacted with an alkyne of the formula $R_1$–C≡C–Y, or, in accordance with stage 2.3a, a compound of the formula

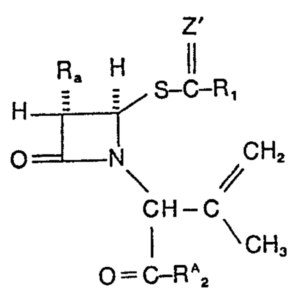

(Xa)

in which $R_a$, $R_1$, Z′ and $R_2^A$ have the meanings given above is isomerised by treating with a suitable basic agent, and, if desired, a group $R_a$ or $R_1$ in a resulting compound is converted into a different group $R_a$ or $R_1$, respectively, and/or, if desired, an optionally substituted methylidene group Z′ is converted into an oxo group Z, in accordance with stage 2.4a for the manufacture of compounds of the formula

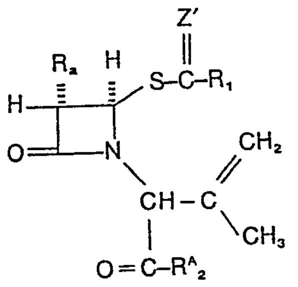

(Xa)

in which $R_a$, $R_1$, Z′ and $R_2^A$ have the meanings given under formula II, a compound of the formula

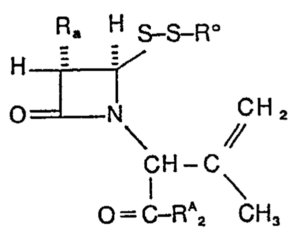

(IX)

in which R°, $R_a$ and $R_2^A$ have the meanings given above, is treated with a suitable reducing agent and simultaneously or subsequently reacted with an acylating derivative of a carboxylic acid of the formula $R_1$–C(=Z)–OH or, when Z′ represents a methylidene group optionally substituted by Y, reacted with an alkyne of the formula $R_1$–C≡C–Y and, if desired, a group $R_a$ or $R_1$ in a resulting compound is converted into a different group $R_a$ or $R_1$, respectively, and/or, if desired, an optionally substituted methylidene group Z′ is converted into an oxo group Z, in accordance with stage 2.5 for the manufacture of compounds of the formula

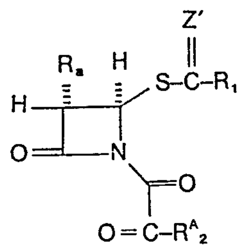

(XII),

in which $R_a$, $R_1$, Z′ and $R_2^A$ have the meanings given under formula II, a compound of the formula

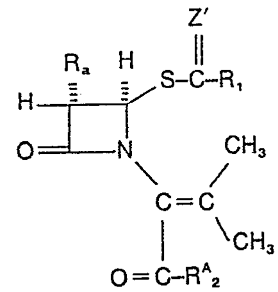

(XI)

in which $R_a$, $R_1$, Z′ and $R_2^A$ have the meanings given above, is ozonised and the ozonide formed is cleaved reductively to form the oxo group and, if desired, a group $R_a$ or $R_1$ in a resulting compound is converted into a different group $R_a$ or $R_1$, respectively, and/or, if desired, an optionally substituted methylidene group Z′ is converted into an oxo group Z, and then the stages 2.6 and 1.2 to 1.4 are carried out or, in accordance with stage 3.1 for the manufacture of compounds of the formula

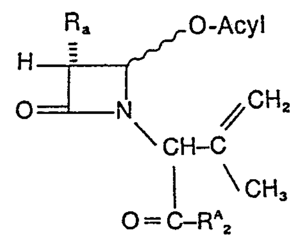

(XIII),

in which $R_a$ and $R_2^A$ have the meanings given under formula II, and acyl is the acyl radical of an organic carboxylic acid, in the cis,trans-, the cis- or the trans-form, a 1-oxide of a penicillanic acid compound of the formula

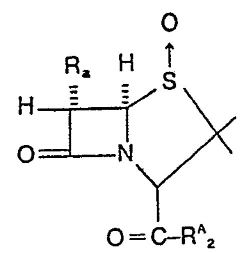

(VIII)

in which $R_a$ and $R_2^A$ have the meanings given above, is treated in the presence of a tri-lower alkyl phosphite with an organic carboxylic acid acyl-OH and, if desired, a group $R_a$ in a resulting compound is converted into a different group $R_a$ and/or the cis- and/or the trans-compound is or are isolated from a resulting cis,trans-compound, in accordance with stage 3.2 for the manufacture of compounds of the formula

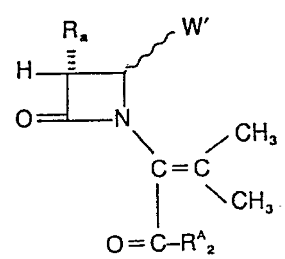

(XIV),

in which $R_a$ and $R_2^A$ have the meanings given under formula II and W′ represents acyloxy in which acyl is the acyl radical of an organic carboxylic acid, and $R_2^A$ together with the carbonyl group $-C(=O)-$ represents a protected carboxy group, in the cis,trans-, the cis- or the trans-form, a compound of the formula

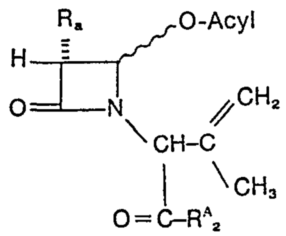

(XIII)

in which $R_a$, acyl and $R_2^A$ have the meanings given above, is isomerised by treating with a suitable basic agent and, if desired, a group $R_a$ in a resulting compound is converted into a different group $R_a$ and/or, if desired, the cis- and/or the trans-compound is or are isolated from a resulting cis,-trans-compound, or, in accordance with stage 3.3 for the manufacture of compounds of the formula

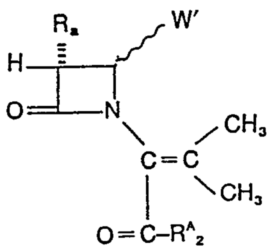

(XIV)

in which $R_a$ and $R_2^A$ have the meanings given under formula II and W′ represents halogen, in the cis,trans-, the cis- or the trans-form, a penicillanic acid compound of the formula

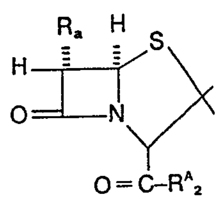

(VII)

in which $R_a$ and $R_2^A$ have the meanings given above, is reacted with a halogenating agent supplying positive halogen ions and, if necessary, a possibly resulting intermediate is treated with a base and, if desired, a group $R_a$ in a resulting compound of the formula XIV is converted into a different group $R_a$ and/or the cis- and/or the trans-compound is or are isolated from a resulting cis,-trans-compound, in accordance with stage 3.4 for the manufacture of compounds of the formula

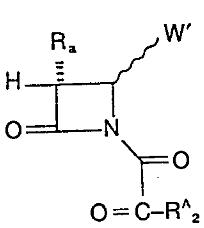

(XV)

in which $R_a$ and $R_2^A$ have the meanings given under formula II and W′ represents halogen or acyloxy in which acyl is the acyl radical of an organic carboxylic acid, in the cis,trans-, the cis- or the trans-form, a compound of the formula

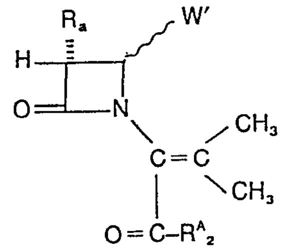

(XIV)

in which $R_a$, W′ and $R_2^A$ have the meanings given above, is ozonised and the ozonide formed is cleaved reductively to form the oxo group and, if desired, a group $R_a$ in a resulting compound is converted into a different group $R_a$ and/or, if desired, the cis- and/or the trans-compound is or are isolated from a resulting cis,trans-compound, in accordance with stage 3.5 for the manufacture of compounds of the formula

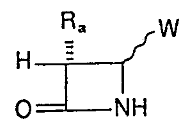

(IIIa)

in which $R_a$ has the meanings given under formula II and W represents a nucleofugal leaving group, in the cis,trans-, the cis- or the trans-form, a compound of the formula

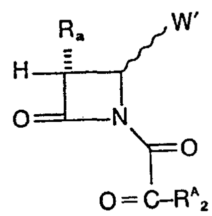

(XV)

in which $R_a$ and $R_2^A$ have the meanings given above and W′ represents acyloxy or halogen, is solvolysed and, if desired, in a resulting compound a group $R_a$ is converted into a different group $R_a$ and/or, if desired, a group W′ is converted into a different group W′ or into W and/or, if desired, a resulting cis-compound is isomerised to form the corresponding trans-compound and/or the cis and/or the trans-compound is or are isolated from a resulting cis,trans-compound, and subsequently stages 1.1 to 1.4 are carried out.

2. Process according to patent claim 1, characterised in that compounds of the formula II are manufactured in which $R_a$ is lower alkyl, hydroxy-lower alkyl, lower alkoxy-lower alkyl, lower alkanoyloxy-lower alkyl, hydroxysulphonyloxy-lower alkyl present in salt form, hydroxy, lower alkoxy, lower alkanoyloxy, lower alkanoyloxy substituted by phenoxy, halogen, amino or cyano, phenyl-lower alkanoyloxy, or phenyl-lower alkanoyloxy

substituted by hydroxy or amino, $R_1$ is hydrogen, lower alkyl, hydroxy-lower alkyl, lower alkoxy-lower alkyl, lower alkanoyloxy-lower alkyl, lower alkylthio-lower alkyl, heterocyclylthio-lower alkyl in which heterocyclyl is a five-membered aromatic diaza-, triaza-, tetraza-, thiaza-, thia-diaza-, thiatriaza-, oxaza- or oxadiaza-cyclic radical optionally substituted by lower alkyl, carboxy-lower alkyl, di-lower alkylamino-lower alkyl, or by sulpho-lower alkyl present in salt form, amino-lower alkyl, acylamino-lower alkyl in which acyl is lower alkanoyl or a substituted oxycarbonyl group that can be used as an amino-protecting group, for example a tert.-butyl-, 2,2,2-trichloroethyl-, diphenylmethyl- or p-nitrobenzyl-oxycarbonyl group, carboxy-lower alkyl, lower alkoxycarbonyl-lower alkyl, phenyl-lower alkyl, phenyl, hydroxyphenyl, aminophenyl, furyl, thienyl, pyridyl, lower alkylthio, lower alkylthio or lower alkenylthio substituted by amino, mono- or di-lower alkylamino or lower alkanoylamino, or tetrazolylthio or thiadiazolylthio optionally substituted by lower alkyl, sulpho-lower alkyl, carboxy-lower alkyl or di-lower alkylamino-lower alkyl, functional groups in such radicals $R_a$ and $R_1$ preferably being in protected form, and Z', $X^{\oplus}$ and $R_2^A$ have the meanings given in patent claim 1, in racemic or optically active form.

3. Compounds of the formula

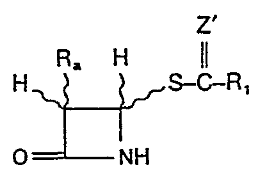

(IV)

in which $R_a$, $R_1$ and Z' have the meanings given in claim 1, in racemic and optically active form, with the first proviso that Z' is other than a methylidene group substituted by a protected carboxy group when $R_a$ represents alkyl, alkoxy, aryl, aralkyl, heterocyclyl or heterocyclylalkyl and $R_1$ represents hydrogen or alkyl, aryl, aralkyl, heterocyclyl, heterocyclylalkyl, alkylthio, arylthio, aralkylthio or heterocyclylthio, the radicals $R_a$ and $R_1$ being unsubstituted or substituted by amino, protected amino, mono- or di-alkylamino, hydroxy, protected hydroxy, alkoxy, mercapto, alkylthio, nitro, chlorine, bromine, fluorine, cyano, carboxy or protected carboxy,

and with the second proviso that Z' is other than oxygen either when

$R_a$ has the meanings given under the first proviso and

$R_1$ is aryl other than phenyl, aralkyl other than phenyl-lower alkyl, heterocyclyl other than pyridyl, thienyl or furyl, heterocyclylalkyl, arylthio, aralkylthio or heterocyclylthio, which radicals are unsubstituted or are substituted by amino, protected amino, mono- or di-alkylamino, hydroxy, protected hydroxy, alkoxy, mercapto, alkylthio, nitro, chlorine, bromine, fluorine, cyano, carboxy or by protected carboxy; alkyl having from 1 bo 6 carbon atoms that is substituted by mono- or di-alkylamino, mercapto, nitro, chlorine, fluorine, bromine or by cyano; phenyl substituted by mono- or di-alkylamino, mercapto, alkylthio, nitro, cyano, carboxy or by protected carboxy; phenyl-lower alkyl, pyridyl, thienyl or furyl each substituted by amino, protected amino, mono- or di-alkylamino, hydroxy, protected hydroxy, alkoxy, mercapto, alkylthio, nitro, chlorine, bromine, fluorine, cyano, carboxy or by protected carboxy; or alkylthio having from 1 to 6 carbon atoms that is substituted by hydroxy, protected hydroxy, mercapto, alkylthio, nitro, chlorine, bromine, fluorine, cyano, carboxy or protected carboxy;

or when $R_1$ has the meanings given under the first proviso and $R_a$ represents substituted alkoxy or optionally substituted aryl, aralkyl, heterocyclyl or heterocyclylalkyl, substituents of such radicals being amino, protected amino, mono- or di-alkylamino, hydroxy, protected hydroxy, alkoxy, mercapto, alkylthio, nitro, chlorine, bromine, fluorine, cyano, carboxy or protected carboxy; or alkyl having from 1 to 6 carbon atoms, which radical is substituted by amino, protected amino, mono- or di-alkylamino, mercapto, alkylthio, nitro, chlorine, bromine, fluorine, cyano, carboxy or by protected carboxy.

4. Compounds of the formula IV according to patent claim 3 in which $R_a$, $R_1$ and Z' have the meanings given in patent claim 2.

5. 4-acetylthio-3-methyl-2-oxoazetidine (racemic cis-compound) according to patent claim 3.

6. 4-acetylthio-3-methyl-2-oxoazetidine (racemic trans-compound) according to patent claim 3.

7. 4-acetylthio-3-isopropyl-2-oxoazetidine (racemic trans-compound) according to patent claim 3.

8. 4-acetylthio-3-benzyl-2-oxoazetidine (racemic trans-compound) according to patent claim 3.

9. 4-ethylthiothiocarbonylthio-3-isopropyl-2-oxoazetidine (racemic trans-compound) according to patent claim 3.

10. (3S,4R)-4-acetylthio-3-methoxy-2-oxoazetidine according to patent claim 3.

11. (3S,4R)-4-acetylthio-3-phenoxyacetoxy-2-oxoazetidine according to patent claim 3.

12. 3-ethyl-4-(4-p-nitrobenzyloxycarbonyl-aminobutyrylthio)-2-oxoazetidine (racemic cis,trans-compound) according to patent claim 3.

13. (3S,4R)-4-(2-p-nitrobenzyloxycarbonyl-aminoethylthiothiocarbonylthio)-3-methoxy-2-oxoazetidine according to patent claim 3.

14. (3S,4R)-4-(4-p-nitrobenzyloxycarbonyl-aminobutyrylthio)-3-methoxy-2-oxoazetidine according to patent claim 3.

15. 3-ethyl-4-(2-acetylaminoethylthiothio-

carbonylthio)-2-oxoazetidine (racemic cis,trans-compound) according to patent claim 3.

16. Compounds of the formula

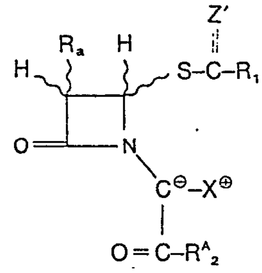

(II)

in which $R_a$, $R_1$, Z', $X^{\oplus}$ and $R_2^A$ have the meanings given in patent claim 1, in racemic or optically active form.

17. Compounds of the formula II, according to patent claim 16, in which $R_a$, $R_1$, Z', $X^{\oplus}$ and $R_2^A$ have the meanings given in patent claim 2, in racemic or optically active form.

18. Compounds of the formula II, according to patent claim 16 or 17, in which $R_a$ represents methyl, ethyl, isopropyl, benzyl, methoxy or phenoxyacetoxy, $R_1$ represents methyl, 3-(p-nitrobenzyloxycarbonylamino)-propyl, ethylthio, 2-acetylaminoethylthio or 2-(p-nitrobenzyloxycarbonylamino)-ethylthio, Z' represents oxygen or sulphur, $R_2^A$ represents p-nitrobenzyloxy and $X^{\oplus}$ represents the group $P^{\oplus}$ (phenyl)$_3$.

19. Compounds of the formula

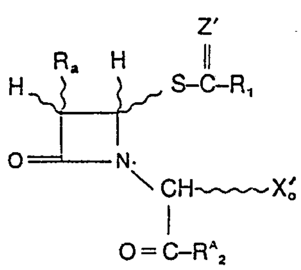

(V) or (VI)

in which $R_a$, $R_1$, Z' and $R_2^A$ have the meanings given in patent claim 1 and $X_o'$ represents hydroxy or a reactive esterified hydroxy group, in racemic or optically active form.

20. Compounds of the formula V or VI according to patent claim 19 in which $R_a$, $R_1$, Z' and $R_2^A$ have the meanings given in patent claim 2 and $X_o'$ has the meanings given in patent claim 19, in racemic or optically active form.

21. Compounds of the formula V or VI according to patent claim 19 or 20 in which $R_a$ represents methyl, ethyl, isopropyl, benzyl, methoxy or phenoxyacetoxy, $R_1$ represents methyl, 3-(p-nitrobenzyloxycarbonyl-amino)-propyl, ethylthio, 2-acetylaminoethylthio or 2-(p-nitrobenzyloxycarbonylamino)-ethylthio, Z' represents oxygen or sulphur, $R_2^A$ represents p-nitrobenzyloxy and $X_o'$ represents hydroxy or chlorine.

22. Compounds of the formula

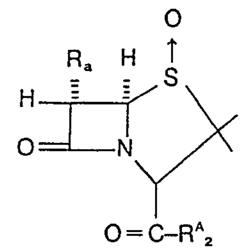

(VIII),

in which $R_a$ and $R_2^A$ have the meanings given in patent claim 1.

23. Compounds of the formula VIII according to patent claim 22 in which $R_a$ and $R_2^A$ have the meanings given in patent claim 2.

24. Compounds of the formula VIII according to patent claim 22 or 23 in which $R_a$ represents methoxy or phenoxyacetoxy and $R_2^A$ represents methoxy or 2,2,2-trichloroethoxy.

25. Compounds of the formula

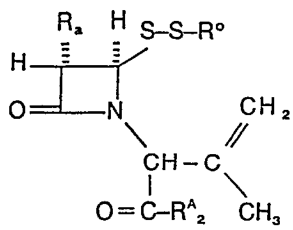

(IX)

in which $R_a$ and $R_2^A$ have the meanings given in patent claim 1 and $R^o$ represents an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–.

26. Compounds of the formula IX according to patent claim 25 in which $R_a$ and $R_2^A$ have the meanings given in patent claim 2 and $R^o$ has the meanings given in patent claim 25.

27. Compounds of the formula IX according to patent claim 25 or 26 in which $R_a$ represents methoxy or phenoxyacetoxy, $R_2^A$ represents methoxy or 2,2,2-trichloroethoxy and $R^o$ represents 2-benzthiazolyl.

28. Compounds of the formula

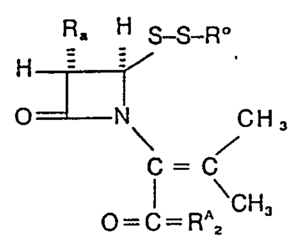

(X)

in which $R_a$ and $R_2^A$ have the meanings given in patent claim 1 and $R^o$ represents an optionally substituted aromatic heterocyclic radical having up to 15 carbon atoms, at least one ring nitrogen atom and optionally one other ring hetero atom, which radical is bonded by one of its ring carbon

atoms, which is bonded to a ring nitrogen atom by a double bond, to the thio group –S–.

29. Compounds of the formula X according to patent claim 28 in which $R_a$ and $R_2^A$ have the meanings given in patent claim 2 and $R^o$ has the meanings given in patent claim 28.

30. Compounds of the formula X according to patent claim 28 or 29 in which $R_a$ represents methoxy or phenoxyacetoxy, $R_2^A$ represents methoxy or 2,2,2-trichloroethoxy and $R^o$ represents 2-benzthiazolyl.

31. Compounds of the formula

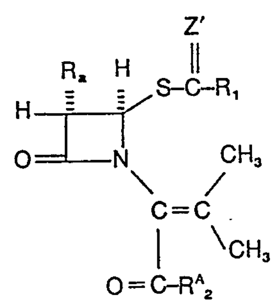

(XI)

in which $R_a$, $R_1$, Z′ and $R_2^A$ have the meanings given in patent claim 1.

32. Compounds of the formula XI according to patent claim 31 in which $R_a$, $R_1$, Z′ and $R_2^A$ have the meanings given in patent claim 2.

33. Compounds of the formula XI according to patent claim 31 or 32 in which $R_a$ represents methyl, methoxy or phenoxyacetoxy, $R_1$ represents methyl, Z′ represents oxygen and $R_2^A$ represents methoxy or 2,2,2-trichloroethoxy.

34. Compounds of the formula

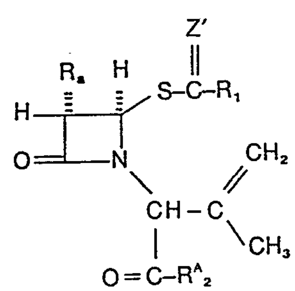

(Xa)

in which $R_a$, $R_1$, Z′ and $R_2^A$ have the meanings given in patent claim 1.

35. Compounds of the formula Xa according to patent claim 34 in which $R_a$, $R_1$, Z′ and $R_2^A$ have the meanings given in patent claim 2.

36. Compounds of the formula

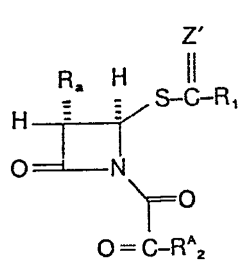

(XII)

in which $R_a$, $R_1$, Z′ and $R_2^A$ have the meanings given in patent claim 1.

37. Compounds of the formula XII according to patent claim 36 in which $R_a$, $R_1$, Z′ and $R_2^A$ have the meanings given in patent claim 2.

38. Compounds of the formula XII according to patent claim 36 or 37 in which $R_a$ represents methyl, methoxy or phenoxyacetoxy, $R_1$ represents methyl, Z′ represents oxygen and $R_2^A$ represents methoxy or 2,2,2-trichloroethoxy.

39. Compounds of the formula

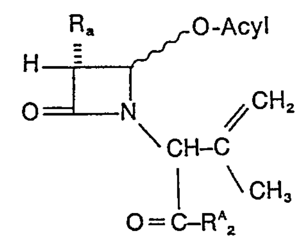

(XIII)

in which $R_a$ and $R_2^A$ have the meanings given in patent claim 1 and acyl is the acyl radical of an organic carboxylic acid in the cis,trans-, the cis- or the trans-form.

40. Compounds of the formula XIII according to patent claim 39 in which $R_a$ represents methyl or methoxy, $R_2^A$ represents 2,2,2-trichloroethoxy and acyl is an acetyl group, in the cis,trans-, ths cis- or the trans-form.

41. Compounds of the formula

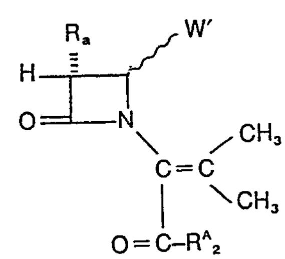

(XIV)

in which $R_a$ and $R_2^A$ have the meanings given in patent claim 1, W′ represents halogen or acyloxy in which acyl is the acyl radical of an organic carboxylic acid, in the cis,trans-, the cis- or the trans-form, with the exception of compounds of the formula XIV in which $R_a$ represents alkyl, alkoxy, aryl, aralkyl, heterocyclyl or heterocyclylalkyl, these radicals being unsubstituted or substituted by amino, protected amino, mono- or di-alkylamino, hydroxy, protected hydroxy, alkoxy, mercapto, alkylthio, nitro, chlorine, bromine, fluorine, cyano, carboxy or by protected carboxy, W′ represents acetoxy and $R_2^A$ together with the carbonyl group –C(=O)– represents a carboxy group protected by a carboxy-protecting group.

42. Compounds of the formula XIV according to patent claim 41 in which $R_a$ represents methoxy, $R_2^A$ represents 2,2,2-trichloroethoxy and W′ represents chlorine, in the cis,trans-, the cis- or the trans-form.

43. Compounds of the formula

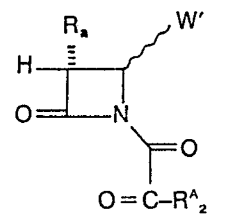

(XV)

in which $R_a$ and $R_2^A$ have the meanings given in patent claim 1, W′ represents halogen or acyloxy in which acyl is the acyl radical of an organic carboxylic acid, in the cis,trans-, the cis- or the trans-form.

44. Compounds of the formula XI according to patent claim 43 in which $R_a$ represents methoxy, $R_2^A$ represents 2,2,2-trichloroethoxy and W′ represents acetoxy or chlorine, in the cis,trans-, the cis- or the trans-form.

45. Compounds of the formula

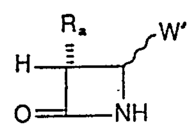

(IIIa)

in which $R_a$ has the meanings given in claim 1 and W represents a nucleofugal leaving group, in the cis, trans-, the cis- or the trans-form, with the exception of compounds of the formula IIIa in which $R_a$ represents alkyl, alkoxy, aryl, aralkyl, heterocyclyl or heterocyclylalkyl, these radicals being unsubstituted or substituted by amino, protected amino, mono- or di-alkylamino, hydroxy, protected hydroxy, alkoxy, mercapto, alkylthio, nitro, chlorine, bromine, fluorine, cyano, carboxy or by protected carboxy, and W represents acetoxy.

46. Compounds of the formula IIIa according to patent claim 45 in which $R_a$ represents methoxy and W represents chlorine, in the cis,trans-, the cis- or the trans-form.

47. The individual process steps according to patent claim 1, with the proviso that in accordance with stage 1.1 compounds of the formula IV are manufactured in which $R_a$, $R_1$ and Z′ have the meanings given in claim 3.

48. The individual process steps according to patent claim 1 in accordance with reaction scheme 3.

49. The individual process steps according to patent claim 1 in accordance with reaction schemes 1 and 2, with the proviso that according to stage 1.1 compounds of the formula IV are manufactured in which $R_a$, $R_1$ and Z′ have the meanings given in claim 3.

**Claims for the contracting state: IT**

1. Process for the manufacture of compounds of the formula

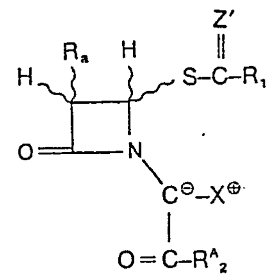

(II)

in which $R_a$ represents a saturated or unsaturated, optionally substituted, aliphatic, cycloaliphatic, cycloaliphatic-aliphatic, aromatic or araliphatic hydrocarbon radical having up to 18, preferably up to 10, carbon atoms.

an optionally substituted heterocyclyl or heterocyclyllower alkyl radical having up to 10 carbon atoms and up to 4 ring hetero atoms selected from the group nitrogen, oxygen and/or sulphur,

in which radicals $R_a$ the substituents optionally present are hydroxy, lower alkoxy, lower alkanoyloxy, hydroxysulphonyloxy present in salt form, halogen, mercapto, lower alkylmercapto, carboxy, lower alkoxycarbonyl, carbamoyl, cyano, nitro, sulpho present in salt form, or amino optionally mono- or di-substituted by lower alkyl or $C_1$–$C_{20}$-acyl or

optionally disubstituted by lower alkylene,

optionally protected carboxy,

hydroxy,

mercapto,

hydroxy or mercapto etherified by an optionally substituted aliphatic, cycloaliphatic, cycloaliphatic-aliphatic, aromatic or araliphatic hydrocarbon radical having up to 18, especially up to 10, carbon atoms, in which the substituents optionally present are hydroxy, lower alkoxy, lower alkanoyloxy, halogen, mercapto, lower alkylmercapto, carboxy, lower alkoxycarbonyl, carbamoyl, cyano, nitro, or amino optionally mono- or disubstituted by lower alkyl or $C_1$–$C_{20}$-acyl or optionally disubstituted by lower alkylene,

hydroxy or mercapto esterified by an acyl radical of an optionally substituted aliphatic, cycloaliphatic, cycloaliphatic-aliphatic, aromatic or araliphatic carboxylic acid having up to 18 carbon atoms. in which the substituents optionally present are hydroxy, lower alkoxy, phenoxy, lower alkanoyloxy, halogen, mercapto, lower alkylmercapto, carboxy, lower alkoxycarbonyl, carbamoyl, cyano, nitro, or amino optionally mono- or disubstituted by lower alkyl or $C_1$–$C_{20}$-acyl or optionally disubstituted by lower alkylene, or halogen,

$R_1$ represents hydrogen, a saturated or unsaturated, optionally substituted aliphatic, cycloaliphatic, cycloaliphatic-aliphatic, aromatic or araliphatic hydrocarbon radical having up to 18, preferably up to 10, carbon atoms, or

an optionally substituted heterocyclyl or heterocyclyl-lower alkyl radical having up to 10 carbon atoms and up to 4 ring hetero atoms selected from the group nitrogen, oxygen and/or sulphur,

in which radicals $R_1$ the substituents optionally present are hydroxy, lower alkoxy, lower alkanoyloxy, halogen, mercapto, lower alkylmercapto, heterocyclylmercapto optionally substituted by lower alkyl, hydroxy-lower alkyl, carboxy-lower alkyl, amino-lower alkyl, di-lower alkylamino-lower alkyl, sulpho-lower alkyl in salt form, cycloalkyl, aryl, aryl-lower alkyl, halogen, amino, mono- or di-lower alkylamino, nitro, hydroxy, lower alkoxy, carboxy, lower alkoxycarbonyl, carbamoyl, N-mono- or N,N-di-lower alkylcarbamoyl, cyano, oxo or by oxido, carboxy, lower alkoxycarbonyl, carbamoyl, cyano, nitro, amino, lower alkylamino, di-lower alkylamino, $C_1$–$C_{20}$-acylamino, di-$C_1$–$C_{20}$-acylamino or lower alkyleneamino,

or in which

$R_1$ represents a mercapto group etherified by an optionally substituted aliphatic, cycloaliphatic, cycloaliphatic-aliphatic, aromatic or araliphatic hydrocarbon radical having up to 18, especially up to 10, carbon atoms, in which the substituents optionally present are hydroxy, lower alkoxy, lower alkanoyloxy, halogen, mercapto, lower alkylmercapto, carboxy, lower alkoxycarbonyl, carbamoyl, cyano, nitro, or amino optionally mono- or di-substituted by lower alkyl or $C_1$–$C_{20}$-acyl or optionally disubstituted by lower alkylene, or in which

$R_1$ represents phenylthio or phenyl-lower alkylthio substituted by lower alkyl or in which

$R_1$ represents optionally protected carboxy, or in which

$R_1$ represents a mercapto group etherified by an optionally substituted heterocyclic radical, in which the heterocyclyl radical is bonded via a ring carbon atom to the mercapto group and contains from 1 to 4 ring nitrogen atoms and optionally one other ring hetero atom selected from the group oxygen and sulphur, and in which the substituents optionally present are lower alkyl, lower alkyl substituted by hydroxy, lower alkanoyloxy, halogen, carboxy, lower alkoxycarbonyl, sulpho, amidated sulpho, amino, mono- or di-lower alkylamino, $C_1$–$C_{20}$-acylamino or by carboxy- or halogen-substituted lower alkanoyl-amino, cycloalkyl, optionally halogen- or nitro-substituted phenyl, benzyl, furyl, thienyl, oxazolyl, halogen, amino optionally mono- or di-substituted by lower alkyl, $C_1$–$C_{20}$-acylamino, nitro, hydroxy, lower alkoxy, carboxy, lower alkoxycarbonyl, optionally N-mono- or N,N-di-lower alkylated carbamoyl, cyano, oxo or oxido, functional groups in these radicals $R_a$ and $R_1$ preferably being in protected form and the groups designated «lower» containing up to 7, especially up to 4, carbon atoms,

Z′ represents oxygen, sulphur, or a methylidene group optionally carrying one or two substituents,

$X^{\oplus}$ represents either a tri-substituted phosphonio group or a di-esterified phosphono group together with a cation, and

$R_2^A$ together with the carbonyl group $-C(=O)-$ represents a protected carboxy group, in racemic or optically active form, characterised in that stages 1.1 to 1.4 in accordance with

Reaction scheme 1

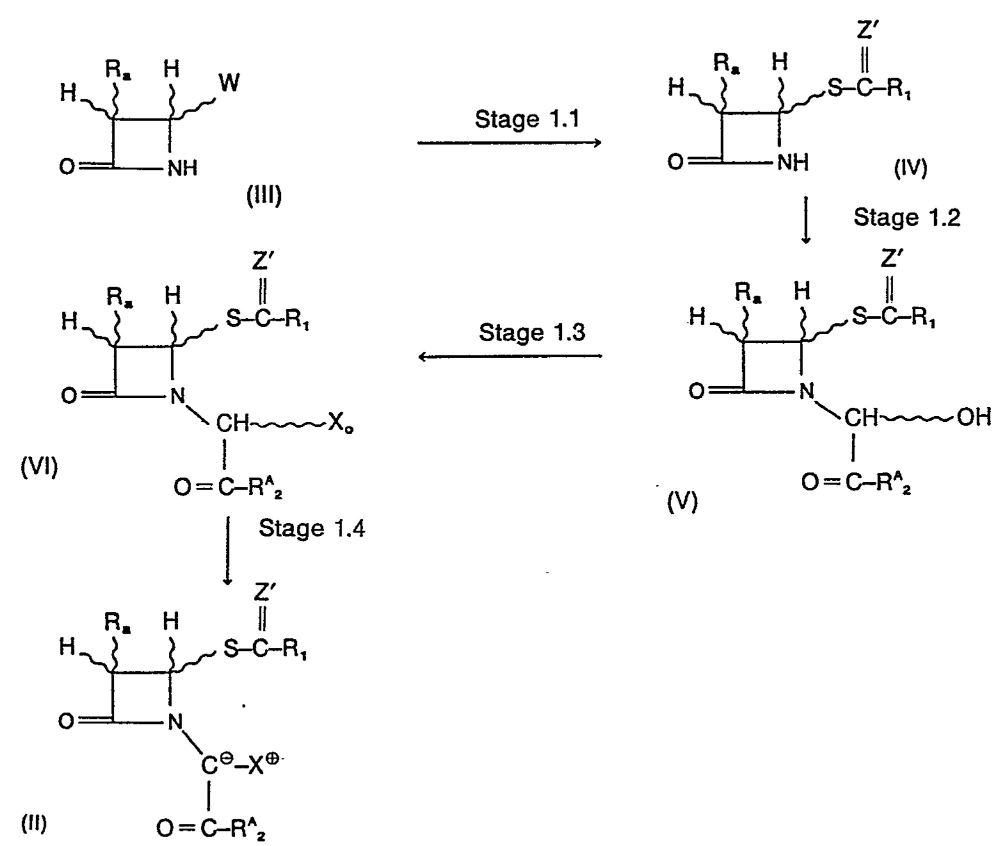


or stages 2.1 to 2.6 in accordance with

Reaction scheme 2

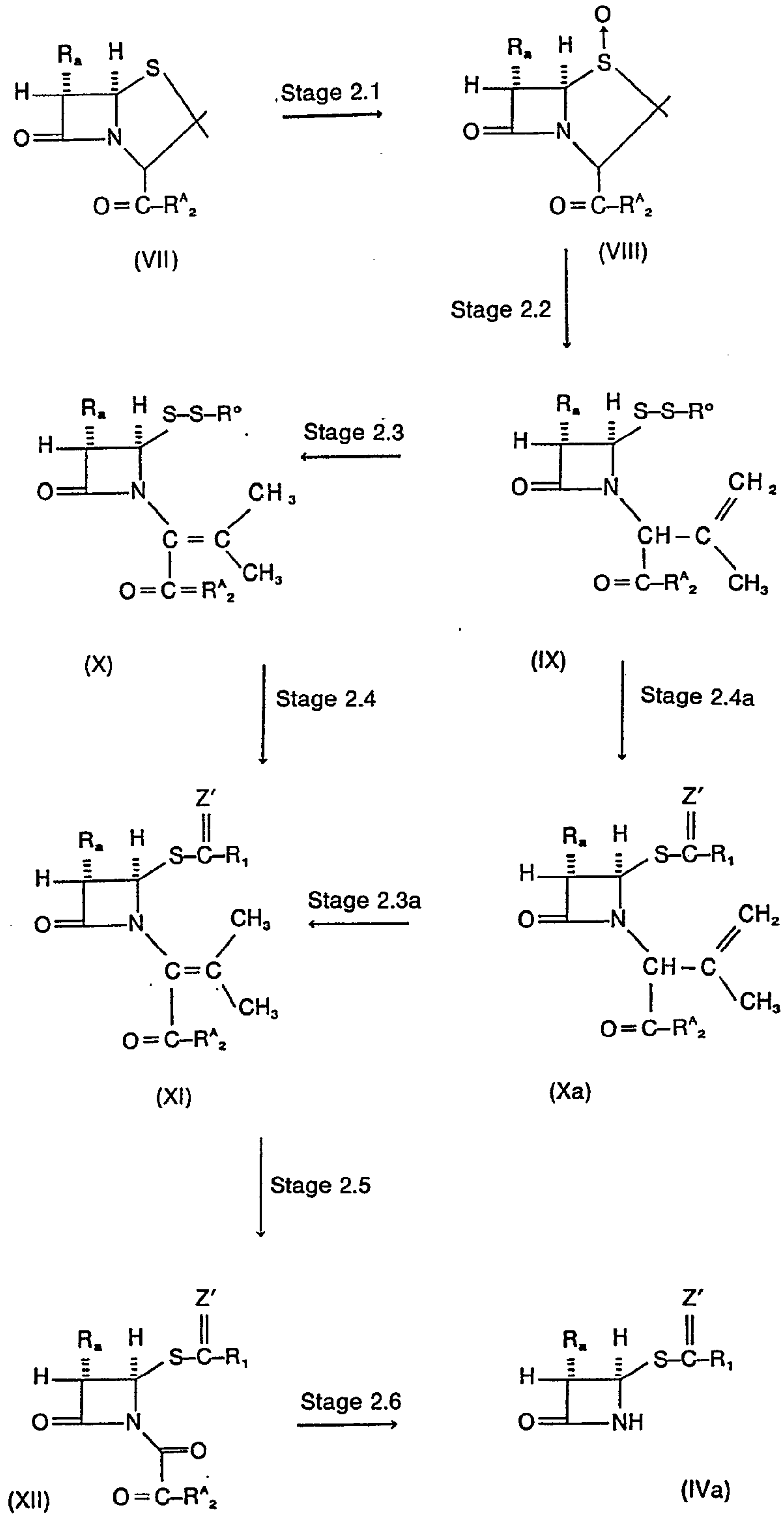


(VII) (VIII) (X) (IX) (XI) (Xa) (XII) (IVa)

followed by stages 1.2 to 1.4, or stages 3.1 to 3.5 in accordance with

Reaction scheme 3

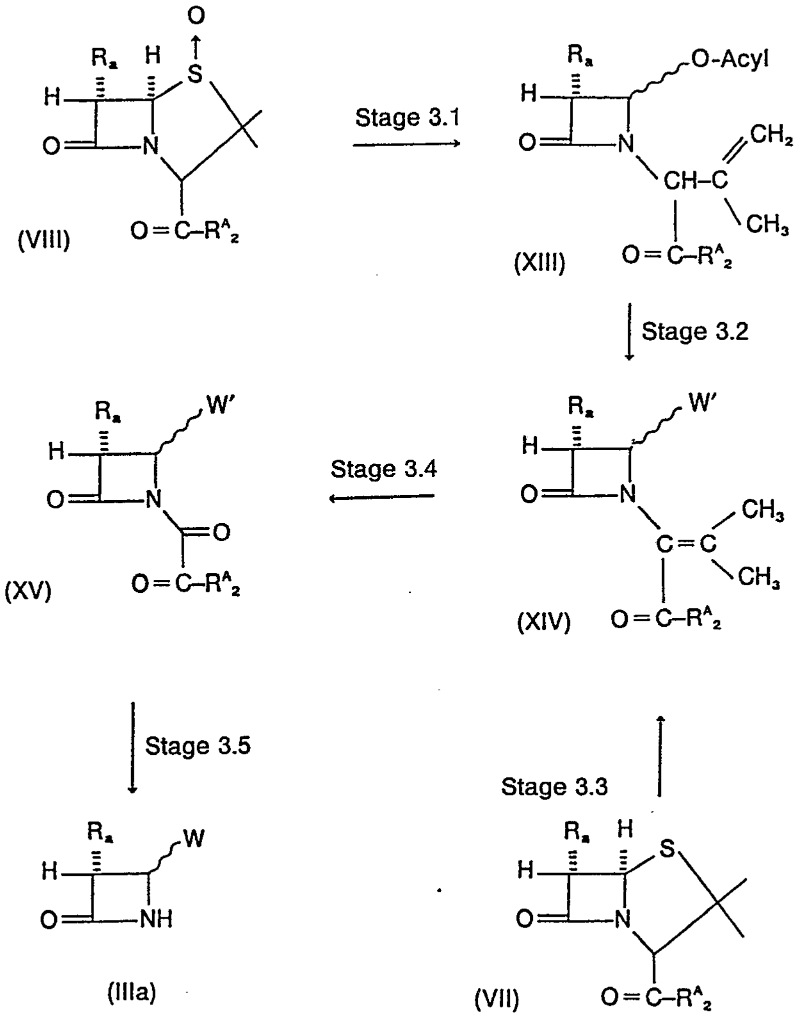


(VIII) (XIII) (XV) (XIV) (IIIa) (VII)

followed by stages 1.1 to 1.4, are carried out; in accordance with stage 1.1 for the manufacture of compounds of the formula

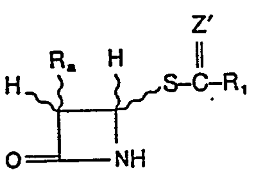

(IV) or (IVa),

in which $R_a$, $R_1$ and Z′ have the meanings given under formula II, in racemic or optically active form, a 4-W-azetidinone of the formula

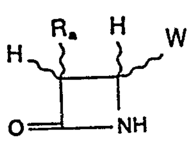

(III)

or

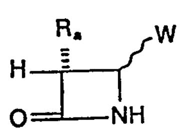

(IIIa)

in which W represents a nucleofugal leaving group, is treated with a mercapto compound $R_1$–C(=Z′)–SH or a salt thereof and, if desired, a resulting isomeric mixture is separated into the individual isomers, or, in accordance with stage 2.6 for the manufacture of optically active (3S, 4R)-compounds of the formula

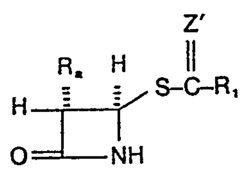

(IVa),

a compound of the formula

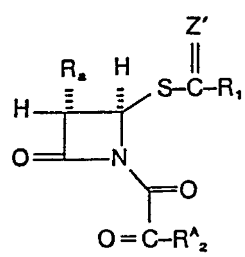

(XII)

in which $R_a$, $R_1$, Z′ and $R_2^A$ have the meanings given under formula II, is solvolysed and, if desir-

ed, a group $R_a$ or $R_1$ in a resulting compound is converted into a different group $R_a$ or $R_1$, respectively, and/or, if desired, an optionally substituted methylidene group Z′ is converted into an oxo group Z, in accordance with stage 1.2 for the manufacture of compounds of the formula

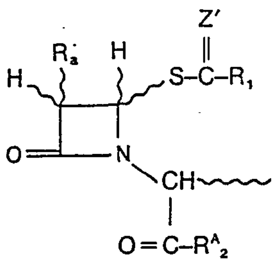
(V)

in which $R_a$, $R_1$, Z′ and $R_2^A$ have the meanings given under formula II, in racemic or optically active form, a compound of the formula

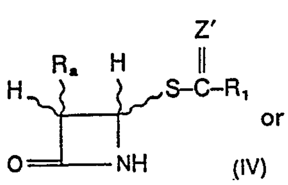
or
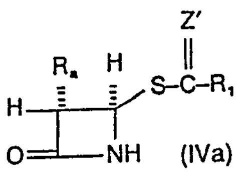
(IV) (IVa)

in which $R_a$, $R_1$ and Z′ have the meanings given above, is reacted with a glyoxylic acid compound of the formula OHC–C(=O)-$R_2^A$ or a suitable derivative thereof, and, if desired, a resulting isomeric mixture is separated into the individual isomers, and/or, if desired, a group $R_a$ or $R_1$ is converted into a different group $R_a$ or $R_1$, respectively, and/or, if desired, an optionally substituted methylidene group Z′ is converted into an oxo group Z,

in accordance with stage 1.3 for the manufacture of compounds of the formula

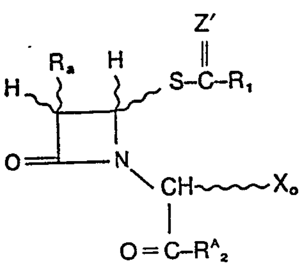
(VI),

in which $R_a$, $R_1$, Z′ and $R_2^A$ have the meanings given under formula II and $X_o$ represents a reactive esterified hydroxy group, in racemic or optically active form, in a compound of the formula

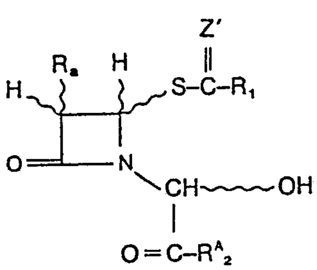
(V)

in which $R_a$, $R_1$, Z′ and $R_2^A$ have the meanings given above, the secondary hydroxy group is converted into a reactive esterified hydroxy group, and, if desired, a resulting isomeric mixture is separated into the individual isomers, and/or, if desired, a group $R_a$ or $R_1$ in a resulting compound is converted into a different group $R_a$ or $R_1$, respectively, and/or, if desired an optionally substituted methylidene group Z′ is converted into an oxo group Z, and

in accordance with stage 1.4 for the manufacture of compounds of the formula II, a compound of the formula

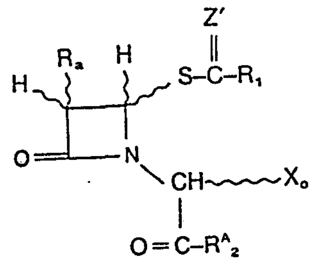
(VI)

in which $X_o$ represents a reactive esterified hydroxy group and $R_a$, $R_1$, Z′ and $R_2^A$ have the meanings given above, is treated with a suitable phosphine compound or a suitable phosphite compound,

or a compound of the formula

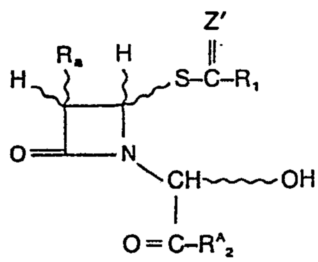
(V)

in which $R_a$, $R_1$, Z′ and $R_2^A$ have the meanings given above is treated with carbon tetrachloride and a phosphine, and, if desired, a group $R_a$ or $R_1$ in a resulting compound is converted into a different group $R_a$ or $R_1$, respectively, and/or, if desired, an optionally substituted methylidene group Z′ is converted into an oxo group Z, or,

in accordance with stage 2.1 for the manufacture of compounds of the formula

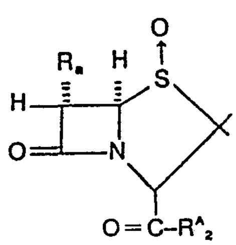
(VIII),

in which $R_a$ and $R_2^A$ have the meanings given under formula II, a penicillanic acid compound of the formula

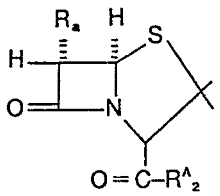

(VII)

is oxidised in the 1-position and, if desired, a group $R_a$ in a resulting compound is converted into a different group $R_a$, in accordance with stage 2.2 for the manufacture of compounds of the formula

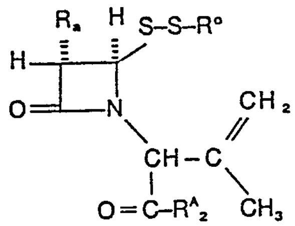

(IX)

in which $R_a$ and $R_2^A$ have the meanings given under formula II and $R^o$ is an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–, a compound of the formula

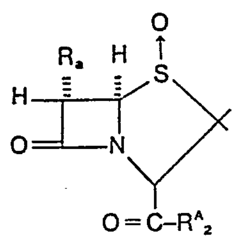

(VIII)

is treated with a mercapto compound $R^o$–SH and, if desired, a group $R_a$ in a resulting compound is converted into a different group $R_a$, in accordance with stage 2.3 for the manufacture of compounds of the formula

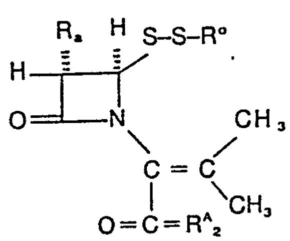

(X)

in which $R_a$ and $R_2^A$ have the meanings given under formula II and $R^o$ is an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–, a 3-methylenebutyric acid compound of the formula

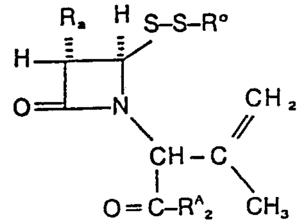

(IX)

is isomerised by treating with a suitable basic agent and, if desired, a group $R_a$ in a resulting compound is converted into a different group $R_a$, in accordance with stage 2.4 for the manufacture of compounds of the formula

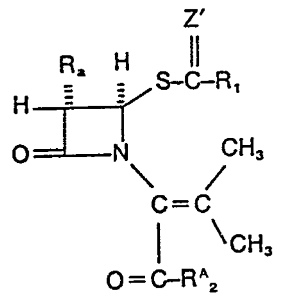

(XI)

in which $R_a$, $R_1$, Z′ and $R_2^A$ have the meanings given under formula II, a compound of the formula

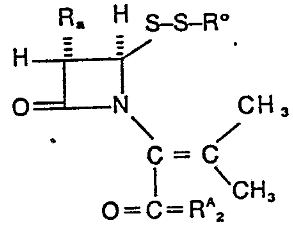

(X)

in which $R^o$ represents an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–, and $R_a$ and $R_2^A$ have the meanings given above, is treated with a suitable reducing agent and simultaneously or subsequently reacted with an acylating derivative of a carboxylic acid of the formula $R_1$–C(=Z)–OH or, when Z′ represents a methylidene group optionally substituted by Y, reacted with an alkyne of the formula $R_1$–C≡C–Y, or, in accordance with stage 2.3a, a compound of the formula

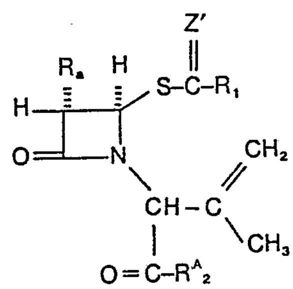

(Xa)

in which $R_a$, $R_1$, Z′ and $R_2^A$ have the meanings given above is isomerised by treating with a suitable basic agent and, if desired, a group $R_a$ or $R_1$ in a resulting compound is converted into a different group $R_a$ or $R_1$, respectively, and/or, if desired, an optionally substituted methylidene group Z′ is converted into an oxo group Z, in accordance with stage 2.4a for the manufacture of compounds of the formula

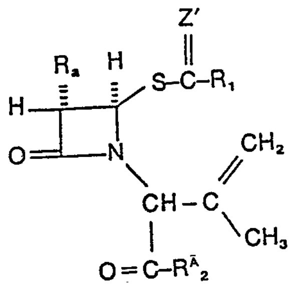

(Xa)

in which $R_a$, $R_1$, Z′ and $R_2^A$ have the meanings given under formula II, a compound of the formula

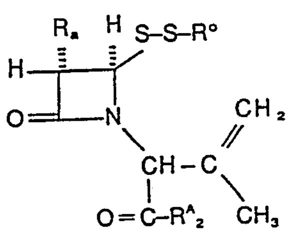

(IX),

in which R°, $R_a$ and $R_2^A$ have the meanings given above, is treated with a suitable reducing agent and simultaneously or subsequently reacted with an acylating derivative of a carboxylic acid of the formula $R_1$–C(=Z)–OH or, when Z′ represents a methylidene group optionally substituted by Y, reacted with an alkyne of the formula $R_1$–C≡C–Y and, if desired, a group $R_a$ or $R_1$ in a resulting compound is converted into a different group $R_a$ or $R_1$, respectively, and/or, if desired, an optionally substituted methylidene group Z′ is converted into an oxo group Z, in accordance with stage 2.5 for the manufacture of compounds of the formula

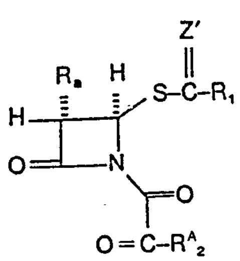

(XII)

in which $R_a$, $R_1$, Z′ and $R_2^A$ have the meanings given under formula II, a compound of the formula

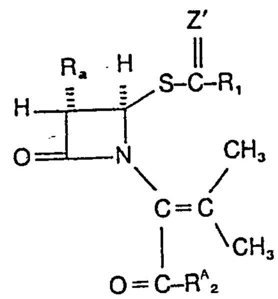

(XI)

in which $R_a$, $R_1$, Z′ and $R_2^A$ have the meanings given above, is ozonised and the ozonide formed is cleaved reductively to form the oxo group and, if desired, a group $R_a$ or $R_1$ in a resulting compound is converted into a different group $R_a$ or $R_1$, respectively, and/or, if desired, an optionally substituted methylidene group Z′ is converted into an oxo group Z, and then the stages 2.6 and 1.2 to 1.4 are carried out or, in accordance with stage 3.1 for the manufacture of compounds of the formula

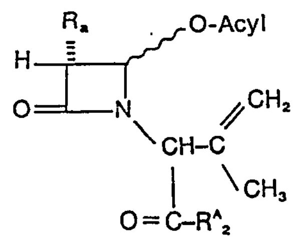

(XIII),

in which $R_a$ and $R_2^A$ have the meanings given under formula II, and acyl is the acyl radical of an organic carboxylic acid, in the cis,trans-, the cis- or the trans-form, a 1-oxide of a penicillanic acid compound of the formula

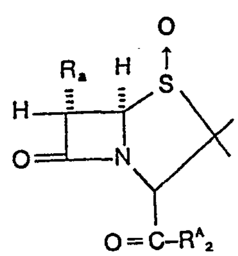

(VIII)

in which $R_a$ and $R_2^A$ have the meanings given above, is treated in the presence of a tri-lower alkyl phosphite with an organic carboxylic acid acyl-OH and, if desired, a group $R_a$ in a resulting compound is converted into a different group $R_a$ and/or the cis- and/or the trans-compound is or are isolated from a resulting cis,trans-compound, in accordance with stage 3.2 for the manufacture of compounds of the formula

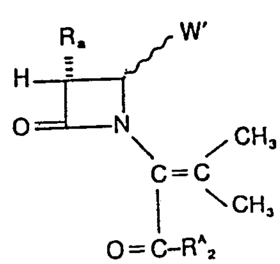

(XIV)

in which $R_a$ and $R_2^A$ have the meanings given under formula II and W′ represents acyloxy in which acyl is the acyl radical of an organic carboxylic acid, and $R_2^A$ together with the carbonyl group $-C(=O)-$ represents a protected carboxy group, in the cis,trans-, the cis- or the trans-form, a compound of the formula

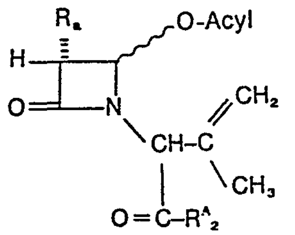

(XIII)

in which $R_a$, acyl and $R_2^A$ have the meanings given above, is isomerised by treating with a suitable basic agent and, if desired, a group $R_a$ in resulting compound is converted into a different group $R_a$ and/or, if desired, the cis- and/or the trans-compound is or are isolated from a resulting cis,-trans-compound, or, in accordance with stage 3.3 for the manufacture of compounds of the formula

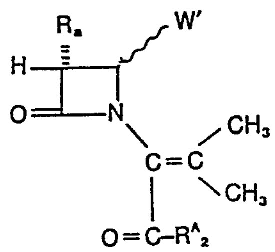

(XIV)

in which $R_a$ and $R_2^A$ have the meanings given under formula II and W′ represents halogen, in the cis,trans-, the cis- or the trans-form, a penicillanic acid compound of the formula

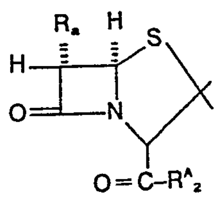

(VII)

in which $R_a$ and $R_2^A$ have the meanings given above, is reacted with a halogenating agent supplying positive halogen ions and, if necessary, a possibly resulting intermediate is treated with a base and, if desired, a group $R_a$ in a resulting compound of the formula XIV is converted into a different group $R_a$ and/or the cis- and/or the trans-compound is or are isolated from a resulting cis,-trans-compound, in accordance with stage 3.4 for the manufacture of compounds of the formula

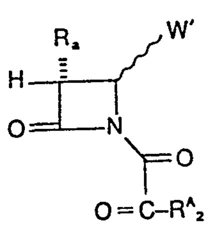

(XV)

in which $R_a$ and $R_2^A$ have the meanings given under formula II and W′ represents halogen or acyloxy in which acyl is the acyl radical of an organic carboxylic acid, in the cis,trans-, the cis- or the trans-form, a compound of the formula

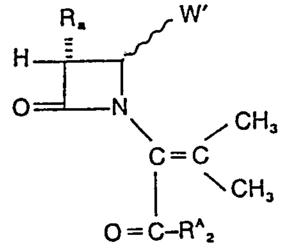

(XIV)

in which $R_a$, W′ and $R_2^A$ have the meanings given above, is ozonised and the ozonide formed is cleaved reductively to form the oxo group and, if desired, a group $R_a$ in a resulting compound is converted into a different group $R_a$ and/or, if desired, the cis- and/or the trans-compound is or are isolated from a resulting cis,trans-compound, in accordance with stage 3.5 for the manufacture of compounds of the formula

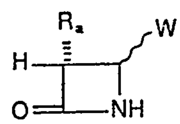

(IIIa)

in which $R_a$ has the meanings given under formula II and W represents a nucleofugal leaving group, in the cis,trans-, the cis- or the trans-form, a compound of the formula

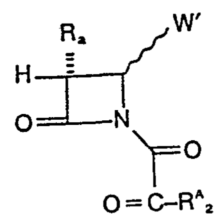

(XV)

in which $R_a$ and $R_2^A$ have the meanings given above and W′ represents acyloxy or halogen, is solvolysed and, if desired, in a resulting compound a group $R_a$ is converted into a different group $R_a$ and/or, if desired, a group W′ is converted into a different group W′ or into W and/or, if desired, a resulting cis-compound is isomerised to form the corresponding trans-compound and/or the cis- and/or the trans-compound is or are isolated from a resulting cis,trans-compound, and subsequently stages 1.1 to 1.4 are carried out.

2. Process according to patent claim 1, characterised in that compounds of the formula II are manufactured in which $R_a$ is lower alkyl, hydroxy-lower alkyl, lower alkoxy-lower alkyl, lower alkanoyloxy-lower alkyl, hydroxysulphonyloxy-lower alkyl present in salt form, hydroxy, lower alkoxy, lower alkanoyloxy, lower alkanoyloxy substituted by phenoxy, halogen, amino or cyano, phenyl-lower alkanoyloxy, or phenyl-lower alkanoyloxy

substituted by hydroxy or amino, $R_1$ is hydrogen, lower alkyl, hydroxy-lower alkyl, lower alkoxy-lower alkyl, lower alkanoyloxy-lower alkyl, lower alkylthio-lower alkyl, heterocyclylthio-lower alkyl in which heterocyclyl is a five-membered aromatic diaza-, triaza-, tetraza-, thiaza-, thiadiaza-, thiatriaza-, oxaza- or oxadiaza-cyclic radical optionally substituted by lower alkyl, carboxy-lower alkyl, di-lower alkylamino-lower alkyl, or by sulpho-lower alkyl present in salt form, amino-lower alkyl, acylamino-lower alkyl in which acyl is lower alkanoyl or a substituted oxycarbonyl group that can be used as an amino-protecting group, for example a tert.-butyl-, 2,2,2-trichloroethyl-, diphenylmethyl- or p-nitrobenzyl-oxycarbonyl group, carboxy-lower alkyl, lower alkoxycarbonyl-lower alkyl, phenyl-lower alkyl, phenyl, hydroxyphenyl, aminophenyl, furyl, thienyl, pyridyl, lower alkylthio, lower alkylthio or lower alkenylthio substituted by amino, mono- or di-lower alkylamino or lower alkanoylamino, or tetrazolylthio or thiadiazolylthio optionally substituted by lower alkyl, sulpho-lower alkyl, carboxy-lower alkyl or di-lower alkylamino-lower alkyl, functional groups in such radicals $R_a$ and $R_1$ preferably being in protected form, and Z′, $X^{\oplus}$ and $R_2^A$ have the meanings given in patent claim 1, in racemic or optically active form.

3. Compounds of the formula

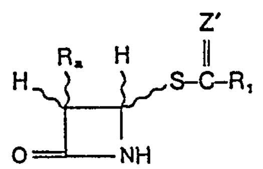

(IV)

in which $R_a$, $R_1$ and Z′ have the meanings given in claim 1, in racemic and optionally active form.

4. Compounds of the formula IV according to patent claim 3 in which $R_a$, $R_1$ and Z′ have the meanings given in patent claim 2.

5. 4-acetylthio-3-methyl-2-oxoazetidine (racemic cis-compound) according to patent claim 3.

6. 4-acetylthio-3-methyl-2-oxoazetidine (racemic trans-compound) according to patent claim 3.

7. 4-acetylthio-3-isopropyl-2-oxoazetidine (racemic trans-compound) according to patent claim 3.

8. 4-acetylthio-3-benzyl-2-oxoazetidine (racemic trans-compound) according to patent claim 3.

9. 4-ethylthiothiocarbonylthio-3-isopropyl-2-oxoazetidine (racemic trans-compound) according to patent claim 3.

10. (3S,4R)-4-acetylthio-3-methoxy-2-oxoazetidine according to patent claim 3.

11. (3S,4R)-4-acetylthio-3-phenoxyacetoxy-2-oxoazetidine according to patent claim 3.

12. 3-ethyl-4-(4-p-nitrobenzyloxycarbonylamino-butyrylthio)-2-oxoazetidine (racemic cis,trans-compound) according to patent claim 3.

13. (3S,4R)-4-(2-p-nitrobenzyloxycarbonylaminoethylthiothiocarbonylthio)3-methoxy-2-oxoazetidine according to patent claim 3.

14. (3S,4R)-4-(4-p-nitrobenzyloxycarbonylaminobutyrylthio)-3-methoxy-2-oxoazetidine according to patent claim 3.

15. 3-ethyl-4-(2-acetylaminoethylthiothiocarbonylthio)-2-oxoazetidine (racemic cis,trans-compound) according to patent claim 3.

16. Compounds of the formula

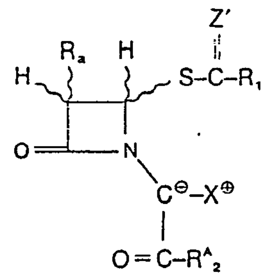

(II)

in which $R_a$, $R_1$, Z′, $X^{\oplus}$ and $R_2^A$ have the meanings given in patent claim 1, in racemic or optically active form.

17. Compounds of the formula II, according to patent claim 16, in which $R_a$, $R_1$, Z′, $X^{\oplus}$ and $R_2^A$ have the meanings given in patent claim 2, in racemic or optically active form.

18. Compounds of the formula II, according to patent claim 16 or 17, in which $R_a$ represents methyl, ethyl, isopropyl, benzyl, methoxy or phenoxyacetoxy, $R_1$ represents methyl, 3-(p-nitrobenzyloxycarbonylamino)-propyl, ethylthio, 2-acetylaminoethylthio or 2-(p-nitrobenzyloxycarbonylamino)-ethylthio, Z′ represents oxygen or sulphur, $R_2^A$ represents p-nitrobenzyloxy and $X^{\oplus}$ represents the group $P^{\ominus}$ (phenyl)$_3$.

19. Compounds of the formula

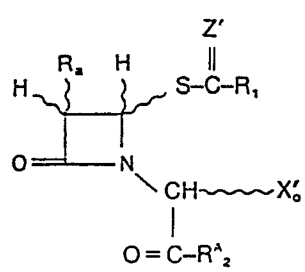

(V) or (VI)

in which $R_a$, $R_1$, Z′ and $R_2^A$ have the meanings given in patent claim 1 and $X_o'$ represents hydroxy or a reactive esterified hydroxy group, in racemic or optically active form.

20. Compounds of the formula V or VI according to patent claim 19 in which $R_a$, $R_1$, Z′ and $R_2^A$ have the meanings given in patent claim 2 and $X_o'$ has the meanings given in patent claim 19, in racemic or optically active form.

21. Compounds of the formula V or VI according to patent claim 19 or 20 in which $R_a$ represents methyl, ethyl, isopropyl, benzyl, methoxy or phenoxyacetoxy, $R_1$ represents methyl, 3-(p-nitrobenzyloxycarbonylamino)-propyl, ethylthio, 2-acetylaminoethylthio or 2-(p-nitrobenzyloxycarbonylamino)-ethylthio, Z′ represents oxygen or sulphur, $R_2^A$ represents p-nitrobenzyloxy and $X_o'$ represents hydroxy or chlorine.

22. Compounds of the formula

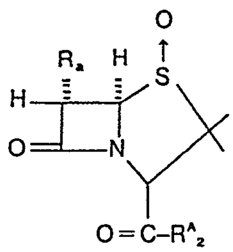

(VIII)

in which $R_a$ and $R_2^A$ have the meanings given in patent claim 1.

23. Compounds of the formula VIII according to patent claim 22 in which $R_a$ and $R_2^A$ have the meanings given in patent claim 2.

24. Compounds of the formula VIII according to patent claim 22 or 23 in which $R_a$ represents methoxy or phenoxyacetoxy and $R_2^A$ represents methoxy or 2,2,2-trichloroethoxy.

25. Compounds of the formula

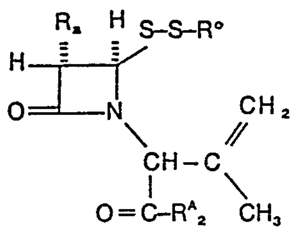

(IX)

in which $R_a$ and $R_2^A$ have the meanings given in patent claim 1 and $R^o$ represents an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–.

26. Compounds of the formula IX according to patent claim 25 in which $R_a$ and $R_2^A$ have the meanings given in patent claim 2 and $R^o$ has the meanings given in patent claim 25.

27. Compounds of the formula IX according to patent claim 25 or 26 in which $R_a$ represents methoxy or phenoxyacetoxy, $R_2^A$ represents methoxy or 2,2,2-trichloroethoxy and $R^o$ represents 2-benzthiazolyl.

28. Compounds of the formula

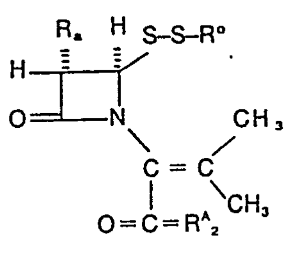

(X)

in which $R_a$ and $R_2^A$ have the meanings given in patent claim 1 and $R^o$ represents an optionally substituted aromatic heterocyclic radical having up to 15 carbon atoms, at least one ring nitrogen atom and optionally one other ring hetero atom, which radical is bonded by one of its ring carbon atoms, which is bonded to a ring nitrogen atom by a double bond, to the thio group –S–.

29. Compounds of the formula X according to patent claim 28 in which $R_a$ and $R_2^A$ have the meanings given in patent claim 2 and $R^o$ has the meanings given in patent claim 28.

30. Compounds of the formula X according to patent claim 28 or 29 in which $R_a$ represents methoxy or phenoxyacetoxy, $R_2^A$ represents methoxy or 2,2,2-trichloroethoxy and $R^o$ represents 2-benzthiazolyl.

31. Compounds of the formula

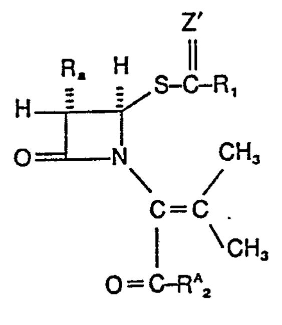

(XI)

in which $R_a$, $R_1$, $Z'$ and $R_2^A$ have the meanings given in patent claim 1.

32. Compounds of the formula XI according to patent claim 31 in which $R_a$, $R_1$, $Z'$ and $R_2^A$ have the meanings given in patent claim 2.

33. Compounds of the formula XI according to patent claim 31 or 32 in which $R_a$ represents methyl, methoxy or phenoxyacetoxy, $R_1$ represents methyl, $Z'$ represents oxygen and $R_2^A$ represents methoxy or 2,2,2-trichloroethoxy.

34. Compounds of the formula

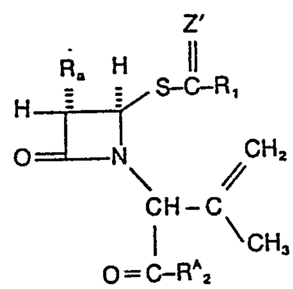

(Xa)

in which $R_a$, $R_1$, $Z'$ and $R_2^A$ have the meanings given in patent claim 1.

35. Compounds of the formula Xa according to patent claim 34 in which $R_a$, $R_1$, $Z'$ and $R_2^A$ have the meanings given in patent claim 2.

36. Compounds of the formula

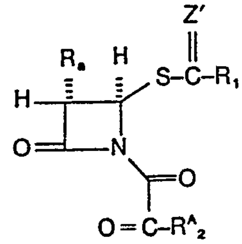

(XII)

in which $R_a$, $R_1$, $Z'$ and $R_2^A$ have the meanings given in patent claim 1.

37. Compounds of the formula XII according to patent claim 36 in which $R_a$, $R_1$, $Z'$ and $R_2^A$ have the

meanings given in patent claim 2.

38. Compounds of the formula XII according to patent claim 36 or 37 in which $R_a$ represents methyl, methoxy or phenoxyacetoxy, $R_1$ represents methyl, Z′ represents oxygen and $R_2^A$ represents methoxy or 2,2,2-trichloroethoxy.

39. Compounds of the formula

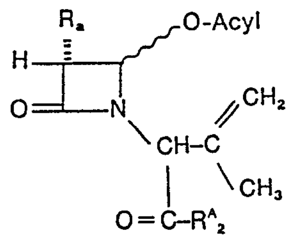

(XIII)

in which $R_a$ and $R_2^A$ have the meanings given in patent claim 1 and acyl is the acyl radical of an organic carboxylic acid in the cis,trans-, the cis- or the trans-form.

40. Compounds of the formula XIII according to patent claim 39 in which $R_a$ represents methyl or methoxy, $R_2^A$ represents 2,2,2-trichloroethoxy and acyl is an acetyl group, in the cis,trans-, the cis- or the trans-form.

41. Compounds of the formula

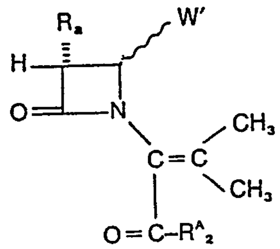

(XIV)

in which $R_a$ and $R_2^A$ have the meanings given in patent claim 1, W′ represents halogen or acyloxy in which acyl is the acyl radical of an organic carboxylic acid, in the cis,trans-, the cis- or the trans-form.

42. Compounds of the formula XIV according to patent claim 41 in which $R_a$ represents methoxy, $R_2^A$ represents 2,2,2-trichloroethoxy and W′ represents chlorine or acetoxy, in the cis,trans-, the cis- or the trans-form.

43. Compounds of the formula

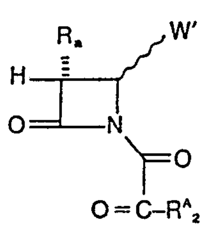

(XV)

in which $R_a$ and $R_2^A$ have the meanings given in patent claim 1, W′ represents halogen or acyloxy in which acyl is the acyl radical of an organic carboxylic acid, in the cis,trans-, the cis- or the trans-form.

44. Compounds of the formula XI according to patent claim 43 in which $R_a$ represents methoxy, $R_2^A$ represents 2,2,2-trichloroethoxy and W′ represents acetoxy or chlorine, in the cis,trans-, the cis- or the trans-form.

45. Compounds of the formula

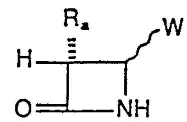

(IIIa)

in which $R_a$ has the meanings given in claim 1 and W represents a nucleofugal leaving group, in the cis,trans-, the cis- or the trans-form.

46. Compounds of the formula IIIa according to patent claim 45 in which $R_a$ represents methoxy and W represents chlorine or acetoxy, in the cis,-trans-, the cis- or the trans-form.

47. The individual process steps according to patent claim 1.

48. The individual process steps according to patent claim 1 in accordance with reaction scheme 3.

49. The individual process steps according to patent claim 1 in accordance with reaction schemes 1 and 2.

**Revendications pour les Etats contractants: BE, CH, DE, FR, GB, LU, NL, SE**

1. Procédé de préparation de composés de formule

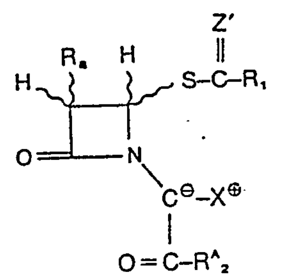

(II),

où Ra représente un radical hydrocarboné saturé ou non saturé, éventuellement substitué, aliphatique, cycloaliphatique, cycloaliphatique-aliphatique, aromatique ou araliphatique ayant jusqu'à 18, de préférence jusqu'à 10, atomes de carbone,

un radical hétérocyclyl- ou hétérocyclyl-alcoyle inférieur éventuellement substitué ayant jusqu'à 10 atomes de carbone et jusqu'à 4 hétéroatomes dans son noyau, du groupe azote, oxygène et/ou soufre,

dans lesquels radicaux Ra les substituants éventuellement présents sont hydroxy, alcoxy inférieur, alcanoyloxy inférieur, hydroxysulfonyloxy présent, sous forme de sel, halogène, mercapto, alcoyle inférieur-mercapto, carboxyle, alcoxy inférieur-carbonyle, carbamoyle, cyano, nitro, sulfo présent sous forme de sel, ou amino éventuellement mono- ou disubstitué par alcoyle inférieur ou acyle en C1 à C20 ou disubstitué par un alcoylène inférieur,

un carboxyle éventuellement protégé,

un hydroxy,

un mercapto,

un hydroxy ou mercapto éthérifié par un radical hydrocarboné éventuellement substitué, aliphatique, cycloaliphatique, cycloaliphatique-aliphatique, aromatique ou araliphatique ayant jusqu'à 18, en particulier jusqu'à 10, atomes de carbone, où les substituants éventuellement présents sont hydroxy, alcoxy inférieur, alcanoyloxy inférieur, halogène, mercapto, alcoyle inférieur-mercapto, carboxyle, alcoxy inférieur-carbonyle, carbamoyle, cyano, nitro ou amino éventuellement mono- ou disubstitué par alcoyle inférieur ou acyle en C1 à C20 ou disubstitué par alcoylène inférieur,

hydroxy ou mercapto estérifié par un radical acyle en C1 à C20 d'un acide carboxylique aliphatique, cycloaliphatique, cycloaliphatique-aliphatique, aromatique ou araliphatique éventuellement substitué ayant jusqu'à 18 atomes de carbone, où

les substituants éventuellement présents sont hydroxy, alcoxy inférieur, phényloxy, alcanoyloxy inférieur, halogène, mercapto, alcoyle inférieur-mercapto, carboxyle, alcoxy inférieur-carbonyle, carbamoyle, cyano, nitro, ou amino éventuellement mono- ou disubstitué par alcoyle inférieur ou acyle en C1 à C20 ou disubstitué par alcoylène inférieur, ou halogène,

R1 représente un hydrogène, un radical hydrocarboné saturé ou non saturé, aliphatique, cycloaliphatique, cycloaliphatique-aliphatique, aromatique ou araliphatique éventuellement substitué ayant jusqu'à 18, de préférence jusqu'à 10, atomes de carbone, ou

un radical hétérocyclyle ou hétérocyclyl-alcoyle inférieur éventuellement substitué ayant jusqu'à 10 atomes de carbone et jusqu'à 4 hétéroatomes dans son noyau, du groupe azote, oxygène et/ou soufre,

dans lesquels radicaux R1 les substituants éventuellement présents sont hydroxy, alcoxy inférieur, alcanoyloxy inférieur, halogène, mercapto, alcoyle inférieur-mercapto, hétérocyclylmercapto éventuellement substitué par alcoyle inférieur, hydroxyalcoyle inférieur, carboxyalcoyle inférieur, aminoalcoyle inférieur, dialcoyle inférieur-aminoalcoyle inférieur, sulfoalcoyle inférieur présent sous forme de sel, cycloalcoyle, aryle, arylalcoyle inférieur, halogène, amino, mono- ou di-alcoyle inférieur-amino, nitro, hydroxy, alcoxy inférieur, carboxyle, alcoxy inférieur-carbonyle, carbamoyle, N-mono- ou N,N-dialcoyle inférieur-carbamoyle, cyano, oxo ou oxydo,

carboxyle, alcoxy inférieur-carbonyle, carbamoyle, cyano, nitro, amino, alcoyle inférieur-amino, dialcoyle inférieur-amino, acylamino en C1 à C20, di-acylamino en C1 à C20 ou alcoylène inférieur-amino, ou bien où R1 est un groupe mercapto éthérifié par un radical hydrocarboné aliphatique, cycloaliphatique, cycloaliphatique-aliphatique, aromatique ou araliphatique éventuellement substitué ayant jusqu'à 18, en particulier jusqu'à 10, atomes de carbone,

où les substituants éventuellement présents sont hydroxy, alcoxy inférieur alcanoyloxy inférieur, halogène, mercapto, alcoyle inférieur-mercapto, carboxyle, alcoxy inférieur-carbonyle, carbamoyle, cyano, nitro, amino éventuellement mono- ou disubstitué par un alcoyle inférieur ou un acyle en C1 à C20 ou disubstitué par un alcoylène inférieur, ou bien où R1 est un phénylthio ou phénylalcoyle inférieur-thio substitué par un alcoyle inférieur, ou bien où R1 est un groupe carboxyle éventuellement protégé, ou bien où R1 est un groupe mercapto éthérifié par un radical hétérocyclique éventuellement substitué, où le radical hétérocyclyle est lié par l'intermédiaire d'un atome de carbone du noyau au groupe mercapto et contient de 1 à 4 atomes de l'azote dans son noyau et éventuellement dans son noyau un autre hétéroatome du groupe oxygène ou soufre et où les substituants éventuellement présents sont alcoyle inférieur, alcoyle inférieur substitué par hydroxy, alcanoyloxy inférieur, halogène, carboxy, alcoxy inférieur-carbonyle, sulfo, sulfo amidé, amino, mono- ou di-alcoyle inférieur-amino, acylamino en C1 à C20 ou alcanoyle inférieur-amino substitué par carboxy ou halogène, cycloalcoyle, phényle éventuellement substitué par halogène ou nitro, benzyle, furyle, thiényle, oxazolyle, halogène, amino éventuellement mono- ou disubstitué par alcoyle inférieur, acylamino en C1 à C20, nitro, hydroxy, alcoxy inférieur, carboxy, alcoxy inférieur-carbonyle, carbamoyle éventuellement substitué par un N-mono- ou N,N-dialcoyle inférieur, cyano, oxo ou oxydo, où les groupes fonctionnels dans ces radicaux Ra et R1 se présentent de préférence sous forme protégée et les groupes décrits par «inférieur» contiennent jusqu'à 7, en particulier jusqu'à 4, atomes de carbone, Z' représente un oxygène, un soufre ou un groupe méthylidène portant éventuellement un ou deux substituants, $X^{\oplus}$ représente soit un groupe phosphonio trisubstitué, soit un groupe phosphono di-estérifié avec un cation et $R_2^A$ représente avec le groupe carbonyle $-C(=O)-$ un groupe carboxyle protégé, sous forme facémique ou optiquement active,

caractérisé en ce qu'on conduit les étapes 1.1 à 1.4 selon

Schéma réactionnel 1

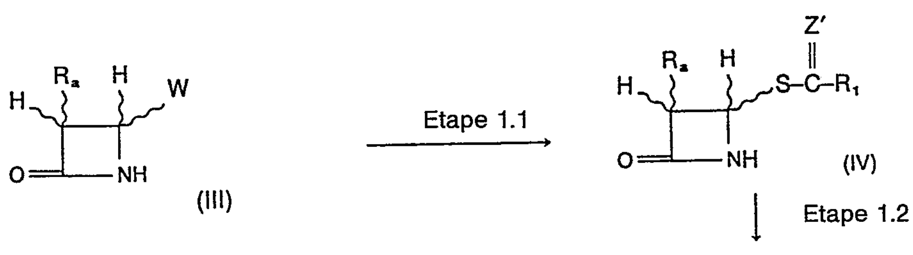


(III) (IV)

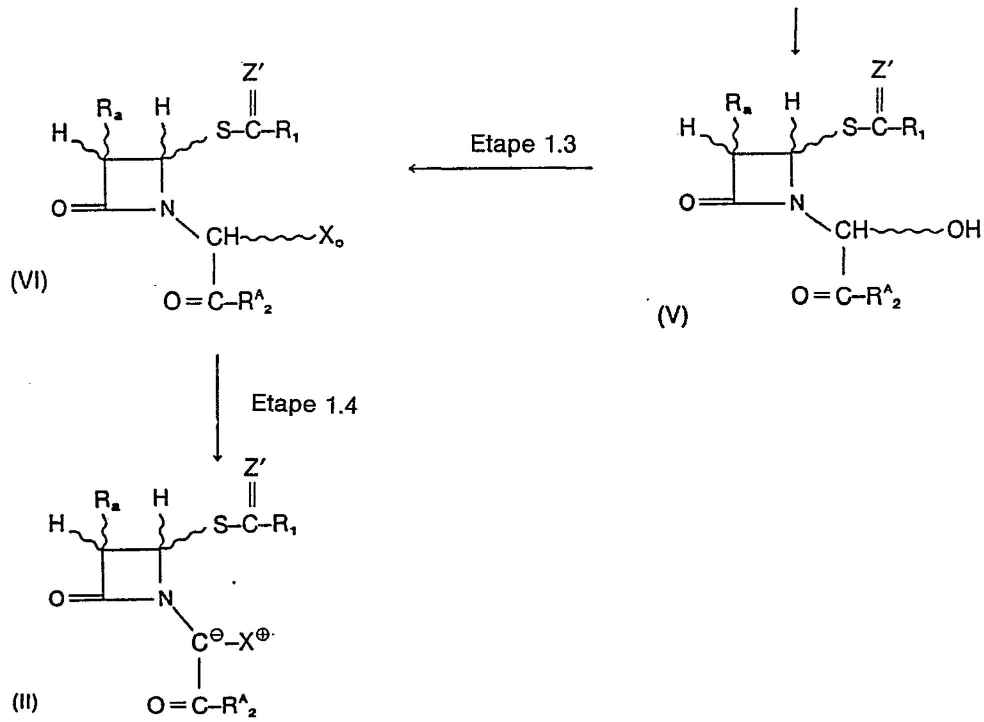


(V)

(VI)

(II)

ou les étapes 2.1 à 2.6 selon

Schéma réactionnel 2

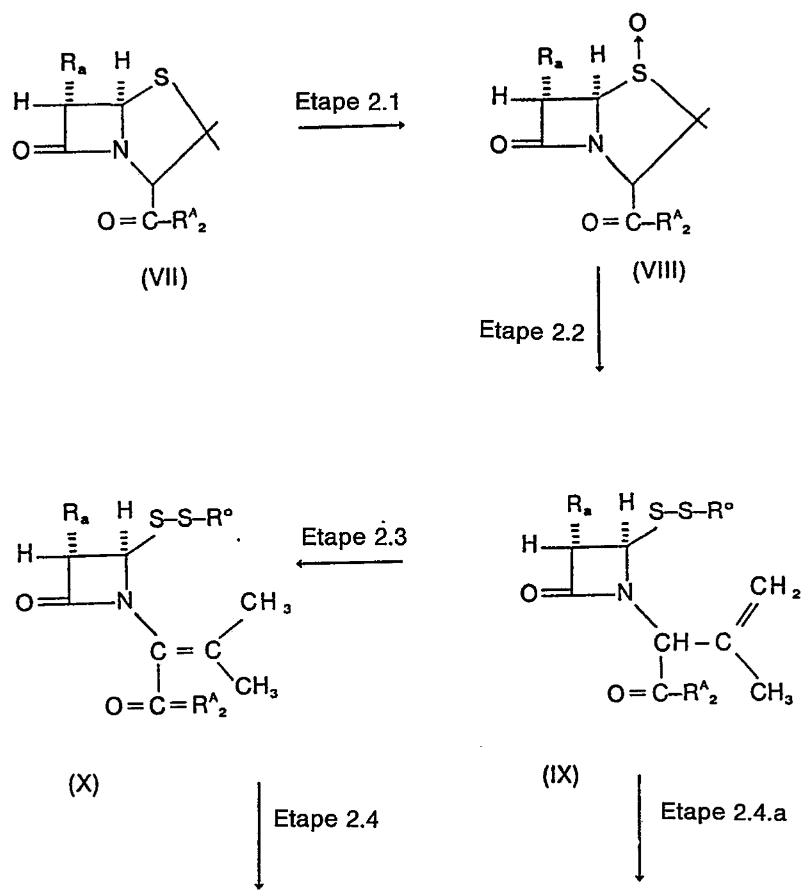


(VII)

(VIII)

(IX)

(X)

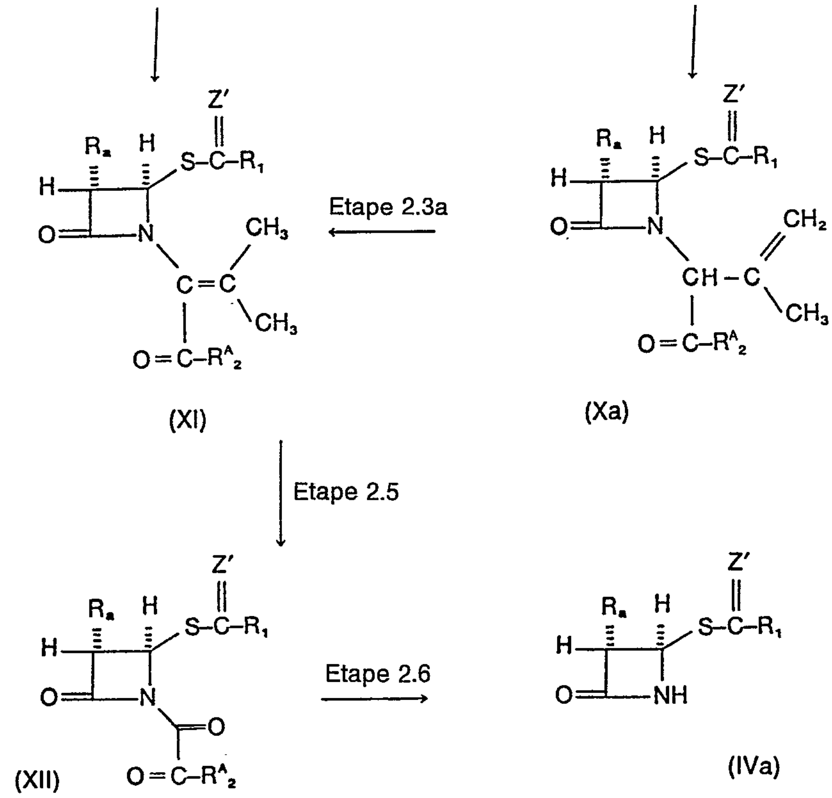


(XI) (Xa) (XII) (IVa)

suivi par les étapes 1.2 à 1.4, ou les étapes 3.1 à 3.5 selon

Schéma réactionnel 3

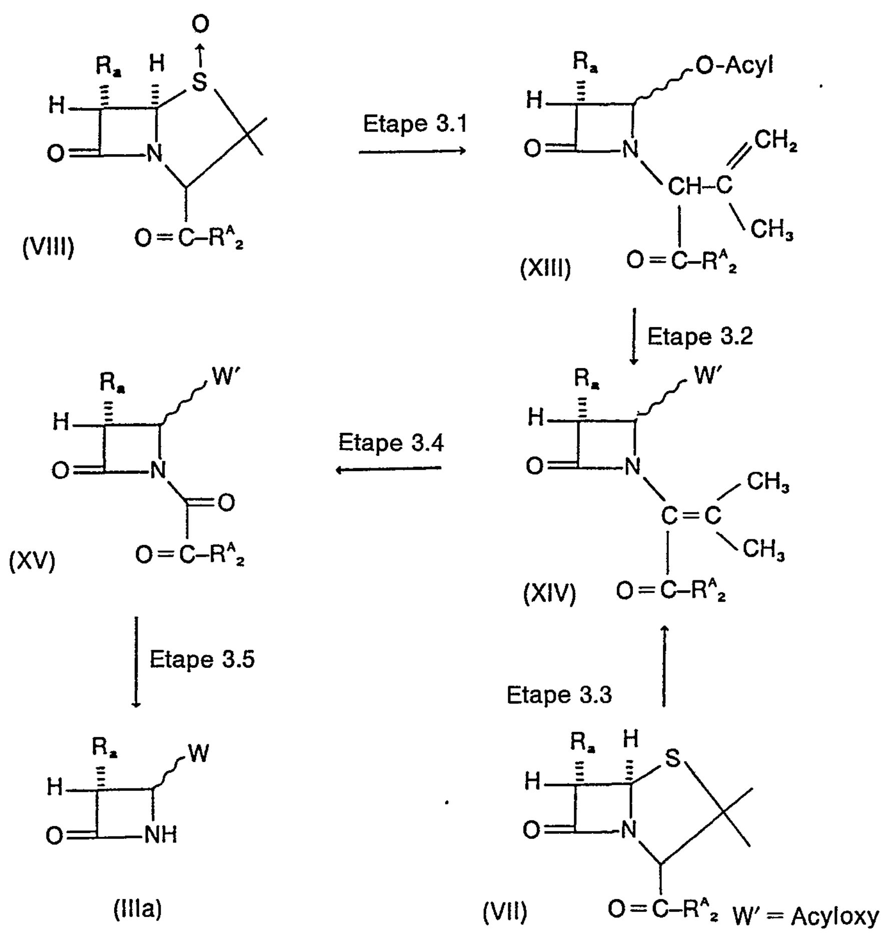


(VIII) (XIII) (XV) (XIV) (IIIa) (VII)

W' = Acyloxy ou Halogène

suivi par les étapes 1.1 à 1.4, où l'on fait réagir selon l'étape 1.1 pour préparer des composés de formule

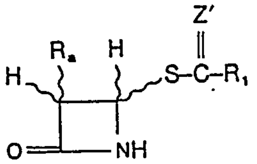

(IV) ou (IVa),

où Ra, R1 et Z' ont les significations mentionnées sous la formule II, sous forme racémique ou optiquement active, une 4-W-azétidinone de formule

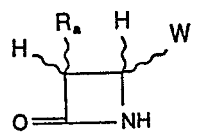

(III)

ou

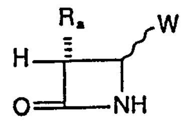

(IIIa),

où représente un groupe nucléofuge, avec un composé mercapto R1–C(=Z')–SH ou un de ses sels, et si on le désire on sépare un mélange d'isomères obtenu en les isomères isolés, ou, selon l'étape 2.6 pour préparer des composés (3S,4R) optiquement actifs de formule

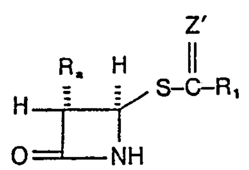

(IVa),

on solvolyse un composé de formule

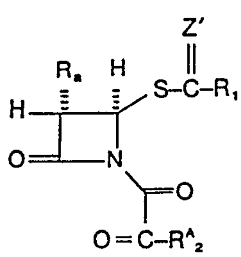

(XII),

où Ra, R1, Z' et $R_2^A$ ont les significations données pour la formule II, et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, selon l'étape 1.2 pour préparer des composés de formule

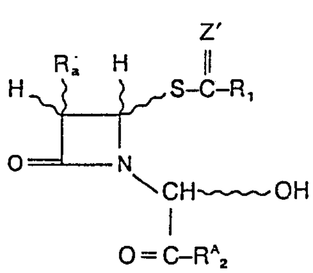

(V)

où Ra, R1, Z' et $R_2^A$ ont les significations données pour la formule II, sous forme racémique ou optiquement active, on fait réagir un composé de formule

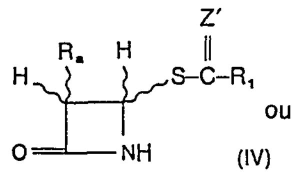

ou

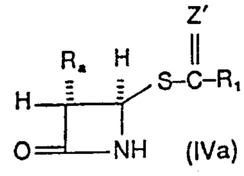

où Ra, R1 et Z' ont les significations données ci-dessus, avec un composé d'acide glyoxylique de formule OHC–C(=O)–$R_2^A$ ou un de ses dérivés appropriés, et si on le désire on sépare un mélange d'isomères obtenu en les isomères isolés, et/ou si on le désire on transforme un groupe Ra ou R1 en un autre groupe Ra ou selon les cas R1, et/ou si on le désire on transforme un groupe méthylidène éventuellement substitué Z' en un groupe oxo Z, selon l'étape 1.3 pour préparer des composés de formule

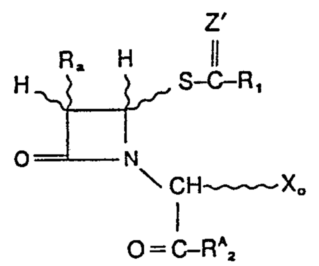

(VI),

où Ra, R1, Z' et $R_2^A$ ont les significations données sous la formule II et Xo représente un groupe hydroxy estérifié réactif, sous forme racémique ou optiquement active, dans un composé de formule

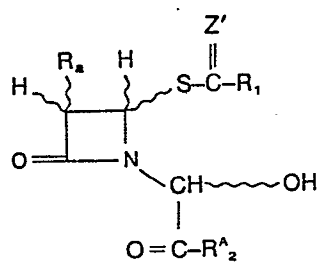

(V),

où Ra, R1, Z' et $R_2^A$ ont les significations données ci-dessus, on transforme le groupe hydroxy secondaire en un groupe hydroxy estérifié réactif, et si on le désire on sépare un mélange d'isomères obtenu en les isomères isolés, et/ou si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, et selon l'étape 1.4, pour préparer des composés de formule II, on traite un composé de formule

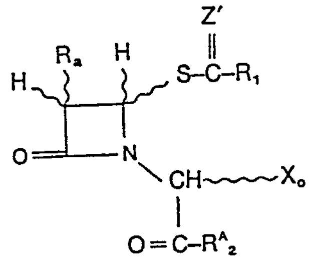

(VI),

où Xo représente un groupe hydroxy estérifié réactif, et

Ra, R1, Z' et $R_2^A$ ont les significations données ci-dessus, avec un composé de phosphine approprié, ou on traite un composé de formule

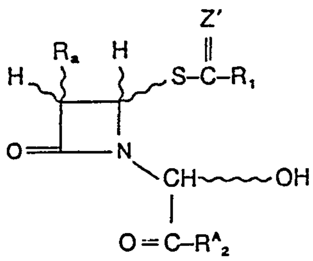

(V),

où Ra, R1, Z' et $R_2^A$ ont les significations données ci-dessus, avec du tétrachlorure de carbone et une phosphine, et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, ou selon l'étape 2.1, pour préparer des composés de formule

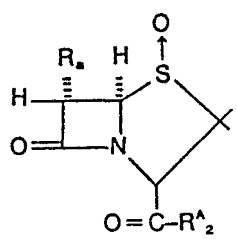

(VIII),

où Ra et $R_2^A$ ont les significations données pour la formule II, on oxyde en position 1 un composé d'acide pénicillanique de formule

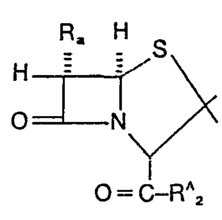

(VII),

et si on le désire on transforme dans un composé obtenu un groupe Ra en un autre groupe Ra, selon l'étape 2.2 pour préparer des composés de formule

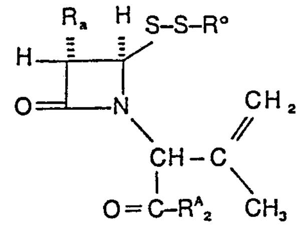

(IX),

où Ra et $R_2^A$ ont les significations données pour la formule II et Ro est un radical hétérocyclique aromatique éventuellement substitué ayant jusqu'à 15 atomes de carbone, au moins un atome d'azote dans son noyau et le cas échéant un autre hétéroatome dans le noyau, lequel radical est lié au groupe thio –S– par un de ses atomes de carbone du noyau, qui est relié à un atome d'azote du noyau par une double liaison, on traite un composé de formule

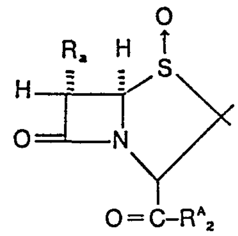

(VIII),

avec un composé mercapto Ro–SH, et si on le désire on transforme dans un composé obtenu un groupe Ra en un autre groupe Ra, selon l'étape 2.3 pour préparer des composés de formule

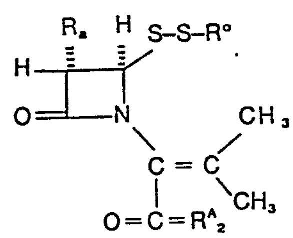

(X),

où Ra et $R_2^A$ ont les significations données pour la formule II et Ro est un radical hétérocyclique aromatique éventuellement substitué ayant jusqu'à 15 atomes de carbone, au moins un atome d'azote dans son noyau et éventuellement un autre hétéroatom dans son noyau, lequel radical est lié au groupe thio –S– par un de ses atomes de carbone du noyau qui est lié à un atome d'azote du noyau par une double liaison, on isomérise un composé d'acide 3-méthylènebutyrique de formule

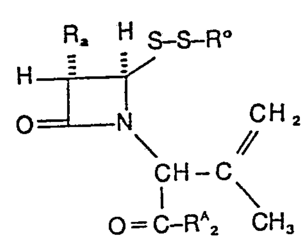

(IX),

par traitement avec un agent basique approprié, et si on le désire on transforme dans un composé obtenu un groupe Ra en un autre groupe Ra, selon l'étape 2.4 pour préparer des composés de formule

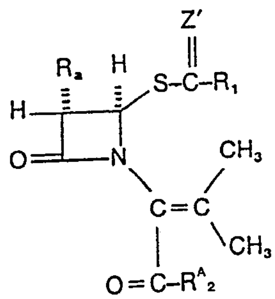

(XI),

où Ra, R1, Z' et $R_2^A$ ont les significations données pour la formule II, on traite avec un agent réducteur approprié un composé de formule

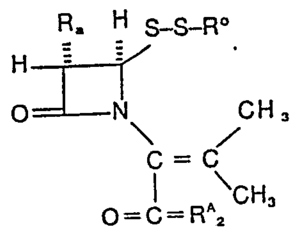

(X),

où Ro est un radical hétérocyclique aromatique éventuellement substitué ayant jusqu'à 15 atomes de carbone, au moins un atome d'azote dans son noyau et éventuellement un autre hétéroatome dans son noyau, lequel radical est lié au groupe thio –S– avec un de ses atomes de carbone du noyau, qui est relié à un atome d'azote du noyau par une double liaison, et Ra et $R_2^A$ ont les significations données ci-dessus, et simultanément ou ultérieurement avec un dérivé d'acylation d'un acide carboxylique de formule R1–C(=Z)–OH, ou, lorsque Z' représente un groupe méthylidène éventuellement substitué par Y, on le fait réagir avec un alcyne de formule R1–C≡C–Y, ou selon l'étape 2.3a on isomérise un composé de formule

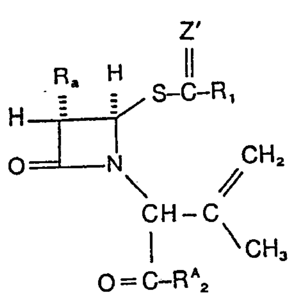

(Xa),

où Ra, R1, Z' et $R_2^A$ ont les significations données ci-dessus, par traitement avec un agent basique approprié, et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, selon l'étape 2.4a pour préparer des composés de formule

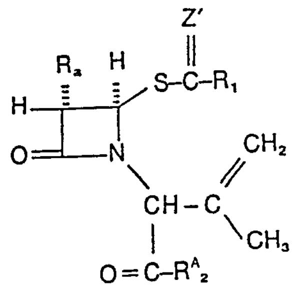

(Xa),

où Ra, R1, Z' et $R_2^A$ ont les significations données pour la formule II, on traite un composé de formule

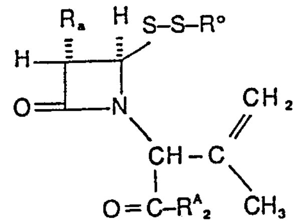

(IX),

où Ro, Ra et $R_2^A$ ont les significations données ci-dessus, avec un agent réducteur approprié et simultanément ou ultérieurement avec un dérivé d'acylation d'un acide carboxylique de formule R1–C(=Z)–OH, ou, lorsque Z' représente un groupe méthylidène éventuellement substitué par Y, on fait réagir avec un alcyne de formule R1–C≡C–Y et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, selon l'étape 2.5 pour préparer des composés de formule

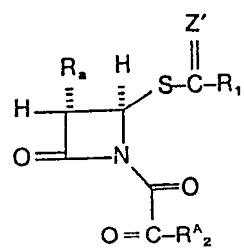

(XII),

où Ra, R1, Z' et $R_2^A$ ont les significations données pour la formule II, on ozonise un composé de formule

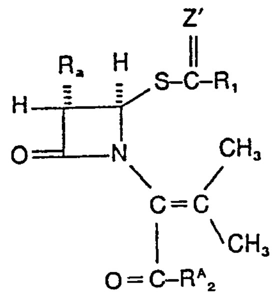

(XI),

où Ra, R1, Z′ et $R_2^A$ ont les significations données ci-dessus et on clive de façon réductrice l'ozonide formé pour donner le groupe oxo, et si on le désire on transforme dans un composé obtenu un groupe Ra ou R1 en un autre groupe Ra ou selon les cas R1, et/ou si on le désire on transforme un groupe méthylidène éventuellement substitué Z′ en un groupe oxo Z, puis on conduit les étapes 2.6 et 1.2 à 1.4, ou, selon l'étape 3.1 pour préparer des composés de formule

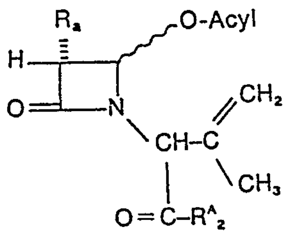

(XIII),

où Ra et $R_2^A$ ont les significations données pour la formule II et acyle est le radical acyle d'un acide carboxylique organique sous forme cis,trans, cis ou trans, on traite un 1-oxyde d'un composé d'acide pénicillanique de formule

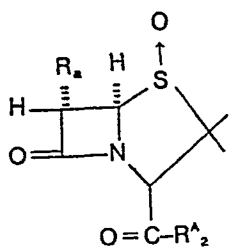

(VIII),

où Ra et $R_2^A$ ont les significations données ci-dessus, en présence d'un trialcoyle inférieur-phosphite avec un acide carboxylique organique acyl-acyl-OH, et si on le désire on transforme dans un composé obtenu un groupe Ra en un autre groupe Ra, et/ou à partir d'un composé cis,trans obtenu on isole le composé cis et/ou le composé trans, selon l'étape 3.2 pour préparer des composés de formule

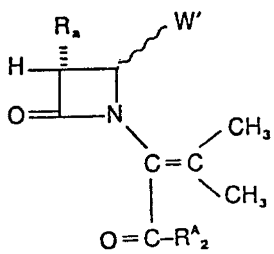

(XIV),

où Ra et $R_2^A$ ont les significations données pour la formule II et W′ représente un acyloxy, où acyle est le radical acyle d'un acide carboxylique organique, et $R_2^A$ avec le groupe carbonyle –C(=O)– représente un groupe carboxyle protégé, sous la forme cis,trans, cis ou trans, on isomérise un composé de formule

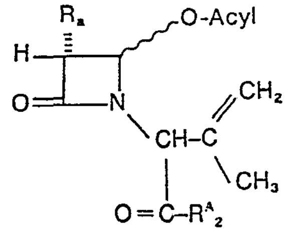

(XIII),

où Ra, acyle et $R_2^A$ ont les significations données ci-dessus, par traitement avec un agent basique approprié, et si on le désire on transforme dans un composé obtenu un groupe Ra en un autre groupe Ra, et/ou si on le désire on isole à partir d'un composé cis,trans obtenu le composé cis et/ou le composé trans, ou selon l'étape 3.3 pour préparer le composé de formule

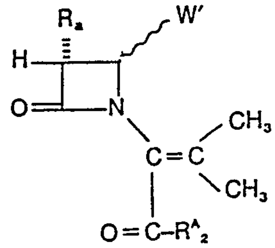

(XIV),

où Ra et $R_2^A$ ont les significations données pour la formule II et W′ représente un halogène, sous la forme cis,trans, cis ou trans, on fait réagir un composé d'acide pénicillanique de formule

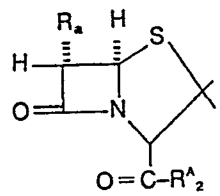

(VII),

où Ra et $R_2^A$ ont les significations données ci-dessus, avec un agent halogénant donneur d'ions halogène positifs et si nécessaire, on traite un produit intermédiaire éventuellement obtenu avec une base, et si on le désire on transforme dans un composé de formule XIV obtenu un groupe Ra en un autre groupe Ra et/ou on isole à partir d'un composé cis,trans obtenu le composé cis et/ou le composé trans, selon l'étape 3.4 pour préparer les composés de formule

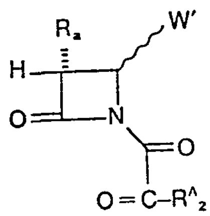

(XV),

où Ra et $R_2^A$ ont les significations données pour la formule II et W′ représente un halogène ou un acyloxy, où acyle est le radical acyle d'un acide carboxylique organique, sous la forme cis,trans, cis ou trans, on ozonise un composé de formule

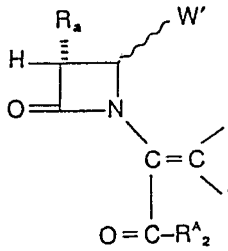

(XIV),

où Ra, W' et $R_2^A$ ont les significations données ci-dessus, eton clive de façon réductrice l'ozonide formé pour donner le groupe oxo, et si on le désire on transforme dans un composé obtenu un groupe Ra en un autre groupe Ra, et/ou si on le désire on isole à partir d'un composé cis,trans obtenu le composé cis ou le composé trans, selon l'étape 3.5 pour préparer les composés de formule

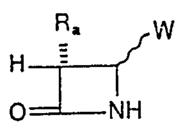

(IIIa),

où Ra a la signification donnée pour la formule II et W représente un groupe sortant nucléofuge, sous la forme cis,trans, cis ou trans, on solvolyse un composé de formule

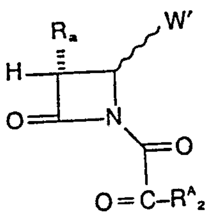

(XV),

où Ra et $R_2^A$ ont les significations données ci-dessus, et W' représente un acyloxy ou un halogène, et si on le désire on transforme dans un composé obtenu un groupe Ra en un autre groupe Ra, et/ou si on le désire on transforme un groupe W' en un autre groupe W' ou en W, et/ou si on le désire on isomérise un composé cis obtenu en le composé trans correspondant, et/ou à partir d'un composé cis,trans obtenu on isole le composé cis et/ou le composé trans, puis on conduit les étapes 1.1 à 1.4

2. Procédé selon la revendication 1, caractérisé en ce qu'on prépare des composés de formule II où Ra représente un alcoyle inférieur, hydroxyalcoyle inférieur, alcoxy inférieur-alcoyle inférieur, alcanoyloxy inférieur-alcoyle inférieur, hydroxysulfonyloxyalcoyle inférieur présent sous forme de sel, hydroxy, alcoxy inférieur, alcanoyloxy inférieur, alcanoyloxy inférieur substitué par phényloxy, halogène, amino ou cyano, phénylalcanoyloxy inférieur, ou phénylalcanoyloxy inférieur substitué par hydroxy ou amino, R1 représente hydrogène, alcoyle inférieur, hydroxyalcoyle inférieur, alcoxy inférieur-alcoyle inférieur, alcanoyloxy inférieur-alcoyle inférieur, alcoyle inférieur-thioalcoyle inférieur, hétérocyclylthioalcoyle inférieur, où hétérocyclyle représente un radical aromatique diaza, triaza-tétraaza, thiaza, thiadiaza-, thiatriaza-, oxaza- ou oxadiazacyclique à 5 chaînons éventuellement substitué par alcoyle inférieur, carboxyalcoyle inférieur, dialcoyle inférieur-aminoalcoyle inférieur ou sulfoalcoyle inférieur présent sous forme de sel, aminoalcoyle inférieur, acylaminoalcoyle inférieur, où acyle est un alcanoyle inférieur ou un groupe oxycarbonyle substitué habituel comme groupe protecteur d'amino, p.ex. un groupe tert-butyle, 2,2,2-trichloréthyle, diphénylméthyle ou p-nitrobenzyloxycarbonyle, carboxyalcoyle inférieur, alcoxy inférieur-carbonylalcoyle inférieur, phénylalcoyle inférieur, phényle, hydroxyphényle, aminophényle, furyle, thiényle, pyridyle, alcoyle inférieur-thio, alcoyle inférieur-thio ou alcényle inférieur-thio substitué par amino, mono- ou dialcoyle inférieur-amino ou alcanoyle inférieur-amino, ou tétrazolylthio ou thiadiazolylthio éventuellement substitué par alcoyle inférieur, sulfoalcoyle inférieur, carboxyalcoyle inférieur ou dialcoyle inférieur-aminoalcoyle inférieur, où les groupes fonctionnels dans de tels radicaux Ra et R1 se présentent de préférence sous forme protégée, et Z', $X^{\oplus}$ et $R_2^A$ ont les significations données

dans la revendication 1, sous forme racémique ou optiquement active.

3. Composés de formule

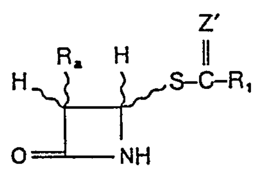

(IV),

où Ra, R1 et Z' ont les significations données dans la revendication 1, sous forme racémique et optiquement active, avec la première précision que Z' est différent d'un groupe méthylidène substitué par un groupe carboxyle protégé lorsque Ra est un alcoyle, alcoxyle, aryle, aralcoyle, hétérocyclyle ou hétérocyclylalcoyle et R1 représente un hydrogène ou un alcoyle, aryle, aralcoyle, hétérocyclyle, hétérocyclylalcoyle, alcoylthio, arylthio, aralcoylthio ou hétérocyclylthio, où ces radicaux Ra et R1 sont non substitués ou substitués par amino, amino protégé, mono- ou dialcoylamino, hydroxyle, hydroxyle protégé, alcoxyle, mercapto, alcoylthio, nitro, chlore, brome, fluor, cyano, carboxyle ou carboxyle protégé,

et avec la seconde précision que Z' est différent d'un oxygène lorsque ou bien Ra a les significations donnée dans la 1ère précision et R1 est un aryle différent d'un phényle, un aralcoyle différent de phénylalcoyle inférieur, hétérocyclyle différent de pyridyle, thiényle et furyle, hétérocyclylalcoyle, arylthio, aralcoylthio ou hétérocyclylthio, radicaux qui sont non substitués ou substitués par amino, amino protégé, mono- ou dialcoylamino, hydroxy, hydroxy protégé, alcoxyle, mercapto, alcoylthio, nitro, chlore, brome, fluor, cyano, carboxyle ou carboxyle protégé; alcoyle en C1 à C6 substitué par mono- ou dialcoylamino, mercapto, nitro,

chlore, fluor, brome ou cyano; phényle substitué par mono- ou dialcoylamino, mercapto, alcoylthio, nitro, cyano, carboxyle, ou carboxyle protégé; phénylalcoyle inférieur, pyridyle, thiényle ou furyle substitué par amino, amino protégé, mono- ou dialcoylamino, hydroxy, hydroxy protégé, alcoxyle, mercapto, alcoylthio, nitro, chlore, brome, fluor, cyano, carboxyle ou carboxyle protégé; ou alcoylthio en C1 à C6, qui est substitué par hydroxy, hydroxy protégé, mercapto, alcoylthio, nitro, chlore, brome, fluor, cyano, carboxyle ou carboxyle protégé; ou bien R1 a les significations données pour la 1ère précision et Ra est un alcoxyle substitué ou un aryle, aralcoyle, hétérocyclyle ou hétérocyclylalcoyle éventuellement substitué, où les substituants de tels radicaux sont amino, amino protégé, mono- ou dialcoylamino, hydroxyle, hydroxyle protégé, alcoxyle, mercapto, alcoylthio, nitro, chlore, brome, fluor, cyano, carboxyle ou carboxyle protégé; ou alcoyle en C1 à C6, lequel radical est substitué par amino, amino protégé, mono- ou dialcoylamino, mercapto, alcoylthio, nitro, chlore, brome, fluor, cyano, carboxyle ou carboxyle protégé.

4. Composés de formule IV selon la revendication 3, où Ra, R1 et Z' ont les significations données dans la revendication 2.

5. 4-Acétylthio-3-méthyl-2-oxo-azétidine (composé cis racémique) selon la revendication 3.

6. 4-Acétylthio-3-méthyl-2-oxo-azétidine (composé trans racémique) selon la revendication 3.

7. 4-Acétylthio-3-isopropyl-2-oxo-azétidine (composé trans racémique) selon la revendication 3.

8. 4-Acétylthio-3-benzyl-2-oxo-azétidine (composé trans racémique) selon la revendication 3.

9. 4-éthylthio-thiocarbonylthio-3-isopropyl-2-oxoazétidine (composé trans racémique) selon la revendication 3.

10. (3S,4R)-4-Acétylthio-3-méthoxy-2-oxo-azétidine selon la revendication 3.

11. (3S,4R)-4-Acétylthio-3-phénoxyacétoxy-2-oxo-azétidine selon la revendication 3.

12. 3-éthyl-4-(4-p-nitrobenzyloxycarbonylamino-butyryl-thio)-2-oxo-azétidine (composé cis-trans racémique) selon la revendication 3.

13. (3S,4R)-4-(2-p-Nitrobenzyloxycarbonylaminoéthyl-thio-thiocarbonylthio)-3-méthoxy-2-oxoazétidine selon la revendication 3.

14. (3S,4R)-4-(4-p-Nitrobenzyloxycarbonylamino-butyrylthio)-3-méthoxy-2-oxo-azétidine selon la revendication 3.

15. 3-éthyl-4-(2-acétylaminoéthylthio-thiocarbonylthio)-2-oxoazétidine (composé cis,trans racémique) selon la revendication 3.

16. Composés de formule

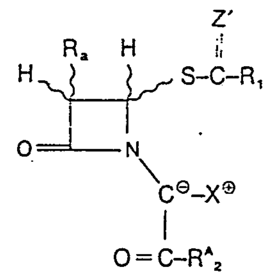

(II),

où Ra, R1, Z', $X^{\oplus}$ et $R_2^A$ ont les significations données dans la revendication 1, sous forme racémique ou optiquement active.

17. Composés de formule II selon la revendication 16, où Ra, R1, Z', $X^{\oplus}$ et $R_2^A$ ont les significations données dans la revendication 2, sous forme racémique ou optiquement active.

18. Composés de formule II selon les revendications 16 et 17, où Ra représente méthyle, éthyle, isopropyle, benzyle, méthoxy ou phénoxyacétoxy, R1 représente méthyle, 3-(p-nitrobenzyloxycarbonylamino)propyle, éthylthio, 2-acétylamino-éthylthio ou 2-(p-nitrobenzyloxycarbonylamino)éthylthio, Z' représente oxygène ou soufre, $R_2^A$ p-nitrobenzyloxy et $X^{\oplus}$ le groupe $P^{\ominus}$ (phényl)$_3$.

19. Composés de formule

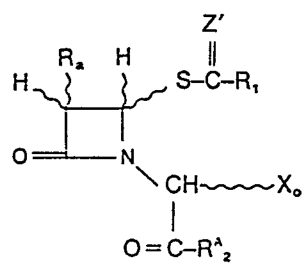

(V) ou (VI),

où Ra, R1, Z' et $R_2^A$ ont les significations données dans la revendication 1 et X'o représente un hydroxy ou un groupe hydroxy estérifié réactif, sous forme racémique ou optiquement active.

20. Composés de formule V ou VI selon la revendication 19, où Ra, R1, Z' et $R_2^A$ ont les significations données dans la revendication 2 et X'o a les significations données dans la revendication 19, sous forme racémique ou optiquement active.

21. Composés de formule V ou VI selon les revendications 19 et 20, où Ra représente méthyle, éthyle, isopropyle, benzyle, méthoxy ou phénoxyacétoxy, R1 représente méthyle, 3-(p-nitrobenzyloxycarbonylamino)propyle, éthylthio, 2-acétylaminoéthylthio ou 2-(p-nitrobenzyloxycarbonylamino)éthylthio, Z' représente oxygène ou soufre, $R_2^A$ p-nitrobenzyloxy et X'o hydroxy ou chlore.

22. Composés de formule

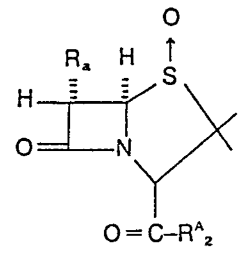

(VIII),

où Ra et $R_2^A$ ont les significations données dans la revendication 1.

23. Composés de formule VIII selon la revendication 22, où Ra et $R_2^A$ ont les significations données dans la revendication 2.

24. Composés de formule VIII selon les revendications 22 et 23, où Ra représente un méthoxy ou un phénoxyacétoxy et $R_2^A$ un méthoxy ou un 2,2,2-trichloréthoxy.

25. Composés de formule

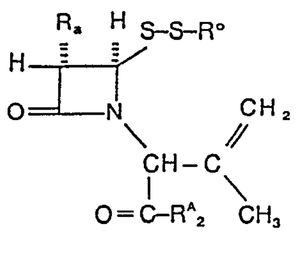

(IX),

où Ra et $R_2^A$ ont les significations données dans la revendication 1 et Ro est un radical hétérocyclique aromatique éventuellement substitué ayant jusqu'à 15 atomes de carbone, au moyen un atome d'azote dans son noyau et éventuellement un autre hétéroatome dans son noyau, radical qui est lié au groupe thio –S– par l'un de ses atomes de carbone du noyau qui est relié à un atome d'azote du noyau par une double liaison.

26. Composés de formule IX selon la revendication 25, où Ra et $R_2^A$ ont les significations données dans la revendication 2 et Ro a les significations données dans la revendication 25.

27. Composés de formule IX selon les revendications 25 et 26, où Ra représente méthoxy ou phénoxyacétoxy, $R_2^A$ méthoxy ou 2,2,2-trichloréthoxy et Ro 2-benzthiazolyle.

28. Composés de formule

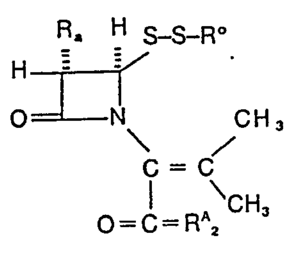

(X)

où Ra et $R_2^A$ ont les significations données dans la revendication 1 et Ro est un radical hétérocyclique aromatique éventuellement substitué ayant jusqu'à 15 atomes de carbone, au moins un atome d'azote dans son noyau et éventuellement un autre hétéroatome dans son noyau, radical qui est lié au groupe thio –S– par un de ses atomes de carbone du noyau qui est lié à un atome d'azote du noyau par une double liaison.

29. Composés de formule X selon la revendication 28, où Ra et $R_2^A$ ont les significations données dans la revendication 2 et Ro a les significations données dans la revendication 28.

30. Composés de formule X selon les revendications 28 et 29, où Ra est un méthoxy ou phénoxyacétoxy, $R_2^A$ méthoxy ou 2,2,2-trichloréthoxy et Ro 2-benzthiazolyle.

31. Composés de formule

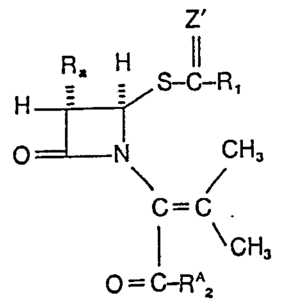

(XI),

où Ra, R1, Z' et $R_2^A$ ont les significations données dans la revendication 1.

32. Composés de formule XI selon la revendication 31, où Ra, R1, Z' et $R_2^A$ ont les significations données dans la revendication 2.

33. Composés de formule XI selon les revendications 31 et 32, où Ra représente méthyle, méthoxy ou phénoxyacétoxy, R1 méthyle, Z' oxygène et $R_2^A$ méthoxy ou 2,2,2-trichloréthoxy.

34. Composés de formule

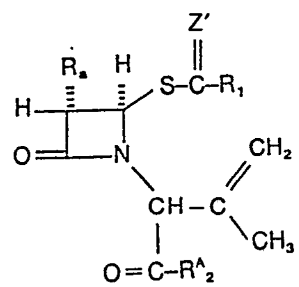

(Xa),

où Ra, R1, Z' et $R_2^A$ ont les significations données dans la revendication 1.

35. Composés de formule Xa selon la revendication 34, où Ra, R1, Z' et $R_2^A$ ont les significations données dans la revendication 2.

36. Composés de formule

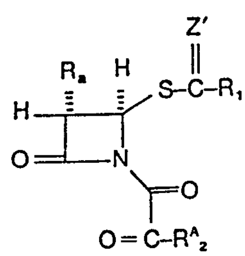

(XII),

où Ra, R1, Z' et $R_2^A$ ont les significations données dans la revendication 1.

37. Composés de formule XII selon la revendication 36, où Ra, R1, Z' et $R_2^A$ ont les significations données dans la revendication 2.

38. Composés de formule XII selon les revendications 36 et 37, où Ra représente méthyle, méthoxy ou phénoxyacétoxy, R1 méthyle, Z' oxygène et $R_2^A$ méthoxy ou 2,2,2-trichloréthoxy.

39. Composés de formule

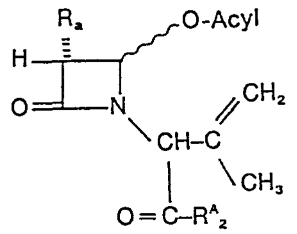

(XIII),

où Ra et $R_2^A$ ont les significations données dans la revendication 1 et acyle le radical acyle d'un acide carboxylique organique en position cis,-trans, cis ou trans.

40. Composés de formule XIII, selon la revendication 39, où Ra représente un radical méthyle ou méthoxy; $R_2^A$ un groupe 2,2,2-trichloroéthoxy et Acyl représente un groupe acétyle, dans leur forme cis,trans-, leur forme cis- ou leur forme trans-.

41. Composés de formule

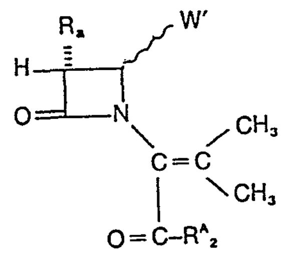

(XIV),

où Ra et $R_2^A$ ont les significations données dans la revendication 1, W′ est halogène ou acyloxy, où acyle est le radical acyle d'un acide carboxylique organique, sous forme cis,trans, cis ou trans, à l'exception des composés de formule XIV où Ra est alcoyle, alcoxyle, aryle, aralcoyle, hétérocyclyle ou hétérocyclylalcoyle, où ces radicaux sont non substitués ou substitués par amino, amino protégé, mono- ou dialcoylamino, hydroxy, hydroxy protégé, alcoxy, mercapto, alcoylthio, nitro, chlore, brome, fluor, cyano, carboxyle ou carboxyle protégé, W′ représente acétyloxy et $R_2^A$ représente avec le groupe carbonyle –C(=O)– un groupe carboxyle protégé par un groupe protecteur de carboxyle.

42. Composés de formule XIV selon la revendication 41, où Ra représente méthoxy, $R_2^A$ 2,2,2-trichloréthoxy et W′ chlore, sous forme cis,trans, cis ou trans.

43. Composés de formule

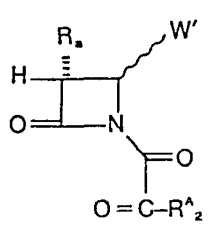

(XV),

où Ra et $R_2^A$ ont les significations données dans la revendication 1, W′ représente halogène ou acyloxy, où acyle est le radical acyle d'un acide carboxylique organique, sous forme cis,trans, cis ou trans.

44. Composés de formule XI selon la revendication 43, où Ra représente méthoxy, $R_2^A$ 2,2,2-trichloréthoxy et W′ acétoxy ou chlore, sous forme cis,trans, cis ou trans.

45. Composés de formule

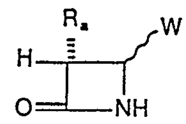

(IIIa),

où Ra a les significations données dans la revendication 1 et W représente un groupe sortant nucléofuge, sous forme cis,trans, cis ou trans, à l'exception des composés de formule IIIa, où Ra est alcoyle, alcoxyle, aryle, aralcoyle, hétérocyclyle ou hétérocyclylalcoyle, où ces radicaux sont non substitués ou substitués par amino, amino protégé, mono- ou dialcoylamino, hydroxy, hydroxyprotégé, alcoxy, mercapto, alcoylthio, nitro, chlore, brome, fluor, cyano, carboxyle ou carboxyle protégé et W est acécyloxy.

46. Composés de formule IIIa selon la revendication 45, où Ra est méthoxy et W chlore, sous forme cis,trans, cis ou trans.

47. Les diverses étapes du procédé selon la revendication 1, avec la précision qu'on prépare des composés de formule IV selon l'étape 1.1 où Ra, R1 et Z′ ont les significations données dans la revendication 3.

48. Les diverses étapes du procédé selon la revendication 1 selon le schéma réactionnel 3.

49. Les diverses étapes du procédé de la revendication 1 selon les schémas réactionnels 1 et 2, avec la précision qu'on prépare selon l'étape 1.1 des composés de formule IV où Ra, R1 et Z′ ont les significations données dans la revendication 3.

**Revendications pour l'Etat contractant: IT**

1. Procédé de préparation de composés de formule

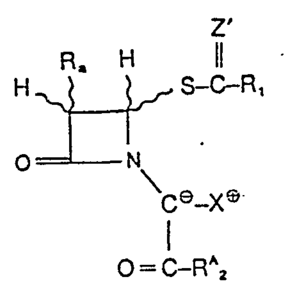

(II),

où Ra représente un radical hydrocarboné saturé ou non saturé, éventuellement substitué, aliphatique, cycloaliphatique, cycloaliphatique-aliphatique, aromatique ou araliphatique ayant jusqu'à 18, de préférence jusqu'à 10, atomes de carbone,

un radical hétérocyclyl- ou hétérocyclyl-alcoyle inférieur éventuellement substitué ayant jusqu'à 10 atomes de carbone et jusqu'à 4 hétéroatomes dans son noyau, du groupe azote, oxygène et/ou soufre,

dans lesquels radicaux Ra les substituants éventuellement présents sont hydroxy, alcoxy inférieur, alcanoyloxy inférieur, hydroxysulfonyloxy présent sous forme de sel, halogène, mercapto, alcoyle inférieur-mercapto, carboxyle, alcoxy inférieur-carbonyle, carbamoyle, cyano, nitro, sulfo présent sous forme de sel, ou amino éventuellement mono- ou disubstitué par alcoyle inférieur ou acyle en C1 à C20 ou disubstitué par un alcoylène inférieur,

un carboxyle éventuellement protégé,

un hydroxy,

un mercapto,

un hydroxy ou mercapto éthérifié par un radical hydrocarboné éventuellement substitué, aliphatique, cycloaliphatique, cycloaliphatique-aliphatique, aromatique ou araliphatique ayant jusqu'à 18, en particulier jusqu'à 10, atomes de carbone, où les substituants éventuellement présents sont hydroxy, alcoxy inférieur, alcanoyloxy inférieur, halogène, mercapto, alcoyle inférieur-mercapto, carboxyle, alcoxy inférieur-carbonyle, carbamoyle, cyano, nitro ou amino éventuellement mono- ou disubstitué par alcoyle inférieur ou acyle en C1 à C20 ou disubstitué par alcoylène inférieur,

hydroxy ou mercapto estérifié par un radical acyle en C1 à C20 d'un acide carboxylique aliphatique, cycloaliphatique, cycloaliphatique-aliphatique, aromatique ou araliphatique éventuellement substitué ayant jusqu'à 18 atomes de carbone, où

les substituants éventuellement présents sont hydroxy, alcoxy inférieur, phényloxy, alcanoyloxy inférieur, halogène, mercapto, alcoyle inférieur-mercapto, carboxyle, alcoxy inférieur-carbonyle, carbamoyle, cyano, nitro, ou amino éventuellement mono- ou disubstitué par alcoyle inférieur ou acyle en C1 à C20 ou disubstitué par alcoylène inférieur, ou halogène,

R1 représente un hydrogène, un radical hydrocarboné saturé ou non saturé, aliphatique, cycloaliphatique, cycloaliphatique-aliphatique, aromatique ou araliphatique éventuellement substitué ayant jusqu'à 18, de préférence jusqu'à 10, atomes de carbone, ou

un radical hétérocyclyle ou hétérocyclyl-alcoyle inférieur éventuellement substitué ayant jusqu'à 10 atomes de carbone et jusqu'à 4 hétéroatomes dans son noyau, du groupe azote, oxygène et/ou soufre,

dans lesquels radicaux R1 les substituants éventuellement présents sont hydroxy, alcoxy inférieur, alcanoyloxy inférieur, halogène, mercapto, alcoyle inférieur-mercapto, hétérocyclylmercapto éventuellement substitué par alcoyle inférieur, hydroxyalcoyle inférieur, carboxyalcoyle inférieur, amino-alcoyle inférieur, dialcoyle inférieur-aminoalcoyle inférieur, sulfoalcoyle inférieur présent sous forme de sel, cycloalcoyle, aryle, arylalcoyle inférieur, halogène, amino, mono- ou di-alcoyle inférieur-amino, nitro, hydroxy, alcoxy inférieur, carboxyle, alcoxy inférieur-carbonyle, carbamoyle, N-mono- ou N,N-dialcoyle inférieur-carbamoyle, cyano, oxo ou oxydo,

carboxyle, alcoxy inférieur-carbonyle, carbamoyle, cyano, nitro, amino, alcoyle inférieur-amino, dialcoyle inférieur-amino, acylamino en C1 à C20, di-acylamino en C1 à C20 ou alcoylène inférieur-amino, ou bien où R1 est un groupe mercapto éthérifié par un radical hydrocarboné aliphatique, cycloaliphatique, cycloaliphatique-aliphatique, aromatique ou araliphatique éventuellement substitué ayant jusqu'à 18, en particulier jusqu'à 10, atomes de carbone,

où les substituants éventuellement présents sont hydroxy, alcoxy inférieur, alcanoyloxy inférieur, halogène, mercapto, alcoyle inférieur-mercapto, carboxyle, alcoxy inférieur-carbonyle, carbamoyle, cyano, nitro, amino éventuellement mono- ou disubstitué par un alcoyle inférieur ou un acyle en C1 à C20 ou disubstitué par un alcoylène inférieur, ou bien où R1 est un phénylthio ou phénylalcoyle inférieur-thio substitué par un alcoyle inférieur, ou bien où R1 est un groupe carboxyle éventuellement protégé, ou bien où R1 est un groupe mercapto éthérifié par un radical hétérocyclique éventuellement substitué, où le radical hétérocyclyle est lié par l'intermédiaire d'un atome de carbone du noyau au groupe mercapto et contient de 1 à 4 atomes de l'azote dans son noyau et éventuellement dans son noyau un autre hétéroatome du groupe oxygène ou soufre et où les substituants éventuellement présents sont alcoyle inférieur, alcoyle inférieur substitué par hydroxy, alcanoyloxy inférieur, halogène, carboxy, alcoxy inférieur-carbonyle, sulfo, sulfo amidé, amino, mono- ou di-alcoyle inférieur-amino, acylamino en C1 à C20 ou alcanoyle inférieur-amino substitué par carboxy ou halogène, cycloalcoyle, phényle éventuellement substitué par halogène ou nitro, benzyle, furyle, thiényle, oxazolyle, halogène, amino éventuellement mono- ou disubstitué par alcoyle inférieur, acylamino en C1 à C20, nitro, hydroxy, alcoxy inférieur, carboxy, alcoxy inférieur-carbonyle, carbamoyle éventuellement substitué par un N-mono- ou N,N-dialcoyle inférieur, cyano, oxo ou oxydo, où les groupes fonctionnels dans ces radicaux Ra et R1 se présentent de préférence sous forme protégée et les groupes décrits par «inférieur» contiennent jusqu'à 7, en particulier jusqu'à 4, atomes de carbone, $Z^{o}$ représente un oxygène, un soufre ou un groupe méthylidène portant éventuellement un ou deux substituants, $X^{\oplus}$ représente soit un groupe phosphonio trisubstitué, soit un groupe phosphono di-estérifié avec un cation et $R_2^A$ représente avec le groupe carbonyle $-C(=O)-$ un groupe carboxyle protégé, sous forme racémique ou optiquement active, caractérisé en ce qu'on conduit les étapes 1.1 à 1.4 selon

Schéma réactionnel 1

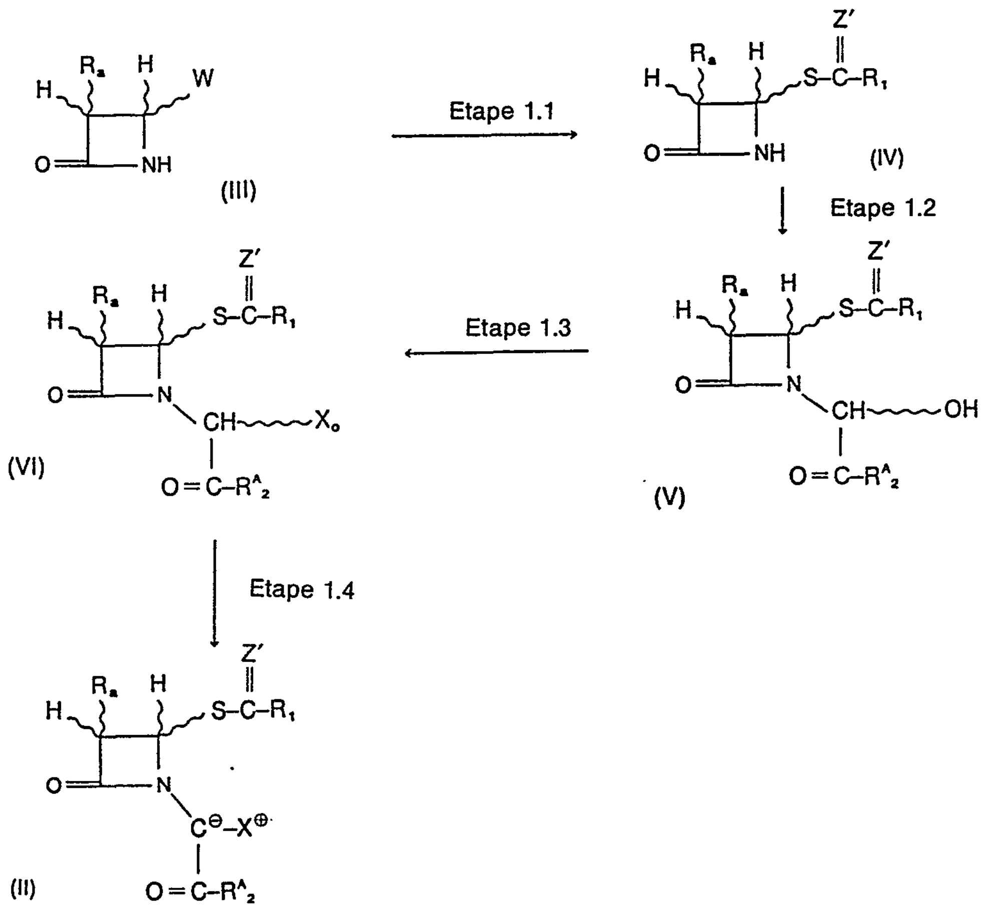


ou les étapes 2.1 à 2.6 selon

Schéma réactionnel 2

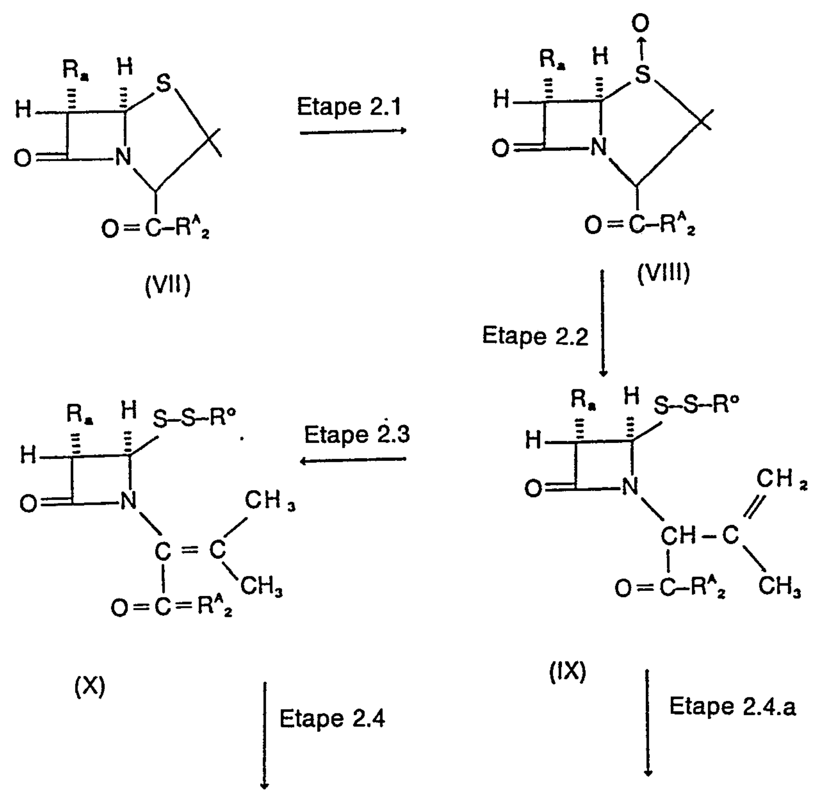

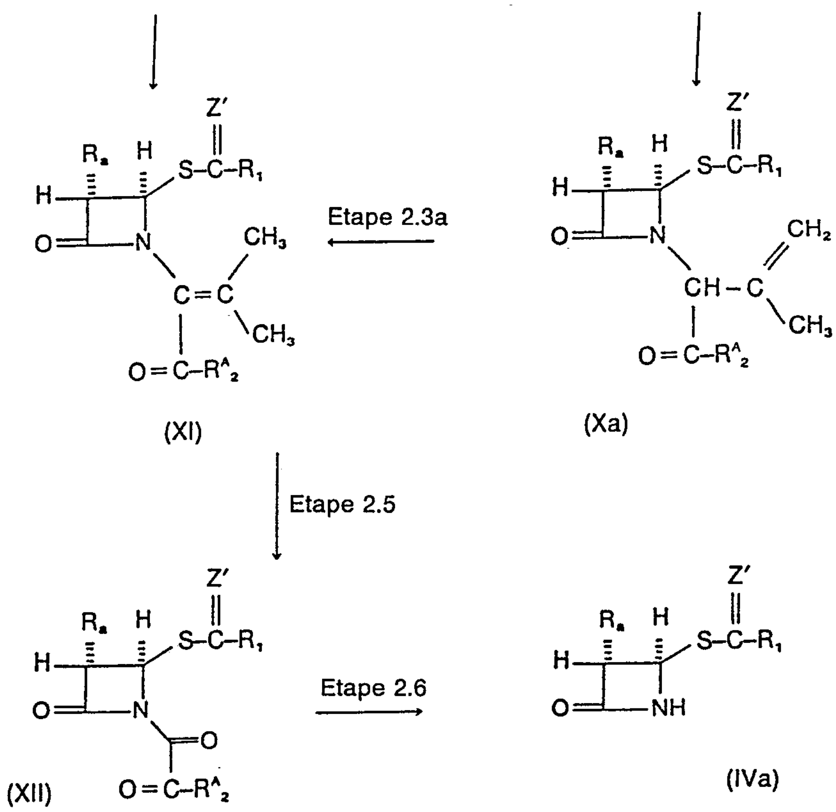


(XI) (Xa)

(XII) (IVa)

suivi par les étapes 1.2 à 1.4, ou les étapes 3.1 à 3.5 selon

Schéma réactionnel 3

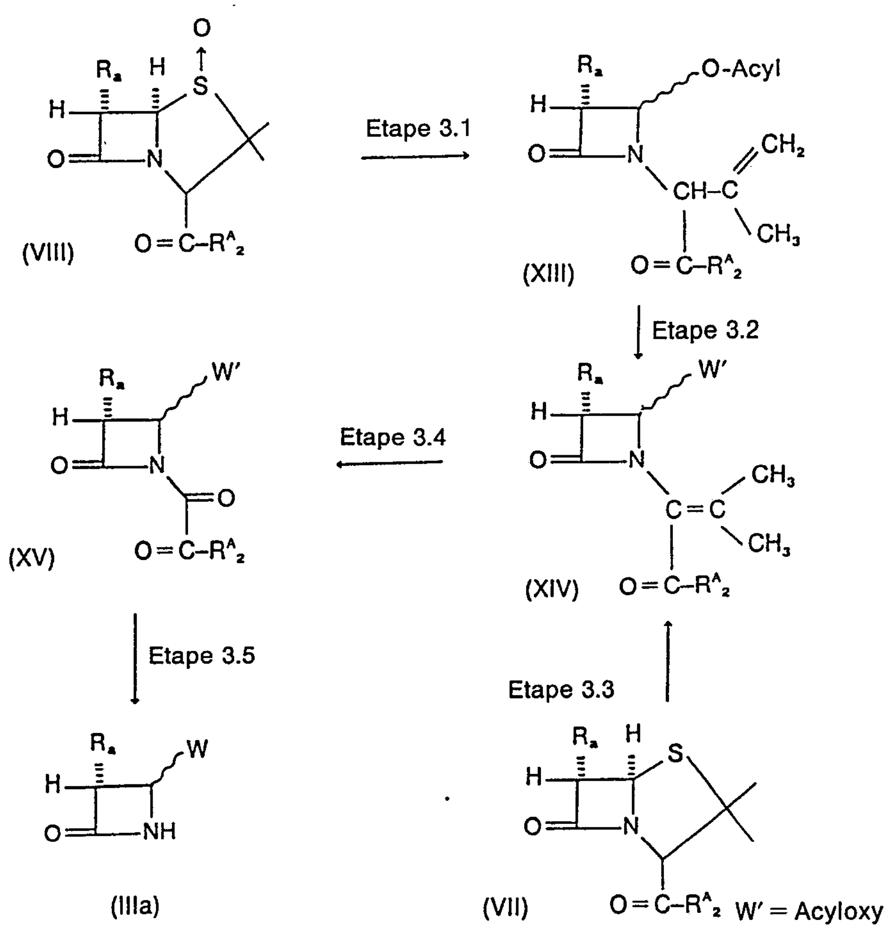


(VIII) (XIII)

(XV) (XIV)

(IIIa) (VII)

W' = Acyloxy ou Halogène

suivi par les étapes 1.1 à 1.4, où l'on fait réagir selon l'étape 1.1 pour préparer des composés de formule

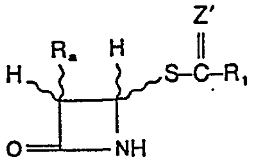

(IV) ou (IVa),

où Ra, R1 et Z' ont les significations mentionnées sous la formule II, sous forme racémique ou optiquement active, une 4-W-azétidinone de formule

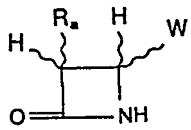

(III)

ou

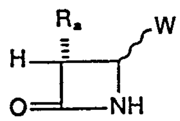

(IIIa),

où W représente un groupe nucléofuge, avec un composé mercapto R1–C(=Z')–SH ou un de ses sels, et si on le désire on sépare un mélange d'isomères obtenu en les isomères isolés, ou, selon l'étape 2.6 pour préparer des composés (3S,4R) optiquement actifs de formule

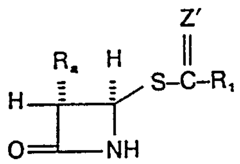

(IVa),

on solvolyse un composé de formule

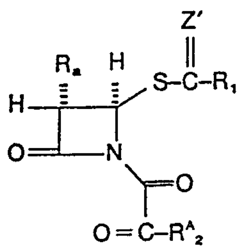

(XII),

où Ra, R1, Z' et $R_2^A$ ont les significations données pour la formule II, et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, selon l'étape 1.2 pour préparer des composés de formule

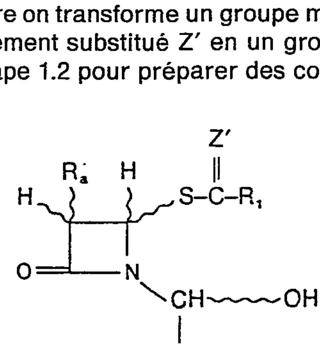

(V),

où Ra, R1, Z' et $R_2^A$ ont les significations données pour la formule II, sous forme racémique ou optiquement active, on fait réagir un composé de formule

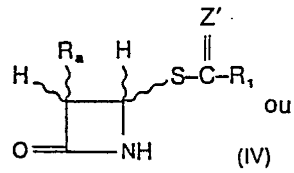

(IV) ou

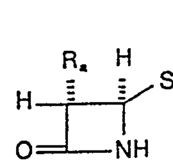

(IVa),

où Ra, R1 et Z' ont les significations données ci-dessus, avec un composé d'acide glyoxylique de formule OHC–C(=O)–$R_2^A$ ou un des dérivés appropriés, et si on le désire on sépare un mélange d'isomères obtenu en les isomères isolés, et/ou si on le désire on transforme un groupe Ra ou R1 en un autre groupe Ra ou selon les cas R1, et/ou si on le désire on transforme un groupe méthylidène éventuellement substitué Z' en un groupe oxo Z, selon l'étape 1.3 pour préparer des composés de formule

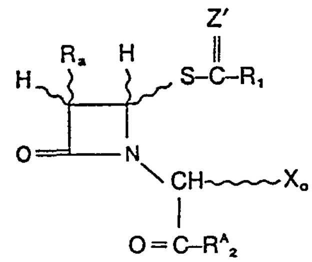

(VI),

où Ra, R1, Z' et $R_2^A$ ont les significations données sous la formule II et Xo représente un groupe hydroxy estérifié réactif, sous forme racémique ou optiquement active, dans un composé de formule

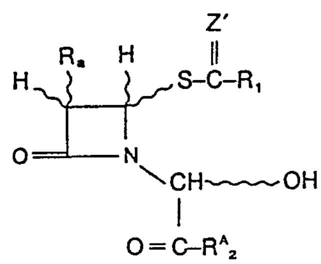

(V),

où Ra, R1, Z' et $R_2^A$ ont les significations données ci-dessus, on transforme le groupe hydroxy secondaire en un groupe hydroxy estérifié réactif, et si on le désire on sépare un mélange d'isomères obtenu en les isomères isolés, et/ou si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, et selon l'étape 1.4, pour préparer des composés de formule II, on traite un composé de formule

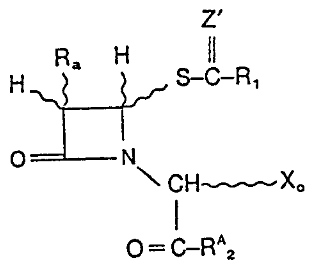

(VI),

où Xo représente un groupe hydroxy estérifié réactif, et

Ra, R1, Z′ et $R_2^A$ ont les significations données ci-dessus, avec un composé de phosphine approprié, ou on traite un composé de formule

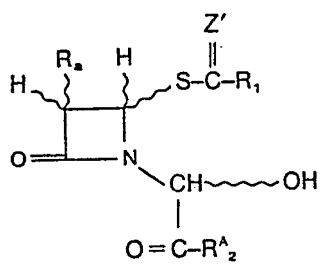

(V),

où Ra, R1, Z′ et $R_2^A$ ont les significations données ci-dessus, avec du tétra-chlorure de carbone et une phosphine, et si on le désire on transforme dans un composé obtenu un groupe Ra ou R1 en un autre groupe Ra ou selon les cas R1, et/ou si on le désire on transforme un groupe méthylidène éventuellement substitué Z′ en un groupe oxo Z, ou selon l'étape 2.1, pour préparer des composés de formule

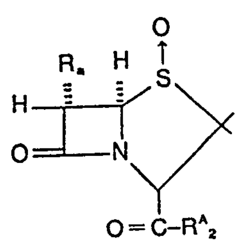

(VIII),

où Ra et $R_2^A$ ont les significations données pour la formule II, on oxyde en position 1 un composé d'acide pénicillanique de formule

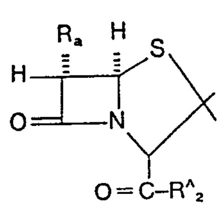

(VII),

et si on le désire on transforme dans un composé obtenu un groupe Ra en un autre groupe Ra, selon l'étape 2.2 pour préparer des composés de formule

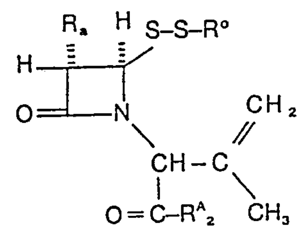

(IX),

où Ra et $R_2^A$ ont les significations données pour la formule II et Ro est un radical hétérocyclique aromatique éventuellement substitué ayant jusqu'à 15 atomes de carbone, au moins un atome d'azote dans son noyau et le cas échéant un autre hétéroatome dans le noyau, lequel radical est lié au groupe thio –S– par un de ses atomes de carbone du noyau, qui est relié à un atome d'azote du noyau par une double liaison, on traite un composé de formule

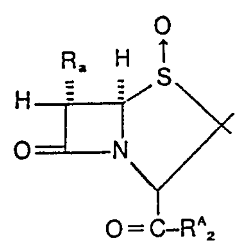

(VIII),

avec un composé mercapto Ro–SH, et si on le désire on transforme dans un composé obtenu un groupe Ra en un autre groupe Ra, selon l'étape 2.3 pour préparer des composés de formule

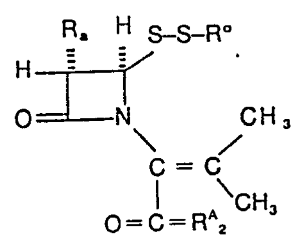

(X),

où Ra et $R_2^A$ ont les significations données pour la formule II et Ro est un radical hétérocyclique aromatique éventuellement substitué ayant jusqu'à 15 atomes de carbone, au moins un atome d'azote dans son noyau et éventuellement un autre hétéroatome dans son noyau, lequel radical est lié au groupe thio –S– par un de ses atomes de carbone du noyau qui est lié à un atome d'azote du noyau par une double liaison, on isomérise un composé d'acide 3-méthylènebutyrique de formule

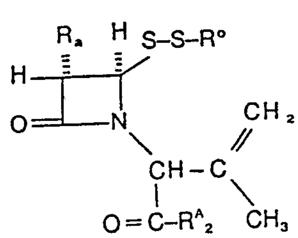

(IX),

par traitement avec un agent basique approprié, et si on le désire on transforme dans un composé obtenu un groupe Ra en un autre groupe Ra, selon l'étape 2.4 pour préparer des composés de formule

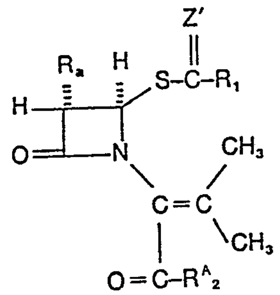 (XI),

où Ra, R1, Z' et $R_2^A$ ont les significations données pour la formule II, on traite avec un agent réducteur approprié un composé de formule

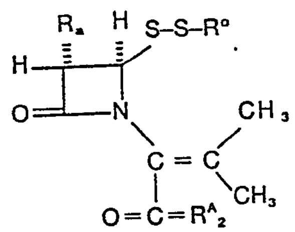 (X),

où Ro est un radical hétérocyclique aromatique éventuellement substitué ayant jusqu'à 15 atomes de carbone, au moins un atome d'azote dans son noyau et éventuellement un autre hétéroatome dans son noyau, lequel radical est lié au groupe thio –S– avec un de ses atomes de carbone du noyau, qui est relié à un atome d'azote du noyau par une double liaison, et Ra et $R_2^A$ ont les significations données ci-dessus, et simultanément ou ultérieurement avec un dérivé d'acylation d'un acide carboxylique de formule R1–C(=Z)–OH, ou, lorsque Z' représente un groupe méthylidène éventuellement substitué par Y, on le fait réagir avec un alcyne de formule R1–C≡C–Y, ou selon l'étape 2.3a on isomérise un composé de formule

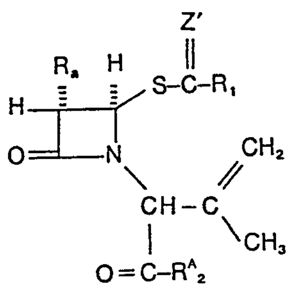 (Xa),

où Ra, R1, Z' et $R_2^A$ ont les significations données ci-dessus, par traitement avec un agent basique approprié, et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, selon l'étape 2.4a pour préparer des composés de formule

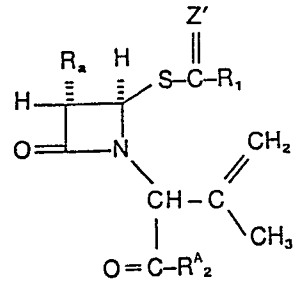 (Xa),

où Ra, R1, Z' et $R_2^A$ ont les significations données pour la formule II, on traite un composé de formule

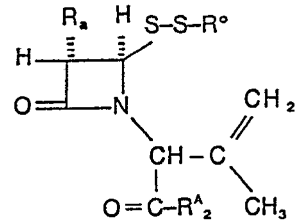 (IX),

où Ro, Ra et $R_2^A$ ont les significations données ci-dessus, avec un agent réducteur approprié et simultanément ou ultérieurement avec un dérivé d'acylation d'un acide carboxylique de formule R1–C(=Z)–OH, ou, lorsque Z' représente un groupe méthylidène éventuellement substitué par Y, on fait réagir avec un alcyne de formule R1–C≡C–Y et si on le désire on transforme dans un composé obtenu un groupe Ra ou R1 en un autre groupe Ra ou selon les cas R1, et/ou si on le désire on transforme un groupe méthylidène éventuellement substitué Z' en un groupe oxo Z, selon l'étape 2.5 pour préparer des composés de formule

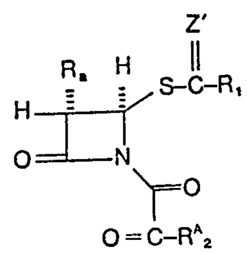 (XII),

où Ra, R1, Z' et $R_2^A$ ont les significations données pour la formule II, on ozonise un composé de formule

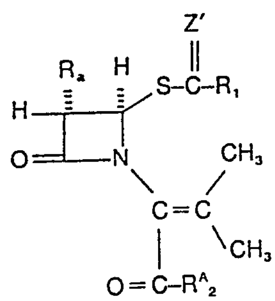 (XI),

où Ra, R1, Z′ et $R_2^A$ ont les significations données ci-dessus et on clive de façon réductrice l'ozonide formé pour donner le groupe oxo, et si on le désire on transforme dans un composé obtenu un groupe Ra ou R1 en un autre groupe Ra ou selon les cas R1, et/ou si on le désire on transforme un groupe méthylidène éventuellement substitué Z′ en un groupe oxo Z, puis on conduit les étapes 2.6 et 1.2 à 1.4, ou, selon l'étape 3.1 pour préparer des composés de formule

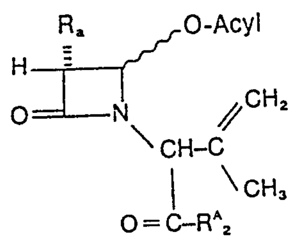

(XIII),

où Ra et $R_2^A$ ont les significations données pour la formule II et acyle est le radical acyle d'un acide carboxylique organique sous forme cis,trans, cis ou trans, on traite un 1-oxyde d'un composé d'acide pénicillanique de formule

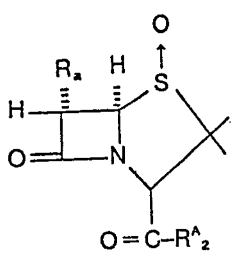

(VIII),

où Ra et $R_2^A$ ont les significations données ci-dessus, en présence d'un trialcoyle inférieur-phosphite avec un acide carboxylique organique acylacyl-OH, et si on le désire on transforme dans un composé obtenu un groupe Ra en un autre groupe Ra, et/ou à partir d'un composé cis,trans obtenu on isole le composé cis et/ou le composé trans, selon l'étape 3.2 pour préparer des composés de formule

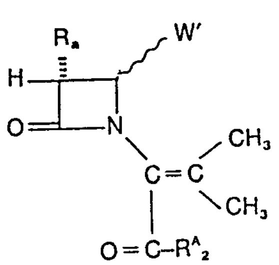

(XIV),

où Ra et $R_2^A$ ont les significations données pour la formule II et W′ représente un acyloxy, où acyle et le radical acyle d'un acide carboxylique organique, et $R_2^A$ avec le groupe carbonyle $-C(=O)-$ représente un groupe carboxyle protégé, sous la forme cis,trans, cis ou trans, on isomérise un composé de formule

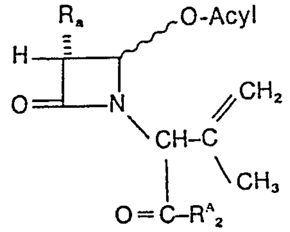

(XIII),

où Ra, acyle et $R_2^A$ ont les significations données ci-dessus, par traitement avec un agent basique approprié, et si on le désire on transforme dans un composé obtenu un groupe Ra en un autre groupe Ra, et/ou si on le désire on isole à partir d'un composé cis,trans obtenu le composé cis et/ou le composé trans, ou selon l'étape 3.3 pour préparer le composé de formule

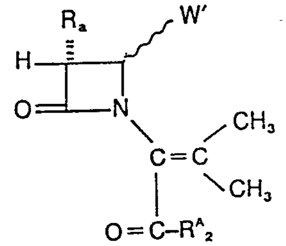

(XIV),

oú Ra et $R_2^A$ ont les significations données pour la formule II et W′ représente un halogène, sous la forme cis,trans, cis ou trans, on fait réagir un composé d'acide pénicillanique de formule

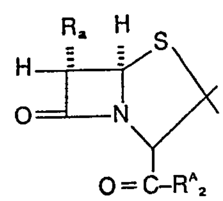

(VII),

où Ra et $R_2^A$ ont les significations données ci-dessus, avec un agent halogénant donneur d'ions halogène positifs et, si nécessaire, on traite un produit intermédiaire éventuellement obtenu avec une base, et si on le désire on transforme dans un composé de formule XIV obtenu un groupe Ra en un autre groupe Ra et/ou on isole à partir d'un composé cis,trans obtenu le composé cis et/ou le composé trans, selon l'étape 3.4 pour préparer les composés de formule

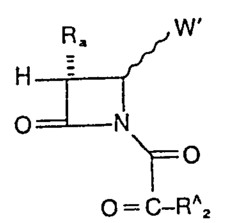

(XV),

où Ra et $R_2^A$ ont les significations données pour la formule II et W′ représente un halogène ou un acyloxy, où acyle est le radical acyle d'un acide carboxylique organique, sous la forme cis,trans, cis ou trans, on ozonise un composé de formule

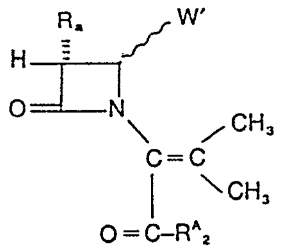

(XIV),

où Ra, W′ et $R_2^A$ ont les significations données ci-dessus, eton clive de façon réductrice l'ozonide formé pour donner le groupe oxo, et si on le désire on transforme dans un composé obtenu un group Ra en un autre groupe Ra, et/ou si on le désire on isole à partir d'un composé cis,trans obtenu le composé cis ou le composé trans, selon l'étape 3.5 pour préparer les composés de formule

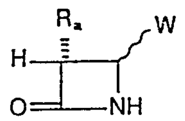

(IIIa),

où Ra a la signification donnée pour la formule II et W représente un groupe sortant nucléofuge, sous la forme cis,trans, cis ou trans, on solvolyse un composé de formule

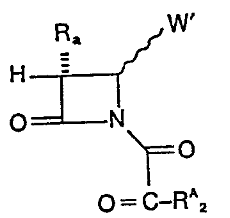

(XV),

où Ra et $R_2^A$ ont les significations données ci-dessus, et W′ représente un acyloxy ou un halogène, et si on le désire on transforme dans un composé obtenu un groupe Ra en un autre groupe Ra, et/ou si on le désire on transforme un groupe W′ en un autre groupe W′ ou en W, et/ou si on le désire on isomérise un composé cis obtenu en le composé trans correspondant, et/ou à partir d'un composé cis,trans obtenu on isole le composé cis et/ou le composé trans, puis on conduit les étapes 1.1 à 1.4.

2. Procédé selon la revendication 1, caractérisé en ce qu'on prépare des composés de formule II où Ra représente un alcoyle inférieur, hydroxyalcoyle inférieur, alcoxy inférieur-alcoyle inférieur, alcanoyloxy inférieur-alcoyle inférieur, hydroxysulfonyloxyalcoyle inférieur présent sous forme de sel, hydroxy, alcoxy inférieur, alcanoyloxy inférieur, alcanoyloxy inférieur substitué par phényloxy, halogène, amino ou cyano, phénylalcanoyloxy inférieur, ou phénylalcanoyloxy inférieur substitué par hydroxy ou amino, R1 représente hydrogène, alcoyle inférieur, hydroxy-alcoyle inférieur, alcoxy inférieur-alcoyle inférieur, alcanoyloxy inférieur-alcoyle inférieur, alcoyle inférieur-thioalcoyle inférieur, hétérocyclylthioalcoyle inférieur, où hétérocyclyle représente un radical aromatique diaza-, triaza-tétraaza-, thiaza-, thiadiaza-, thiatriaza-, oxaza- ou oxadiazacyclique à 5 chaînons éventuellement substitué par alcoyle inférieur, carboxyalcoyle inférieur, dialcoyle inférieur-aminoalcoyle inférieur ou sulfoalcoyle inférieur présent sous forme de sel, aminoalcoyle inférieur, acylaminoalcoyle inférieur, où acyle est un alcanoyle inférieur ou un groupe oxycarbonyle substitué habituel comme groupe protecteur d'amino, p.ex. un groupe tert-butyle, 2,2,2-trichloroéthyle, diphénylméthyle ou p-nitrobenzyloxycarbonyle, carboxy-alcoyle inférieur, alcoxy inférieur-carbonylalcoyle inférieur, phénylalcoyle inférieur, phényle, hydroxyphényle, aminophényle, furyle, thiényle, pyridyle, alcoyle inférieur-thio, alcoyle inférieur-thio ou alcényle inférieur-thio substitué par amino, mono- ou dialcoyle inférieur-amino ou alcanoyle inférieur-amino, ou tétrazolylthio ou thiadiazolylthio éventuellement substitué par alcoyle inférieur, sulfoalcoyle inférieur, carboxyalcoyle inférieur ou dialcoyle inférieur-aminoalcoyle inférieur, où les groupes fonctionnels dans de tels radicaux Ra et R1 se présentent de préférence sous forme protégée, et Z′, $X^{\oplus}$ et $R_2^A$ ont les significations données

dans la revendication 1, sous forme racémique ou optiquement active.

3. Composés de formule

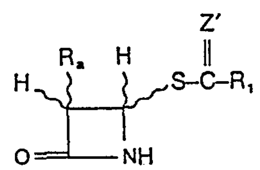

(IV),

où Ra, R1 et Z′ ont les significations données dans la revendication 1, sous forme racémique et optiquement active.

4. Composés de formule IV selon la revendication 3, où Ra, R1 et Z′ ont les significations données dans la revendication 2.

5. 4-Acétylthio-3-méthyl-2-oxo-azétidine (composé cis racémique) selon la revendication 3.

6. 4-Acétylthio-3-méthyl-2-oxo-azétidine (composé trans racémique selon la revendication 3.

7. 4-Acétylthio-3-isopropyl-2-oxo-azétidine (composé trans racémique) selon la revendication 3.

8. 4-Acétylthio-3-benzyl-2-oxo-azétidine (composé trans racémique) selon la revendication 3.

9. 4-éthylthio-thiocarbonylthio-3-isopropyl-2-oxoazétidine (composé trans racémique) selon la revendication 3.

10. (3S,4R)-4-Acétylthio-3-méthoxy-2-oxo-azétidine selon la revendication 3.

11. (3S,4R)-4-Acétylthio-3-phénoxyacétoxy-2-oxo-azétidine selon la revendication 3.

12. 3-éthyl-4-(4-p-nitrobenzyloxycarbonyl-amino-butyryl-thio)-2-oxo-azétidine (composé cis-trans racémique) selon la revendication 3.

13. (3S,4R)-4-(2-p-Nitrobenzyloxycarbonyl-aminoéthyl-thio-thiocarbonylthio)-3-méthoxy-2-oxoazétidine selon la revendication 3.

14. (3S,4R)-4-(4-p-Nitrobenzyloxycarbonyl-amino-butyrylthio)-3-méthoxy-2-oxoazétidine selon la revendication 3.

15. 3-éthyl-4-(2-acétylaminoéthylthio-thiocarbonylthio)-2-oxoazétidine (composé cis,trans racémique) selon la revendication 3.

16. Composés de formule

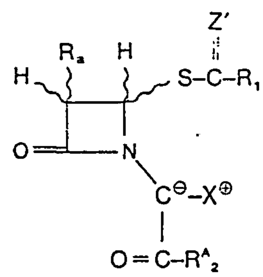

(II),

où Ra, R1, Z', $X^{\oplus}$ et $R_2^A$ ont les significations données dans la revendication 1, sous forme racémique ou optiquement active.

17. Composés de formule II selon la revendication 16, où Ra, R1, Z', $X^{\oplus}$ et $R_2^A$ ont les significations données dans la revendication 2, sous forme racémique ou optiquement active.

18. Composés de formule II selon les revendications 16 et 17, où Ra représente méthyle, éthyle, isopropyle, benzyle, méthoxy ou phénoxyacétoxy, R1 représente méthyle, 3-(p-nitrobenzyloxycarbonylamino)propyle, éthylthio, 2-acétylamino-éthylthio ou 2-(p-nitrobenzyloxycarbonylamino)éthylthio, Z' représente oxygène ou soufre, $R_2^A$ p-nitrobenzyloxy et $X^{\oplus}$ le groupe $P^{\ominus}$ (phényl)$_3$.

19. Composés de formule

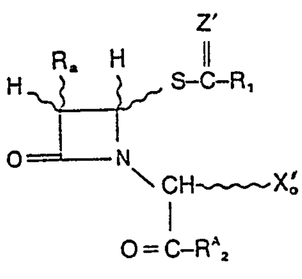

(V) ou (VI),

où Ra, R1, Z' et $R_2^A$ ont les significations données dans la revendication 1 et X'o représente un hydroxy ou un groupe hydroxy estérifié réactif, sous forme racémique ou optiquement active.

20. Composés de formule V ou VI selon la revendication 19, où Ra, R1, Z' et $R_2^A$ ont les significations données dans la revendication 2 et X'o a les significations données dans la revendication 19, sous forme racémique ou optiquement active.

21. Composés de formule V ou VI selon les revendications 19 et 20, où Ra représente méthyle, éthyle, isopropyle, benzyle, méthoxy ou phénoxyacétoxy, R1 représente méthyle, 3-(p-nitrobenzyloxycarbonylamino)propyle, éthylthio, 2-acétylaminoéthylthio ou 2-(p-nitrobenzyloxycarbonylamino)éthylthio, Z' représente oxygène ou soufre, $R_2^A$ p-nitrobenzyloxy et X'o hydroxy ou chlore.

22. Composés de formule

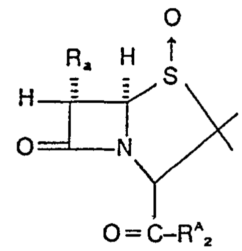

(VIII),

où Ra et $R_2^A$ ont les significations données dans la revendication 1.

23. Composés de formule VIII selon la revendication 22, où Ra et $R_2^A$ ont les significations données dans la revendication 2.

24. Composés de formule VIII selon les revendications 22 et 23, où Ra représente un méthoxy ou un phénoxyacétoxy et $R_2^A$ un méthoxy ou un 2,2,2-trichloréthoxy.

25. Composés de formule

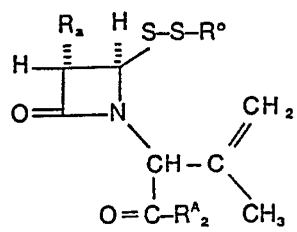

(IX),

où Ra et $R_2^A$ ont les significations données dans la revendication 1 et Ro est un radical hétérocyclique aromatique éventuellement substitué ayant jusqu'à 15 atomes de carbone, au moyen un atome d'azote dans son noyau et éventuellement un autre hétéroatome dans son noyau, radical qui est lié au groupe thio –S– par l'un de ses atomes de carbone du noyau qui est relié à un atome d'azote du noyau par une double liaison.

26. Composés de formule IX selon la revendication 25, où Ra et $R_2^A$ ont les significations données dans la revendication 2 et Ro a les significations données dans la revendication 25.

27. Composés de formule IX selon les revendications 25 et 26, où Ra représente méthoxy ou phénoxyacétoxy, $R_2^A$ méthoxy ou 2,2,2-trichloréthoxy et Ro 2-benzthiazolyle.

28. Composés de formule

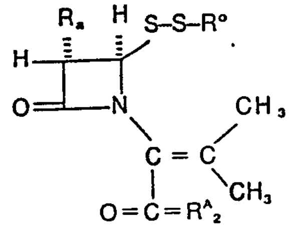

(X),

où Ra et $R_2^A$ ont les significations données dans la revendication 1 et Ro est un radical hétérocyclique aromatique éventuellement substitué ayant jusqu'à 15 atomes de carbone, au moins un atome d'azote dans son noyau et éventuellement un autre hétéroatome dans son noyau, radical qui est lié au groupe thio –S– par un de ses atomes de carbone du noyau qui est lié à un atome d'azote du noyau par une double liaison.

29. Composés de formule X selon la revendication 28, où Ra et $R_2^A$ ont les significations données dans la revendication 2 et Ro a les significations données dans la revendication 28.

30. Composés de formule X selon les revendications 28 et 29, où Ra est un méthoxy ou phénoxyacétoxy, $R_2^A$ méthoxy ou 2,2,2-trichloréthoxy et Ro 2-benzthiazolyle.

31. Composés de formule

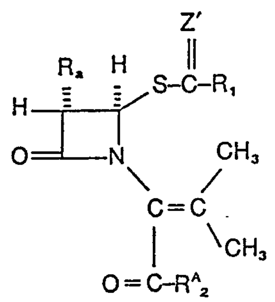

(XI),

où Ra, R1, Z′ et $R_2^A$ ont les significations données dans la revendication 1.

32. Composés de formule XI selon la revendication 31, où Ra, R1, Z′ et $R_2^A$ ont les significations données dans la revendication 2.

33. Composés de formule XI selon les revendications 31 et 32, où Ra représente méthyle, méthoxy ou phénoxyacétoxy, R1 méthyle, Z′ oxygène et $R_2^A$ méthoxy ou 2,2,2-trichloréthoxy.

34. Composés de formule

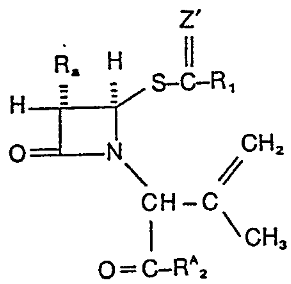

(Xa),

où Ra, R1, Z′ et $R_2^A$ ont les significations données dans la revendication 1.

35. Composés de formule Xa selon la revendication 34, où Ra, R1, Z′ et $R_2^A$ ont les significations données dans la revendication 2.

36. Composés de formule

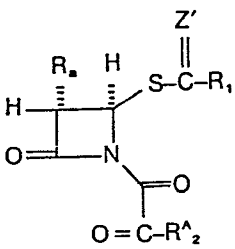

(XII),

où Ra, R1, Z′ et $R_2^A$ ont les significations données dans la revendication 1.

37. Composés de formule XII selon la revendication 36, où Ra, R1, Z′ et $R_2^A$ ont les significations données dans la revendication 2.

38. Composés de formule XII selon les revendications 36 et 37, où Ra représente méthyle, méthoxy ou phénoxyacétoxy, R1 méthyle, Z′ oxygène et $R_2^A$ méthoxy ou 2,2,2-trichloréthoxy.

39. Composés de formule

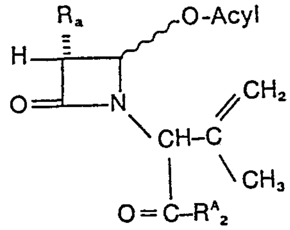

(XIII),

où Ra et $R_2^A$ ont les significations données dans la revendication 1 et acyle le radical acyle d'un acide carboxylique organique en position cis,-trans, cis ou trans.

40. Composés de formule XIII, selon la revendication 39, où Ra représente un radical méthyle ou méthoxy; $R_2^A$ un groupe 2,2,2-trichloréthoxy et Acyl représente un groupe acétyle, dans leur forme cis,trans-, leur forme cis- ou leur forme trans-.

41. Composés de formule

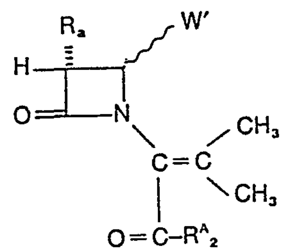

(XIV),

où Ra et $R_2^A$ ont les significations données dans la revendication 1, W′ est halogène ou acyloxy, où acyle est le radical acyle d'un acide carboxylique organique, sous forme cis,trans, cis ou trans.

42. Composés de formule XIV selon la revendication 41, où Ra représente méthoxy, $R_2^A$ 2,2,2-trichloréthoxy et W′ chlore ou acétoxy, sous forme cis,trans, cis ou trans.

43. Composés de formule

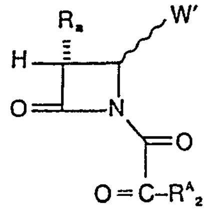

(XV),

où Ra et $R_2^A$ ont les significations données dans la revendication 1, W′ représente halogène ou acyloxy, où acyle est le radical acyle d'un acide carboxylique organique, sous forme cis,trans, cis ou trans.

44. Composés de formule XI selon la revendication 43, où Ra représente méthoxy, $R_2^A$ 2,2,2-trichloréthoxy et W′ acétoxy ou chlore, sous forme cis,trans, cis ou trans.

45. Composés de formule

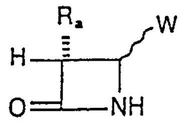

(IIIa),

où Ra a les significations données dans la revendication 1 et W représente un groupe sortant nucléofuge, sous forme cis,trans, cis ou trans.

46. Composés de formule IIIa selon la revendication 45, où Ra est méthoxy, W chlore ou acétoxy, sous forme cis,trans, cis ou trans.

47. Les diverses étapes du procédé selon la revendication 1.

48. Les diverses étapes du procédé selon la revendication 1 selon le schéma réactionnel 3.

49. Les diverses étapes du procédé selon la revendication 1 selon les schémas réactionnels 1 et 2.